(19) **Europäisches Patentamt European Patent Office Office européen des brevets**

(11) **EP 3 337 819 B1**

(12) **EUROPEAN PATENT SPECIFICATION**

(45) Date of publication and mention
of the grant of the patent:
**21.02.2024 Bulletin 2024/08**

(21) Application number: **16758343.4**

(22) Date of filing: **19.08.2016**

(51) International Patent Classification (IPC):
*C07K 16/06* (2006.01)    *C07K 1/16* (2006.01)
*C07K 1/18* (2006.01)    *C07K 1/22* (2006.01)
*C07K 16/12* (2006.01)    *C07K 14/245* (2006.01)

(52) Cooperative Patent Classification (CPC):
**C07K 16/065; A61P 43/00; C07K 1/165;
C07K 1/18; C07K 1/22; C07K 14/245;
C07K 16/1232; C12N 9/90;** C12Y 502/01008

(86) International application number:
**PCT/US2016/047912**

(87) International publication number:
**WO 2017/031476 (23.02.2017 Gazette 2017/08)**

(54) **METHOD OF PURIFYING FOR PRODUCING RECOMBINANT POLYPEPTIDES USING FKPA**

VERFAHREN ZUR AUFREINIGUNG VON REKOMBINANTEN POLYPEPTIDEN UNTER
VERWENDUNG VON FKPA

MÉTHODE DE PURIFICATION POUR LA PRODUCTION DE POLYPEPTIDES RECOMBINANTS
UTILISANT FKPA

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.08.2015 US 201562207910 P
20.08.2015 US 201562207908 P
26.08.2015 US 201562210378 P**

(43) Date of publication of application:
**27.06.2018 Bulletin 2018/26**

(73) Proprietor: **F. Hoffmann-La Roche AG
4070 Basel (CH)**

(72) Inventors:
• **FONG, Chris, B.
South San Francisco, CA 94080 (US)**
• **LADIWALA, Asif
South San Francisco, CA 94080 (US)**
• **MCKAY, Patrick, A.
South San Francisco, CA 94080 (US)**

(74) Representative: **Klaus, Michaela et al
F. Hoffmann-La Roche AG
Patent Department
Grenzacherstrasse 124
4070 Basel (CH)**

(56) References cited:
**WO-A1-2015/024896    WO-A1-2015/038888
WO-A1-2015/070068    WO-A2-02/061090
WO-A2-2009/136286    WO-A2-2013/102042
WO-A2-2014/165771    WO-A2-2015/127405
US-A1- 2014 315 245**

• **ARIE J-P ET AL: "Chaperone function of FkpA, a
heat shock prolyl isomerase, in the periplasm of
escherichia coli", MOLECULAR
MICROBIOLOGY, WILEY-BLACKWELL
PUBLISHING LTD, GB, vol. 39, no. 1, 1 January
2001 (2001-01-01), pages 199-210, XP002973578,
ISSN: 0950-382X, DOI:
10.1046/J.1365-2958.2001.02250.X**

EP 3 337 819 B1

**(Cont. next page)**

- **RAMM K ET AL: "High enzymatic activity and chaperone function are mechanistically related features of the dimeric E. coli peptidyl-prolyl-isomerase FkpA", JOURNAL OF MOLECULAR BIOLOGY, ACADEMIC PRESS, UNITED KINGDOM, vol. 310, no. 2, 6 July 2001 (2001-07-06), pages 485-498, XP004479226, ISSN: 0022-2836, DOI: 10.1006/JMBI.2001.4747**
- **PETE GAGNON: "Technology trends in antibody purification", JOURNAL OF CHROMATOGRAPHY A, vol. 1221, 1 January 2012 (2012-01-01), pages 57-70, XP055027826, ISSN: 0021-9673, DOI: 10.1016/j.chroma.2011.10.034**

Remarks:

The complete document including Reference Table(s) and the Sequence Listing(s) can be downloaded from the EPO website

**Description**

FIELD OF THE INVENTION

The present invention provides methods for purifying polypeptides (*e.g.*, multispecific antibodies) from a composition comprising the polypeptide and at least one impurity and formulations comprising the product purified by the methods. In some embodiments, the impurity is a product-specific impurity; for example, a precursor, aggregate, and/or variant of the multispecific antibody.

BACKGROUND OF THE INVENTION

The production yield and quality of eukaryotic polypeptides produced in bulk by expression in bacterial host cells under fermentation conditions is often modified to improve the proper assembly and folding of the secreted heteromultimeric proteins (*e.g.*, antibodies). Overexpression of chaperone proteins, such as FKBP-type peptidyl-prolyl cis-trans isomerase (FkpA) facilitates the proper folding and solubility of heterologous proteins, such as antibodies, produced in bacterial host cells. FkpA catalyzes the cis-trans isomerization of proline imidic peptide bonds in oligopeptides (Missiakas, D. et al., Molecular Microbiology (1996) 21(4), 871-884).

For recombinant biopharmaceutical proteins to be acceptable for administration to human patients, it is important that residual impurities resulting from the manufacture and purification process are removed from or reduced to small quantities in the final biological product. These process components include culture medium proteins, immunoglobulin affinity ligands, viruses, endotoxin, DNA, and host cell proteins. These host cell impurities include process-specific host cell proteins (HCPs), which are process-related impurities in the biologic product derived from recombinant DNA technology, for example, chaperones such as FkpA, DsbA and DsbC that are overexpressed to facilitate protein folding. While HCPs are typically present in the final drug substance in small quantities (in parts-per-million or nanograms per milligram of the intended recombinant polypeptide), it is recognized that HCPs are undesirable and their quantities should be minimized. For example, the U.S. Food and Drug Administration (FDA) requires that biopharmaceuticals intended for in vivo human use should be as free as possible of extraneous impurities, and requires tests for detection and quantitation of potential impurities, such as HCPs. WO2015/038888 describes combinations of various chromatographic methods to remove process- related impurities.

In addition, the International Conference on Harmonization (ICH) provides guidelines on test procedures and acceptance criteria for biotechnological/biological products. The guidelines suggest that for HCPs, a sensitive immunoassay capable of detecting a wide range of protein impurities be utilized. Assays and reagents to detect immunoglobulins, DNA, endotoxins, viruses, and total HCPs, *e.g.*, total *E. coli* proteins (ECP) or total CHO proteins (CHOP) have been developed but such assays and reagents may not accurately detect accessory protein impurities such as FkpA. There are currently no commercial reagents or analytical methods of sufficient specificity and sensitivity for the detection and quantification of proteins such as FkpA that are typically not expressed at high levels in bacteria but may be overexpressed in recombinant bacterial host cells to facilitate folding and secretion of biologic products.

Reagents, methods and kits for the detection of FkpA are particularly needed where there are no existing assays and reagents of sufficient consistency, sensitivity, specificity or efficiency. The invention described herein meets certain of the above described needs and provides other benefits. In addition, the preparation of multispecific antibodies have unique product-specific impurities such as non-paired antibody arms and misassembled antibodies. As such, there is a need to develop purification schemes for multispecific antibodies that remove these product-specific impurities.

BRIEF SUMMARY

The technical disclosure set out below may in some respects go beyond the scope of the claims. Elements of the disclosure which do not fall within the scope of the claims are provided for information.

Disclosed herein are methods for purifying a FkpA polypeptide from a cell lysate comprising the FkpA polypeptide, the method comprising a) clarifying the cell lysate by centrifugation, b) applying the clarified cell lysate comprising the FkpA polypeptide to a cation exchange chromatography material, c) eluting the FkpA polypeptide from the cation exchange chromatography material to generate an cation exchange eluate comprising the FkpA polypeptide, d) applying the cation exchange eluate comprising the FkpA polypeptide to a hydrophobic interaction chromatography (HIC) material, e) eluting the FkpA polypeptide from the HIC material to generate a HIC eluate, f) applying the HIC eluate comprising the FkpA polypeptide to a size exclusion chromatography material, g) collecting fraction from the size exclusion chromatography comprising the purified FkpA polypeptide. In some aspects of the disclosure, the lysate comprising the FkpA polypeptide is at about pH 6.0.

In some aspects of the disclosure, the cation exchange chromatography material is a strong cation exchanger. In some aspects of the disclosure, the strong cation exchanger comprises a sulfopropyl group. In some aspects of the disclosure, the sulphopropyl group is linked to crosslinked agarose. In some aspects of the disclosure, the cation exchange material is washed in 25 mM 2-(N-morpholino)ethanesulfonic acid (MES) prior to elution. In some aspects of the disclosure, the FkpA is eluted from the cation chromatography material using a salt gradient. In some aspects of the disclosure, the salt gradient is a linear gradient. In some aspects of the disclosure, the salt gradient is a 0-30% gradient from about 25 mM MES, 300 mM NaCl to 25 mM MES 300 mM NaCl over 9 column volumes. In some aspects of the disclosure, the cation exchange eluate is collected in fractions. In some aspects of the disclosure, the fractions are analyzed by size exclusion chromatography prior to hydrophobic interaction chromatography. In some aspects of the disclosure, fractions comprising at least about 25% FkpA are selected for further purification.

In some aspects of the disclosure, the HIC material comprises a butyl moiety. In some aspects of the disclosure, the butyl moiety is linked to a crosslinked agarose. In some aspects of the disclosure, the cation exchange eluate is conditioned to comprise about 0.6 M sodium sulfate and about 50 mM $PO_4$, about pH 7 before HIC. In some aspects of the disclosure, the FkpA-bound HIC material is washed with 40 mM phosphate, 300 mM sodium sulfate, pH 7.0 prior to elution. In some aspects of the disclosure, the FkpA is eluted from the HIC material using water. In some aspects of the disclosure, the HIC eluate is collected in fractions. In some aspects of the disclosure, fractions comprising FkpA are pooled. In some aspects of the disclosure, fractions comprising at least about 25% FkpA are pooled.

In some aspects of the disclosure, the size exclusion chromatography material comprises a spherical composite of cross-linked agarose and dextran. In some aspects of the disclosure, the size exclusion flow through is collected in fractions. In some aspects of the disclosure, the HIC eluate is concentrated prior to size exclusion chromatography. In some aspects of the disclosure, the HIC eluate is concentrated about 6-fold to about 10-fold prior to size exclusion chromatography. In some aspects of the disclosure, the size exclusion fractions comprising FkpA are pooled.

In some aspects of the disclosure of the above embodiments, the FkpA polypeptide is an *Escherichia coli* FkpA polypeptide. In some aspects of the disclosure, the FkpA polypeptide comprises the amino acid sequence of SEQ ID NO: 1. In some aspects of the disclosure, the amino acid sequence of the FkpA polypeptide comprises at least about 80% identical to the amino acid sequence of SEQ ID NO: 1. In some aspects of the disclosure, the FkpA is expressed in the cell. In some aspects of the disclosure, the cell is a prokaryotic cell. In some aspects of the disclosure, the cell is an *E. coli* cell. In some aspects of the disclosure, the cell is engineered to express FkpA at levels greater than endogenous expression of FkpA. In some aspects of the disclosure, the cell is lysed using a microfluidizer.

In some aspects, the disclosure provides a composition comprising a FkpA polypeptide purified by the methods described herein. In some aspects of the disclosure, the composition comprises at least about 98% FkpA polypeptide (*e.g.*, 98% monomeric FkpA polypeptide; for example, as measured by size exclusion chromatography). In some aspects of the disclosure, the composition comprises less than about 2% low molecular weight species. In some aspects of the disclosure, the composition comprises no more than about 5% high molecular weight species. In some aspects of the disclosure, the composition comprises less than about 2% high molecular weight species. In some aspects of the disclosure, the percentage of FkpA polypeptide (*e.g.*, monomeric FkpA) is detected by size exclusion chromatography. In some aspects of the disclosure, the composition comprises less than about 5%, about 4%, about 3%, about 2%, about 1% impurities or is substantially free of impurities. In some aspects of the disclosure, the impurities are high molecular weight and/or low molecular weight polypeptide species relative to FkpA. In some aspects of the disclosure, the impurities are one or more of an *E. coli* protein (ECP), aggregates of FkpA, fragments of FkpA, a nucleic acid or a cell culture media component.

In some aspects of the disclosure of the above compositions, the purified FkpA is stable to one or more freeze-thaw cycles. In some aspects of the disclosure, the FkpA is stable to three freeze-thaw cycles. In some aspects of the disclosure, the composition comprises at least about 70% FkpA after freeze-thaw. In some aspects of the disclosure, the composition comprises at least about 80% FkpA after freeze-thaw.

In some aspects of the disclosure of the above compositions, the purity of the FkpA polypeptide in the composition is measured by one or more of chromatography, SDS polyacrylamide gel electrophoresis or western blot analysis. In some aspects of the disclosure, the purity of the FkpA polypeptide in the composition is measured by high performance liquid chromatography (HPLC). In some aspects of the disclosure, the purity of the FkpA polypeptide in the composition is measured by size exclusion chromatography (SEC). In some aspects of the disclosure, the purity of the FkpA polypeptide in the composition is measured by SDS gel electrophoresis using a fluorescent imaging or a silver stain. In some aspects of the disclosure, the presence of non-FkpA polypeptides in the composition are identified by the presence of species identified by gel electrophoresis that are not immunoreactive with anti-FkpA antibodies as shown by western blot analysis. In some aspects of the disclosure, the presence of aggregates of the FkpA polypeptide in the composition are identified by the presence of species with a molecular weight greater than the native FkpA by western blot analysis.

In some aspects, the disclosure provides methods for generating antibodies that specifically bind FkpA, comprising exposing or immunizing an animal to a composition comprising FkpA purified by the methods described herein. In some aspects of the disclosure, the methods for generating antibodies that specifically bind FkpA comprise exposing or immunizing an animal to a composition of FkpA as described herein. In some aspects of the disclosure, the methods further comprise collecting sera from the animal wherein the sera comprises antibodies that specifically bind FkpA. In some aspects of the disclosure, the sera comprises polyclonal antibodies that specifically bind FkpA. In other aspects of the disclosure, one or more monoclonal antibodies are isolated from the sera. In some aspects of the disclosure, the animal is a goat, a rabbit, a mouse, a guinea pig, a hamster, a rat, a donkey or a chicken.

In some aspects the disclosure provides compositions comprising polyclonal antibodies that specifically bind FkpA, wherein the polyclonal antibodies are generated by immunizing an animal to a composition comprising FkpA purified by any of the methods described herein. In some aspects of the disclosure, the polyclonal antibodies are generated by immunizing an animal to a purified FkpA composition as described herein. In some aspects of the disclosure, the polyclonal antibodies are collected from sera of the animal. In other aspects, the disclosure provides monoclonal antibodies that specifically bind FkpA, wherein the monoclonal antibodies are generated by immunizing an animal to a composition comprising FkpA purified by any of the methods described herein. In some aspects of the disclosure, the monoclonal antibodies are generated by immunizing an animal to a purified FkpA composition as described herein. In some aspects of the disclosure, the animal is a goat, a rabbit, a mouse, a guinea pig, a hamster, a rat, a donkey or a chicken.

In some aspects, the disclosure provides methods for purifying antibodies that specifically bind FkpA, comprising contacting a composition that comprises anti-FkpA antibodies with ammonium sulfate to a final concentration of ammonium sulfate of about 60%, centrifuging the solutions, removing the supernatants and dissolving the pellets in phosphate buffer. In some aspects of the disclosure, the phosphate buffer comprises about 50 mM sodium phosphate at about pH 7.0. In some aspects of the disclosure, the method for purifying antibodies that specifically bind FkpA comprise a) contacting a composition that comprises anti-FkpA antibodies with ammonium sulfate to a final concentration of ammonium sulfate of about 60%, centrifuging the solutions, removing the supernatants and dissolving the pellets in phosphate buffer to generate an anti-FkpA antibody solution; b) applying the anti-FkpA antibody solution to an affinity chromatography material, c) eluting the antibodies from the affinity material to generate an affinity eluate comprising the anti-FkpA antibodies; and d) subjecting the affinity eluate to size exclusion chromatography, wherein fractions comprising the purified antibodies are collected from the size exclusion chromatography. In some aspects of the disclosure, the phosphate buffer comprises about 50 mM sodium phosphate at about pH 7.0. In some aspects of the disclosure, the affinity chromatography material comprises purified FkpA linked to a chromatography media. In some aspects of the disclosure, the chromatography media is a cross-linked dextran. In some aspects of the disclosure, the FkpA is purified by any of the method described herein. In some aspects of the disclosure, the FkpA is derived from any of the compositions described herein. In some aspects of the disclosure, the FkpA is covalently linked to the chromatography media. In some aspects of the disclosure, the FkpA is linked to the chromatography media using Glyceryl Controlled Pore Glass (Glyceryl-CPG). In some aspects of the disclosure, the anti-FkpA antibodies are eluted from the affinity column using phosphate buffered saline at about pH 2.0. In some aspects of the disclosure, the elution pool is collected into about 1 M Tris, about pH 8.0. In some aspects of the disclosure, the size exclusion chromatography material comprises a spherical composite of cross-linked agarose and dextran. In some aspects of the disclosure, the spherical composite of cross-linked agarose and dextran has a separation range for molecules with molecular weights between 10,000 daltons and 600,000 daltons. In some aspects of the disclosure, the size exclusion flow through is collected in fractions. In some aspects of the disclosure, the size exclusion fractions comprising anti-FkpA antibodies are pooled. In some aspects of the disclosure, the antibodies are polyclonal antibodies. In some aspects of the disclosure, the antibodies are prepared according to any of the methods described herein. In some aspects of the disclosure, less than about 1% of the antibodies specifically bind non-FkpA compounds. In some aspects of the disclosure, the invention provides isolated anti-FkpA antibodies purified by any of the methods described herein.

In some aspects which are not explicitly claimed, the disclosure provides methods for quantifying FkpA in a sample, comprising detecting FkpA in the sample using a detection system and comparing the amount of FkpA detected in the sample with the detection of one or more concentrations of an ultrapure FkpA reference standard. In some aspects of the disclosure, the ultrapure FkpA reference standard comprises at least about 95%, about 96%, about 97%, or about 98% monomeric FkpA polypeptide. In some aspects of the disclosure, the ultrapure FkpA reference standard is prepared by any of the methods described herein or comprises any of the FkpA compositions described herein. In some aspects of the disclosure, the detection system is an immunoassay. In some aspects of the disclosure, the immunoassay comprises antibodies that specifically binds the ultrapure FkpA reference standard.

In some aspects which are not explicitly claimed, the disclosure provides methods for analyzing a recombinant polypeptide sample for the presence of and/or quantity of FkpA, comprising detecting FkpA in the sample using an immunoassay and comparing the amount of FkpA detected in the sample with the detection of one or more concentrations of an ultrapure FkpA reference standard. In some aspects of the disclosure, the ultrapure FkpA reference standard comprises at least about 98% monomeric FkpA polypeptide. In some aspects of the disclosure, the ultrapure FkpA reference standard is prepared by any of the methods described herein. In some aspects of the disclosure, the immunoassay comprises antibodies that specifically bind the ultrapure FkpA reference standard. In some aspects of the disclosure, the antibodies that specifically bind the ultrapure FkpA are polyclonal antibodies. In some aspects of the disclosure, the antibodies that specifically bind the ultrapure FkpA are used as capture antibodies in the immunoassay. In some aspects of the disclosure, the antibodies that specifically bind ultrapure FkpA are used as detection antibodies.

In some aspects of the disclosure, the detection antibodies are conjugated to a detection agent. In some aspects of the disclosure, the detection agent is a horseradish peroxidase. In some aspects of the disclosure, the antibodies that specifically bind FkpA are prepared according to any of the methods described herein. In some aspects of the disclosure, the FkpA is an *E. coli* FkpA. In some aspects of the disclosure, the sample comprises a recombinant polypeptide prepared in a host cell. In some aspects of the disclosure, the host cell is an *E. coli* cell. In some aspects of the disclosure, the host cell overexpresses FkpA. In some aspects of the disclosure, the sample is a cell lysate. In some aspects of the disclosure, the sample is obtained from a recombinant polypeptide preparation and wherein the recombinant polypeptide preparation has been subjected to one or more chromatographic purification steps. In some aspects of the disclosure, the recombinant polypeptide preparation is a final purified product. In some aspects of the disclosure, the recombinant polypeptide contained in the recombinant polypeptide sample is an antibody or an immunoadhesin. In some aspects of the disclosure, the antibody is a multispecific antibody, a bispecific antibody, a half antibody or an antibody fragment. In some aspects of the disclosure, the recombinant polypeptide is an IgG 1, an IgG2, an IgG3, or an IgG4.

In some aspects which are not explicitly claimed, the disclosure provides immunoassay methods for detecting FkpA in a sample, wherein the sample is obtained from a recombinant polypeptide preparation from a host cell line, the method comprising: (a) contacting a capture antibody that binds FkpA with the sample thereby generating a sample-capture antibody combination material; (b) contacting a detection antibody that binds FkpA with the sample-capture antibody combination material; and (c) detecting the antibody bound to the sample-capture antibody combination material. In some aspects of the disclosure, the immunoassay further comprises quantifying the level of the detection antibody bound using a standard titration curve. In some aspects of the disclosure, the immunoassay further comprises calculating an amount of FkpA present in the sample based on the level of the detection antibody bound. In some aspects of the disclosure, the amount of FkpA present in the sample is determined by comparing the standard titration curve with a standard titration curve generated with an ultrapure FkpA composition. In some aspects of the disclosure, the ultrapure FkpA composition comprises at least about 98% FkpA polypeptide (*e.g.*, monomeric FkpA). In some aspects of the disclosure, the ultrapure FkpA in the composition is prepared by any of the methods described herein. In some aspects of the disclosure, the capture antibody specifically binds the ultrapure FkpA. In some aspects of the disclosure, the detection antibody specifically binds the ultrapure FkpA. In some aspects of the disclosure, the antibody that specifically binds the ultrapure FkpA is a polyclonal antibody. In some aspects of the disclosure, the second detection antibody that binds FkpA is conjugated to a horseradish peroxidase. In some aspects of the disclosure, the polyclonal antibody is generated according to any of the methods described herein. In some aspects of the disclosure, the immunoassay is a sandwich assay. In some aspects of the disclosure, the sandwich assay is an enzyme-linked immunosorbent assay (ELISA). In some aspects of the disclosure, the FkpA is an *E. coli* FkpA. In some aspects of the disclosure, the recombinant polypeptide preparation or the host cell line is obtained from *E. coli.* In some aspects of the disclosure, the host cell line expresses exogenous FkpA. In some embodiments, the sample is a cell lysate. In some aspects of the disclosure, the sample is obtained from the recombinant polypeptide preparation and wherein the recombinant polypeptide preparation has been subjected to one or more chromatographic purification steps. In some aspects of the disclosure, the recombinant polypeptide preparation is a final purified product.

In some aspects of the disclosure which are not explicitly claimed, the immunoassay is used to analyze preparation of an antibody or an immunoadhesin. In some aspects of the disclosure, the antibody is a multispecific antibody, a bispecific antibody, a half antibody or an antibody fragment. In some aspects of the disclosure, the antibody is an IgG1, an IgG2, an IgG3, or an IgG4. In some aspects of the disclosure, the antibody is a bispecific antibody comprising a first binding domain that binds IL13 and a second binding domain that binds IL17. In some aspects of the disclosure, the IL13 comprises the amino acid sequence of SEQ ID NO:4 or SEQ ID NO:5. In some aspects of the disclosure, the first binding domain of the bispecific antibody comprises HVR-H1 comprising the amino acid sequence of SEQ ID NO: 12, HVR-H2 comprising the amino acid sequence of SEQ ID NO: 13, HVR-H3 comprising the amino acid sequence of SEQ ID NO: 14, HVR-L1 comprising the amino acid sequence of SEQ ID NO: 15, HVR-L2 comprising the amino acid sequence of SEQ ID NO: 16, and HVR-L3 comprising the amino acid sequence of SEQ ID NO:17. In some aspects of the disclosure, the first binding domain of the bispecific antibody comprises a VH sequence comprising the amino acid sequence of SEQ ID NO:10 and a VL sequence comprising the amino acid sequence of SEQ ID NO:11. In some aspects of the disclosure, the first binding domain of the bispecific antibody comprises a heavy chain comprising the amino acid sequence of SEQ ID NO:18. In some aspects of the disclosure, the first binding domain of the bispecific antibody comprises a heavy chain comprising the amino acid sequence of SEQ ID NO:19. In some aspects of the disclosure, the first binding domain of the bispecific antibody comprises a light chain comprising the amino acid sequence of SEQ ID NO:20. In some aspects of the disclosure, the IL17 is human IL17. In some aspects of the disclosure, the second binding domain binds human IL17AA, IL17FF and IL17AF. In some embodiments, the IL17A comprises the amino acid sequence of SEQ ID NO:6 or SEQ ID NO:7, and the IL17F comprises the amino acid sequence of SEQ ID NO:8 or SEQ ID NO:9. In some aspects of the disclosure, the second binding domain of the bispecific antibody comprises HVR-H1 comprising the amino acid sequence of SEQ ID NO:23, HVR-H2 comprising the amino acid sequence of SEQ ID NO:24, HVR-H3 comprising the amino acid sequence of SEQ ID NO:25, HVR-L1 comprising the amino acid sequence of SEQ ID NO:26, HVR-L2 comprising the amino acid sequence of SEQ ID NO:27, and HVR-L3 comprising the amino acid sequence of SEQ ID NO:28. In some aspects of the disclosure, the second binding domain of the bispecific antibody comprises a VH sequence comprising the amino acid sequence of SEQ ID NO:21 and a VL sequence comprising the amino acid sequence of SEQ ID NO:22. In some aspects of the disclosure, the second binding domain of the bispecific antibody comprises a heavy chain comprising the amino acid sequence of SEQ ID NO:29. In some aspects of the disclosure, the second binding domain of the bispecific antibody comprises a heavy chain comprising the amino acid sequence of SEQ ID NO: 30. In some aspects of the disclosure, the second binding domain of the bispecific antibody comprises a light chain comprising the amino acid sequence of SEQ ID NO:31.

In some aspects which are not explicitly claimed, the disclosure provides a quality assay for a pharmaceutical composition comprising a recombinant polypeptide prepared from a bacterial cell, the quality assay comprising subjecting a sample of the pharmaceutical composition to the immunoassay of as described herein for detecting presence or amount of FkpA in the pharmaceutical composition. In some aspects of the disclosure, the amount of FkpA detected in the pharmaceutical composition is no more than about 30 ppm, about 25 ppm, about 20 ppm, about 15 ppm, about 14 ppm, about 13 ppm, about 12 ppm, about 11 ppm, about 10 ppm, about 9 ppm, about 8 ppm, about 7 ppm, about 6 ppm, about 5 ppm, about 4 ppm, about 3 ppm, about 2 ppm, about 1 ppm, about 0.9 ppm, about 0.8 ppm, about 0.7 ppm, about 0.6 ppm, about 0.5 ppm, about 0.4 ppm, about 0.3 ppm, about 0.2 ppm, or about 0.1 ppm as determined by immunoassay. In some aspects of the disclosure, the bacterial cell is an *E. coli* cell. In some aspects of the disclosure, the bacterial cell expresses exogenous FkpA. In some aspects of the disclosure, the sample is a cell lysate. In some aspects of the disclosure, the sample is obtained from the recombinant polypeptide preparation and wherein the recombinant polypeptide preparation has been subjected to one or more chromatographic purification steps. In some aspects of the disclosure, the recombinant polypeptide preparation is a final purified product. In some aspects of the disclosure, the recombinant polypeptide is an antibody or an immunoadhesin. In some aspects of the disclosure, the antibody is a multispecific antibody, a bispecific antibody, a half antibody or an antibody fragment. In some aspects of the disclosure, the recombinant polypeptide is an IgG 1, an IgG2, an IgG3, or an IgG4.

In some aspects which have not been explicitly claimed, the disclosure provides a quality assay for a bispecific antibody comprising a first binding domain that binds IL13 and a second binding domain that binds IL17. In some aspects of the disclosure, the IL13 comprises the amino acid sequence of SEQ ID NO:4 or SEQ ID NO:5. In some aspects of the disclosure, the first binding domain of the bispecific antibody comprises HVR-H1 comprising the amino acid sequence of SEQ ID NO:12, HVR-H2 comprising the amino acid sequence of SEQ ID NO:13, HVR-H3 comprising the amino acid sequence of SEQ ID NO:14, HVR-L1 comprising the amino acid sequence of SEQ ID NO:15, HVR-L2 comprising the amino acid sequence of SEQ ID NO:16, and HVR-L3 comprising the amino acid sequence of SEQ ID NO:17. In some aspects of the disclosure, the first binding domain of the bispecific antibody comprises a VH sequence comprising the amino acid sequence of SEQ ID NO:10 and a VL sequence comprising the amino acid sequence of SEQ ID NO:11. In some aspects of the disclosure, the first binding domain of the bispecific antibody comprises a heavy chain comprising the amino acid sequence of SEQ ID NO:18. In some aspects of the disclosure, the first binding domain of the bispecific antibody comprises a heavy chain comprising the amino acid sequence of SEQ ID NO:19. In some aspects of the disclosure, the first binding domain of the bispecific antibody comprises a light chain comprising the amino acid sequence of SEQ ID NO:20. In some embodiments, the IL17 is human IL17. In some aspects of the disclosure, the second binding domain binds human IL17AA, IL17FF and IL17AF. In some aspects of the disclosure, the IL17A comprises the amino acid sequence of SEQ ID NO:6 or SEQ ID NO:7 and IL17F comprises the amino acid sequence of SEQ ID NO:8 or SEQ ID NO:9. In some aspects of the disclosure, the second binding domain of the bispecific antibody comprises HVR-H1 comprising the amino acid sequence of SEQ ID NO:23, HVR-H2 comprising the amino acid sequence of SEQ ID NO:24, HVR-H3 comprising the amino acid sequence of SEQ ID NO:25, HVR-L1 comprising the amino acid sequence of SEQ ID NO:26, HVR-L2 comprising the amino acid sequence of SEQ ID NO:27, and HVR-L3 comprising the amino acid sequence of SEQ ID NO:28. In some aspects of the disclosure, the second binding domain of the bispecific antibody comprises a VH sequence comprising the amino acid sequence of SEQ ID NO:21 and a VL sequence comprising the amino acid sequence of SEQ ID NO:22. In some aspects of the disclosure, the second binding domain of the bispecific antibody comprises a heavy chain comprising the amino acid sequence of SEQ ID NO:29. In some aspects of the disclosure, the second binding domain of the bispecific antibody comprises a heavy chain comprising the amino acid sequence of SEQ ID NO:30. In some aspects of the disclosure, the second binding domain of the bispecific antibody comprises a light chain comprising the amino acid sequence of SEQ ID NO:31. In some aspects of the disclosure, the assay further comprising the step of detecting an amount of DsbA and/or DsbC in the pharmaceutical composition. In some aspects of the disclosure, the amount of DsbA is no more than about 5 ppm, about 4 ppm, about 3 ppm, about 2 ppm, about 1 ppm, about 0.9 ppm, about 0.8 ppm, about 0.7 ppm, about 0.6 ppm, about 0.5 ppm, about 0.4 ppm, about 0.3 ppm, about 0.2 ppm, or about 0.1 ppm. In some aspects of the disclosure, the amount of DsbC is no more than about 5 ppm, about 4 ppm, about 3 ppm, about 2 ppm, about 1 ppm, about 0.9 ppm, about 0.8 ppm, about 0.7 ppm, about 0.6 ppm, about 0.5 ppm, about 0.4 ppm, about 0.3 ppm, about 0.2 ppm, or about 0.1 ppm as determined by immunoassay.

In some aspects which are not explicitly claimed, the disclosure provides kits for the detection of FkpA in a pharmaceutical composition comprising a recombinant polypeptide prepared from a bacterial cell, said kit comprising anti-FkpA antibodies prepared by any of the methods described herein or any of the compositions of anti-FkpA antibodies described herein. In some aspects of the disclosure, the kit further comprises ultrapure FkpA for use as a reference standard in generating standard curves for quantitating FkpA in a sample and/or for use as a positive control. In some aspects of the disclosure, the ultrapure FkpA is prepared according to any of the methods described herein.

In some aspects which are not explicitly claimed, the disclosure provides compositions comprising a recombinant polypeptide, wherein the recombinant polypeptide was produced in a host cell that expresses endogenous FkpA, wherein the composition comprises FkpA at a concentration of no more than about 6 ppm, about 5 ppm, about 4 ppm, about 3 ppm, about 2 ppm, about 1 ppm, about 0.9 ppm, about 0.8 ppm, about 0.7 ppm, about 0.6 ppm, about 0.5 ppm, about 0.4 ppm, about 0.3 ppm, about 0.2 ppm, or about 0.1 ppm. In some aspects of the disclosure, the concentration of FkpA is determined by an immunoassay. In some aspects of the disclosure, the concentration of FkpA is determined by any of the immunoassays described herein. In some aspects of the disclosure, the recombinant polypeptide was produced in a host cell that is engineered to express exogenous FkpA, wherein the composition comprises no more than about 15 ppm FkpA, about 6 ppm, about 5 ppm, about 4 ppm, about 3 ppm, about 2 ppm, about 1 ppm, about 0.9 ppm, about 0.8 ppm, about 0.7 ppm, about 0.6 ppm, about 0.5 ppm, about 0.4 ppm, about 0.3 ppm, about 0.2 ppm, or about 0.1 ppm. In some aspects of the disclosure, the recombinant polypeptide is an antibody. In some aspects of the disclosure, the antibody is a bispecific antibody. In some aspects of the disclosure, the composition comprises at least about 0.1 ppm FkpA. In some aspects of the disclosure, the host cell expresses exogenous FkpA, wherein the composition comprises no more than about 13 ppm. In some aspects of the disclosure, the host cell expresses exogenous FkpA, wherein the composition comprises no more than about 0.7 ppm. In some aspects of the disclosure, the host cell expresses exogenous FkpA, wherein the composition comprises no more than about 6 ppm.

In some aspects which are not explicitly claimed, the disclosure provides compositions comprising a bispecific antibody, wherein the bispecific antibody comprises a first binding domain that binds IL13, and a second binding domain that binds IL17. In some aspects of the disclosure, the IL13 comprises the amino acid sequence of SEQ ID NO:4 or SEQ ID NO:5. In some aspects of the disclosure, the first binding domain of the bispecific antibody comprises HVR-H1 comprising the amino acid sequence of SEQ ID NO: 12, HVR-H2 comprising the amino acid sequence of SEQ ID NO: 13, HVR-H3 comprising the amino acid sequence of SEQ ID NO: 14, HVR-L1 comprising the amino acid sequence of SEQ ID NO: 15, HVR-L2 comprising the amino acid sequence of SEQ ID NO: 16, and HVR-L3 comprising the amino acid sequence of SEQ ID NO: 17. In some aspects of the disclosure, the first binding domain of the bispecific antibody comprises a VH sequence comprising the amino acid sequence of SEQ ID NO: 10 and a VL sequence comprising the amino acid sequence of SEQ ID NO:11. In some embodiaspects of the disclosurements, the first binding domain of the bispecific antibody comprises a heavy chain comprising the amino acid sequence of SEQ ID NO:18. In some aspects of the disclosure, the first binding domain of the bispecific antibody comprises a heavy chain comprising the amino acid sequence of SEQ ID NO:19. In some aspects of the disclosure, the first binding domain of the bispecific antibody comprises a light chain comprising the amino acid sequence of SEQ ID NO:20. In some aspects of the disclosure, the IL17 is human IL17. In some embodiments, the second binding domain binds human IL17AA, IL17FF and IL17AF. In some aspects of the disclosure, the IL17A comprises the amino acid sequence of SEQ ID NO:6 or SEQ ID NO:7 and IL17F comprises the amino acid sequence of SEQ ID NO:8 or SEQ ID NO:9. In some aspects of the disclosure, the second binding domain of the bispecific antibody comprises HVR-H1 comprising the amino acid sequence of SEQ ID NO:23, HVR-H2 comprising the amino acid sequence of SEQ ID NO:24, HVR-H3 comprising the amino acid sequence of SEQ ID NO:25, HVR-L1 comprising the amino acid sequence of SEQ ID NO:26, HVR-L2 comprising the amino acid sequence of SEQ ID NO:27, and HVR-L3 comprising the amino acid sequence of SEQ ID NO:28. In some aspects of the disclosure, the second binding domain of the bispecific antibody comprises a VH sequence comprising the amino acid sequence of SEQ ID NO:21 and a VL sequence comprising the amino acid sequence of SEQ ID NO:22. In some aspects of the disclosure, the second binding domain of the bispecific antibody comprises a heavy chain comprising the amino acid sequence of SEQ ID NO:29. In some aspects of the disclosure, the second binding domain of the bispecific antibody comprises a heavy chain comprising the amino acid sequence of SEQ ID NO:30. In some aspects of the disclosure, the second binding domain of the bispecific antibody comprises a light chain comprising the amino acid sequence of SEQ ID NO:31. In some aspects of the disclosure, the amount of DsbA is no more than about 5 ppm, about 4 ppm, about 3 ppm, about 2 ppm, about 1 ppm, about 0.9 ppm, about 0.8 ppm, about 0.7 ppm, about 0.6 ppm, about 0.5 ppm, about 0.4 ppm, about 0.3 ppm, about 0.2 ppm, or about 0.1 ppm. In some aspects of the disclosure, the amount of DsbA is determined by an immunoassay. In some aspects of the disclosure, the amount of DsbC is no more than about 5 ppm, about 4 ppm, about 3 ppm, about 2 ppm, about 1 ppm, about 0.9 ppm, about 0.8 ppm, about 0.7 ppm, about 0.6 ppm, about 0.5 ppm, about 0.4 ppm, about 0.3 ppm, about 0.2 ppm, or about 0.1 ppm. In some aspects of the disclosure, the amount of DsbC is determined by an immunoassay. In some aspects of the disclosure, the composition is substantially free of FkpA. In some embodiments, the composition is substantially free of DsbA. In some aspects of the disclosure, the composition is substantially free of DsbC.

In some aspects which are not explicitly claimed, the disclosure provides compositions comprising a bispecific antibody, wherein the recombinant polypeptide was produced by a method comprising a) introducing to a host cell i) nucleic acid encoding a first heavy chain and a first light chain, wherein the first heavy chain and the first light chain form a first antigen binding domain; ii) nucleic acid encoding a second heavy chain and a second light chain, wherein the second heavy chain and the second light chain form a second antigen binding domain; iii) nucleic acid encoding a FkpA polypeptide; wherein the first heavy chain and the second heavy chain form an Fc domain; and b) purifying the antibody, wherein the composition comprises less than about 5 ppm FkpA. In some aspects of the disclosure, the invention provides a composition comprising a bispecific antibody, wherein the bispecific antibody was produced by a method comprising a) introducing to a first host cell i) nucleic acid encoding a first heavy chain and a first light chain, wherein the first heavy chain and the first light chain form a first antigen binding domain; ii) nucleic acid encoding a FkpA polypeptide; b) introducing to a second host cell i) nucleic acid encoding a second heavy chain and a second light chain, wherein the second heavy chain and the second light chain form a second antigen binding domain; ii) nucleic acid encoding a FkpA polypeptide; b) isolating the first antigen binding domain and the second binding domain; c) combining the first antigen binding domain and the second antigen binding domain under conditions where the first antigen binding domain and the second antigen binding domain assemble to form a bispecific antibody; and b) purifying the bispecific antibody, wherein the composition comprises less than about 5 ppm FkpA. In some aspects of the disclosure, the composition comprises less than about 1 ppm FkpA. In some embodiments, the host cell is a prokaryotic host cell. In some aspects of the disclosure, the first host cell and the second host cell are a prokaryotic host cell. In some aspects of the disclosure, the host cell is a gram-negative bacterium. In some aspects of the disclosure, the gram-negative bacterium is *E. coli.* In some aspects of the disclosure, the FkpA is *E. coli* FkpA. In some aspects of the disclosure, the bispecific antibody comprises a first binding domain that binds IL13 and a second binding domain that binds IL17. In some aspects of the disclosure, the IL13 comprises the amino acid sequence of SEQ ID NO:4 or SEQ ID NO:5. In some aspects of the disclosure, the first binding domain of the bispecific antibody comprises HVR-H1 comprising the amino acid sequence of SEQ ID NO:12, HVR-H2 comprising the amino acid sequence of SEQ ID NO:13, HVR-H3 comprising the amino acid sequence of SEQ ID NO:14, HVR-L1 comprising the amino acid sequence of SEQ ID NO:15, HVR-L2 comprising the amino acid sequence of SEQ ID NO:16, and HVR-L3 comprising the amino acid sequence of SEQ ID NO:17. In some aspects of the disclosure, the first binding domain of the bispecific antibody comprises a VH sequence comprising the amino acid sequence of SEQ ID NO:10 and a VL sequence comprising the amino acid sequence of SEQ ID NO:11. In some aspects of the disclosure, the first binding domain of the bispecific antibody comprises a heavy chain comprising the amino acid sequence of SEQ ID NO:18. In some aspects of the disclosure, the first binding domain of the bispecific antibody comprises a heavy chain comprising the amino acid sequence of SEQ ID NO:19. In some aspects of the disclosure, the first binding domain of the bispecific antibody comprises a light chain comprising the amino acid sequence of SEQ ID NO:20. In some aspects of the disclosure, the IL17 is human IL17. In some aspects of the disclosure, the second binding domain binds human IL17AA, IL17FF and IL17AF. In some aspects of the disclosure, wherein the IL17A comprises the amino acid sequence of SEQ ID NO:6 or SEQ ID NO:7 andIL17F comprises the amino acid sequence of SEQ ID NO:8 or SEQ ID NO:9. In some aspects of the disclosure, the second binding domain of the bispecific antibody comprises HVR-H1 comprising the amino acid sequence of SEQ ID NO:23, HVR-H2 comprising the amino acid sequence of SEQ ID NO:24, HVR-H3 comprising the amino acid sequence of SEQ ID NO:25, HVR-L1 comprising the amino acid sequence of SEQ ID NO:26, HVR-L2 comprising the amino acid sequence of SEQ ID NO:27, and HVR-L3 comprising the amino acid sequence of SEQ ID NO:28. In some aspects of the disclosure, the second binding domain of the bispecific antibody comprises a VH sequence comprising the amino acid sequence of SEQ ID NO:21 and a VL sequence comprising the amino acid sequence of SEQ ID NO:22. In some aspects of the disclosure, the second binding domain of the bispecific antibody comprises a heavy chain comprising the amino acid sequence of SEQ ID NO:29. In some aspects of the disclosure, the second binding domain of the bispecific antibody comprises a heavy chain comprising the amino acid sequence of SEQ ID NO:30. In some aspects of the disclosure, the second binding domain of the bispecific antibody comprises a light chain comprising the amino acid sequence of SEQ ID NO:31. In some aspects of the disclosure, the composition comprises no more than about 15 ppm DsbA. In some aspects of the disclosure, the composition comprises no more than about 5 ppm DsbA. In some aspects of the disclosure, the composition comprises no more than about 15 ppm DsbC. In some aspects of the disclosure, the composition comprises no more than about 5 ppm DsbC. In some aspects of the disclosure, the DsbA is determined by immunoassay. In some aspects of the disclosure, the DsbC is measured by immunoassay.

Herein disclosed are methods for purifying a polypeptide from a composition comprising the multispecific antibody and one or more impurities, the method comprising the sequential steps of a) subjecting the composition to affinity chromatography to produce an affinity eluate, b) subjecting the affinity eluate to mixed-mode chromatography to generate a mixed-mode eluate, and c) subjecting the mixed-mode eluate to hydrophobic interaction chromatography and collecting a fraction comprising the polypeptide, wherein the method reduces the amount of product-specific impurities from the composition. In some aspects of the disclosure, the mixed-mode chromatography is a mixed-mode anion exchange chromatography. In some aspects of the disclosure, the affinity chromatography is carried out in bind and elute mode.

In some aspects of the disclosure, the mixed-mode chromatography is carried out in bind and elute mode. In some aspects of the disclosure, the HIC is carried out in flow-through mode.

Herein disclosed are methods for purifying a multispecific antibody from a composition comprising the multispecific antibody and an impurity, wherein the multispecific antibody comprises multiple arms, each arm comprising a VH/VL unit, the method comprising the sequential steps of a) subjecting the composition to protein A chromatography to produce a protein A eluate, b) subjecting the protein A eluate to mixed-mode chromatography to generate a mixed-mode eluate, and c) subjecting the mixed-mode eluate to hydrophobic interaction chromatography and collecting a fraction comprising the multispecific antibody, wherein the method reduces the amount of product-specific impurities from the composition. In some aspects of the disclosure, the mixed-mode chromatography is a mixed-mode anion exchange chromatography. In some aspects of the disclosure, the affinity chromatography is carried out in bind and elute mode. In some aspects of the disclosure, the mixed-mode chromatography is carried out in bind and elute mode. In some aspects of the disclosure, the HIC is carried out in flow-through mode.

In some aspects, the disclosure provides methods for purifying a multispecific antibody from a composition comprising the multispecific antibody and an impurity, wherein the multispecific antibody comprises multiple arms, each arm comprising a VH/VL unit, wherein each arm of the multispecific antibody is produced separately, the method comprising the sequential steps of a) subjecting each arm of the multispecific antibody to protein A chromatography to produce protein A eluates for each arm of the multispecific antibody, b) forming a mixture comprising protein A eluates of each arm of the multispecific antibody under conditions sufficient to produce a composition comprising the multispecific antibody, c) subjecting the composition comprising the multispecific antibody to mixed-mode chromatography to generate a mixed-mode eluate, and d) subjecting the mixed-mode eluate to hydrophobic interaction chromatography (HIC) and collecting a fraction comprising the multispecific antibody wherein the method reduces the amount of product-specific impurities from the composition. In some aspects of the disclosure, the mixed-mode chromatography is a mixed-mode anion exchange chromatography. In some aspects of the disclosure, the affinity chromatography is carried out in bind and elute mode. In some aspects of the disclosure, the mixed-mode chromatography is carried out in bind and elute mode. In some aspects of the disclosure, the hydrophobic interaction chromatography is carried out in flow-through mode. In some aspects of the disclosure, the mixed-mode eluate is subjected to anion exchange chromatography prior to hydrophobic interaction chromatography. In some aspects of the disclosure, the anion exchange chromatography is carried out in bind and elute mode.

In some aspects of the above disclosures, the methods further comprising the step of subjecting the hydrophobic interaction chromatography fraction comprising the multispecific antibody to cation exchange chromatography and collecting a fraction comprising the multispecific antibody. In some aspects of the disclosure, the cation exchange chromatography is carried out in bind and elute mode.

In some aspects, the disclosure provides methods for purifying a polypeptide from a composition comprising the polypeptide and one or more impurities, the method comprising the sequential steps of a) subjecting the composition to affinity chromatography to produce an affinity eluate, b) subjecting the affinity eluate to mixed-mode chromatography to generate a mixed-mode eluate, and c) subjecting the mixed-mode eluate to anion exchange chromatography to generate an anion exchange eluate, d) subjecting the anion exchange eluate to hydrophobic interaction chromatography and collecting a fraction comprising the polypeptide, wherein the method reduces the amount of product-specific impurities from the composition.

In some aspects, the invention provides methods for purifying a multispecific antibody from a composition comprising the multispecific antibody and an impurity, wherein the multispecific antibody comprises multiple arms, each arm comprising a VH/VL unit, the method comprising the sequential steps of a) subjecting the composition to protein A chromatography to produce a protein A eluate, b) subjecting the protein A eluate to mixed-mode chromatography to generate a mixed-mode eluate, and c) subjecting the mixed-mode eluate to anion exchange chromatography to generate an anion exchange eluate, d) subjecting the anion exchange eluate to hydrophobic interaction chromatography and collecting a fraction comprising the multispecific antibody, wherein the method reduces the amount of product-specific impurities as defined in the claims from the composition.

In some aspects, the invention provides methods for purifying a multispecific antibody from a composition comprising the multispecific antibody and an impurity, wherein the multispecific antibody comprises multiple arms, each arm comprising a VH/VL unit, wherein each arm of the multispecific antibody is produced separately, the method comprising the sequential steps of a) subjecting each arm of the multispecific antibody to protein A chromatography to produce protein A eluates for each arm of the multispecific antibody, b) forming a mixture comprising protein A eluates of each arm of the multispecific antibody under conditions sufficient to produce a composition comprising the multispecific antibody c) subjecting the composition comprising the multispecific antibody to mixed-mode chromatography to generate a mixed-mode eluate, and d) subjecting the mixed-mode eluate to anion exchange chromatography to generate an anion exchange eluate, e) subjecting the anion exchange eluate to hydrophobic interaction chromatography in and collecting a fraction comprising the multispecific antibody and collecting a fraction comprising the multispecific antibody, wherein the method reduces the amount of product-specific impurities as defined in the claims from the composition.

In some embodiments of the above aspects, the mixed-mode chromatography is a mixed-mode anion exchange chromatography. In some embodiments, the affinity chromatography is carried out in bind and elute mode. In some embodiments, the mixed-mode chromatography is carried out in bind and elute mode. In some embodiments, the anion exchange chromatography is carried out in bind and elute mode. In some embodiments, the hydrophobic interaction chromatography is carried out in flow-through mode. The methods further comprise the step of subjecting the hydrophobic interaction chromatography fraction comprising the multispecific antibody to cation exchange chromatography. In some embodiments, the cation exchange chromatography is carried out in bind and elute mode. In some embodiments, the methods further comprise the step of subjecting the hydrophobic interaction chromatography fraction comprising the multispecific antibody to ultrafiltration. In some embodiments, the methods further comprise the step of subjecting the cation exchange fraction comprising the multispecific antibody to ultrafiltration. In some embodiments, the ultrafiltration comprises sequentially a first ultrafiltration, a diafiltration and a second ultrafiltration.

In some aspects, the disclosure provides a method for purifying a polypeptide from a composition comprising the polypeptide and one or more impurities, the method comprising: a) subjecting the composition to affinity chromatography to produce a affinity eluate, b) subjecting the affinity eluate to mixed-mode chromatography to generate a mixed-mode eluate, and c) subjecting the mixed-mode eluate to anion exchange chromatography to generate an anion exchange eluate, d) subjecting the anion exchange eluate to hydroxyapapatite chromatography and collecting a fraction comprising the polypeptide, wherein the method reduces the amount of product-specific impurities from the composition. In some aspects, the disclosure provides a method for purifying a multispecific antibody from a composition comprising the multispecific antibody and one or more impurities, wherein the multispecific antibody comprises multiple arms, each arm comprising a VH/VL unit, the method comprising the sequential steps of a) subjecting the composition to protein A chromatography to produce a protein A eluate, b) subjecting the protein A eluate to mixed-mode chromatography to generate a mixed-mode eluate, and c) subjecting the mixed-mode eluate to anion exchange chromatography to generate an anion exchange eluate, d) subjecting the anion exchange eluate to hydroxyapatite chromatography and collecting a fraction comprising the multispecific antibody, wherein the method reduces the amount of product-specific impurities from the composition. In some embodiments, the invention provides a method for purifying a multispecific antibody from a composition comprising the multispecific antibody and one or more impurities, wherein the multispecific antibody comprises multiple arms, each arm comprising a VH/VL unit, wherein each arm of the multispecific antibody is produced separately, the method comprising the sequential steps of a) subjecting each arm of the multispecific antibody to protein A chromatography to produce protein A eluates for each arm of the multispecific antibody, b) forming a mixture comprising protein A eluates of each arm of the multispecific antibody under conditions sufficient to produce a composition comprising the multispecific antibody c) subjecting the composition comprising the multispecific antibody to mixed-mode chromatography to generate a mixed-mode eluate, and d) subjecting the mixed-mode eluate to anion exchange chromatography to generate an anion exchange eluate, e) subjecting the anion exchange eluate to hydroxyapatite chromatography in and collecting a fraction comprising the multispecific antibody and collecting a fraction comprising the multispecific antibody, wherein the method reduces the amount of product-specific impurities from the composition. In some embodiments, the mixed-mode chromatography is a mixed-mode anion exchange chromatography. In some embodiments, the affinity chromatography is carried out in bind and elute mode. In some embodiments, the protein A chromatography is carried out in bind and elute mode. In some embodiments, the mixed-mode chromatography is carried out in bind and elute mode. In some embodiments, the anion exchange chromatography is carried out in bind and elute mode. In some embodiments, the hydroxyapatite chromatography is carried out in flow-through mode.

In some embodiments of the above aspects and embodiments, the protein A chromatography comprises protein A linked to agarose. In some embodiments, the protein A chromatography is a MabSelect™, MabSelect SuRe™ and MabSelect SuRe™ LX, ProSep®-vA, ProSep® Ultra Plus, Protein A Sepharose® Fast Flow, or Toyopearl® AF-rProtein A chromatography. In some embodiments, the protein A chromatography uses one or more of a protein A equilibration buffer, a protein A loading buffer or a protein A wash buffer wherein the equilibration buffer, a loading buffer, and/or wash buffer is between about pH 7 and about pH 8. In some embodiments, the protein A equilibration buffer is about pH 7.7. In some embodiments, the protein A equilibration buffer comprises about 25 mM Tris and about 25 mM NaCl. In some embodiments, the protein A chromatography is washed with equilibration buffer following load. In some embodiments, the multispecific antibody is eluted from the protein A chromatography by pH gradient. In some embodiments, the multispecific antibody is eluted from the protein A by applying a protein A elution buffer with low pH to the protein A chromatography. In some embodiments, the protein A elution buffer comprises about 150 mM acetic acid, about pH 2.9. In some embodiments, the protein A eluate is pooled where the OD280 of the eluate is greater than about 0.5.

In some embodiments of the above aspects and embodiments, the mixed-mode chromatography comprises a quaternary amine and a hydrophobic moiety. In some embodiments, the mixed-mode chromatography comprises a quaternary amine and a hydrophobic moiety linked to highly crosslinked agarose. In some embodiments, the mixed-mode chromatography comprises N-benzyl-n-methyl ethanolamine. In some embodiments, the mixed-mode chromatography is a Capto™ adhere chromatography. In some embodiments, the mixed-mode chromatography uses one or more of a mixed-mode pre-equilibration buffer, a mixed-mode equilibration buffer, a mixed-mode loading buffer or a mixed-mode wash buffer wherein the mixed-mode pre-equilibration buffer, the mixed-mode equilibration buffer, and/or mixed-mode wash buffer is between about pH 6 and about pH 7. In some embodiments, the mixed-mode pre-equilibration buffer, the mixed-mode equilibration buffer, and/or mixed-mode wash buffer is about pH 6.5. In some embodiments, the mixed-mode pre-equilibration buffer comprises about 500 mM acetate. In some embodiments, the mixed-mode equilibration buffer comprises about 50 mM acetate. In some embodiments, the mixed-mode chromatography is washed with wash buffer following load. In some embodiments, the multispecific antibody is eluted from the mixed-mode chromatography by pH gradient. In some embodiments, the multispecific antibody is eluted from the mixed-mode chromatography by applying a mixed-mode elution buffer with low pH to the mixed-mode anion exchange chromatography. In some embodiments, the mixed-mode elution buffer comprises about 25 mM acetate, about pH 5.2. In some embodiments, the mixed-mode eluate is pooled where the OD280 of the eluate is greater than about 0.5 to about 1.0.

In some embodiments of the above aspects and embodiments, the hydrophobic interaction chromatography (HIC) comprises a hydrophobic moiety. In some embodiments, the hydrophobic interaction chromatography comprises a phenyl moiety. In some embodiments, the hydrophobic interaction chromatography comprises a hydrophobic moiety linked to crosslinked agarose. In some embodiments, the hydrophobic interaction chromatography comprises a phenyl moiety or a butyl moiety linked to crosslinked agarose. In some embodiments, the hydrophobic interaction chromatography is Phenyl Sepharose® chromatography or Butyl Sepharose® chromatography. In some embodiments, the hydrophobic interaction exchange chromatography uses one or more of a HIC equilibration buffer, a HIC loading buffer or a HIC wash buffer wherein the HIC equilibration buffer, the HIC load buffer and/or the HIC wash buffer is between about pH 5 and about pH 6. In some embodiments, the HIC equilibration buffer, the load buffer, and/or the wash buffer is about pH 5.5. In some embodiments, the HIC equilibration buffer and the wash buffer comprises about 170 mM acetate. In some embodiments, the HIC load buffer is 50 mM Tris, 100 mM sodium acetate, pH about 5.5.

In some embodiments, the hydrophobic interaction chromatography comprises a hexyl moiety. In some embodiments, the hydrophobic interaction chromatography comprises a hydrophobic moiety linked to hydroxylated methacrylic polymer. In some embodiments, the hydrophobic interaction chromatography comprises a hexyl moiety linked to hydroxylated methacrylic polymer. In some embodiments, the hydrophobic interaction chromatography is TOYOPEARL® Hexyl 650C. In some embodiments, the hydrophobic interaction exchange chromatography uses one or more of a HIC equilibration buffer, a HIC loading buffer or a HIC wash buffer wherein the HIC equilibration buffer, the HIC load buffer and/or the HIC wash buffer is between about pH 6 and about pH 8. In some embodiments, the HIC equilibration buffer, the load buffer, and/or the wash buffer is about pH 7.0. In some embodiments, the HIC equilibration buffer and the wash buffer comprises about 50 mM 3-(N-Morpholino)propanesulfonic acid (MOPS) and 125 mM acetate.

In some embodiments, the hydrophobic interaction chromatography is washed with HIC wash buffer following load. In some embodiments, the hydrophobic interaction eluate is pooled where the OD280 of the eluate is greater than about 0.5 to about 1.0.

In some embodiments of the above aspects and embodiments, the anion exchange chromatography comprises a quaternary amine. In some embodiments, the anion exchange chromatography comprises a quaternary amine linked to crosslinked agarose. In some embodiments, the mixed-mode anion exchange chromatography is a Q Sepharose® Fast Flow (QSFF) chromatography. In some embodiments, the anion exchange chromatography uses one or more of an anion exchange pre-equilibration buffer, an anion exchange equilibration buffer or an anion exchange loading buffer wherein the anion exchange pre-equilibration buffer, the anion exchange equilibration buffer and/or anion exchange the load buffer is between about pH 8 and about pH 9. In some embodiments, the anion exchange pre-equilibration buffer, the anion exchange equilibration buffer and/or the anion exchange load buffer is about pH 8.5. In some embodiments, the anion exchange pre-equilibration buffer comprises about 50 mM Tris, 500 mM sodium acetate. In some embodiments, the anion exchange equilibration buffer comprises about 50 mM Tris. In some embodiments, the anion exchange chromatography is washed with anion exchange equilibration buffer following load. In some embodiments, the multispecific antibody is eluted from the anion exchange chromatography by salt gradient. In some embodiments, the multispecific antibody is eluted from the anion exchange chromatography by applying an anion exchange elution buffer with increased salt concentration to the anion exchange chromatography. In some embodiments, the anion exchange elution buffer comprises about 50 mM Tris, 100 mM sodium acetate at about pH 8.5. In some embodiments, the anion exchange eluate is pooled where the OD280 of the eluate is greater than about 0.5 to about 1.0.

In some embodiments of the above aspects and embodiments, the hydroxyapatite chromatography is a ceramic hydroxyapatite chromatography. In some embodiments, hydroxyapatite chromatography is a CHT Type I ceramic hydroxyapaptite chromatography. In some embodiments, the hydroxyapatite chromatography uses one or more of a hydroxyapatite buffer A or hydroxyapatite buffer B wherein the hydroxyapatite buffer B has a higher conductivity compared to hydroxyapatite buffer A. In some embodiments, the hydroxyapatite buffer A is an equilibration buffer comprising about 10 mM sodium phosphate at about pH 6.8. In some embodiments, the hydroxyapatite buffer B comprises about 10 mM sodium phosphate and about 1 M sodium chloride at about pH 6.8. In some embodiments, the polypeptide or multispecific antibody is eluted from the hydroxyapaptide chromatography using a gradient of hydroxyapatite buffer B in hydroxyapatite buffer A. In some embodiments, the gradient is a linear gradient. In some embodiments, the gradient increases from about 0% hydroxyapaptite buffer B to about 100% hydroxyapaptite buffer B. In some embodiments, the gradient increases from 0% hydroxyapaptite buffer B to 100% hydroxyapaptite buffer B in about 20 column volumes. In some embodiments the hydroxyapatite eluate is pooled where the $OD_{280}$ of the eluate is greater than about 0.5 to about 1.0.

In some embodiments of the above aspects and embodiments, the cation exchange chromatography comprises a carboxylic acid moiety or a sulfonic acid moiety. In some embodiments, the carboxylic acid moiety or the sulfonic acid moiety is a sulphopropyl, asulfoethyl, a sulfoisobutyl, or a carboxyl moiety. In some embodiments, the cation exchange chromatography comprises a carboxylic acid moiety or a sulfonic acid moiety linked to crosslinked agarose. In some embodiments, the cation exchange material is POROS® HS 50, POROS® HS 20, $SO_3$ Monolith, S Ceramic HyperD®, sulphopropyl-Sepharose® Fast Flow (SPSFF), SP-Sepharose® XL (SPXL), CM Sepharose® Fast Flow, Capto™ S, Fractogel® EMD Se Hicap, Fractogel® EMD $SO_3^-$, or Fractogel® EMD $COO^-$. In some embodiments, the cation exchange loading density is less than about 20 g/L. In some embodiments, the cation exchange chromatography uses one or more of a cation exchange pre-equilibration buffer, an cation exchange equilibration buffer, cation exchange loading buffer or cation exchange wash buffer wherein the cation exchange pre-equilibration buffer, the cation exchange equilibration buffer, the cation exchange load buffer and/or cation exchange wash buffer is between about pH 5.0 and about pH 6.0. In some embodiments, the cation exchange cation exchange the equilibration buffer, the cation exchange load buffer and/or cation exchange wash buffer is about pH 5.5. In some embodiments, the cation exchange equilibration buffer comprises 50 mM sodium acetate. In some embodiments, the cation exchange wash buffer comprises 50 mM sodium acetate. In some embodiments, the cation exchange chromatography is washed with equilibration buffer following load. In some embodiments, the multispecific antibody is eluted from the cation exchange chromatography by salt gradient. In some embodiments, the multispecific antibody is eluted from the cation exchange chromatography by applying a cation exchange elution buffer with increased salt concentration to the cation exchange chromatography. In some embodiments, the cation exchange elution buffer comprises 500 mM sodium acetate, pH 5.8. In some embodiments, the elution is a gradient elution of 10% to 100% cation exchange elution buffer. In some embodiments, the cation exchange eluate is pooled where the OD280 of the eluate is greater than about 0.5 to about 1.0.

In some embodiments of the above aspects and embodiments, the arms of the multispecific antibodies are produced in a cell. In some embodiments, the cell is a prokaryotic cell. In some embodiments, the prokaryotic cell is in *E. coli* cell. In some embodiments, the cell is engineered to express one or more chaperones. In some embodiments, the chaperone is one or more of FkpA, DsbA or DsbC. In some embodiments, the chaperone is an *E. coli* chaperone. In some embodiments, the cells are lysed to generate a cell lysate comprising the multispecific antibody or an arm of the multispecific antibody prior to protein A chromatography. In some embodiments, the cells are lysed using a microfluidizer. In some embodiments, polyethlyeneimine (PEI) is added to the cell lysate prior to chromatography. In some embodiments, the PEI is added to the lysate to a final concentration of about 0.4%. In some embodiments, the cell lysate is clarified by centrifugation.

In some embodiments of the above aspects and embodiments, the product specific impurity is one or more of non-paired antibody arms, antibody homodimers, aggregates, high molecular weight species (HMW), low molecular weight species (LMW), acidic variants, or basic variants. In some embodiments, the methods reduce the amount of any one of *E. coli* protein (ECP), FkpA, DsbA, DsbC, leached protein A, nucleic acid, cell culture media components, or viral impurities in the composition.

In some embodiments, the multispecific antibody is a bispecific antibody. In some embodiments, a first arm of the multispecific antibody binds IL13. In some embodiments, the IL13 is human IL13. In some embodiments, the IL13 comprises the amino acid sequence of SEQ ID NO:1 or SEQ ID NO:2. In some embodiments, the first arm of the multispecific antibody comprises HVR-H1 comprising the amino acid sequence of SEQ ID NO:7, HVR-H2 comprising the amino acid sequence of SEQ ID NO:8, HVR-H3 comprising the amino acid sequence of SEQ ID NO:9, HVR-L1 comprising the amino acid sequence of SEQ ID NO:10, HVR-L2 comprising the amino acid sequence of SEQ ID NO:11, and HVR-L3 comprising the amino acid sequence of SEQ ID NO:12. In some embodiments, the first arm of the multispecific antibody comprises a VH sequence comprising the amino acid sequence of SEQ ID NO:13 and a VL sequence comprising the amino acid sequence of SEQ ID NO:14. In some embodiments, the first arm of the multispecific antibody comprises a heavy chain comprising the amino acid sequence of SEQ ID NO:15. In some embodiments, the first arm of the multispecific antibody comprises a heavy chain comprising the amino acid sequence of SEQ ID NO:16. In some embodiments, the first arm of the multispecific antibody comprises a light chain comprising the amino acid sequence of SEQ ID NO:17. In some embodiments, a second arm of the antibody binds IL17. In some embodiments, the IL17 is human IL17. In some embodiments, the IL17 comprises the amino acid sequence of SEQ ID NO:3, SEQ ID NO: 4, SEQ ID NO:5 or SEQ ID NO:6. In some embodiments, a second arm of the multispecific antibody comprises HVR-H1 comprising the amino acid sequence of SEQ ID NO:18, HVR-H2 comprising the amino acid sequence of SEQ ID NO:19, HVR-H3 comprising the amino acid sequence of SEQ ID NO:20, HVR-L1 comprising the amino acid sequence of SEQ ID NO:21, HVR-L2 comprising the amino acid sequence of SEQ ID NO:22, and HVR-L3 comprising the amino acid sequence of SEQ ID NO:23. In some embodiments, the second arm of the multispecific antibody comprises a VH sequence comprising the amino acid sequence of SEQ ID NO:25 and a VL sequence comprising the amino acid sequence of SEQ ID NO:26. In some embodiments, the second arm of the multispecific antibody comprises a heavy chain comprising the amino acid sequence of SEQ ID NO:27. In some embodiments, the second arm of the multispecific antibody comprises a heavy chain comprising the amino acid sequence of SEQ ID NO:28. In some embodiments, the second arm of the multispecific antibody comprises a light chain comprising the amino acid sequence of SEQ ID NO:29.

In some aspects, the invention provides a composition comprising a multispecific antibody purified by any of the method described herein, wherein the composition comprises no more than about 5%, 4%, 3%, 2% or 1% product specific impurities. In some embodiments, the product specific impurity is one or more of non-paired antibody arms, antibody homodimers, aggregates, high molecular weight species (HMW), low molecular weight species (LMW), acidic variants, or basic variants. In some embodiments, the composition comprises less than about composition comprises no more than about 5%, 4%, 3%, 2% or 1% of any one of *E. coli* protein (ECP), FkpA, DsbA, DsbC, leached protein A, nucleic acid, cell culture media components, or viral impurities in the composition.

BRIEF DESCRIPTION OF THE DRAWINGS

FIG. 1 shows SDS polyacrylamide gel electrophoresis (PAGE) of fractions from a gravity-flow column packed with the strong cation-exchange media SP Sepharose® Fast Flow (GE) for the purification of FkpA. Lane markers as indicated on right.

FIG. 2 shows a chromatogram from SP Sepharose® Fast Flow (GE) chromatography using step elution for the purification of FkpA.

FIGs. 3A and 3B show SDS polyacrylamide gel electrophoresis (PAGE) of fractions 3-13 (FIG. 3A) and 14-23 (FIG. 3B) from SP Sepharose® Fast Flow (GE) chromatography using step elution for the purification of FkpA. Lane markers as indicated on right.

FIG. 4 shows a chromatogram from SP Sepharose® Fast Flow (GE) chromatography using a gradient elution for the purification of FkpA.

FIG. 5 shows SDS polyacrylamide gel electrophoresis (PAGE) of fractions from a gravity-flow column packed with the anion-exchange chromatography media Q Sepharose® Fast Flow (GE) and the hydrophobic interaction chromatography (HIC) media Phenyl Sepharose® Fast Flow (low substitution) (GE) for the purification of FkpA. Lane markers as indicated below.

FIG. 6 shows SDS polyacrylamide gel electrophoresis (PAGE) of fractions from a gravity-flow column packed with the hydrophobic interaction chromatography (HIC) media Butyl-S Sepharose® 6 Fast Flow (GE) for the purification of FkpA. Lane markers as indicated below.

FIGs. 7A and 7B show SDS polyacrylamide gel electrophoresis (PAGE) of fractions from gravity-flow column packed with the hydrophobic interaction chromatography (HIC) media Butyl Sepharose® 4 Fast Flow (GE) for the purification of FkpA. Lane markers as indicated on right.

FIG. 8 shows SDS polyacrylamide gel electrophoresis (PAGE) of fractions from gravity-flow column packed with the hydrophobic interaction chromatography (HIC) media Butyl-S Sepharose® 6 Fast Flow (GE) for the purification of FkpA. Lane markers as indicated below.

FIG. 9 shows a chromatogram from Butyl-S Sepharose® Fast Flow (GE) chromatography for the purification of FkpA.

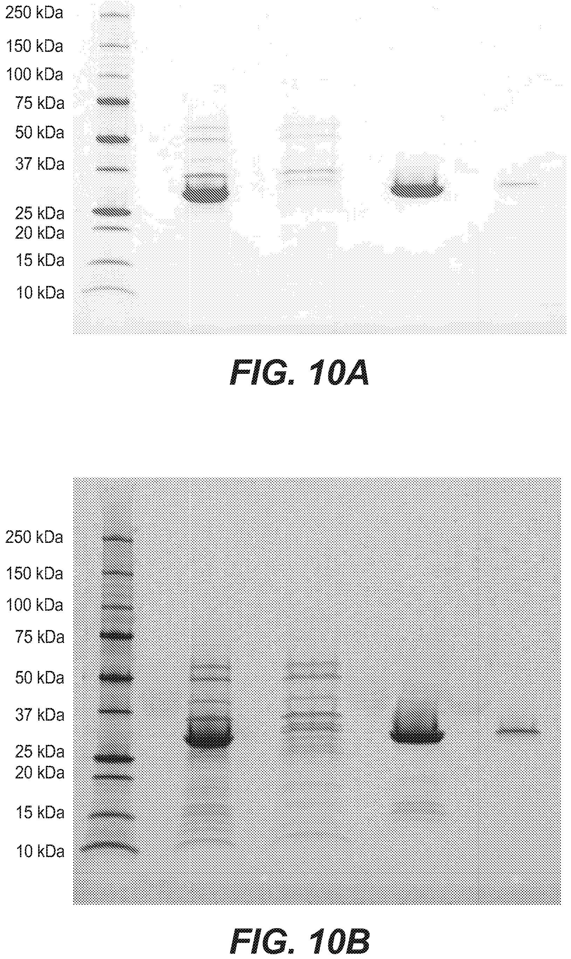

FIG. 10A shows SDS polyacrylamide gel electrophoresis (PAGE) of fractions from a gravity-flow column packed with Butyl-S Sepharose® material for the purification of FkpA. Lane markers as indicated. FIG. 10B shows SDS polyacrylamide gel electrophoresis (PAGE) imaged with SYPRO Ruby of fractions from a gravity-flow column packed with Butyl-S Sepharose® material for the purification of FkpA. Lane 1-blank, Lane 2-precision standards, Lane 3-blank, Lane 4-SP fr. 22-50 10 μL sample, Lane 5-blank, Lane 6-F-T/W 20 10 μL sample, Lane 7-blank, Lane 8-PW eluate 20 μL sample, Lane 9-blank, Lane 10-post pool 20 μL sample.

FIG. 11 shows high performance liquid chromatography (HPLC) size exclusion chromatography (SEC) chromatogram of pooled fractions from Butyl-S Sepharose® purification of FkpA

FIG. 12 shows a chromatogram from Run 1 of Superdex 200 (GE) chromatography for purification of FkpA.

FIG. 13A shows SDS polyacrylamide gel electrophoresis (PAGE) of fractions from Superdex 200 Run 1 for the purification of FkpA. FIG. 13B shows SDS polyacrylamide gel electrophoresis (PAGE) imaged with SYPRO Ruby of fractions from Superdex 200 Run 1 for the purification of FkpA. Lane markers as indicated at the bottom of FIG. 13B..

FIG. 14 shows SDS polyacrylamide gel electrophoresis (PAGE) imaged with SYPRO Ruby and western blot of fraction 37 from Superdex 200 Run 1 for the purification of FkpA. Lane markers as indicated.

FIG. 15 shows a chromatogram from Run 2 of Superdex 200 for purification of FkpA.

FIG. 16 shows a chromatogram from high performance liquid chromatography (HPLC) size exclusion chromatography (SEC) of pooled fractions 37-44 from Superdex 200 Run 2 for the purification of FkpA.

FIG. 17 shows SDS polyacrylamide gel electrophoresis (PAGE) of pooled fractions from 35-40 from Superdex 200 Run 1 and 37-44 from Superdex 200 Runs 2-4 for the purification of FkpA. Lane markers as indicated.

FIG. 18 shows SDS polyacrylamide gel electrophoresis (PAGE) of ultrapure FkpA after 0-3 freeze-thaws. Lane markers as indicated.

FIG. 19A shows a chromatogram from high performance liquid chromatography (HPLC) size exclusion chromatography (SEC) of Ultrapure FkpA after 0 freeze-thaws. FIG.19B shows a chromatogram from high performance liquid chromatography (HPLC) size exclusion chromatography (SEC) of Ultrapure FkpA after 3 freeze-thaws.

FIG. 20 shows the results of a direct binding ELISA using the pre-immunization sera and antisera of Rabbit A, B, and C. Open circles represent pre-immunization serum from Rabbit A, open squares represent pre-immunization serum from Rabbit B, open triangles represent pre-immunization serum from Rabbit C, open diamonds represent antisera from Rabbit A, closed circles represent antisera from Rabbit B, closed squares represent antisera from Rabbit C.

FIG. 21 shows SDS polyacrylamide gel electrophoresis (PAGE) of ASP anti-FkpA antibodies. Lane markers as indicated.

FIG. 22 shows the results of a direct binding ELISA using ASP anti-FkpA antibodies.

FIG. 23 shows the results of a sandwich ELISA using ASP anti-FkpA antibodies. Open circles represent detection antibody diluted to 1:60, open squares represent detection antibody diluted to 1:80, open triangles represent detection antibody diluted to 1:100, open diamonds represent detection antibody diluted to 1:110.

FIG. 24 shows a chromatogram from the affinity purification of ASP anti-FkpA antibodies

FIG. 25 shows a chromatogram from Superdex 200purification of AF-purified anti-FkpA antibodies.

FIG. 26A shows high performance liquid chromatography (HPLC) size exclusion chromatography (SEC) chromatogram of fractions 41-63 of Superdex 200 purification of AF-purified anti-FkpA antibodies. FIG. 26B shows high performance liquid chromatography (HPLC) size exclusion chromatography (SEC) chromatogram of fractions 64-86 of Superdex 200 purification of AF-purified anti-FkpA antibodies.

FIG. 27 shows the results of a sandwich ELISA using AF anti-FkpA antibodies. Open circles represent detection antibody diluted to 1:2000, open squares represent detection antibody diluted to 1:4000, open triangles represent detection antibody diluted to 1:6000, open diamonds represent detection antibody diluted to 1:8000.

FIG. 28A shows the results of a sandwich ELISA using ASP anti-FkpA antibodies. FIG. 28B shows the results of a sandwich ELISA using AF anti-FkpA antibodies.

FIG. 29 shows a chromatogram from high performance liquid chromatography (HPLC) size exclusion chromatography of AF anti-FkpA antibodies.

FIG. 30 shows SDS polyacrylamide gel electrophoresis (PAGE) of AF-purified anti-FkpA antibodies after 0-3 freeze-thaws. Lane markers as indicated.

FIG. 31A shows a chromatogram from high performance liquid chromatography (HPLC) size exclusion chromatography (SEC) of AF-purified anti-FkpA antibodies after 0 freeze-thaws. FIG. 31B shows a chromatogram from high performance liquid chromatography (HPLC) size exclusion chromatography (SEC) of AF-purified anti-FkpA antibodies after 3 freeze-thaws.

FIG. 32 shows the functional testing of freeze thaw stability samples of AF anti-FkpA antibodies.

FIG. 33 shows the results of a sandwich ELISA using ASP anti-FkpA antibodies from subsequent bleeds of Rabbits A, B, and C. Open circles represent affinity purified coat antibodies, open squares represent non-lysed coat antibodies, open triangles represent lysed coat antibodies.

FIG. 34 shows monitoring of control stability over time for ultrapure FkpA at -70 °C and 2-8 °C.

FIG. 35A shows monitoring of control stability over time for ultrapure FkpA when diluted to 3 ng/mL in 0.2% fish gelatin, 1 mg/mL ApoMab, or buffer C. FIG. 35B shows monitoring of control stability over time for ultrapure FkpA when diluted to 15 ng/mL in 0.2% fish gelatin, 1 mg/mL ApoMab or buffer C.

FIG. 36 shows the results of a sandwich ELISA using different FkpA standards. Open circles represent ultrapure FkpA standard, open squares represent MyBioSource FkpA standard catalog number MBS1037402, open triangles represent MyBioSource FkpA standard catalog number MBS1182120.

FIGs. 37A and 37B show high-throughput screening for resolution of product-related impurities on mixed-mode anion exchange (Capto™ Adhere, Fig. 37B) and traditional ion-exchange (QSFF, Fig. 37A) resin.

FIGs. 38A and 38B shows high-throughput screening results for product binding capacity in sodium acetate on hydrophobic interaction chromatography resins.

FIG. 39 shows high-throughput screening results for FkpA levels in flow-through on hydrophobic interaction chromatography resins in the presence of sodium acetate. Complete testing was not performed for WP HI-Propyl, Toyopearl® PPG-600M, Toyopearl® Phenyl-650M, Toyopearl® Ether-650M due to suboptimal clearance.

FIGs. 40A, 40B, 40C and 40D show flow diagrams of bispecific antibody production processes 1, 2, 3, and 4 respectively.

DETAILED DESCRIPTION OF THE INVENTION

Herein disclosed are methods to generate ultrapure compositions of FkpA. Such compositions are used to generate antibodies that are highly specific and sensitive to FkpA. The antibodies in turn are useful for the detection of FkpA in recombinant polypeptides prepared in bacterial fermentation cultures where FkpA is expressed to facilitate recombinant polypeptide folding and assembly. For example, the antibodies may be useful in release assays in the development of pharmaceutical formulation of the recombinant polypeptides such as antibodies including multispecific antibodies.

In some aspects, the disclosure provides compositions of recombinant polypeptides produced in host cells that express exogenous FkpA wherein the composition comprises no more than about 10 ppm of FkpA , about 9 ppm, about 8 ppm, about 7 ppm, about 6 ppm, about 5 ppm, about 4 ppm, about 3 ppm, about 2 ppm, or about 1 ppm. In some embodiments, the recombinant polypeptide is a multispecific antibody such as a bispecific antibody that binds IL13 and IL17.

In some aspects, the disclosure provides methods for purifying a multispecific antibodies comprising the sequential steps of subjecting a composition comprising the multispecific antibody to a) protein A chromatography, b) mixed-mode chromatography and c) hydrophobic interaction chromatography. In some aspects, the invention provides methods for purifying a multispecific antibodies wherein individual arms of the multispecific antibody are produced in separate cultures and purified by protein A chromatography. The purified antibody arms are then assembled to produce the multispecific antibody. The assembled multispecific antibody is then subjected to mixed-mode anion exchange chromatography followed by hydrophobic interaction chromatography.

In some aspects, the disclosure provides comprising multispecific antibodies that are essentially free of product specific impurities such as unpaired antibody arms, homodimers, aggregates, low molecular weight species, acidic and basic variants. In some embodiments, the composition is essentially free of process specific impurities such as *E. coli* protein and DNA and chaperones including FkpA, DsbA and DsbC.

I. Definitions

The term "detecting" is used herein in the broadest sense to include both qualitative and quantitative measurements of a target molecule. Detecting includes identifying the mere presence of the target molecule in a sample as well as determining whether the target molecule is present in the sample at detectable levels.

The terms "polypeptide" and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc.*), as well as other modifications known in the art. The terms "polypeptide" and "protein" as used herein specifically encompass antibodies.

"Purified" polypeptide (*e.g.*, antibody or immunoadhesin, *etc.*) means that the polypeptide has been increased in purity, such that it exists in a form that is more pure than it exists in its natural environment and/or when initially synthesized and/or amplified under laboratory conditions. Purity is a relative term and does not necessarily mean absolute purity.

As used herein, "ultrapure FkpA" refers to FkpA compositions in which at least about 95% of the protein in the composition is the desired protein, FkpA. In some examples, ultrapure FkpA comprises at least about 96%, 97%, 98%, 99%, or 99.5% FkpA. In some embodiments, FkpA purity is determined by SEC.

When used in reference to a polypeptide, "FkpA" proteins refer to bacterial FKBP-type peptidyl-prolyl cis-trans isomerase. FkpA is a periplasmic protein disulfide isomerase I exhibits both cis/trans peptidyl-prolyl isomerase (PPIase) and chaperone activities.

As used herein, "FkpA" may also be known as FKBP-type peptidyl-prolyl cis-trans isomerase (FkpA)periplasmic protein disulfide isomerase I. An exemplary FkpA protein is *E. coli* FkpA. The amino acid sequence of *E. coli* FkpA is provided SEQ ID NO: 1 or SEQ ID NO:2. The nucleic acid sequence of *E. coli* FkbA gene is provided by (SEQ ID NO:3). In some embodiments, *E. coli* FkpA refers a protein encoded by an fkpA gene described by EcoGene Accession Number EG12900. In some embodiments, *E. coli* FkpA refers a protein having the sequence described by the NCBI RefSeq Accession Number NP_417806. Other FkpA proteins are known in the art. Examples of FkpA proteins may include, without limitation, S. boydii peptidyl-prolyl isomerase (NCBI RefSeq No. WP_000838252), C. youngae peptidyl-prolyl isomerase (NCBI RefSeq No. WP_006687366), K. oxytoca peptidyl-prolyl isomerase (NCBI RefSeq No. WP_004125943), S. enterica peptidyl-prolyl isomerase (NCBI RefSeq No. WP_000838233), K. pneumoniae peptidyl-prolyl isomerase (NCBI RefSeq No. WP_019704642), S. cerevisiae FPR3p (NCBI RefSeq No. NP_013637), M. musculus Fkpb1a (NCBI RefSeq No. NP_032045), M. musculus Fkpb2 (NCBI RefSeq No. NP_032046), H. sapiens FKBP2 (NCBI RefSeq No. NP_001128680), and D. melanogaster CG14715 (NCBI RefSeq No. NP_650101). In some embodiments, an FkpA protein of the present disclosure has at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, or at least about 99% identity to *E. coli* FkpA. In certain embodiments, the *E. coli* FkpA comprises the amino acid sequences of SEQ ID NO: 1.

When used in reference to a polypeptide, "Dsb" proteins refer to bacterial disulfide oxidoreductases. Bacterial disulfide oxireductases are members of the disulfide bond family of enzymes. Members of the Dsb family include DsbA, DsbB, DsbC, DsbD and DsbG. DsbA forms intrachain disulfide bonds as peptides emerge into the cell's periplasm and DsbC serves as a disulfide bond isomerase during oxidative protein-folding in cell's periplasm.

As used herein, "DsbA" may also be known as periplasmic protein disulfide isomerase I. An exemplary DsbA protein is *E. coli* DsbA. The amino acid sequence of *E. coli* DsbA is provided by NCBI Accession No. NP_418297 and the nucleic acid sequence of *E. coli* dsbA gene is provided by NCBI Accession No. NC_000913.3 or EcoGene:EG11297.

As used herein, "DsbC" may also be known as periplasmic protein disulfide isomerase II. An exemplary DsbC protein is *E. coli* DsbC. The amino acid sequence of *E. coli* DsbC is provided by NCBI Accession No. NP_417369.1 and the nucleic acid sequence of *E. coli* dsbC gene is provided by NCBI Accession No. NC_000913.3 or EcoGene:EG11070.

A "sample" refers to a small portion of a larger quantity of material. Generally, testing according to the methods described herein is performed on a sample. The sample is typically obtained from a recombinant polypeptide preparation obtained, for example, from cultured recombinant polypeptide-expressing cell lines, also referred to herein as "product cell lines," or from cultured host cells. As used herein, "host cells" do not contain genes for the expression of recombinant polypeptides of interest or products. A sample may be obtained from, for example but not limited to, harvested cell culture fluid, from an in-process pool at a certain step in a purification process, or from the final purified product.

A "capture antibody" refers to an antibody that specifically binds a target molecule in a sample. Under certain conditions, the capture antibody forms a complex with the target molecule such that the antibody-target molecule complex can be separated from the rest of the sample. In certain embodiments, such separation may include washing away substances or material in the sample that did not bind the capture antibody. In certain embodiments, a capture antibody may be attached to a solid support surface, such as, for example but not limited to, a plate or a bead.

A "detection antibody" refers to an antibody that specifically binds a target molecule in a sample or in a sample-capture antibody combination material. Under certain conditions, the detection antibody forms a complex with the target molecule or with a target molecule-capture antibody complex. A detection antibody is capable of being detected either directly through a label, which may be amplified, or indirectly, *e.g.*, through use of another antibody that is labeled and that binds the detection antibody. For direct labeling, the detection antibody is typically conjugated to a moiety that is detectable by some means, for example, including but not limited to, biotin or ruthenium.

The terms "label" or "detectable label" refers to any chemical group or moiety that can be linked to a substance that is to be detected or quantitated, *e.g.*, an antibody. Typically, a label is a detectable label that is suitable for the sensitive detection or quantification of a substance. Examples of detectable labels include, but are not limited to, luminescent labels, *e.g.*, fluorescent, phosphorescent, chemiluminescent, bioluminescent and electrochemiluminescent labels, radioactive labels, enzymes, particles, magnetic substances, electroactive species and the like. Alternatively, a detectable label may signal its presence by participating in specific binding reactions. Examples of such labels include haptens, antibodies, biotin, streptavidin, His-tag, nitrilotriacetic acid, glutathione S-transferase, glutathione and the like.

The term "detection means" refers to a moiety or technique used to detect the presence of the detectable antibody through signal reporting that is then read out in an assay. Typically, detection means employ reagents that amplify an immobilized label such as the label captured onto a microtiter plate, *e.g.*, avidin or streptavidin-HRP.

"Photoluminescence" refers to a process whereby a material luminesces subsequent to the absorption by that material of light (alternatively termed electromagnetic radiation). Fluorescence and phosphorescence are two different types of photoluminescence.

"Chemiluminescent" processes involve the creation of the luminescent species by a chemical reaction. "Electrochemiluminescence" or "ECL" is a process whereby a species, *e.g.*, an antibody, luminesces upon the exposure of that species to electrochemical energy in an appropriate surrounding chemical environment.

An antibody "which binds" an antigen of interest, *e.g.* a host cell protein, is one that binds the antigen with sufficient affinity such that the antibody is useful as an assay reagent, *e.g.*, as a capture antibody or as a detection antibody. Typically, such an antibody does not significantly cross-react with other polypeptides.

With regard to the binding of a polypeptide to a target molecule, the term "specific binding" or "specifically binds to" or is "specific for" a particular polypeptide or an epitope on a particular polypeptide target means binding that is measurably different from a non-specific interaction. Specific binding can be measured, for example, by determining binding of a target molecule compared to binding of a control molecule, which generally is a molecule of similar structure that does not have binding activity. In some embodiments the invention provides a preparation of polyclonal antibodies that specifically binds FkpA. For example, at least about 95%, about 96%, about 97%, about 98%, or about 99% of the antibodies in the polyclonal antibody preparation bind the desired polypeptide (*e.g.*, FkpA).

"Affinity" refers to the strength of the sum total of noncovalent interactions between a single binding site of a molecule (*e.g.*, an antibody) and its binding partner (*e.g.*, an antigen). Unless indicated otherwise, as used herein, "binding affinity" refers to intrinsic binding affinity which reflects a 1:1 interaction between members of a binding pair (*e.g.*, antibody and antigen). The affinity of a molecule X for its partner Y can generally be represented by the dissociation constant (Kd). Affinity can be measured by common methods known in the art, including those described herein.

"Active" or "activity" for the purposes herein refers to form(s) of a polypeptide which retain a biological and/or an immunological activity of native or naturally-occurring polypeptide, wherein "biological" activity refers to a biological function (either inhibitory or stimulatory) caused by a native or naturally-occurring polypeptide other than the ability to induce the production of an antibody against an antigenic epitope possessed by a native or naturally-occurring polypeptide and an "immunological" activity refers to the ability to induce the production of an antibody against an antigenic epitope possessed by a native or naturally-occurring polypeptide.

The term "antagonist" is used in the broadest sense, and includes any molecule that partially or fully blocks, inhibits, or neutralizes a biological activity of a native polypeptide. In a similar manner, the term "agonist" is used in the broadest sense and includes any molecule that mimics a biological activity of a native polypeptide. Suitable agonist or antagonist molecules specifically include agonist or antagonist antibodies or antibody fragments, fragments or amino acid sequence variants of native polypeptides, *etc.* Methods for identifying agonists or antagonists of a polypeptide may comprise contacting a polypeptide with a candidate agonist or antagonist molecule and measuring a detectable change in one or more biological activities normally associated with the polypeptide.

The term "antibody" herein is used in the broadest sense and specifically covers monoclonal antibodies, polyclonal antibodies, half-antibodies, multispecific antibodies (*e.g.* bispecific antibodies) formed from at least two intact antibodies, and antibody fragments so long as they exhibit the desired biological activity. The term "immunoglobulin" (Ig) is used interchangeable with antibody herein.

Antibodies are naturally occurring immunoglobulin molecules which have varying structures, all based upon the immunoglobulin fold. For example, IgG antibodies have two "heavy" chains and two "light" chains that are disulfide-bonded to form a functional antibody. As another example, one heavy chain and one light chain linked by one or more disulfide bonds forms a functional half-antibody. Each heavy and light chain itself comprises a "constant" (C) and a "variable" (V) region. The V regions determine the antigen binding specificity of the antibody, whilst the C regions provide structural support and function in non-antigen-specific interactions with immune effectors. The antigen binding specificity of an antibody or antigen-binding fragment of an antibody is the ability of an antibody to specifically bind to a particular antigen.

The antigen binding specificity of an antibody is determined by the structural characteristics of the V region. The variability is not evenly distributed across the 110-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (*see* Kabat et al., Sequences of Proteins of Immunological Interest, 5th Ed. Public Health Service, National Institutes of Health, Bethesda, Md. (1991)). The constant domains are not involved directly in binding an antibody to an antigen, but exhibit various effector functions, such as participation of the antibody in antibody dependent cellular cytotoxicity (ADCC).

Each V region typically comprises three complementarity determining regions ("CDRs", each of which contains a "hypervariable loop"), and four framework regions. An antibody binding site, the minimal structural unit required to bind with substantial affinity to a particular desired antigen, will therefore typically include the three CDRs, and at least three, preferably four, framework regions interspersed there between to hold and present the CDRs in the appropriate conformation. Classical four chain antibodies have antigen binding sites which are defined by $V_H$ and $V_L$ domains in cooperation. Certain antibodies, such as camel and shark antibodies, lack light chains and rely on binding sites formed by heavy chains only. Single domain engineered immunoglobulins can be prepared in which the binding sites are formed by heavy chains or light chains alone, in absence of cooperation between $V_H$ and $V_L$.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (*see* Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody that are responsible for antigen binding. The hypervariable region may comprise amino acid residues from a "complementarity determining region" or "CDR" (*e.g.*, around about residues 24-34 (L1), 50-56 (L2) and 89-97 (L3) in the $V_L$, and around about 31-35B (H1), 50-65 (H2) and 95-102 (H3) in the $V_H$ (Kabat et al., Sequences of Proteins of Immunological Interest, 5th Ed. Public Health Service, National Institutes of Health, Bethesda, Md. (1991)) and/or those residues from a "hypervariable loop" (*e.g.* residues 26-32 (L1), 50-52 (L2) and 91-96 (L3) in the $V_L$, and 26-32 (H1), 52A-55 (H2) and 96-101 (H3) in the $V_H$ (Chothia and Lesk J. Mol. Biol. 196:901-917 (1987)).

"Framework" or "FR" residues are those variable domain residues other than the hypervariable region residues as herein defined.

"Hinge region" in the context of an antibody or half-antibody is generally defined as stretching from Glu216 to Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions.

The "lower hinge region" of an Fc region is normally defined as the stretch of residues immediately C-terminal to the hinge region, *i.e.* residues 233 to 239 of the Fc region. Prior to the present invention, FcγR binding was generally attributed to amino acid residues in the lower hinge region of an IgG Fc region.

The "CH2 domain" of a human IgG Fc region usually extends from about residues 231 to about 340 of the IgG. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. (Burton, Molec. Immunol.22:161-206 (1985)).

The "CH3 domain" comprises the stretch of residues C-terminal to a CH2 domain in an Fc region (*i.e.* from about amino acid residue 341 to about amino acid residue 447 of an IgG).

"Antibody fragments" comprise a portion of an intact antibody, preferably comprising the antigen binding region thereof. Examples of antibody fragments include Fab, Fab', F(ab')$_2$, and Fv fragments; diabodies; tandem diabodies (taDb), linear antibodies(*e.g.*, U.S. Patent No. 5,641,870, Example 2; Zapata et al., Protein Eng. 8(10): 1057-1062 (1995)); one-armed antibodies, single variable domain antibodies, minibodies, single-chain antibody molecules; multispecific antibodies formed from antibody fragments (*e.g.*, including but not limited to, Db-Fc, taDb-Fc, taDb-CH3, (scFV)4-Fc, di-scFv, bi-scFv, or tandem (di,tri)-scFv); and Bi-specific T-cell engagers (BiTEs).

Papain digestion of antibodies produces two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (CH1). Pepsin treatment of an antibody yields a single large F(ab')2 fragment which roughly corresponds to two disulfide linked Fab fragments having divalent antigen-binding activity and is still capable of cross-linking antigen. Fab' fragments differ from Fab fragments by having additional few residues at the carboxy terminus of the CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

"Fv" is the minimum antibody fragment that contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the $V_H$-$V_L$ dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')$_2$ antibody fragments originally were produced as pairs of Fab' fragments that have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, antibodies can be assigned to different classes. There are five major classes of intact antibodies: IgA, IgD, IgE, IgG, and IgM, and several of these may be further divided into subclasses (isotypes), *e.g.*, IgG1, IgG2, IgG3, IgG4, IgA, and IgA2. The heavy chain constant domains that correspond to the different classes of antibodies are called $\alpha$, $\delta$, $\varepsilon$, $\gamma$, and $\mu$, respectively. The subunit structures and three-dimensional configurations of different classes of immunoglobulins are well known.

"Single-chain Fv" or "scFv" antibody fragments comprise the $V_H$ and $V_L$ domains of antibody, wherein these domains are present in a single polypeptide chain. In some embodiments, the Fv polypeptide further comprises a polypeptide linker between the $V_H$ and $V_L$ domains that enables the scFv to form the desired structure for antigen binding. For a review of scFv *see* Plückthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy chain variable domain ($V_H$) connected to a light chain variable domain ($V_L$) in the same polypeptide chain ($V_H$ - $V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

The term "half-antibody" or "hemimer" as used herein refers to a monovalent antigen binding polypeptide. In certain embodiments, a half antibody or hemimer comprises a VH/VL unit and optionally at least a portion of an immunoglobulin constant domain. In certain embodiments, a half antibody or hemimer comprises one immunoglobulin heavy chain associated with one immunoglobulin light chain, or an antigen binding fragment thereof. In certain embodiments, a half antibody or hemimer is mono-specific, i.e., binds to a single antigen or epitope. In certain such embodiments, a half antibody binds to IL-13 and does not bind to IL-17. In certain other embodiments, a half antibody binds to IL-17 and does not bind to IL-13. One skilled in the art will readily appreciate that a half-antibody may have an antigen binding domain consisting of a single variable domain, *e.g.*, originating from a camelidae.

The term "VH/VL unit" refers to the antigen-binding region of an antibody that comprises at least one VH HVR and at least one VL HVR. In certain embodiments, the VH/VL unit comprises at least one, at least two, or all three VH HVRs and at least one, at least two, or all three VL HVRs. In certain embodiments, the VH/VL unit further comprises at least a portion of a framework region (FR). In some embodiments, a VH/VL unit comprises three VH HVRs and three VL HVRs. In some such embodiments, a VH/VL unit comprises at least one, at least two, at least three or all four VH FRs and at least one, at least two, at least three or all four VL FRs.

The term "multispecific antibody" is used in the broadest sense and specifically covers an antibody comprising an antigen-binding domain that has polyepitopic specificity (i.e., is capable of specifically binding to two, or more, different epitopes on one biological molecule or is capable of specifically binding to epitopes on two, or more, different biological molecules). In some embodiments, an antigen-binding domain of a multispecific antibody (such as a bispecific antibody) comprises two VH/VL units, wherein a first VH/VL unit specifically binds to a first epitope and a second VH/VL unit specifically binds to a second epitope, wherein each VH/VL unit comprises a heavy chain variable domain (VH) and a light chain variable domain (VL). Such multispecific antibodies include, but are not limited to, full length antibodies, antibodies having two or more VL and VH domains, antibody fragments such as Fab, Fv, dsFv, scFv, diabodies, bispecific diabodies and triabodies, antibody fragments that have been linked covalently or non-covalently. A VH/VL unit that further comprises at least a portion of a heavy chain constant region and/or at least a portion of a light chain constant region may also be referred to as a "hemimer" or "half antibody." In some embodiments, a half antibody comprises at least a portion of a single heavy chain variable region and at least a portion of a single light chain variable region. In some such embodiments, a bispecific antibody that comprises two half antibodies and binds to two antigens comprises a first half antibody that binds to the first antigen or first epitope but not to the second antigen or second epitope and a second half antibody that binds to the second antigen or second epitope and not to the first antigen or first epitope. According to some embodiments, the multispecific antibody is an IgG antibody that binds to each antigen or epitope with an affinity of 5 M to 0.001 pM, 3 M to 0.001 pM, 1 M to 0.001 pM, 0.5 M to 0.001 pM, or 0.1 M to 0.001 pM. In some embodiments, a hemimer comprises a sufficient portion of a heavy chain variable region to allow intramolecular disulfide bonds to be formed with a second hemimer. In some embodiments, a hemimer comprises a knob mutation or a hole mutation, for example, to allow heterodimerization with a second hemimer or half antibody that comprises a complementary hole mutation or knob mutation. Knob mutations and hole mutations are discussed further below.

A "bispecific antibody" is a multispecific antibody comprising an antigen-binding domain that is capable of specifically binding to two different epitopes on one biological molecule or is capable of specifically binding to epitopes on two different biological molecules. A bispecific antibody may also be referred to herein as having "dual specificity" or as being "dual specific." Unless otherwise indicated, the order in which the antigens bound by a bispecific antibody are listed in a bispecific antibody name is arbitrary. That is, in some embodiments, the terms "anti-IL-13/IL-17 bispecific antibody" and "anti-IL-17/IL-13 bispecific antibody" may be used interchangeably. In some embodiments, a bispecific antibody comprises two half antibodies, wherein each half antibody comprises a single heavy chain variable region and optionally at least a portion of a heavy chain constant region, and a single light chain variable region and optionally at least a portion of a light chain constant region. In certain embodiments, a bispecific antibody comprises two half antibodies, wherein each half antibody comprises a single heavy chain variable region and a single light chain variable region and does not comprise more than one single heavy chain variable region and does not comprise more than one single light chain variable region. In some embodiments, a bispecific antibody comprises two half antibodies, wherein each half antibody comprises a single heavy chain variable region and a single light chain variable region, and wherein the first half antibody binds to a first antigen and not to a second antigen and the second half antibody binds to the second antigen and not to the first antigen.

The term "knob-into-hole" or "KnH" technology as used herein refers to the technology directing the pairing of two polypeptides together in vitro or in vivo by introducing a protuberance (knob) into one polypeptide and a cavity (hole) into the other polypeptide at an interface in which they interact. For example, KnHs have been introduced in the Fc:Fc binding interfaces, CL:CH1 interfaces or VH/VL interfaces of antibodies (see, *e.g.,* US 2011/0287009, US2007/0178552, WO 96/027011, WO 98/050431, and Zhu et al., 1997, Protein Science 6:781-788). In some embodiments, KnHs drive the pairing of two different heavy chains together during the manufacture of multispecific antibodies. For example, multispecific antibodies having KnH in their Fc regions can further comprise single variable domains linked to each Fc region, or further comprise different heavy chain variable domains that pair with similar or different light chain variable domains. KnH technology can also be used to pair two different receptor extracellular domains together or any other polypeptide sequences that comprises different target recognition sequences (*e.g.*, including affibodies, peptibodies and other Fc fusions).

The term "knob mutation" as used herein refers to a mutation that introduces a protuberance (knob) into a polypeptide at an interface in which the polypeptide interacts with another polypeptide. In some embodiments, the other polypeptide has a hole mutation (see *e.g.*, US 5,731,168, US 5,807,706, US 5,821,333, US 7,695,936, US 8,216,805, WO2011/133886, WO2013055958).

The term "hole mutation" as used herein refers to a mutation that introduces a cavity (hole) into a polypeptide at an interface in which the polypeptide interacts with another polypeptide. In some embodiments, the other polypeptide has a knob mutation (see *e.g.*, US 5,731,168, US 5,807,706, US 5,821,333, US 7,695,936, US 8,216,805).

The expression "single domain antibodies" (sdAbs) or "single variable domain (SVD) antibodies" generally refers to antibodies in which a single variable domain (VH or VL) can confer antigen binding. In other words, the single variable domain does not need to interact with another variable domain in order to recognize the target antigen. Examples of single domain antibodies include those derived from camelids (lamas and camels) and cartilaginous fish (e.g., nurse sharks) and those derived from recombinant methods from humans and mouse antibodies (Nature (1989) 341:544-546; Dev Comp Immunol (2006) 30:43-56; Trend Biochem Sci (2001) 26:230-235; Trends Biotechnol (2003):21:484-490; WO 2005/035572; WO 03/035694; FEBS Lett (1994) 339:285-290; WO00/29004; WO 02/051870).

The term "monoclonal antibody" as used herein refers to an antibody obtained from a population of substantially homogeneous antibodies, *i.e.*, the individual antibodies comprising the population are identical and/or bind the same epitope, except for possible variants that may arise during production of the monoclonal antibody, such variants generally being present in minor amounts. In contrast to polyclonal antibody preparations that typically include different antibodies directed against different determinants (epitopes), each monoclonal antibody is directed against a single determinant on the antigen. In addition to their specificity, the monoclonal antibodies are advantageous in that they are uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the methods provided herein may be made by the hybridoma method first described by Kohler et al., Nature 256:495 (1975), or may be made by recombinant DNA methods (*see*, *e.g.*, U.S. Patent No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA 81:6851-6855 (1984)). Chimeric antibodies of interest herein include "primatized" antibodies comprising variable domain antigen-binding sequences derived from a non-human primate (*e.g.* Old World Monkey, such as baboon, rhesus or cynomolgus monkey) and human constant region sequences (US Pat No. 5,693,780).

"Humanized" forms of non-human (*e.g.*, murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence, except for FR substitution(s) as noted above. The humanized antibody optionally also will comprise at least a portion of an immunoglobulin constant region, typically that of a human immunoglobulin. For further details, *see* Jones et al., Nature 321:522-525 (1986); Riechmann et al., Nature 332:323-329 (1988); and Presta, Curr. Op. Struct. Biol. 2:593-596 (1992).

For the purposes herein, an "intact antibody" is one comprising heavy and light variable domains as well as an Fc region. The constant domains may be native sequence constant domains (*e.g.* human native sequence constant domains) or amino acid sequence variant thereof. Preferably, the intact antibody has one or more effector functions.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

A "naked antibody" is an antibody (as herein defined) that is not conjugated to a heterologous molecule, such as a cytotoxic moiety or radiolabel.

As used herein, the term "immunoadhesin" designates molecules which combine the binding specificity of a heterologous protein (an "adhesin") with the effector functions of immunoglobulin constant domains. Structurally, the immunoadhesins comprise a fusion of an amino acid sequence with a desired binding specificity, which amino acid sequence is other than the antigen recognition and binding site of an antibody (*i.e.*, is "heterologous" compared to a constant region of an antibody), and an immunoglobulin constant domain sequence (*e.g.*, CH2 and/or CH3 sequence of an IgG). Exemplary adhesin sequences include contiguous amino acid sequences that comprise a portion of a receptor or a ligand that binds to a protein of interest. Adhesin sequences can also be sequences that bind a protein of interest, but are not receptor or ligand sequences (*e.g.*, adhesin sequences in peptibodies). Such polypeptide sequences can be selected or identified by various methods, include phage display techniques and high throughput sorting methods. The immunoglobulin constant domain sequence in the immunoadhesin can be obtained from any immunoglobulin, such as IgG-1, IgG-2, IgG-3, or IgG-4 subtypes, IgA (including IgA-1 and IgA-2), IgE, IgD, or IgM.

In some embodiments, antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity; Fc receptor binding; antibody-dependent cell-mediated cytotoxicity (ADCC); phagocytosis; down regulation of cell surface receptors.

"Complement dependent cytotoxicity" or "CDC" refers to the ability of a molecule to lyse a target in the presence of complement. The complement activation pathway is initiated by the binding of the first component of the complement system (C1q) to a molecule (*e.g.* polypeptide (*e.g.*, an antibody)) complexed with a cognate antigen. To assess complement activation, a CDC assay, *e.g.* as described in Gazzano-Santoro et al., J. Immunol. Methods 202:163 (1996), may be performed.

"Antibody-dependent cell-mediated cytotoxicity" and "ADCC" refer to a cell-mediated reaction in which non-specific cytotoxic cells that express Fc receptors (FcRs) (*e.g.* Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells in summarized is Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo*, *e.g.*, in an animal model such as that disclosed in Clynes et al., Proc. Natl. Acad. Sci. (USA) 95:652-656 (1998).

"Human effector cells" are leukocytes that express one or more FcRs and perform effector functions. In some embodiments, the cells express at least FcγRIII and carry out ADCC effector function. Examples of human leukocytes that mediate ADCC include peripheral blood mononuclear cells (PBMC), natural killer (NK) cells, monocytes, cytotoxic T cells and neutrophils; with PBMCs and NK cells being preferred.

The terms "Fc receptor" or "FcR" are used to describe a receptor that binds to the Fc region of an antibody. In some embodiments, the FcR is a native sequence human FcR. Moreover, a preferred FcR is one that binds an IgG antibody (a gamma receptor) and includes receptors of the FcγRI, FcγRII, and Fcγ RIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

The term "impurities" as used herein refers to materials or substances that are different from the desired polypeptide product. In certain embodiments, the polypeptide product comprises an antibody, including a bispecific antibody. The impurities include, without limitation: host cell materials, such as E. coli host cell protein (ECP); leached Protein A; nucleic acid; a variant, fragment, aggregate or derivative of the desired polypeptide; another polypeptide; endotoxin; virus; cell culture media component, etc. In some examples, the impurity may be a host cell protein (HCP) from, for example but not limited to, a bacterial cell such as an E. coli cell (e.g., ECP), a eukaryotic cell, a yeast cell, a mammalian cell, an avian cell, a fungal cell. In some embodiments, the impurity may be clipped FkpA and/or aggregates of FkpA. In some embodiments, the impurity may refer to accessory proteins used to facilitate expression, folding or assembly of multispecific antibodies; for example, chaperones such as FkpA, DsbA and DsbC. In some embodiments, the impurity may refer to product-specific polypeptides such as one-armed antibodies and misassembled antibodies, antibody variants including basic variants and acidic variants, and aggregates.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. In certain embodiments, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, *e.g.*, Kindt et al., Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, *e.g.,* Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The term "sequential" as used herein with regard to chromatography refers to having a first chromatography step followed by a second chromatography steps. In some embodiments, the term "sequential" is used in the context where a first chromatography step is followed by more than one additional step. In some examples, term "sequential" as used herein with regard to chromatography refers to chromatography steps in a specific sequence; *e.g.*, a first chromatography step followed by a second chromatography step followed by a third chromatography step, *etc.* Additional steps, including, but not limited to non-chromatography steps (*e.g.*, pH and/or conductivity adjustment, filtration, concentration) may be included between the first chromatography step and the additional chromatography steps.

The term "continuous" as used herein with regard to chromatography refers to having a first chromatography material and a second chromatography material either directly connected or some other mechanism which allows for continuous flow between the two chromatography materials.

"Loading density" refers to the mass amount, *e.g.* grams, of composition put in contact with a volume of chromatography material, *e.g.* liters. In some examples, loading density is expressed in g/L.

Reference to "about" a value or parameter herein includes (and describes) variations that are directed to that value or parameter per se. For example, description referring to "about X" includes description of "X".

As used herein and in the appended claims, the singular forms "a," "or," and "the" include plural referents unless the context clearly dictates otherwise. It is understood that aspects and variations of the invention described herein include "consisting" and/or "consisting essentially of" aspects and variations.

II. Methods of Purification of FkpA

Disclosed herein are methods for generating ultrapure preparations of FkpA. In some embodiments, the preparations comprise more than about any of 95.0%, 96.0%, 97.0%, 98.0%, 99.0%, 99.1%, 99.2%, 99.3%, 99.4%, 99.5%, 99.6%, 99.7%, 99.8%, 99.9% pure FkpA. In some aspects of the disclosure, a preparation that is 99% pure FkpA indicates that no more than 1% of the material in the preparation are substances present in the cells or cell lysates (*e.g.*, protein, nucleic acids, lipids, *etc.*) present during the production of the FkpA. A 99% pure preparation of FkpA may contain buffers, salts or other excipients used in the purification or formulation of FkpA.

In some aspects, the disclosure provides methods for generating ultrapure preparations of FkpA. In some aspects of the disclosure, the FkpA is produced by overexpressing FkpA in a bacterial fermentation culture such as an *E. coli* culture. In some embodiments, the bacterial fermentation culture such as an *E. coli* culture expresses in addition to endogenous FkpA, exogenous FkpA. In some aspects of the disclosure, the FkpA is produced by expressing an exogenous FkpA in a bacterial fermentation culture such as an *E. coli* culture. Accordingly, in certain aspects of the disclosure, the method further comprises the step of culturing an *E. coli* culture under conditions that allow expression of exogenous FkpA. Following fermentation, bacterial cells are collected and centrifuged. The resultant cell paste is resuspended in a lysis buffer (e.g., 25 mM MES pH 6.0) and lysed (e.g., by using a microfluidizer). In some aspects of the disclosure, the cell lysate is conditioned with polyethyleneimine (PEI). In some aspects of the disclosure, the cell lysate is conditioned with PEI at a final concentration of more than about any of 0.01%, 0.05%, 0.1%, 0.2%, 0.3%, 0.4%, 0.5%, 0.6%, 0.7%, 0.8%, 0.9%, or 1.0%. In some aspects of the disclosure, the cell lysate is conditioned with PEI for more than about any of 15 min., 30 min., 45 min., 1 hr., 2 hr., 3 hr., 4 hr., 5 hr., 6 hr., 8 hr., 12 hr., 16 hr., 20 hr., or 24 hr. In some aspects of the disclosure, the cell lysate is conditioned with PEI at more than about any of 0 °C, 4 °C, or 21 °C. In some aspects of the disclosure, the cell lysate is conditioned with PEI at ambient temperature (~21 °C). In some aspects of the disclosure, the PEI-conditioned lysate is centrifuged to remove particulates. In some embodaspects of the disclosureiments, the PEI-conditioned lysate is filtered prior to chromatography. In some aspects of the disclosure, the PEI-conditioned lysate is filtered through a 22 $\mu$m filter prior to chromatography.

In some aspects of the disclosure, the method comprises methods for purifying a FkpA polypeptide from a cell lysate comprising the FkpA polypeptide and impurities, the method comprising b) clarifying the cell lysate by centrifugation, c) applying the clarified cell lysate comprising the FkpA polypeptide to an cation exchange chromatography material, d) eluting the FkpA polypeptide from the cation exchange chromatography material to generate a cation exchange eluate comprising the FkpA polypeptide, e) applying the cation exchange eluate comprising the FkpA polypeptide to a hydrophobic interaction chromatography (HIC) material, f) eluting the FkpA polypeptide from the HIC material to generate a HIC eluate, g) applying the HIC eluate comprising the FkpA polypeptide to a size exclusion chromatography, h) collecting fractions from the size exclusion chromatography comprising the purified FkpA polypeptide.

In some embodiments of any of the methods described herein, the cation exchange chromatography material is a solid phase that contains a negatively charged group (a "ligand") and has free cations for exchange with cations in an aqueous solution passed over or through the solid phase. In some embodiments of any of the methods described herein, the cation exchange material may be a membrane, a monolith, or resin. In an embodiment, the cation exchange material may be a resin. In some embodiments of the above, the cation exchange chromatography material is a cation exchange chromatography column. In some embodiments of the above, the cation exchange chromatography material is a cation exchange chromatography membrane.

In some embodiments of any of the methods described herein, the cation exchange material may utilize a conventional chromatography material or a convective chromatography material. The conventional chromatography materials include, for example, perfusive materials (*e.g.*, poly (styrene-divinylbenzene) resin) and diffusive materials (*e.g.*, cross-linked agarose resin). In some embodiments, the poly (styrene-divinylbenzene) resin can be POROS® resin. In some embodiments, the cross-linked agarose resin may be sulfopropyl-Sepharose® Fast Flow ("SPSFF") resin. The convective chromatography material may be a membrane (*e.g.*, polyethersulfone) or monolith material (*e.g.* cross-linked polymer). The polyethersulfone membrane may be Mustang. The cross-linked polymer monolith material may be cross-linked poly(glycidyl methacrylate-co-ethylene dimethacrylate).

In some embodiments of any of the methods, the cation exchange material comprises a carboxylic acid functional group or a sulfonic acid functional group. In some embodiments, the functional group is sulfopropyl, sulfoethyl, sulfoisobutyl, or caboxyl. In some embodiments, the cation exchange material is a membrane, a monolith, or resin particles. In some embodiments, the cation exchange material is a resin. In some embodiments, the cation exchange material is Mustang S, Sartobind S, SO3 Monolith, S Ceramic HyperD, POROS® HS50, POROS® HS20, sulfopropyl-Sepharose® Fast Flow (SPSFF), SP-Sepharose® XL (SPXL), CM Sepharose® Fast Flow, Capto™ S, Fractogel Se HiCap, Fractogel SO3, or Fractogel COO. In some embodiments, the cation exchange material is POROS® HS50. Examples of anion exchange materials are known in the art and include, but are not limited to POROS® HQ 50, POROS® PI 50, POROS® D, Mustang Q, Q Sepharose® FF (QSFF), and DEAE Sepharose® or equivalents thereof. In some embodiments, the anion exchange material is a weak anion exchange material; *e.g.* DEAE. In other embodiments, the anion exchange material is a strong anion exchange material; *e.g.*, QSFF. In some embodiments, the anion exchange material used for the purification of FkpA is a weak anion exchange material. In some embodiments the weak anion exchange material comprises a quarternary amine. In some embodiments, the quarternary amine is linked to crosslinked agarose. In some embodiments, the anion exchange material used for the purification of FkpA is DEAE Sepharose®.

In some embodiments of any of the methods described herein, the hydrophobic interaction chromatography (HIC) is a liquid chromatography technique that separates biomolecules according to hydrophobicity. Examples of HIC materials include, but are not limited to, Toyopearl® hexyl 650, Toyopearl® butyl 650, Toyopearl® phenyl 650, Toyopearl® ether 650, Source, Resource, Sepharose® Hi-Trap, butyl sepharose®, Octyl sepharose®, phenyl sepharose®. In some embodiments of the above, the HIC material is a HIC column. In some embodiments of the above, the HIC material is a HIC membrane. In some embodiments the HIC material comprises a butyl moiety. In some embodiments, the butyl moiety is linked to crosslinked agarose. In some embodiments, the HIC material used for the purification of FkpA is butyl Sepharose®.

In some embodiments of any of the methods described herein, the size exclusion chromatography is a liquid chromatography technique that separates biomolecules according to their size and shape. Size exclusion chromatography materials come in different pore size for efficient separation of molecules of particular weight ranges. Examples of size exclusion chromatography materials include, but are not limited to, dextran, porous agarose particles, crosslinked agarose, crosslinked agarose and dextran, and crosslinked acrylamide. Examples of size exclusion chromatography materials include, but are not limited to Sephadex, Superdex, Sephacryl, TSKgel, and Bio-Gel. In some embodiments, Superdex 75 size exclusion chromatography is used in the purification of FkpA.

Loading of the composition comprising FkpA on the chromatography material may be optimized for separation of the FkpA product from impurities. In some embodiments, loading on the composition onto the chromatography material is optimized for binding of the impurities to the chromatography material. For example, the composition may be loaded onto the chromatography material, *e.g.* a chromatography column, in a load buffer at a number of different pH values while the conductivity of the load buffer is constant. Alternatively, the composition may be loaded onto the chromatography material in a load buffer at a number of different conductivities while the pH of the load buffer is constant. Upon completion of loading the composition on the chromatography material and later elution of the product from the chromatography material into a pool fraction, the amount of impurities in the pool fraction provides information regarding the separation of the product from the impurities for a given pH or conductivity.

In some embodiments of any of the methods described herein, the composition comprising FkpA is loaded onto a cation exchange chromatography material at a loading density of the polypeptide of less than or equal to about any of 10 mg/mL, 9 mg/mL, 8 mg/mL, 7 mg/mL, 6 mg/mL, 5 mg/mL, 4 mg/mL, 3 mg/mL, 2 mg/mL, or 1 mg/mL of the cation exchange chromatography material. The composition may be loaded onto an cation chromatography material at a loading density of the polypeptide of between about any of 1 mg/mL and 2 mg/mL, 2 mg/mL and 3 mg/mL, 3 mg/mL and 4 mg/mL, 4 mg/mL and 5 mg/mL, 5 mg/mL and 6 mg/mL, 6 mg/mL and 7 mg/mL, 7 mg/mL and 8 mg/mL, 8 mg/mL and 9 mg/mL, or 9 mg/mL and 10 mg/mL of cation exchange chromatography material. In some embodiments, the cation exchange chromatography material a sulfopropyl group crosslinked to agarose; e.g., SP Sepharose®.

In some embodiments of any of the methods described herein, the composition comprising FkpA is loaded onto a HIC material at a loading density of the polypeptide of less than or equal to about any of 10 mg/mL, 9 mg/mL, 8 mg/mL, 7 mg/mL, 6 mg/mL, 5 mg/mL, 4 mg/mL, 3 mg/mL, 2 mg/mL, or 1 mg/mL of HIC material. The composition may be loaded onto HIC material at a loading density of the polypeptide of between about any of 1 mg/mL and 2 mg/mL, 2 mg/mL and 3 mg/mL, 3 mg/mL and 4 mg/mL, 4 mg/mL and 5 mg/mL, 5 mg/mL and 6 mg/mL, 6 mg/mL and 7 mg/mL, 7 mg/mL and 8 mg/mL, 8 mg/mL and 9 mg/mL, or 9 mg/mL and 10 mg/mL of HIC material. In some embodiments, the HIC material is a butyl moiety crosslinked to crosslinked agarose; e.g., Butyl Sepharose®.

Elution of the FkpA from the chromatography material may be optimized for yield of FkpA with minimal impurities and at minimal pool volume. For example, the composition may be loaded onto the chromatography material, e.g. a chromatography column, in a load buffer. Upon completion of load, the chromatography material is initially washed and then eluted with buffers at a number of different pH while the conductivity of the elution buffer is constant. Alternatively, the product may be eluted from the chromatography material in an elution buffer at a number of different conductivities while the pH of the elution buffer is constant. Upon completion of elution of the product from the chromatography material, the amount of impurities in the pool fraction provides information regarding the separation of the product from the impurities for a given pH or conductivity. Elution of the product in a high number of fractions (e.g. eight column volumes) indicates "tailing" of the elution profile. In some embodiments of the invention, tailing of the elution is minimized.

Various buffers which can be employed depending, for example, on the desired pH of the buffer, the desired conductivity of the buffer, the characteristics of the protein of interest, and the purification method. In some embodiments of any of the methods described herein, the methods comprise using a buffer. The buffer can be a loading buffer, an equilibration buffer, or a wash buffer. In some embodiments, one or more of the loading buffer, the equilibration buffer, and/or the wash buffer are the same. In some embodiments, the loading buffer, the equilibration buffer, and/or the wash buffer are different. In some embodiments of any of the methods described herein, the buffer comprises a salt. The loading buffer may comprise sodium chloride, sodium acetate, or a mixture thereof. In some embodiments, the loading buffer is a sodium chloride buffer. In some embodiments, the loading buffer is a sodium acetate buffer.

Load, as used herein, is the composition loaded onto a chromatography material. Loading buffer is the buffer used to load the composition comprising FkpA onto a chromatography material. The chromatography material may be equilibrated with an equilibration buffer prior to loading the composition which is to be purified. In some examples, the wash buffer is used after loading the composition onto a chromatography material and before elution of the polypeptide of interest from the solid phase.

Elution, as used herein, is the removal of the product, e.g. FkpA, from the chromatography material. Elution buffer is the buffer used to elute the polypeptide or other product of interest from a chromatography material. In many cases, an elution buffer has a different physical characteristic than the load buffer. For example, the elution buffer may have a different conductivity than load buffer or a different pH than the load buffer. In some embodiments, the elution buffer has a lower conductivity than the load buffer. In some embodiments, the elution buffer has a higher conductivity than the load buffer. In some embodiments, the elution buffer has a lower pH than the load buffer. In some embodiments, the elution buffer has a higher pH than the load buffer. In some embodiments the elution buffer has a different conductivity and a different pH than the load buffer. The elution buffer can have any combination of higher or lower conductivity and higher or lower pH.

Conductivity refers to the ability of an aqueous solution to conduct an electric current between two electrodes. In solution, the current flows by ion transport. Therefore, with an increasing amount of ions present in the aqueous solution, the solution will have a higher conductivity. The basic unit of measure for conductivity is the Siemen (or mho), mho (mS/cm), and can be measured using a conductivity meter, such as various models of Orion conductivity meters. Since electrolytic conductivity is the capacity of ions in a solution to carry electrical current, the conductivity of a solution may be altered by changing the concentration of ions therein. For example, the concentration of a buffering agent and/or the concentration of a salt (e.g. sodium chloride, sodium acetate, or potassium chloride) in the solution may be altered in order to achieve the desired conductivity. Preferably, the salt concentration of the various buffers is modified to achieve the desired conductivity.

In some embodiments of any of the methods described herein, the cation exchange load buffer has a conductivity of greater than about any of 4.0 mS/cm, 4.5 mS/cm, 5.0 mS/cm, 5.5 mS/cm, 6.0 mS/cm, 6.5 mS/cm, 7.0 mS/cm, 7.5 mS/cm, 8.0 mS/cm, 8.5 mS/cm, 9.0 mS/cm, 9.5 mS/cm, or 10 mS/cm. The conductivity may be between about any of 4 mS/cm and 17 mS/cm, 4 mS/cm and 10 mS/cm, 4 mS/cm and 7 mS/cm, 5 mS/cm and 17 mS/cm, 5 mS/cm and 10 mS/cm, or 5 mS/cm and 7 mS/cm. In some embodiments, the conductivity is about any of 4 mS/cm, 4.5 mS/cm, 5.0 mS/cm, 5.5 mS/cm, 6.0 mS/cm, 6.5 mS/cm, 7.0 mS/cm, 7.5 mS/cm, 8.0 mS/cm, 8.5 mS/cm, 9.0 mS/cm, 9.5 mS/cm, or 10 mS/cm. In one aspect, the conductivity is the conductivity of the loading buffer, the equilibration buffer, and/or the wash buffer. In some embodiments, the conductivity of one or more of the loading buffer, the equilibration buffer, and the wash buffer are the same. In some embodiments, the conductivity of the loading buffer is different from the conductivity of the wash buffer and/or equilibration buffer.

In some embodiments, the cation exchange elution buffer has a conductivity greater than the conductivity of the load buffer. In some embodiments of any of the methods described herein, the elution buffer has a conductivity of greater than about any of 5 mS/cm, 10 mS/cm, 15 mS/cm, 20 mS/cm, 25 mS/cm, 30 mS/cm, 35 mS/cm, 40 mS/cm, 45 mS/cm, 50 mS/cm, 55 mS/cm, 60 mS/cm, 65 mS/cm, 70 mS/cm, 75 mS/cm, 80 mS/cm, 85 mS/cm, 90 mS/cm, 95 mS/cm, or 100 mS/cm. In some embodiments, the conductivity of the elution buffer is altered by altering the salt concentration of the elution buffer.

In some embodiments, the HIC loading buffer has a conductivity greater than the conductivity of the elution buffer. In some embodiments of any of the methods described herein, the HIC loading buffer has a conductivity of greater than about any of 5 mS/cm, 10 mS/cm, 15 mS/cm, 20 mS/cm, 25 mS/cm, 30 mS/cm, 35 mS/cm, 40 mS/cm, 45 mS/cm, 50 mS/cm, 55 mS/cm, 60 mS/cm, 65 mS/cm, 70 mS/cm, 75 mS/cm, 80 mS/cm, 85 mS/cm, 90 mS/cm, 95 mS/cm, or 100 mS/cm. In some embodiments, the conductivity of the elution buffer is altered by altering the salt concentration of the elution buffer.

In some embodiments of any of the methods described herein, the HIC elution buffer has a conductivity of less than about any of 4.0 mS/cm, 4.5 mS/cm, 5.0 mS/cm, 5.5 mS/cm, 6.0 mS/cm, 6.5 mS/cm, 7.0 mS/cm, 7.5 mS/cm, 8.0 mS/cm, 8.5 mS/cm, 9.0 mS/cm, 9.5 mS/cm, or 10 mS/cm.

In some aspects of any of the above embodiments, the conductivity of the elution buffer changed from the load and/or wash buffer isocratically, by step gradient or by linear gradient.

In some embodiments, FkpA is eluted from the cation exchange chromatography material with a salt gradient from about 0% to about 60% 10 mM MES and 300 mM NaCl over 9 column volumes.

In some embodiments, FkpA is eluted from the HIC material using purified water until the FkpA elutes from the HIC material.

The calculated isoelectric point of FkpA is about 7.01 based on the amino acid content of the protein. In some embodiments of any of the methods described herein, the cation exchange load buffer has a pH of less than about any of 10, 9, 8, 7, 6, or 5. In some embodiments of any of the methods described herein, the load buffer has a pH of greater than about any of 4, 5, 6, 7, 8, or 9. In some embodiments, the pH of one or more of the loading buffer, the equilibration buffer, and/or the wash buffer are the same. In some embodiments, the pH of the loading buffer is different from the pH of the equilibration buffer and/or the wash buffer. In some embodiments, the pH of the load buffer for cation exchange chromatography is about pH 6.0.

In some embodiments, the elution buffer has a pH less than the pH of the load buffer. In some embodiments of any of the methods described herein, the elution buffer has a pH of less than about any of 8, 7, 6, 5, 4, 3 or 2. The pH of the elution buffer may be between about any of 4 and 9, 4 and 8, 4 and 7, 4 and 6, 4 and 5, 5 and 9, 5 and 8, 5 and 7, 5 and 6, 6 and 9, 6 and 8, 6 and 7. In some embodiments, the pH of the elution buffer is about any of 4.0, 4.5, 5.0, 5.5, 6.0, 6.5, 7.0, 7.5, 8.0, 8.5 or 9.0. In some embodiments, the composition comprising FkpA is loaded onto the anion exchange material in loading buffer at pH 8 and eluted from the anion exchange material in elution buffer at pH about 7.0 or 7.1.

In some embodiments of any of the methods described herein, the HIC load buffer has a pH of less than about any of 6.0, 7.0, or 8.0. In some embodiments of any of the methods described herein, the load buffer has a pH of greater than about any of 6.0, 7.0, or 8.0. In some embodiments, the pH of one or more of the loading buffer, the equilibration buffer, and/or the wash buffer are the same. In some embodiments, the pH of the loading buffer is different from the pH of the equilibration buffer and/or the wash buffer.

In some embodiments, size exclusion chromatography is used in a flow-through mode or in a differential partitioning mode. In some embodiments, the buffer used for size exclusion chromatography is PBS pH 7.5 $\pm 0.4$.

In some embodiments of any of the methods described herein, the flow rate is less than about any of 50 CV/hr, 40 CV/hr, or 30 CV/hr. The flow rate may be between about any of 5 CV/hr and 50 CV/hr, 10 CV/hr and 40 CV/hr, or 18 CV/hr and 36 CV/hr. In some embodiments, the flow rate is about any of 9 CV/hr, 18 CV/hr, 25 CV/hr, 30 CV/hr, 36 CV/hr, or 40 CV/hr. In some embodiments of any of the methods described herein, the flow rate is less than about any of 200 cm/hr, 150 cm/hr, 100 cm/hr, 75 cm/hr, or 50 cm/hr. The flow rate may be between about any of 25 cm/hr and 200 cm/hr, 25 cm/hr and 175 cm/hr, 25 cm/hr and 150 cm/hr, 25 cm/hr and 100 cm/hr, 50 cm/hr and 100 cm/hr, or 65 cm/hr and 85 cm/hr. In some embodiments, the flow rate is about more than about 0.1 mL/min, 0.25 mL/min, 0.5 mL/min, 0.75 mL/min,1 mL/min, 2 mL/min, 3 mL/min, 4 mL/min, 5 mL/min, 6 mL/min, 7 mL/min, 8 mL/min, 9 mL/min, 10 mL/min, 11 mL/min, 12 mL/min, 13 mL/min, 14 mL/min, 15 mL/min, 20 mL/min, 25 mL/min, and 50 mL/min. In some embodiments, the flow rate is between about 0.1 mL/min and 1 mL/min, 1 mL/min and 5 mL/min, 1 mL/min and 10 mL/min, 5 mL/min and 10 mL/min, 10 mL/min and 15 mL/min, 10 mL/min and 25 mL/min, and 15 mL/min and 25 mL/min. In some embodiments, the flow rate for anion exchange chromatography of FkpA is about 13.3 ml/min. In some embodiments, the flow rate for hydrophobic interaction chromatography of FkpA is about 13.2 ml/min. In some embodiments, the flow rate for size exclusion chromatography of FkpA is about 1 ml/min.

Bed height is the height of chromatography material used. In some embodiments of any of the method described herein, the bed height is greater than about any of 3 cm, 10 cm, 15 cm, 20 cm, 25 cm, 30 cm, 35 cm, 40 cm, 45 cm, 50 cm, 60 cm, 70 cm, 80 cm, 90 cm or 100 cm. The bed height may be between about any of 3 cm and 50 cm, 5 cm and 35 cm, 3 cm and 35 cm, or 5 cm and 50 cm. In some embodiments, bed height is determined based on the amount of polypeptide or impurities in the load.

In some embodiments, the chromatography is in a column ofr vessel with a volume of greater than about 1 mL, 2 mL, 3 mL, 4 mL, 5 mL, 6 mL, 7 mL, 8 mL, 9 mL, 10 mL, 15 mL, 20 mL, 25 mL, 30 mL, 40 mL, 50 mL, 75 mL, 100 mL, or 200 mL.

In some embodiments of the invention, fractions are collected from the chromatography. In some embodiments, fractions collected are greater than about 0.01 CV, 0.02 CV, 0.03 CV, 0.04 CV, 0.05 CV, 0.06 CV, 0.07 CV, 0.08 CV, 0.09 CV, 0.1 CV, 0.2 CV, 0.3 CV, 0.4 CV, 0.5 CV, 0.6 CV, 0.7 CV, 0.8 CV, 0.9 CV, 1.0 CV, 2.0 CV, 3.0 CV, 4.0 CV, 5.0 CV, 6.0 CV, 7.0 CV, 8.0 CV, 9.0 CV, or 10.0 CV. In some embodiments, fractions containing the product, e.g. polypeptide, are pooled. In some embodiments, fractions containing the polypeptide from the load fractions and from the elution fractions are pooled. The amount of polypeptide in a fraction can be determined by one skilled in the art; for example, the amount of polypeptide in a fraction can be determined by UV spectroscopy. In some embodiments, fractions containing detectable polypeptide fragment are pooled. In some embodiments, the presence and purity of FkpA in a fraction is determined by size exclusion chromatography.

Exemplary embodiment

In some embodiments, the invention provides the following exemplary but nonlimiting method for producing an ultrapure preparation of FkpA. FkpA is expressed in *E. coli.* The cell paste is suspended in 10 volumes 25 mM MES pH 6.0 (lysis buffer) and mixed until the suspension is homogenous. Cell lysis is performed using a Microfluidizer HC-8000 with 4 passes at house pressure (6000-8000 psi). The suspension is centrifuged in a RC6 centrifuge using a SS-34 rotor at 15,000 rpm for 30 min.

In other embodiments, The cell paste is suspended (50 g cell paste/1 L) in 50 mM MES pH 6.0 and mixed until the suspension is homogenous. Cell lysis is performed using a Microfluidizer 110F with 3 passes at house pressure (~7000 psi). The homogenate is conditioned to 0.1% PEI (using a 10% PEI stock solution) and mixed for 30 min at ambient temperature (~21°C). The suspension is centrifuged in a Sorval RC-5B+ centrifuge using a "GSA" rotor at 8500 rpm for 30 min. The centrate is collected and filtered through a 0.22um Durapore filter.

(a) SP Sepharose® Fast Flow

The clarified centrate (conditioned with 1.5 M Tris base to pH 8.0) is loaded to a column containing SP Sepharose® Fast Flow (GE Healthcare) in bind and elute mode. The column has the following properties: column was 27.0 cm (height) × 2.6 cm (diameter), volume 145 mL, protein capacity ≤50 mg/mL resin. The column is pre-equilibrated with 3 CV 250 mM MES pH 6.0 and equilibrated with 25 mM MES, pH 6.0. At the end of loading, the column is washed with 3 CV of equilibration buffer. The FkpA is eluted using a 9 CV gradient of 0 - 30% buffer B (25 mM MES, 300 mM NaCl. Fractions are analyzed by SDS-PAGE.

(b) Butyl-S Sepharose®

The pooled SP fractions are then applied to Butyl-S Sepharose® chromatography material in bind and elute mode. The column has the following properties: column dimensions were 9.4 cm (height) × 2.6 cm (diameter), volume 50 mL, protein capacity ≤20 mg/mL resin. The column is equilibrated with 300 mM sodium sulfate, 50 mM sodium phosphate, pH 7.0. The SP pool was conditioned by the addition of an equal volume of 50 mM phosphate, 0.6 M sodium sulfate, pH 7.0 prior to loading on the Butyl-S Sepharose® column. The column is then washed with 50 mM phosphate, 300 mM sodium sulfate, pH 7.0 for 3 CV. FkpA is eluted from the column with purified water. Fractions are collected and analyzed by SDS-PAGE analysis of the column fractions.

(c) Superdex 200

The pooled Butyl Sepharose® fractions are then subjected to size exclusion chromatography, using Superdex 200. This step is used to remove any residual high MW and low MW species and to formulate FkpA. Superdex 200 has fractionation range for molecules with molecular weights between 10 and 600 kDal and is suited to a protein like FkpA. The column is a Superdex 200 and has the following properties: column was 60 cm × 2.6 cm diameter, volume 320 mL, load volume ≤16mL. The HIC pool (fractions 7-11) is concentrated to a volume of ≤ 16 mL (≤ 5% of the SEC CV) using centrifugal filters. The units are centrifuged at 3000 rpm using an Eppendorf clinical centrifuge for 20 min intervals until the target volume is reached. The Superdex 200 size exclusion column is equilibrated with 3 CV PBS, pH 7.5 ± 0.4. The Butyl Sepharose® fraction is loaded on the column and eluted with PBS, pH 7.5 ± 0.4 at a flow rate of 1 mL/min.

Methods to determine the purity of FkpA

Methods to determine the purity of preparations of FkpA are known in the art. In some aspects of the disclosure, the purity of FkpA in a preparation is determined by size exclusion chromatography (SEC). In some aspects of the disclosure, the purity of FkpA in a preparation is determined by high performance liquid chromatography size exclusion chromatography (HPLC SEC). In some aspects of the disclosure, the purity of FkpA in a preparation is determined by sodium dodecyl sulfate polyacrylamide gel electrophoresis (SDS-PAGE). In some aspects of the disclosure, protein on an SDS PAGE gel is identified using a fluorescent protein imaging. In some aspects of the disclosure, the SDS PAGE is imaged using Sypro®Ruby. In some aspects of the disclosure, proteins on the SDS-PAGE are visualized using Heukeshoven Silver staining. In other aspects of the disclosure, the identity of the FkpA molecule is confirmed using characterization assays including but not limited to N-terminal sequence analysis, Peptide Mass Fingerprinting (PMF), intact/reduced Mass by CHIP TOF, and western blot analysis.

In some aspects of the disclosure, the invention provides ultrapure preparations of FkpA. In some aspects of the disclosure, the invention provides a preparation of FpkA wherein FkpA makes up at least about any of 95%, 96%, 97%, 98%, 99%, or 99.5% of the preparation. In some aspects of the disclosure, the FkpA is about any of 95%, 96%, 97%, 98%, 99%, and/or 99.5% of the preparation. In some aspects of the disclosure, the preparation comprises less than about any of 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, or 0.01%, of impurities. Impurities may include, but are not limited to host cell proteins such as *E. coli* host cell proteins, nucleic acids, viruses, cell culture components such as cell culture media components as well as aggregates of FkpA or fragments of FkpA (*e.g.*, non-functional fragments of FkpA). In some aspects of the disclosure, the preparation comprises less than about any of 2%, 1.5%, or 1% low molecular weight species. In some aspects of the disclosure, the preparation comprises less than about 1%, 0.5%, or 0.1% high molecular weight species. In some aspects of the disclosure, the high molecular weight species are undetectable. In some aspects of the disclosure, the presence of FkpA, low molecular weight species, and/or high molecular weight species are detected by SEC.

In some aspects of the disclosure, the invention provides ultrapure preparations of FkpA. In some aspects of the disclosure, the invention provides a preparation of FkpA wherein FkpA makes up at least about any of 95%, 96%, 97%, 98%, 99%, or 99.5% of the preparation. In some aspects of the disclosure, the FkpA is about any of 95%, 96%, 97%, 98%, 99%, and/or 99.5% of the preparation. In some aspects of the disclosure, the preparation comprises less than about any of 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, or 0.01%, of impurities. Impurities may include, but are not limited to host cell proteins such as *E. coli* host cell proteins, nucleic acids, viruses, cell culture components such as cell culture media components as well as aggregates of FkpA or fragments of FkpA (*e.g.*, non-functional fragments of FkpA). In some aspects of the disclosure, the preparation comprises less than about any of 1%, 0.5%, or 0.1% low molecular weight species. In some aspects of the disclosure, the preparation comprises less than about 1%, 0.5%, or 0.1% high molecular weight species. In some aspects of the disclosure, the presence of FkpA, low molecular weight species, and/or high molecular weight species are detected by SEC.

Methods of measuring DNA such as host cell DNA are known in the art and described in the examples section. In some aspects of the disclosureof any of the methods described herein, the amount of DNA is reduced by greater than about any of 10%, 20%, 30%, 40%, 50%, 60%, 70%, 80%, or 90%. The amount of DNA may be reduced by between about any of 10% and 99%, 30% and 95%, 30% and 99%, 50% and 95%, 50% and 99%, 75% and 99%, or 85% and 99%. The amount of DNA may be reduced by about any of 10%, 20%, 30%, 40%, 50%, 60%, 70%, 80%, 90%, 95%, or 99%. In some aspects of the disclosure, the reduction is determined by comparing the amount of DNA in the composition recovered from a purification step(s) to the amount of DNA in the composition before the purification step(s).

Cell culture media component refers to a component present in a cell culture media. A cell culture media may be a cell culture media at the time of harvesting cells. In some aspects of the disclosure, the cell culture media component is gentamicin. The amount of gentamicin may be measured by ELISA. In some aspects of the disclosureof any of the methods described herein, the amount of cell culture media component is reduced by greater than about any of 10%, 20%, 30%, 40%, 50%, 60%, 70%, 80%, or 90%. The amount of cell culture media component may be reduced by between about any of 10% and 99%, 30% and 95%, 30% and 99%, 50% and 95%, 50% and 99%, 75% and 99%, or 85% and 99%. In some aspects of the disclosure, the amount of cell culture media component is reduced by about any of 10%, 20%, 30%, 40%, 50%, 60%, 70%, 80%, 90%, 95%, or 98%. In some aspects of the disclosure, the reduction is determined by comparing the amount of cell culture media component in the composition recovered from a purification step(s) to the amount of cell culture media component in the composition before the purification step(s).

## III. Polypeptides-FkpA

Herein disclosed are methods to generate ultrapure preparations of FkpA. The term "FkpA" proteins refer to bacterial FKBP-type peptidyl-prolyl cis-trans isomerase. FkpA is a periplasmic protein disulfide isomerase I exhibits both cis/trans peptidyl-prolyl isomerase (PPIase) and chaperone activities. FkpA may assist in polypeptide folding as peptides emerge into the cell's periplasm. In some aspects of the disclosure, FkpA is derived from bacteria. In some aspects of the disclosure, the FkpA polypeptide is an *E. coli* FkpA polypeptide. In other aspects of the disclosure, the FkpA polypeptide is derived from any species of *Enterobacteria*, *Azoarcus*, *Salmonella*, *Buchnera*, *Xanthmonas*, *Campylobacter*, *Shigella*, *Pseudomonas*, *Yersina*, *Erwinia*, and *Neisseria.* In some aspects of the disclosure, the FkpA polypeptide comprises the amino acid sequence of SEQ ID NO: 1 or SEQ ID NO:2. In some aspects of the disclosure, the FkpA polypeptide comprises an amino acid sequence that has at least about any of 70%, 75%, 80%, 85%, 90%, 95%, or 99% identity to the amino acid sequence of SEQ ID NO: 1 or SEQ ID NO:2.

The FkpA polypeptides to be purified using the methods described herein is generally produced using recombinant techniques. Methods for producing recombinant polypeptides in bacteria are known in the art. When using recombinant techniques, the polypeptides can be produced intracellularly, in the periplasmic space, or directly secreted into the medium. In some aspects of the disclosure, nucleic acid encoding FkpA is introduced to the host cell for overexpression of the polypeptide. In certain aspects of the disclosure, the host cell engineered to overexpress FkpA expresses FkpA at a level greater than the level produced by the host cell without the genetic engineering. In some aspects of the disclosure, the nucleic acid encoding FkpA is expressed from an expression vector; *e.g.*, a plasmid. In some aspects of the disclosure, the nucleic acid encoding the FkpA polypeptide comprises the nucleic acid sequence of SEQ ID NO:3. In some aspects of the disclosure, the nucleic acid encoding FkpA comprises a nucleic acid sequence that has at least about any of 70%, 75%, 80%, 85%, 90%, 95%, or 99% identity to the nucleic acid sequence of SEQ ID NO:3.

The polypeptides may be recovered from culture medium or from host cell lysates. Cells employed in expression of the polypeptides can be disrupted by various physical or chemical means, such as freeze-thaw cycling, sonication, mechanical disruption, or cell lysing agents. If the polypeptide is produced intracellularly, as a first step, the particulate debris, either host cells or lysed fragments, are removed, for example, by centrifugation or ultrafiltration. Carter et al., Bio/Technology 10: 163-167 (1992) describe a procedure for isolating polypeptides which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the polypeptide is secreted into the medium, supernatants from such expression systems are generally first concentrated using a commercially available polypeptide concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

IV. Polypeptides for which FkpA may be used in production.

Examples of polypeptides that may be produced in bacteria (*e.g.* E coli) where protein folding and assembly may be aided by the expression of FkpA (*e.g.*, exogenous FkpA) include but are not limited to immunoglobulins, immunoadhesins, antibodies, half-antibodies, antibody fragments, enzymes, hormones, fusion proteins, Fc-containing proteins, immunoconjugates, cytokines and interleukins. Examples of polypeptide include, but are not limited to, mammalian proteins, such as, e.g., renin; a hormone; a growth hormone, including human growth hormone and bovine growth hormone; growth hormone releasing factor; parathyroid hormone; thyroid stimulating hormone; lipoproteins; alpha-1-antitrypsin; insulin A-chain; insulin B-chain; proinsulin; follicle stimulating hormone; calcitonin; luteinizing hormone; glucagon; clotting factors such as factor VIIIC, factor IX, tissue factor, and von Willebrands factor; anti-clotting factors such as Protein C; atrial natriuretic factor; lung surfactant; a plasminogen activator, such as urokinase or human urine or tissue-type plasminogen activator (t-PA); bombesin; thrombin; hemopoietic growth factor; tumor necrosis factor-alpha and -beta; enkephalinase; RANTES (regulated on activation normally T-cell expressed and secreted); human macrophage inflammatory protein (MIP-1-alpha); a serum albumin such as human serum albumin; Muellerian-inhibiting substance; relaxin A-chain; relaxin B-chain; prorelaxin; mouse gonadotropin-associated peptide; an enzyme; a microbial protein, such as beta-lactamase; DNase; IgE; a cytotoxic T-lymphocyte associated antigen (CTLA), such as CTLA-4; inhibin; activin; vascular endothelial growth factor (VEGF); receptors for hormones or growth factors; protein A or D;

rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-b; platelet-derived growth factor (PDGF); fibroblast growth factor such as aFGF and bFGF; epidermal growth factor (EGF); transforming growth factor (TGF) such as TGF-alpha and TGF-beta, including TGF-β1, TGF-β2, TGF-β3, TGF-β4, or TGF-β5; insulin-like growth factor-I and -II (IGF-I and IGF-II); des(1-3)-IGF-I (brain IGF-I), insulin-like growth factor binding proteins (IGFBPs); a cytokine; CD proteins such as CD3, CD4, CD8, CD19 and CD20; erythropoietin; osteoinductive factors; immunotoxins; a fusion polypeptide, *i.e.* a polypeptide comprised on two or more heterologous polypeptides or fragments thereof and encoded by a recombinant nucleic acid; an Fc-containing polypeptide, for example, a fusion protein comprising an immunoglobulin Fc region, or fragment thereof, fused to a second polypeptide; an immunoconjugate; a bone morphogenetic protein (BMP); an interferon such as interferon-alpha, -beta, and -gamma; colony stimulating factors (CSFs), *e.g.*, M-CSF, GM-CSF, and G-CSF; interleukins (ILs), *e.g.*, IL-1 to IL-10, IL13, IL17; superoxide dismutase; T-cell receptors; surface membrane proteins; decay accelerating factor; viral antigen such as, for example, a portion of the AIDS envelope; transport proteins; homing receptors; addressins; regulatory proteins; integrins such as CD11a, CD11b, CD11c, CD18, an ICAM, VLA-4 and VCAM; a tumor associated antigen such as CA125 (ovarian cancer antigen) or HER2, HER3 or HER4 receptor; immunoadhesins; and fragments and/or variants of any of the above-listed proteins as well as antibodies, including antibody fragments, binding to a protein, including, for example, any of the above-listed proteins.

In some embodiments, the polypeptide preparation for use in any of the assay methods described herein contains an antibody of interest, *i.e.* the recombinant polypeptide produced by a host cell is an antibody.

Molecular targets for such antibodies include CD proteins and their ligands, such as, but not limited to: (i) CD3, CD4, CD13, CD8, CD19, CD11a, CD20, CD22, CD34, CD40, CD79α (CD79a), and CD79β (CD79b); (ii) members of the ErbB receptor family such as the EGF receptor, HER2, HER3 or HER4 receptor; (iii) cell adhesion molecules such as LFA-1, Mac1, p150,95, VLA-4, ICAM-1, VCAM and αv/β3 integrin, including either alpha or beta subunits thereof (e.g., anti-CD11a, anti-CD18 or anti-CD11b antibodies); (iv) growth factors such as VEGF; IgE; blood group antigens; flk2/flt3 receptor; obesity (OB) receptor; mpl receptor; CTLA-4; protein C, BR3, c-met, tissue factor, β7 *etc*; and (v) cell surface and transmembrane tumor-associated antigens (TAA), such as those described in U.S. Patent No. 7,521,541.

Other exemplary antibodies include those selected from, and without limitation, anti-estrogen receptor antibody, anti-progesterone receptor antibody, anti-p53 antibody, anti-HER-2/neu antibody, anti-EGFR antibody, anti-cathepsin D antibody, anti-Bcl-2 antibody, anti-E-cadherin antibody, anti-CA125 antibody, anti-CA15-3 antibody, anti-CA19-9 antibody, anti-c-erbB-2 antibody, anti-P-glycoprotein antibody, anti-CEA antibody, anti-retinoblastoma protein antibody, anti-ras oncoprotein antibody, anti-Lewis X antibody, anti-Ki-67 antibody, anti-PCNA antibody, anti-CD3 antibody, anti-CD4 antibody, anti-CD5 antibody, anti-CD7 antibody, anti-CD8 antibody, anti-CD9/p24 antibody, anti-CD 10 antibody, anti-CD11a antibody, anti-CD11c antibody, anti-CD 13 antibody, anti-CD 14 antibody, anti-CD 15 antibody, anti-CD 19 antibody, anti-CD20 antibody, anti-CD22 antibody, anti-CD23 antibody, anti-CD30 antibody, anti-CD31 antibody, anti-CD33 antibody, anti-CD34 antibody, anti-CD35 antibody, anti-CD38 antibody, anti-CD41 antibody, anti-LCA/CD45 antibody, anti-CD45RO antibody, anti-CD45RA antibody, anti-CD39 antibody, anti-CD100 antibody, anti-CD95/Fas antibody, anti-CD99 antibody, anti-CD106 antibody, anti-ubiquitin antibody, anti-CD71 antibody, anti-c-myc antibody, anti-cytokeratins antibody, anti-vimentins antibody, anti-HPV proteins antibody, anti-kappa light chains antibody, anti-lambda light chains antibody, anti-melanosomes antibody, anti-prostate specific antigen antibody, anti-S-100 antibody, anti-tau antigen antibody, antifibrin antibody, anti-keratins antibody and anti-Tn-antigen antibody.

Monoclonal antibodies

In some embodiments, antibodies are monoclonal antibodies. Monoclonal antibodies are obtained from a population of substantially homogeneous antibodies, i.e., the individual antibodies comprising the population are identical and/or bind the same epitope except for possible variants that arise during production of the monoclonal antibody, such variants generally being present in minor amounts. Thus, the modifier "monoclonal" indicates the character of the antibody as not being a mixture of discrete or polyclonal antibodies.

For example, the monoclonal antibodies may be made using the hybridoma method first described by Kohler et al., Nature 256:495 (1975), or may be made by recombinant DNA methods (U.S. Patent No. 4,816,567).

In the hybridoma method, a mouse or other appropriate host animal, is immunized as herein described to elicit lymphocytes that produce or are capable of producing antibodies that will specifically bind to the polypeptide used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp. 59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

In some embodiments, the myeloma cells are those that fuse efficiently, support stable high-level production of antibody by the selected antibody-producing cells, and are sensitive to a medium such as HAT medium. Among these, in some embodiments, the myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol. 133:3001 (1984); Brodeur et al., Monoclonal Antibody Production Techniques and Applications pp. 51-63 (Marcel Dekker, Inc., New York, 1987)).

Culture medium in which hybridoma cells are growing is assayed for production of monoclonal antibodies directed against the antigen. In some embodiments, the binding specificity of monoclonal antibodies produced by hybridoma cells is determined by immunoprecipitation or by an in vitro binding assay, such as radioimmunoassay (RIA) or enzyme-linked immunoabsorbent assay (ELISA).

The binding affinity of the monoclonal antibody can, for example, be determined by the Scatchard analysis of Munson et al., Anal. Biochem. 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice pp. 59-103 (Academic Press, 1986)). Suitable culture media for this purpose include, for example, D-MEM or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional immunoglobulin purification procedures such as, for example, polypeptide A-Sepharose®, hydroxylapatite chromatography, gel electrophoresis, dialysis, or affinity chromatography.

DNA encoding the monoclonal antibodies is readily isolated and sequenced using conventional procedures (e.g., by using oligonucleotide probes that are capable of binding specifically to genes encoding the heavy and light chains of murine antibodies). In some embodiments, the hybridoma cells serve as a source of such DNA. Once isolated, the DNA may be placed into expression vectors, which are then transfected into host cells such as E. coli cells, simian COS cells, human embryonic kidney (HEK) 293 cells, Chinese Hamster Ovary (CHO) cells, or myeloma cells that do not otherwise produce immunoglobulin polypeptide, to obtain the synthesis of monoclonal antibodies in the recombinant host cells. Review articles on recombinant expression in bacteria of DNA encoding the antibody include Skerra et al., Curr. Opinion in Immunol. 5:256-262 (1993) and Plückthun, Immunol. Revs., 130:151-188 (1992).

In a further embodiment, antibodies or antibody fragments can be isolated from antibody phage libraries generated using the techniques described in McCafferty et al., Nature 348:552-554 (1990). Clackson et al., Nature 352:624-628 (1991) and Marks et al., J. Mol. Biol. 222:581-597 (1991) describe the isolation of murine and human antibodies, respectively, using phage libraries. Subsequent publications describe the production of high affinity (nM range) human antibodies by chain shuffling (Marks et al., Bio/Technology 10:779-783 (1992)), as well as combinatorial infection and in vivo recombination as a strategy for constructing very large phage libraries (Waterhouse et al., Nuc. Acids. Res. 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy- and light chain constant domains in place of the homologous murine sequences (U.S. Patent No. 4,816,567; Morrison et al., Proc. Natl Acad. Sci. USA 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

In some embodiments of any of the methods described herein, the antibody is IgA, IgD, IgE, IgG, or IgM. In some embodiments, the antibody is an IgG monoclonal antibody.

Antibody fragments

In some embodiments, an antibody is an antibody fragment. Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992) and Brennan et al., Science 229:81 (1985)). However, these fragments can now be produced directly by recombinant host cells. For example, the antibody fragments can be isolated from the antibody phage libraries discussed above. Alternatively, Fab'-SH fragments can be directly recovered from E. coli and chemically coupled to form F(ab')2 fragments (Carter et al., Bio/Technology 10: 163-167 (1992)). According to another approach, F(ab')2 fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; US Patent No. 5,571,894; and US Patent No. 5,587,458. The antibody fragment may also be a "linear antibody," *e.g.*, as described in US Patent 5,641,870 for example. Such linear antibody fragments may be monospecific or bispecific.

In some embodiments, fragments of the antibodies described herein are provided. In some embodiments, the antibody fragment is an antigen binding fragment. In some embodiments, the antigen binding fragment is selected from the group consisting of a Fab fragment, a Fab' fragment, a F(ab')2 fragment, a scFv, a Fv, and a diabody.

Polypeptide Variants and Modifications

In certain embodiments, amino acid sequence variants of the proteins herein are contemplated. For example, it may be desirable to improve the binding affinity and/or other biological properties of the protein. Amino acid sequence variants of a protein may be prepared by introducing appropriate modifications into the nucleotide sequence encoding the protein, or by peptide synthesis. Such modifications include, for example, deletions from, and/or insertions into and/or substitutions of residues within the amino acid sequences of the protein. Any combination of deletion, insertion, and substitution can be made to arrive at the final construct, provided that the final construct possesses the desired characteristics.

Variant Polypeptides

"Polypeptide variant" means a polypeptide, for example, an active polypeptide, as defined herein having at least about 80% amino acid sequence identity with a full-length native sequence of the polypeptide, a polypeptide sequence lacking the signal peptide, an extracellular domain of a polypeptide, with or without the signal peptide. Such polypeptide variants include, for instance, polypeptides wherein one or more amino acid residues are added, or deleted, at the N or C-terminus of the full-length native amino acid sequence. Ordinarily, a polypeptide variant will have at least about 80% amino acid sequence identity, alternatively at least about any of 85%, 90%, 95%, 96%, 97%, 98%, or 99% amino acid sequence identity, to a full-length native sequence polypeptide sequence, a polypeptide sequence lacking the signal peptide, an extracellular domain of a polypeptide, with or without the signal peptide. Optionally, variant polypeptides will have no more than one conservative amino acid substitution as compared to the native polypeptide sequence, alternatively no more than about any of 2, 3, 4, 5, 6, 7, 8, 9, or 10 conservative amino acid substitution as compared to the native polypeptide sequence.

The variant polypeptide may be truncated at the N-terminus or C-terminus, or may lack internal residues, for example, when compared with a full length native polypeptide. Certain variant polypeptides may lack amino acid residues that are not essential for a desired biological activity. These variant polypeptides with truncations, deletions, and insertions may be prepared by any of a number of conventional techniques. Desired variant polypeptides may be chemically synthesized. Another suitable technique involves isolating and amplifying a nucleic acid fragment encoding a desired variant polypeptide, by polymerase chain reaction (PCR). Oligonucleotides that define the desired termini of the nucleic acid fragment are employed at the 5' and 3' primers in the PCR. Preferably, variant polypeptides share at least one biological and/or immunological activity with the native polypeptide disclosed herein.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic polypeptide. Other insertional variants of the antibody molecule include the fusion to the N- or C-terminus of the antibody to an enzyme or a polypeptide which increases the serum half-life of the antibody.

For example, it may be desirable to improve the binding affinity and/or other biological properties of the polypeptide. Amino acid sequence variants of the polypeptide are prepared by introducing appropriate nucleotide changes into the antibody nucleic acid, or by peptide synthesis. Such modifications include, for example, deletions from, and/or insertions into and/or substitutions of, residues within the amino acid sequences of the polypeptide. Any combination of deletion, insertion, and substitution is made to arrive at the final construct, provided that the final construct possesses the desired characteristics. The amino acid changes also may alter post-translational processes of the polypeptide (*e.g.*, antibody), such as changing the number or position of glycosylation sites.

Guidance in determining which amino acid residue may be inserted, substituted or deleted without adversely affecting the desired activity may be found by comparing the sequence of the polypeptide with that of homologous known polypeptide molecules and minimizing the number of amino acid sequence changes made in regions of high homology.

A useful method for identification of certain residues or regions of the polypeptide (*e.g.*, antibody) that are preferred locations for mutagenesis is called "alanine scanning mutagenesis" as described by Cunningham and Wells, Science 244: 1081-1085 (1989). Here, a residue or group of target residues are identified (*e.g.*, charged residues such as Arg, Asp, His, Lys, and Glu) and replaced by a neutral or negatively charged amino acid (most preferably Alanine or Polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed antibody variants are screened for the desired activity.

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated. If such substitutions result in a change in biological activity, then more substantial changes, denominated "exemplary substitutions" in the Table 1, or as further described below in reference to amino acid classes, may be introduced and the products screened.

Table 1.

| Original Residue | Exemplary Substitutions | Conservative Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in the biological properties of the polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, Biochemistry second ed., pp. 73-75, Worth Publishers, New York (1975)):

(1) non-polar: Ala (A), Val (V), Leu (L), Ile (I), Pro (P), Phe (F), Trp (W), Met (M)
(2) uncharged polar: Gly (G), Ser (S), Thr (T), Cys (C), Tyr (Y), Asn (N), Gln (Q)
(3) acidic: Asp (D), Glu (E)
(4) basic: Lys (K), Arg (R), His(H)

Alternatively, naturally occurring residues may be divided into groups based on common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

Any cysteine residue not involved in maintaining the proper conformation of the antibody also may be substituted, generally with serine, to improve the oxidative stability of the molecule and prevent aberrant crosslinking. Conversely, cysteine bond(s) may be added to the polypeptide to improve its stability (particularly where the antibody is an antibody fragment such as an Fv fragment).

One example of substitutional variant involves substituting one or more hypervariable region residues of a parent antibody (e.g., a humanized antibody). Generally, the resulting variant(s) selected for further development will have improved biological properties relative to the parent antibody from which they are generated. A convenient way for generating such substitutional variants involves affinity maturation using phage display. Briefly, several hypervariable region sites (e.g., 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (e.g., binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and target. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the polypeptide alters the original glycosylation pattern of the antibody. The polypeptide may comprise non-amino acid moieties. For example, the polypeptide may be glycosylated. Such glycosylation may occur naturally during expression of the polypeptide in the host cell or host organism, or may be a deliberate modification arising from human intervention. By altering is meant deleting one or more carbohydrate moieties found in the polypeptide, and/or adding one or more glycosylation sites that are not present in the polypeptide.

Glycosylation of polypeptide is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the polypeptide is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Removal of carbohydrate moieties present on the polypeptide may be accomplished chemically or enzymatically or by mutational substitution of codons encoding for amino acid residues that serve as targets for glycosylation. Enzymatic cleavage of carbohydrate moieties on polypeptides can be achieved by the use of a variety of endo- and exo-glycosidases.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the $\gamma$-amino groups of lysine, arginine, and histidine side chains, acetylation of the N-terminal amine, and amidation of any C-terminal carboxyl group.

Chimeric Polypeptides

The polypeptide described herein may be modified in a way to form chimeric molecules comprising the polypeptide fused to another, heterologous polypeptide or amino acid sequence. In some embodiments, a chimeric molecule comprises a fusion of the polypeptide with a tag polypeptide which provides an epitope to which an anti-tag antibody can selectively bind. The epitope tag is generally placed at the amino- or carboxyl-terminus of the polypeptide. The presence of such epitope-tagged forms of the polypeptide can be detected using an antibody against the tag polypeptide. Also, provision of the epitope tag enables the polypeptide to be readily purified by affinity purification using an anti-tag antibody or another type of affinity matrix that binds to the epitope tag.

Multispecific Antibodies

In certain embodiments, an antibody provided herein is a multispecific antibody, e.g. a bispecific antibody. In certain embodiments, multispecific antibodies are monoclonal antibodies that have binding specificities for at least two different sites. In certain embodiments, one of the binding specificities is for one antigen and the other is for any other antigen. In certain embodiments, bispecific antibodies may bind to two different epitopes of the same antigen. Bispecific antibodies may also be used to localize cytotoxic agents to cells which express a particular antigen. Bispecific antibodies can be prepared as full length antibodies or antibody fragments. In some embodiments, the multispecific antibody is a bispecific antibody where one arm binds IL13 and one arm binds IL17.
Techniques for making multispecific antibodies include, but are not limited to, recombinant co-expression of two immunoglobulin heavy chain-light chain pairs having different specificities (see Milstein and Cuello, Nature 305: 537 (1983), WO 93/08829, and Traunecker et al., EMBO J. 10: 3655 (1991)), and "knob-in-hole" engineering (see, *e.g.,* U.S. Patent No. 5,731,168), and in addition exchanging one or more heavy chain and light chain domains within the antigen-binding fragment (Fab) of one half of the bi-specific antibody (CrossMab, see WO2009/080251, WO2009/080252, WO2009/080253, and WO2009/080254). Multi-specific antibodies may also be made by engineering electrostatic steering effects for making antibody Fc-heterodimeric molecules (WO 2009/089004A1); cross-linking two or more antibodies or fragments (see, *e.g.,* US Patent No. 4,676,980, and Brennan et al., Science, 229: 81 (1985)); using leucine zippers to produce bi-specific antibodies (see, *e.g.,* Kostelny et al., J. Immunol., 148(5): 1547-1553 (1992)); using "diabody" technology for making bispecific antibody fragments (see, *e.g.,* Hollinger et al., Proc. Natl. Acad. Sci. USA, 90:6444-6448 (1993)); and using single-chain Fv (sFv) dimers (see, *e.g.* Gruber et al., J. Immunol., 152:5368 (1994)); and preparing trispecific antibodies as described, *e.g.,* in Tutt et al. J. Immunol. 147: 60 (1991). As discussed below, in some embodiments, multispecific antibodies are prepared by generating half antibodies in cells (*e.g.,* E. coli cells expressing FkpA), isolating the half antibodies, and assembling the half antibodies into a bispecific antibody.
Engineered antibodies with three or more functional antigen binding sites, including "Octopus antibodies," are also included herein (see, *e.g.* US 2006/0025576A1).
The antibody or fragment herein also includes a "Dual Acting FAb" or "DAF" comprising an antigen binding site that binds to one antigen as well as another, different antigen (see, US 2008/0069820, for example).
Methods for making bispecific antibodies are known in the art. Traditionally, the recombinant production of bispecific antibodies is based on the co-expression of two immunoglobulin heavy chain-light chain pairs, where the two heavy chains have different specificities (Milstein and Cuello, Nature, 305: 537 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in WO 93/08829 published May 13, 1993, and in Traunecker et al., EMBO J., 10: 3655 (1991).
According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1), containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.
In a one embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).
According to another approach, the interface between a pair of antibody molecules can be engineered to maximize the percentage of heterodimers which are recovered from recombinant cell culture. The preferred interface comprises at least a part of the CH3 domain of an antibody constant domain. In this method, one or more small amino acid side chains from the interface of the first antibody molecule are replaced with larger side chains (*e.g.,* tyrosine or tryptophan) (knobs or protuberances). Compensatory "cavities" (holes) of identical or similar size to the large side chain(s) are created on the interface of the second antibody molecule by replacing large amino acid side chains with smaller ones (*e.g.*, alanine or threonine). This provides a mechanism for increasing the yield of the heterodimer over other unwanted end-products such as homodimers. Knobs and holes are further described herein.

Knobs in holes

The use of knobs into holes as a method of producing multispecific antibodies and/or one-armed antibodies and/or immunoadhesins is well known in the art. See US Pat. No. 5,731,168 granted 24 March 1998 assigned to Genentech. Other references that describe methods to generate multispecific antibodies include PCT Pub. No. WO2009089004 published 16 July 2009 and assigned to Amgen, and US Pat. Pub. No. 20090182127 published 16 July 2009 and assigned to Novo Nordisk A/S. See also Marvin and Zhu, Acta Pharmacologica Sincia (2005) 26(6):649-658 and Kontermann (2005) Acta Pharacol. Sin., 26:1-9. A brief discussion is provided here.

A "protuberance" refers to at least one amino acid side chain which projects from the interface of a first polypeptide and is therefore positionable in a compensatory cavity in the adjacent interface (*i.e.* the interface of a second polypeptide) so as to stabilize the heteromultimer, and thereby favor heteromultimer formation over homomultimer formation, for example. The protuberance may exist in the original interface or may be introduced synthetically (*e.g.* by altering nucleic acid encoding the interface). Normally, nucleic acid encoding the interface of the first polypeptide is altered to encode the protuberance. To achieve this, the nucleic acid encoding at least one "original" amino acid residue in the interface of the first polypeptide is replaced with nucleic acid encoding at least one "import" amino acid residue which has a larger side chain volume than the original amino acid residue. It will be appreciated that there can be more than one original and corresponding import residue. The upper limit for the number of original residues which are replaced is the total number of residues in the interface of the first polypeptide. The side chain volumes of the various amino residues are shown in the following table.

Table 2. Properties of Amino Acids

| Amino Acid | One-Letter Abbreviation | MASS[a] (daltons) | VOLUME[b] (Angstrom$^3$) | Accessible Surface Area[c] (Angstrom$^2$) |
|---|---|---|---|---|
| Alanine (Ala) | A | 71.08 | 88.6 | 115 |
| Arginine (Arg) | R | 156.20 | 173.4 | 225 |
| Asparagine (Asn) | N | 114.11 | 117.7 | 160 |
| Aspartic acid (Asp) | D | 115.09 | 111.1 | 150 |
| Cysteine (Cys) | C | 103.14 | 108.5 | 135 |
| Glutamine (Gln) | Q | 128.14 | 143.9 | 180 |
| Glutamic acid (Glu) | E | 129.12 | 138.4 | 190 |
| Glycine (Gly) | G | 57.06 | 60.1 | 75 |
| Histidine (His) | H | 137.15 | 153.2 | 195 |
| Isoleucine (Ile) | I | 113.17 | 166.7 | 175 |
| Leucine (Leu) | L | 113.17 | 166.7 | 170 |
| Lysine (Lys) | K | 128.18 | 168.6 | 200 |
| Methionine (Met) | M | 131.21 | 162.9 | 185 |
| Phenylalinine (Phe) | F | 147.18 | 189.9 | 210 |
| Proline (Pro) | P | 97.12 | 122.7 | 145 |
| Serine (Ser) | S | 87.08 | 89.0 | 115 |

(continued)

| Amino Acid | One-Letter Abbreviation | MASS[a] (daltons) | VOLUME[b] (Angstrom$^3$) | Accessible Surface Area[c] (Angstrom$^2$) |
|---|---|---|---|---|
| Threonine (Thr) | T | 101.11 | 116.1 | 140 |
| Tryptophan (Trp) | W | 186.21 | 227.8 | 255 |
| Tyrosine (Tyr) | Y | 163.18 | 193.6 | 230 |
| Valine (Val) | V | 99.14 | 140.0 | 155 |

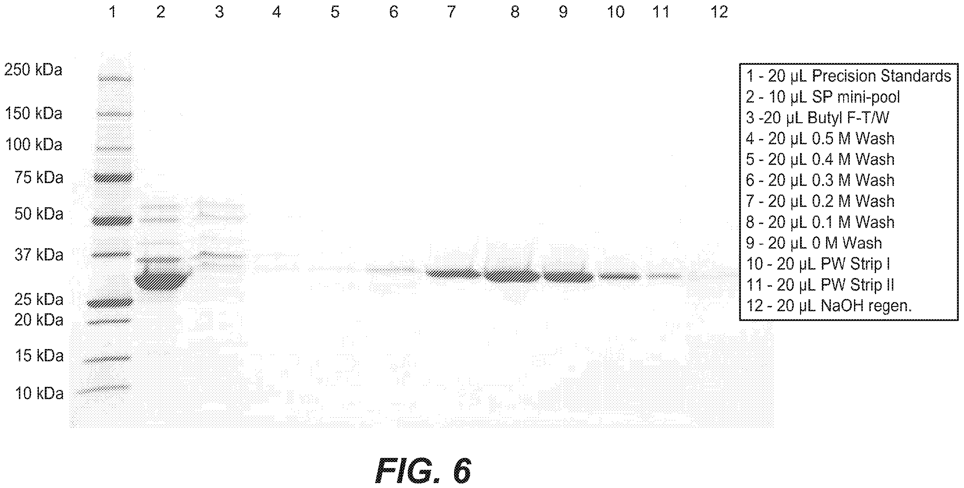

[a]Molecular weight amino acid minus that of water. Values from Handbook of Chemistry and Physics, 43rd ed. Cleveland, Chemical Rubber Publishing Co., 1961.
[b]Values from A.A. Zamyatnin, Prog. Biophys. Mol. Biol. 24: 107-123, 1972.
[c]Values from C. Chothia, J. Mol. Biol. 105: 1-14, 1975. The accessible surface area is defined in FIGs. 6-20 of this reference.

The preferred import residues for the formation of a protuberance are generally naturally occurring amino acid residues and are preferably selected from arginine (R), phenylalanine (F), tyrosine (Y) and tryptophan (W). Most preferred are tryptophan and tyrosine. In one embodiment, the original residue for the formation of the protuberance has a small side chain volume, such as alanine, asparagine, aspartic acid, glycine, serine, threonine or valine. Exemplary amino acid substitutions in the CH3 domain for forming the protuberance include without limitation the T366W substitution.

A "cavity" refers to at least one amino acid side chain which is recessed from the interface of a second polypeptide and therefore accommodates a corresponding protuberance on the adjacent interface of a first polypeptide. The cavity may exist in the original interface or may be introduced synthetically (e.g. by altering nucleic acid encoding the interface). Normally, nucleic acid encoding the interface of the second polypeptide is altered to encode the cavity. To achieve this, the nucleic acid encoding at least one "original" amino acid residue in the interface of the second polypeptide is replaced with DNA encoding at least one "import" amino acid residue which has a smaller side chain volume than the original amino acid residue. It will be appreciated that there can be more than one original and corresponding import residue. The upper limit for the number of original residues which are replaced is the total number of residues in the interface of the second polypeptide. The side chain volumes of the various amino residues are shown in Table 2 above. The preferred import residues for the formation of a cavity are usually naturally occurring amino acid residues and are preferably selected from alanine (A), serine (S), threonine (T) and valine (V). Most preferred are serine, alanine or threonine. In one embodiment, the original residue for the formation of the cavity has a large side chain volume, such as tyrosine, arginine, phenylalanine or tryptophan. Exemplary amino acid substitutions in the CH3 domain for generating the cavity include without limitation the T366S, L368A and Y407A substitutions.

An "original" amino acid residue is one which is replaced by an "import" residue which can have a smaller or larger side chain volume than the original residue. The import amino acid residue can be a naturally occurring or non-naturally occurring amino acid residue, but preferably is the former. "Naturally occurring" amino acid residues are those residues encoded by the genetic code and listed in Table 2 above. By "non-naturally occurring" amino acid residue is meant a residue which is not encoded by the genetic code, but which is able to covalently bind adjacent amino acid residue(s) in the polypeptide chain. Examples of non-naturally occurring amino acid residues are norleucine, ornithine, norvaline, homoserine and other amino acid residue analogues such as those described in Ellman et al., Meth. Enzym. 202:301-336 (1991), for example. To generate such non-naturally occurring amino acid residues, the procedures of Noren et al. Science 244: 182 (1989) and Ellman et al., supra can be used. Briefly, this involves chemically activating a suppressor tRNA with a non-naturally occurring amino acid residue followed by in vitro transcription and translation of the RNA. The method of the instant invention involves replacing at least one original amino acid residue, but more than one original residue can be replaced. Normally, no more than the total residues in the interface of the first or second polypeptide will comprise original amino acid residues which are replaced. Typically, original residues for replacement are "buried". By "buried" is meant that the residue is essentially inaccessible to solvent. Generally, the import residue is not cysteine to prevent possible oxidation or mispairing of disulfide bonds.

The protuberance is "positionable" in the cavity which means that the spatial location of the protuberance and cavity on the interface of a first polypeptide and second polypeptide respectively and the sizes of the protuberance and cavity are such that the protuberance can be located in the cavity without significantly perturbing the normal association of the first and second polypeptides at the interface. Since protuberances such as Tyr, Phe and Trp do not typically extend perpendicularly from the axis of the interface and have preferred conformations, the alignment of a protuberance with a corresponding cavity relies on modeling the protuberance/cavity pair based upon a three-dimensional structure such as that obtained by X-ray crystallography or nuclear magnetic resonance (NMR). This can be achieved using widely accepted techniques in the art.

By "original or template nucleic acid" is meant the nucleic acid encoding a polypeptide of interest which can be "altered" (*i.e.* genetically engineered or mutated) to encode a protuberance or cavity. The original or starting nucleic acid may be a naturally occurring nucleic acid or may comprise a nucleic acid which has been subjected to prior alteration (*e.g.* a humanized antibody fragment). By "altering" the nucleic acid is meant that the original nucleic acid is mutated by inserting, deleting or replacing at least one codon encoding an amino acid residue of interest. Normally, a codon encoding an original residue is replaced by a codon encoding an import residue. Techniques for genetically modifying a DNA in this manner have been reviewed in Mutagenesis: a Practical Approach, M.J. McPherson, Ed., (IRL Press, Oxford, UK. (1991), and include site-directed mutagenesis, cassette mutagenesis and polymerase chain reaction (PCR) mutagenesis, for example. By mutating an original/template nucleic acid, an original/template polypeptide encoded by the original/template nucleic acid is thus correspondingly altered.

The protuberance or cavity can be "introduced" into the interface of a first or second polypeptide by synthetic means, *e.g.* by recombinant techniques, in vitro peptide synthesis, those techniques for introducing non-naturally occurring amino acid residues previously described, by enzymatic or chemical coupling of peptides or some combination of these techniques. Accordingly, the protuberance or cavity which is "introduced" is "non-naturally occurring" or "non-native", which means that it does not exist in nature or in the original polypeptide (*e.g.* a humanized monoclonal antibody).

Generally, the import amino acid residue for forming the protuberance has a relatively small number of "rotamers" (*e.g.* about 3-6). A "rotamer" is an energetically favorable conformation of an amino acid side chain. The number of rotamers of the various amino acid residues is reviewed in Ponders and Richards, J. Mol. Biol. 193: 775-791 (1987).

In one embodiment, a first Fc polypeptide and a second Fc polypeptide meet/interact at an interface. In some embodiments wherein the first and second Fc polypeptides meet at an interface, the interface of the second Fc polypeptide (sequence) comprises a protuberance (also termed a "knob") which is positionable in a cavity (also termed a "hole") in the interface of the first Fc polypeptide (sequence). In one embodiment, the first Fc polypeptide has been altered from a template/original polypeptide to encode the cavity or the second Fc polypeptide has been altered from a template/original polypeptide to encode the protuberance, or both. In one embodiment, the first Fc polypeptide has been altered from a template/original polypeptide to encode the cavity and the second Fc polypeptide has been altered from a template/original polypeptide to encode the protuberance. In one embodiment, the interface of the second Fc polypeptide comprises a protuberance which is positionable in a cavity in the interface of the first Fc polypeptide, wherein the cavity or protuberance, or both, have been introduced into the interface of the first and second Fc polypeptides, respectively. In some embodiments wherein the first and second Fc polypeptides meet at an interface, the interface of the first Fc polypeptide (sequence) comprises a protuberance which is positionable in a cavity in the interface of the second Fc polypeptide (sequence). In one embodiment, the second Fc polypeptide has been altered from a template/original polypeptide to encode the cavity or the first Fc polypeptide has been altered from a template/original polypeptide to encode the protuberance, or both. In one embodiment, the second Fc polypeptide has been altered from a template/original polypeptide to encode the cavity and the first Fc polypeptide has been altered from a template/original polypeptide to encode the protuberance. In one embodiment, the interface of the first Fc polypeptide comprises a protuberance which is positionable in a cavity in the interface of the second Fc polypeptide, wherein the protuberance or cavity, or both, have been introduced into the interface of the first and second Fc polypeptides, respectively.

In one embodiment, the protuberance and cavity each comprise a naturally occurring amino acid residue. In one embodiment, the Fc polypeptide comprising the protuberance is generated by replacing an original residue from the interface of a template/original polypeptide with an import residue having a larger side chain volume than the original residue. In one embodiment, the Fc polypeptide comprising the protuberance is generated by a method comprising a step wherein polynucleotide encoding an original residue from the interface of said polypeptide is replaced with polynucleotide encoding an import residue having a larger side chain volume than the original. In one embodiment, the original residue is threonine. In one embodiment, the original residue is T366. In one embodiment, the import residue is arginine (R). In one embodiment, the import residue is phenylalanine (F). In one embodiment, the import residue is tyrosine (Y). In one embodiment, the import residue is tryptophan (W). In one embodiment, the import residue is R, F, Y or W. In one embodiment, a protuberance is generated by replacing two or more residues in a template/original polypeptide. In one embodiment, the Fc polypeptide comprising a protuberance comprises replacement of threonine at position 366 with tryptophan, amino acid numbering according to the EU numbering scheme of Kabat et al. (pp. 688-696 in Sequences of proteins of immunological interest, 5th ed., Vol. 1 (1991; NIH, Bethesda, MD)).

In some embodiments, the Fc polypeptide comprising a cavity is generated by replacing an original residue in the interface of a template/original polypeptide with an import residue having a smaller side chain volume than the original residue. For example, the Fc polypeptide comprising the cavity may be generated by a method comprising a step wherein polynucleotide encoding an original residue from the interface of said polypeptide is replaced with polynucleotide encoding an import residue having a smaller side chain volume than the original. In one embodiment, the original residue is threonine. In one embodiment, the original residue is leucine. In one embodiment, the original residue is tyrosine. In one embodiment, the import residue is not cysteine (C). In one embodiment, the import residue is alanine (A). In one embodiment, the import residue is serine (S). In one embodiment, the import residue is threonine (T). In one embodiment, the import residue is valine (V). A cavity can be generated by replacing one or more original residues of a template/original polypeptide. For example, in one embodiment, the Fc polypeptide comprising a cavity comprises replacement of two or more original amino acids selected from the group consisting of threonine, leucine and tyrosine. In one embodiment, the Fc polypeptide comprising a cavity comprises two or more import residues selected from the group consisting of alanine, serine, threonine and valine. In some embodiments, the Fc polypeptide comprising a cavity comprises replacement of two or more original amino acids selected from the group consisting of threonine, leucine and tyrosine, and wherein said original amino acids are replaced with import residues selected from the group consisting of alanine, serine, threonine and valine. In some embodiments, an original amino acid that is replaced is T366, L368 and/or Y407. In one embodiment, the Fc polypeptide comprising a cavity comprises replacement of threonine at position 366 with serine, amino acid numbering according to the EU numbering scheme of Kabat *et al. supra*. In one embodiment, the Fc polypeptide comprising a cavity comprises replacement of leucine at position 368 with alanine, amino acid numbering according to the EU numbering scheme of Kabat *et al. supra*. In one embodiment, the Fc polypeptide comprising a cavity comprises replacement of tyrosine at position 407 with valine, amino acid numbering according to the EU numbering scheme of Kabat *et al. supra*. In one embodiment, the Fc polypeptide comprising a cavity comprises two or more amino acid replacements selected from the group consisting of T366S, L368A and Y407V, amino acid numbering according to the EU numbering scheme of Kabat *et al. supra*. In some embodiments of these antibody fragments, the Fc polypeptide comprising the protuberance comprises replacement of threonine at position 366 with tryptophan, amino acid numbering according to the EU numbering scheme of Kabat *et al. supra*.

In one embodiment, the antibody comprises Fc mutations constituting "knobs" and "holes" as described in WO2005/063816. For example, a hole mutation can be one or more of T366S, L368A and/or Y407V in an Fc polypeptide, and a knob mutation can be T366W.

In one aspect of the invention, multispecific antibodies are purified, wherein the antibodies comprise a first half antibody and a second half antibody, wherein the first half-antibody comprises a first VH/VL unit that binds IL-17 and the second half antibody comprises a second VH/VL unit that binds IL-13. In some embodiments, the first half antibody does not bind IL-13, and wherein the second half antibody does not bind IL-17.

There are six members in the IL-17 family, IL-17A, B, C, D, E and F. Members of the IL-17 family form homodimers, and IL-17A and IL-17F further form heterodimer. See *e.g.*, Gaffen, S. L., 2009, Nature Review 9:556-567; Hymowitz et al., 2001, EMBO J. 20:5332-41 and WO2005/010044. IL-17 is sometimes used in the field to refer to the prototype of the IL-17 family member, IL-17A. In certain embodiments, the multispecific antibody provided herein binds and inhibits the activities of IL-13 and IL-17AA.

In certain particular embodiments, the multispecific antibody provided herein binds to IL-17AA, IL-17AF and IL-17FF, inhibits IL-17AA-, IL-17AF-, and IL-17FF - induced activity, and inhibits IL-13-induced activity. In certain embodiments, the anti-IL-13/IL-17 bispecific antibody refers to an anti-IL-13/IL-17AA, FF and AF antibody. In certain such embodiments, the anti-IL-13/IL-17AA, AF and FF bispecific antibody advantageously blocks activities induced by all of IL-17A and F cytokines as opposed to activities induced by IL-17A or IL-17F alone. In some embodiments, a multispecific antibody provided herein binds to IL-17AA, IL-17AF, and IL-17FF. In some embodiments, the IL-17AA-induced activity is IL-17AA-induced gene expression and/or proliferation of cells in vivo or in vitro. In some embodiments, the IL-17AF-induced activity is IL-17AF-induced gene expression and/or proliferation of cells in vivo or in vitro. In some such embodiments, the IL-17FF-induced activity is IL-17FF-induced gene expressing and/or proliferation of cells in vivo or in vitro. In some embodiments, the IL-13-induced activity is IL-13-induced gene expression and/or proliferation of cells in vivo or in vitro. In some embodiments, a multispecific antibody provided herein does not inhibit binding of IL-13 to IL-13Rα1.

In some embodiments, the anti-IL13/IL17AA AF FF bispecific antibody comprises a first half antibody and a second half antibody, wherein the first half-antibody comprises a first VH/VL unit that binds IL-17AA, AF and FF and the second half antibody comprises a second VH/VL unit that binds IL-13, wherein the first VH/VL unit comprises HVR-H1 comprising the amino acid sequence of SEQ ID NO: 20, HVR-H2 comprising the amino acid sequence of SEQ ID NO: 21, HVR-H3 comprising the amino acid sequence of SEQ ID NO: 22, HVR-L1 comprising the amino acid sequence of SEQ ID NO: 23, HVR-L2 comprising the amino acid sequence of SEQ ID NO: 24, and HVR-L3 comprising the amino acid sequence of SEQ ID NO: 25, and wherein the second VH/VL unit comprises HVR-H1 comprising the amino acid sequence of SEQ ID NO: 9, HVR-H2 comprising the amino acid sequence of SEQ ID NO: 10, HVR-H3 comprising the amino acid sequence of SEQ ID NO:11, HVR-L1 comprising the amino acid sequence of SEQ ID NO: 12, HVR-L2 comprising the amino acid sequence of SEQ ID NO: 13, and HVR-L3 comprising the amino acid sequence of SEQ ID NO: 14. In some embodiments, the first half antibody does not bind IL-13, and the second half antibody does not bind IL-17.

In some embodiments, the first VH/VL unit comprises a VH sequence having at least 95%, at least 96%, at least 97%, at least 98%, or at least 99% sequence identity to the amino acid sequence of SEQ ID NO: 18 and a VL sequence having at least 95%, at least 96%, at least 97%, at least 98%, or at least 99% sequence identity to the amino acid sequence of SEQ ID NO: 19, and wherein the second VH/VL unit comprises a VH sequence having at least 95%, at least 96%, at least 97%, at least 98%, or at least 99% sequence identity to the amino acid sequence of SEQ ID NO:7 and a VL sequence having at least 95%, at least 96%, at least 97%, at least 98%, or at least 99% sequence identity to the amino acid sequence of SEQ ID NO: 8. In some embodiments, a multispecific antibody is provided, wherein the first VH/VL unit comprises a VH sequence having the amino acid sequence of SEQ ID NO: 18 and a VL sequence having the amino acid sequence of SEQ ID NO: 19, and wherein the second VH/VL unit comprises a VH sequence having the amino acid sequence of SEQ ID NO:7 and a VL sequence having the amino acid sequence of SEQ ID NO:8.

In certain embodiments, the first half antibody binds IL17AA AF and FF comprising a heavy chain that comprises the amino acid sequence of SEQ ID NO:26 and a light chain that comprises the amino acid sequence of SEQ ID NO: 28, and the second half antibody binds IL13 comprising a heavy chain that comprises the amino acid sequence of SEQ ID NO: 15 and a light chain that comprises the amino acid sequence of SEQ ID NO: 17. In certain embodiments, the first half antibody binds IL17AA AF and FF comprising a heavy chain that comprises the amino acid sequence of SEQ ID NO:27 and a light chain that comprises the amino acid sequence of SEQ ID NO:28, and the second half antibody binds IL13 comprising a heavy chain that comprises the amino acid sequence of SEQ ID NO: 16 and a light chain that comprises the amino acid sequence of SEQ ID NO: 17. Sequences related to anti-IL13/anti-IL17 antibodies are presented in Table 3. See PCT/US2015/01716 (WO2015/127405).

Table 3

| SEQ ID NO: | Description | Sequence |
| --- | --- | --- |
| 4 | human IL-13 precursor (Swiss-Prot Accession No. P35225.2) | MALLLT  TVIALTCLGG  FASPGPVPPS  TALRELIEEL VNITQNQKAP  LCNGSMVWSI  NLTAGMYCAA  LESLINVSGC SAIEKTQRML  SGFCPHKVSA  GQFSSLHVRD  TKIEVAQFVK DLLLHLKKLF  REGRFN |
| 5 | human IL-13, mature form (without signal sequence) | SP  GPVPPSTALR  ELIEELVNIT  QNQKAPLCNG SMVWSINLTA  GMYCAALESL  INVSGCSAIE  KTQRMLSGFC PHKVSAGQFS  SLHVRDTKIE  VAQFVKDLLL  HLKKLFREGR FN |
| 6 | human IL-17A precursor (Swiss-Prot Accession No. Q16552.1) | MTPGKTSLVS  LLLLLLSLEAI  VKAGITIPRN  PGCPNSEDKN FPRTVMVNLN  IHNRNTNTNP  KRSSDYYNRS  TSPWNLHRNE DPERYPSVIW  EAKCRHLGCI  NADGNVDYHM  NSVPIQQEIL VLRREPPHCP  NSFRLEKILV  SVGCTCVTPI  VHHVA |
| 7 | human IL-17A, mature form (without signal sequence) | GITIPRN  PGCPNSEDKN  FPRTVMVNLN  IHNRNTNTNP KRSSDYYNRS  TSPWNLHRNE  DPERYPSVIW  EAKCRHLGCI NADGNVDYHM  NSVPIQQEIL  VLRREPPHCP  NSFRLEKILV SVGCTCVTPI  VHHVA |
| 8 | human IL-17F precursor (Swiss-Prot Accession No. Q96PD4.3) | MTVKTLHGPA  MVKYLLLSIL  GLAFLSEAAA  RKIPKVGHTF FQKPESCPPV  PGGSMKLDIG  IINENQRVSM  SRNIESRSTS PWNYTVTWDP  NRYPSEVVQA  QCRNLGCINA  QGKEDISMNS VPIQQETLVV  RRKHQGCSVS  FQLEKVLVTV  GCTCVTPVIH HVQ |
| 9 | human IL-17F, mature form (without signal sequence) | RKIPKVGHTF  FQKPESCPPV  PGGSMKLDIG  IINENQRVSM SRNIESRSTS  PWNYTVTWDP  NRYPSEVVQA  QCRNLGCINA QGKEDISMNS  VPIQQETLVV  RRKHQGCSVS  FQLEKVLVTV GCTCVTPVIH  HVQ |
| 10 | Anti-IL13 VH | EVTLRESGPA  LVKPTQTLTL  TCTVSGFSLS  AYSVNWIRQP PGKALEWLAM  IWGDGKIVYN  SALKSRLTIS  KDTSKNQVVL TMTNMDPVDT  ATYYCAGDGY  YPYAMDNWGQ  GSLVTVSS |
| 11 | Anti-IL13 VL | DIVLTQSPDS  LSVSLGERAT  INCRASKSVD  SYGNSFMHWY QQKPGQPPKL  LIYLASNLES  GVPDRFSGSG  SGTDFTLTIS SLQAEDVAVY  YCQQNNEDPR  TFGGGTKVEI  KR |
| 12 | Anti-IL13 HVRH1 | AYSVN |

(continued)

| SEQ ID NO: | Description | Sequence |
|---|---|---|
| 13 | Anti-IL13 HVRH2 | MIWGDGKIVYNSALKS |
| 14 | Anti-IL13 HVRH3 | DGYYPYAMDN |
| 15 | Anti-IL13 HVRL1 | RASKSVDSYGNSFMH |
| 16 | Anti-IL13 HVRL2 | LASNLES |
| 17 | Anti-IL13 HVRL3 | QQNNEDPRT |
| 18 | Anti-IL13 IgG4 heavy chain with knob T366W S228P in hinge Without C-ter K | EVTLRESGPA LVKPTQTLTL TCTVSGFSLS AYSVNWIRQP PGKALEWLAM IWGDGKIVYN SALKSRLTIS KDTSKNQVVL TMTNMDPVDT ATYYCAGDGY YPYAMDNWGQ GSLVTVSSAS TKGPSVFPLA PCSRSTSEST AALGCLVKDY FPEPVTVSWN SGALTSGVHT FPAVLQSSGL YSLSSVVTVP SSSLGTKTYT CNVDHKPSNT KVDKRVESKY GPPCPPCPAP EFLGGPSVFL FPPKPKDTLM ISRTPEVTCV VVDVSQEDPE VQFNWYVDGV EVHNAKTKPR EEQFNSTYRV VSVLTVLHQD WLNGKEYKCK VSNKGLPSSI EKTISKAKGQ PREPQVYTLP PSQEEMTKNQ VSLWCLVKGF YPSDIAVEWE SNGQPENNYK TTPPVLDSDG SFFLYSRLTV DKSRWQEGNV FSCSVMHEAL HNHYTQKSLS LSLG |
| 19 | Anti-IL13 IgG4 heavy chain with knob T366W S228P in hinge | EVTLRESGPA LVKPTQTLTL TCTVSGFSLS AYSVNWIRQP PGKALEWLAM IWGDGKIVYN SALKSRLTIS KDTSKNQVVL TMTNMDPVDT ATYYCAGDGY YPYAMDNWGQ GSLVTVSSAS TKGPSVFPLA PCSRSTSEST AALGCLVKDY FPEPVTVSWN SGALTSGVHT FPAVLQSSGL YSLSSVVTVP SSSLGTKTYT CNVDHKPSNT KVDKRVESKY GPPCPPCPAP EFLGGPSVFL FPPKPKDTLM ISRTPEVTCV VVDVSQEDPE VQFNWYVDGV EVHNAKTKPR EEQFNSTYRV VSVLTVLHQD WLNGKEYKCK VSNKGLPSSI EKTISKAKGQ PREPQVYTLP PSQEEMTKNQ VSLWCLVKGF YPSDIAVEWE SNGQPENNYK TTPPVLDSDG SFFLYSRLTV DKSRWQEGNV FSCSVMHEAL HNHYTQKSLS LSLGK |
| 20 | Anti-IL13 light chain | DIVLTQSPDS LSVSLGERAT INCRASKSVD SYGNSFMHWY QQKPGQPPKL LIYLASNLES GVPDRFSGSG SGTDFTLTIS SLQAEDVAVY YCQQNNEDPR TFGGGTKVEI KRTVAAPSVF IFPPSDEQLK SGTASVVCLL NNFYPREAKV QWKVDNALQS GNSQESVTEQ DSKDSTYSLS STLTLSKADY EKHKVYACEV THQGLSSPVT KSFNRGEC |
| 21 | Anti-IL17A and F cross reactive VH | EVQLVESGGG LVQPGRSLRL SCAASGFTFD DYAMHWVRQA PGKGLEWVSG INWSSGGIGY ADSVKGRFTI SRDNAKNSLY LQMNSLRAED TALYYCARDI GGFGEFYWNF GLWGRGTLVT VSS |
| 22 | Anti-IL17A and F cross reactive VL | EIVLTQSPAT LSLSPGERAT LSCRASQSVR SYLAWYQQKP GQAPRLLIYD ASNRATGIPA RFSGSGSGTD FTLTISSLEP EDFAVYYCQQ RSNWPPATFG GGTKVEIK |
| 23 | Anti-IL17A and F cross reactive HVR-H1 | DYAMH |
| 24 | Anti-IL17A and F cross reactive HVR-H2 | GINWSSGGIGYADSVKG |
| 25 | Anti-IL17A and F cross reactive HVR-H3 | DIGGFGEFYWNFGL |

(continued)

| SEQ ID NO: | Description | Sequence |
|---|---|---|
| 26 | Anti-IL17A and F cross reactive HVR-L1 | RASQSVRSYLA |
| 27 | Anti-IL17A and F cross reactive HVR-L2 | DASNRAT |
| 28 | Anti-IL17A and F cross reactive HVR-L3 | QQRSNWPPAT |
| 29 | Anti-IL17A and F cross reactive IgG4 heavy chain with hole T366S/ L368A/Y407V and hinge S228P Without C-ter K | EVQLVESGGG LVQPGRSLRL SCAASGFTFD DYAMHWVRQA PGKGLEWVSG INWSSGGIGY ADSVKGRFTI SRDNAKNSLY LQMNSLRAED TALYYCARDI GGFGEFYWNF GLWGRGTLVT VSSASTKGPS VFP LAPCSRSTSE STAALGCLVK DYFPEPVTVS WNSGALTSGV HTFPAVLQSS GLYSLSSVVT VPSSSLGTKT YTCNVDHKPS NTKVDKRVES KYGPPCPPCP APEFLGGPSV FLFPPKPKDT LMISRTPEVT CVVVDVSQED PEVQFNWYVD GVEVHNAKTK PREEQFNSTY RVVSVLTVLH QDWLNGKEYK CKVSNKGLPS SIEKTISKAK GQPREPQVYT LPPSQEEMTK NQVSLSCAVK GFYPSDIAVE WESNGQPENN YKTTPPVLDS DGSFFLVSRL TVDKSRWQEG NVFSCSVMHE ALHNHYTQKS LSLSLG |
| 30 | Anti-IL17A and F cross reactive IgG4 heavy chain with hole T366S/ L368A/Y407V and hinge S228P | EVQLVESGGG LVQPGRSLRL SCAASGFTFD DYAMHWVRQA PGKGLEWVSG INWSSGGIGY ADSVKGRFTI SRDNAKNSLY LQMNSLRAED TALYYCARDI GGFGEFYWNF GLWGRGTLVT VSSASTKGPS VFP LAPCSRSTSE STAALGCLVK DYFPEPVTVS WNSGALTSGV HTFPAVLQSS GLYSLSSVVT VPSSSLGTKT YTCNVDHKPS NTKVDKRVES KYGPPCPPCP APEFLGGPSV FLFPPKPKDT LMISRTPEVT CVVVDVSQED PEVQFNWYVD GVEVHNAKTK PREEQFNSTY RVVSVLTVLH QDWLNGKEYK CKVSNKGLPS SIEKTISKAK GQPREPQVYT LPPSQEEMTK NQVSLSCAVK GFYPSDIAVE WESNGQPENN YKTTPPVLDS DGSFFLVSRL TVDKSRWQEG NVFSCSVMHE ALHNHYTQKS LSLSLGK |
| 31 | Anti-IL17A and F cross reactive light chain | EIVLTQSPAT LSLSPGERAT LSCRASQSVR SYLAWYQQKP GQAPRLLIYD ASNRATGIPA RFSGSGSGTD FTLTISSLEP EDFAVYYCQQ RSNWPPATFG GGTKVEIKRT VAAPSVF IFPPSDEQLK SGTASVVCLL NNFYPREAKV QWKVDNALQS GNSQESVTEQ DSKDSTYSLS STLTLSKADY EKHKVYACEV THQGLSSPVT KSFNRGEC |
| 32 | Anti-IL13 VH coding sequence | GAAGTTACCC TGCGCGAGAG CGGCCCAGCC CTGGTGAAGC CAACCCAGAC CCTGACCCTG ACCTGCACCG TCAGCGGCTT CAGCCTGAGC GCCTACAGCG TGAACTGGAT CCGCCAGCCA CCAGGCAAGG CCCTGGAGTG GCTGGCCATG ATCTGGGGCG ACGGCAAGAT CGTGTACAAC AGCGCCCTGA AGAGCCGCCT GACCATCAGC AAGGACACCA GCAAGAACCA GGTGGTGCTG ACCATGACCA ACATGGACCC AGTGGACACC GCCACCTACT ACTGCGCCGG CGACGGCTAC TACCCATACG CCATGGACAA CTGGGGCCAG GGCAGCCTGG TGACCGTGAG CAGC |

(continued)

| SEQ ID NO: | Description | Sequence |
|---|---|---|
| 33 | Anti-IL13 VL coding sequence | GATATCGTGC TGACCCAGAG CCCAGACAGC CTGTCTGTGA GCCTGGGCGA GCGCGCCACC ATCAACTGCC GCGCCAGCAA AAGCGTGGAC AGCTACGGCA ACAGCTTCAT GCACTGGTAT CAGCAGAAGC CAGGCCAGCC ACCCAAGCTG CTGATCTACC TGGCCAGCAA CCTGGAGAGC GGCGTGCCAG ACCGCTTCAG CGGCAGCGGC AGCGGCACCG ACTTCACCCT GACCATCAGC TCTCTGCAGG CCGAGGATGT GGCCGTGTAC TACTGCCAGC AGAACAACGA GGACCCACGC ACCTTCGGTG GCGGTACCAA GGTGGAGATC AAA |
| 34 | Anti-IL13 IgG4 T366W/S228P heavy chain coding sequence, no C-terminal Lys | GAAGTTACCC TGCGCGAGAG CGGCCCAGCC CTGGTGAAGC CAACCCAGAC CCTGACCCTG ACCTGCACCG TCAGCGGCTT CAGCCTGAGC GCCTACAGCG TGAACTGGAT CCGCCAGCCA CCAGGCAAGG CCCTGGAGTG GCTGGCCATG ATCTGGGGCG ACGGCAAGAT CGTGTACAAC AGCGCCCTGA GAGCCGCCT GACCATCAGC AAGGACACCA GCAAGAACCA GGTGGTGCTG ACCATGACCA ACATGGACCC AGTGGACACC GCCACCTACT ACTGCGCCGG CGACGGCTAC TACCCATACG CCATGGACAA CTGGGGCCAG GGCAGCCTGG TGACCGTGAG CAGCGCTTCC ACCAAGGGCC CATCGGTCTT CCCCCTGGCA CCCTGCTCCC GCAGTACTTC TGAGTCCACA GCGGCCCTGG GCTGCCTGGT CAAGGACTAC TTCCCCGAAC CGGTGACGGT GTCGTGGAAC TCAGGCGCCC TGACCAGCGG CGTGCACACC TTCCCGGCTG TCCTACAGTC CTCAGGACTC TACTCCCTCA GCAGCGTGGT GACTGTGCCC TCTAGCAGCT TGGGCACCAA GACCTACACG TGCAACGTGG ATCACAAGCC CAGCAACACC AAGGTGGACA AACGCGTTGA GTCCAAATAT GGTCCCCCAT GCCCACCATG CCCAGCACCT GAGTTCCTGG GGGGACCATC AGTCTTCCTG TTCCCCCCAA AACCCAAGGA CACTCTCATG ATCTCCCGGA CCCCTGAGGT CACGTGCGTG GTGGTGGACG TGAGCCAGGA AGACCCCGAG GTCCAGTTCA ACTGGTACGT GGATGGCGTG GAGGTGCATA ATGCCAAGAC AAAGCCGCGG GAGGAGCAGT TCAACAGCAC GTACCGTGTG GTCAGCGTCC TCACCGTCCT GCACCAGGAC TGGCTGAACG GCAAGGAGTA CAAGTGCAAG GTCTCCAACA AAGGCCTCCC GTCCTCCATC GAGAAAACCA TCTCCAAAGC CAAAGGGCAG CCCCGAGAGC CACAGGTGTA CACCCTGCCC CCATCCCAGG AGGAGATGAC CAAGAACCAG GTCAGCCTGT GGTGCCTGGT CAAAGGCTTC TACCCCAGCG ACATCGCCGT GGAGTGGGAG AGCAATGGGC AGCCGGAGAA CAACTACAAG ACCACGCCTC CCGTGCTGGA CTCCGACGGC TCCTTCTTCC TCTACAGCAG GCTAACCGTG GACAAGAGCA GGTGGCAGGA GGGGAATGTC TTCTCATGCT CCGTGATGCA TGAGGCTCTG CACAACCACT ACACACAGAA GAGCCTCTCC CTGTCTCTGG GT |

(continued)

| SEQ ID NO: | Description | Sequence |
|---|---|---|
| 35 | Anti-IL13 IgG4 T366W/S228P heavy chain coding sequence | GAAGTTACCC TGCGCGAGAG CGGCCCAGCC CTGGTGAAGC CAACCCAGAC CCTGACCCTG ACCTGCACCG TCAGCGGCTT CAGCCTGAGC GCCTACAGCG TGAACTGGAT CCGCCAGCCA CCAGGCAAGG CCCTGGAGTG GCTGGCCATG ATCTGGGGCG ACGGCAAGAT CGTGTACAAC AGCGCCCTGA AGAGCCGCCT GACCATCAGC AAGGACACCA GCAAGAACCA GGTGGTGCTG ACCATGACCA ACATGGACCC AGTGGACACC GCCACCTACT ACTGCGCCGG CGACGGCTAC TACCCATACG CCATGGACAA CTGGGGCCAG GGCAGCCTGG TGACCGTGAG CAGCGCTTCC ACCAAGGGCC CATCGGTCTT CCCCCTGGCA CCCTGCTCCC GCAGTACTTC TGAGTCCACA GCGGCCCTGG GCTGCCTGGT CAAGGACTAC TTCCCCGAAC CGGTGACGGT GTCGTGGAAC TCAGGCGCCC TGACCAGCGG CGTGCACACC TTCCCGGCTG TCCTACAGTC CTCAGGACTC TACTCCCTCA GCAGCGTGGT GACTGTGCCC TCTAGCAGCT TGGGCACCAA GACCTACACG TGCAACGTGG ATCACAAGCC CAGCAACACC AAGGTGGACA AACGCGTTGA GTCCAAATAT GGTCCCCCAT GCCCACCATG CCCAGCACCT GAGTTCCTGG GGGGACCATC AGTCTTCCTG TTCCCCCCAA AACCCAAGGA CACTCTCATG ATCTCCCGGA CCCCTGAGGT CACGTGCGTG GTGGTGGACG TGAGCCAGGA AGACCCCGAG GTCCAGTTCA ACTGGTACGT GGATGGCGTG GAGGTGCATA ATGCCAAGAC AAAGCCGCGG GAGGAGCAGT TCAACAGCAC GTACCGTGTG GTCAGCGTCC TCACCGTCCT GCACCAGGAC TGGCTGAACG GCAAGGAGTA CAAGTGCAAG GTCTCCAACA AAGGCCTCCC GTCCTCCATC GAGAAAACCA TCTCCAAAGC CAAAGGGCAG CCCCGAGAGC CACAGGTGTA CACCCTGCCC CCATCCCAGG AGGAGATGAC CAAGAACCAG GTCAGCCTGT GGTGCCTGGT CAAAGGCTTC TACCCCAGCG ACATCGCCGT GGAGTGGGAG AGCAATGGGC AGCCGGAGAA CAACTACAAG ACCACGCCTC CCGTGCTGGA CTCCGACGGC TCCTTCTTCC TCTACAGCAG GCTAACCGTG GACAAGAGCA GGTGGCAGGA GGGGAATGTC TTCTCATGCT CCGTGATGCA TGAGGCTCTG CACAACCACT ACACACAGAA GAGCCTCTCC CTGTCTCTGG GTAAATAA |
| 36 | Anti-IL13 light chain coding sequence | GATATCGTGC TGACCCAGAG CCCAGACAGC CTGTCTGTGA GCCTGGGCGA GCGCGCCACC ATCAACTGCC GCGCCAGCAA AAGCGTGGAC AGCTACGGCA ACAGCTTCAT GCACTGGTAT CAGCAGAAGC CAGGCCAGCC ACCCAAGCTG CTGATCTACC TGGCCAGCAA CCTGGAGAGC GGCGTGCCAG ACCGCTTCAG CGGCAGCGGC AGCGGCACCG ACTTCACCCT GACCATCAGC TCTCTGCAGG CCGAGGATGT GGCCGTGTAC TACTGCCAGC AGAACAACGA GGACCCACGC ACCTTCGGTG GCGGTACCAA GGTGGAGATC AAACGAACTG TGGCTGCACC ATCTGTCTTC ATCTTCCCGC CATCTGATGA GCAGTTGAAA TCTGGAACTG CTTCTGTTGT GTGCCTGCTG AATAACTTCT ATCCCCGTGA GGCCAAAGTA CAGTGGAAGG TGGATAACGC CCTCCAATCG GGTAACTCCC AGGAGAGTGT CACAGAGCAG GACAGCAAGG ACAGCACCTA CAGCCTCAGC AGCACCCTGA CGCTGAGCAA AGCAGACTAC GAGAAACACA AAGTCTACGC CTGCGAAGTC ACCCATCAGG GCCTGAGCTC GCCCGTCACA AAGAGCTTCA ACAGGGGAGA GTGTTAA |

(continued)

| SEQ ID NO: | Description | Sequence |
|---|---|---|
| 37 | Anti-IL17A and F cross reactive VH coding sequence | GAAGTGCAGC TGGTGGAGTC TGGGGGAGGC TTGGTACAGC CTGGCAGGTC CCTGAGACTC TCCTGTGCAG CCTCTGGATT CACCTTTGAT GATTATGCCA TGCACTGGGT CCGGCAAGCT CCAGGGAAGG GCCTGGAGTG GGTCTCAGGT ATTAATTGGA GCAGTGGTGG CATAGGCTAT GCGGACTCTG TGAAGGGCCG ATTCACCATC TCCAGAGACA ACGCCAAGAA CTCCCTGTAT CTGCAAATGA ACAGTCTGAG AGCTGAGGAC ACGGCCTTGT ATTACTGTGC AAGAGATATC GGGGGGTTCG GGGAGTTTTA CTGGAACTTC GGTCTCTGGG GCCGTGGCAC CCTGGTCACT GTCTCCTCA |
| 38 | Anti-IL17A and F cross reactive VL coding sequence | GAAATTGTGT TGACACAGTC TCCAGCCACC CTGTCTTTGT CTCCAGGGGA AAGAGCCACC CTCTCCTGCA GGGCCAGTCA GAGTGTTAGA AGCTACTTAG CCTGGTACCA ACAGAAACCT GGCCAGGCTC CCAGGCTCCT CATCTATGAT GCATCCAACA GGGCCACTGG CATCCCAGCC AGGTTCAGTG GCAGTGGGTC TGGGACAGAC TTCACTCTCA CCATCAGCAG CCTAGAGCCT GAAGATTTTG CAGTTTATTA CTGTCAGCAG CGTAGCAACT GGCCTCCGGC CACTTTCGGC GGAGGGACCA AGGTGGAGAT CAAA |
| 39 | Anti-IL17A and F cross reactive IgG4 T366S/ L368A/Y407V/ S228P heavy chain coding sequence, no C-terminal Lys | GAAGTGCAGC TGGTGGAGTC TGGGGGAGGC TTGGTACAGC CTGGCAGGTC CCTGAGACTC TCCTGTGCAG CCTCTGGATT CACCTTTGAT GATTATGCCA TGCACTGGGT CCGGCAAGCT CCAGGGAAGG GCCTGGAGTG GGTCTCAGGT ATTAATTGGA GCAGTGGTGG CATAGGCTAT GCGGACTCTG TGAAGGGCCG ATTCACCATC TCCAGAGACA ACGCCAAGAA CTCCCTGTAT CTGCAAATGA ACAGTCTGAG AGCTGAGGAC ACGGCCTTGT ATTACTGTGC AAGAGATATC GGGGGGTTCG GGGAGTTTTA CTGGAACTTC GGTCTCTGGG GCCGTGGCAC CCTGGTCACT GTCTCCTCAG CCTCCACCAA GGGCCCATCG GTCTTCCCCC TGGCACCCTG CTCCCGCAGT ACTTCTGAGT CCACAGCGGC CCTGGGCTGC CTGGTCAAGG ACTACTTCCC CGAACCGGTG ACGGTGTCGT GGAACTCAGG CGCCCTGACC AGCGGCGTGC ACACCTTCCC GGCTGTCCTA CAGTCCTCAG GACTCTACTC CCTCAGCAGC GTGGTGACTG TGCCCTCTAG CAGCTTGGGC ACCAAGACCT ACACGTGCAA CGTGGATCAC AAGCCCAGCA ACACCAAGGT GGACAAACGC GTTGAGTCCA AATATGGTCC CCCATGCCCA CCATGCCCAG CACCTGAGTT CCTGGGGGGA CCATCAGTCT TCCTGTTCCC CCCAAAACCC AAGGACACTC TCATGATCTC CCGGACCCCT GAGGTCACGT GCGTGGTGGT GGACGTGAGC CAGGAAGACC CCGAGGTCCA GTTCAACTGG TACGTGGATG GCGTGGAGGT GCATAATGCC AAGACAAAGC CGCGGGAGGA GCAGTTCAAC AGCACGTACC GTGTGGTCAG CGTCCTCACC GTCCTGCACC AGGACTGGCT GAACGGCAAG GAGTACAAGT GCAAGGTCTC CAACAAAGGC CTCCCGTCCT CCATCGAGAA AACCATCTCC AAAGCCAAAG GGCAGCCCCG AGAGCCACAG GTGTACACCC TGCCCCCATC CCAGGAGGAG ATGACCAAGA ACCAGGTCAG CCTGAGCTGC CTGGTCAAAG GCTTCTACCC CAGCGACATC GCCGTGGAGT GGGAGAGCAA TGGGCAGCCG GAGAACAACT ACAAGACCAC GCCTCCCGTG CTGGACTCCG ACGGCTCCTT CTTCCTCGTT AGCAGGCTAA CCGTGGACAA GAGCAGGTGG CAGGAGGGGA ATGTCTTCTC ATGCTCCGTG ATGCATGAGG CTCTGCACAA CCACTACACA CAGAAGAGCC TCTCCCTGTC TCTGGGT |

(continued)

| SEQ ID NO: | Description | Sequence |
|---|---|---|
| 40 | Anti-IL17A and F cross reactive IgG4 T366S/ L368A/Y407V/ S228P heavy chain coding sequence | GAAGTGCAGC TGGTGGAGTC TGGGGGAGGC TTGGTACAGC<br>CTGGCAGGTC CCTGAGACTC TCCTGTGCAG CCTCTGGATT<br>CACCTTTGAT GATTATGCCA TGCACTGGGT CCGGCAAGCT<br>CCAGGGAAGG GCCTGGAGTG GGTCTCAGGT ATTAATTGGA<br>GCAGTGGTGG CATAGGCTAT GCGGACTCTG TGAAGGGCCG<br>ATTCACCATC TCCAGAGACA ACGCCAAGAA CTCCCTGTAT<br>CTGCAAATGA ACAGTCTGAG AGCTGAGGAC ACGGCCTTGT<br>ATTACTGTGC AAGAGATATC GGGGGGTTCG GGGAGTTTTA<br>CTGGAACTTC GGTCTCTGGG GCCGTGGCAC CCTGGTCACT<br>GTCTCCTCAG CCTCCACCAA GGGCCCATCG GTCTTCCCCC<br>TGGCACCCTG CTCCCGCAGT ACTTCTGAGT CCACAGCGGC<br>CCTGGGCTGC CTGGTCAAGG ACTACTTCCC CGAACCGGTG<br>ACGGTGTCGT GGAACTCAGG CGCCCTGACC AGCGGCGTGC<br>ACACCTTCCC GGCTGTCCTA CAGTCCTCAG GACTCTACTC<br>CCTCAGCAGC GTGGTGACTG TGCCCTCTAG CAGCTTGGGC<br>ACCAAGACCT ACACGTGCAA CGTGGATCAC AAGCCCAGCA<br>ACACCAAGGT GGACAAACGC GTTGAGTCCA AATATGGTCC<br>CCCATGCCCA CCATGCCCAG CACCTGAGTT CCTGGGGGGA<br>CCATCAGTCT TCCTGTTCCC CCCAAAACCC AAGGACACTC<br>TCATGATCTC CCGGACCCCT GAGGTCACGT GCGTGGTGGT<br>GGACGTGAGC CAGGAAGACC CCGAGGTCCA GTTCAACTGG<br>TACGTGGATG GCGTGGAGGT GCATAATGCC AAGACAAAGC<br>CGCGGGAGGA GCAGTTCAAC AGCACGTACC GTGTGGTCAG<br>CGTCCTCACC GTCCTGCACC AGGACTGGCT GAACGGCAAG<br>GAGTACAAGT GCAAGGTCTC CAACAAAGGC CTCCCGTCCT<br>CCATCGAGAA AACCATCTCC AAAGCCAAAG GGCAGCCCCG<br>AGAGCCACAG GTGTACACCC TGCCCCCATC CCAGGAGGAG<br>ATGACCAAGA ACCAGGTCAG CCTGAGCTGC GCTGTCAAAG<br>GCTTCTACCC CAGCGACATC GCCGTGGAGT GGGAGAGCAA<br>TGGGCAGCCG GAGAACAACT ACAAGACCAC GCCTCCCGTG<br>CTGGACTCCG ACGGCTCCTT CTTCCTCGTT AGCAGGCTAA<br>CCGTGGACAA GAGCAGGTGG CAGGAGGGGA ATGTCTTCTC<br>ATGCTCCGTG ATGCATGAGG CTCTGCACAA CCACTACACA<br>CAGAAGAGCC TCTCCCTGTC TCTGGGTAAA TAA |
| 41 | Anti-IL17A F cross reactive light chain coding sequence | GAAATTGTGT TGACACAGTC TCCAGCCACC CTGTCTTTGT<br>CTCCAGGGGA AAGAGCCACC CTCTCCTGCA GGGCCAGTCA<br>GAGTGTTAGA AGCTACTTAG CCTGGTACCA ACAGAAACCT<br>GGCCAGGCTC CCAGGCTCCT CATCTATGAT GCATCCAACA<br>GGGCCACTGG CATCCCAGCC AGGTTCAGTG GCAGTGGGTC<br>TGGGACAGAC TTCACTCTCA CCATCAGCAG CCTAGAGCCT<br>GAAGATTTTG CAGTTTATTA CTGTCAGCAG CGTAGCAACT<br>GGCCTCCGGC CACTTTCGGC GGAGGGACCA AGGTGGAGAT<br>CAAACGAACT GTGGCTGCAC CATCTGTCTT CATCTTCCCG<br>CCATCTGATG AGCAGTTGAA ATCTGGAACT GCTTCTGTTG<br>TGTGCCTGCT GAATAACTTC TATCCCCGTG AGGCCAAAGT<br>ACAGTGGAAG GTGGATAACG CCCTCCAATC GGGTAACTCC<br>CAGGAGAGTG TCACAGAGCA GGACAGCAAG GACAGCACCT<br>ACAGCCTCAG CAGCACCCTG ACGCTGAGCA AAGCAGACTA<br>CGAGAAACAC AAAGTCTACG CCTGCGAAGT CACCCATCAG<br>GGCCTGAGCT CGCCCGTCAC AAAGAGCTTC AACAGGGGAG<br>AGTGTTAA |

(continued)

| SEQ ID NO: | Description | Sequence |
|---|---|---|
| 42 | Anti-IL17A and F cross reactive IgG4 T366S/ L368A/Y407V/ S228P heavy chain codon optimized coding sequence, no C-terminal Lys | GAAGTTCAGC TGGTTGAAAG CGGTGGTGGT CTGGTTCAGC CTGGTCGTAG CCTGCGTCTG AGCTGTGCAG CAAGCGGTTT TACCTTTGAT GATTATGCCA TGCATTGGGT TCGTCAGGCA CCGGGTAAAG GTCTGGAATG GGTTAGCGGT ATTAATTGGA GCAGCGGTGG TATTGGTTAT GCAGATAGCG TTAAAGGTCG TTTTACCATT AGCCGTGATA ATGCCAAAAA TAGCCTGTAC CTGCAGATGA ATAGTCTGCG TGCAGAAGAT ACCGCACTGT ATTATTGTGC ACGTGATATT GGTGGTTTTG GCGAATTCTA TTGGAATTTT GGTCTGTGGG GTCGTGGCAC CCTGGTTACC GTTAGCAGCG CCTCCACCAA GGGCCCATCG GTCTTCCCCC TGGCACCCTG CTCCCGCAGT ACTTCTGAGT CCACAGCGGC CCTGGGCTGC CTGGTCAAGG ACTACTTCCC CGAACCGGTG ACGGTGTCGT GGAACTCAGG CGCCCTGACC AGCGGCGTGC ACACCTTCCC GGCTGTCCTA CAGTCCTCAG GACTCTACTC CCTCAGCAGC GTGGTGACTG TGCCCTCTAG CAGCTTGGGC ACCAAGACCT ACACGTGCAA CGTGGATCAC AAGCCCAGCA ACACCAAGGT GGACAAACGC GTTGAGTCCA AATATGGTCC CCCATGCCCA CCATGCCCAG CACCTGAGTT CCTGGGGGGA CCATCAGTCT TCCTGTTCCC CCCAAAACCC AAGGACACTC TCATGATCTC CCGGACCCCT GAGGTCACGT GCGTGGTGGT GGACGTGAGC CAGGAAGACC CCGAGGTCCA GTTCAACTGG TACGTGGATG GCGTGGAGGT GCATAATGCC AAGACAAAGC CGCGGGAGGA GCAGTTCAAC AGCACGTACC GTGTGGTCAG CGTCCTCACC GTCCTGCACC AGGACTGGCT GAACGGCAAG GAGTACAAGT GCAAGGTCTC CAACAAAGGC CTCCCGTCCT CCATCGAGAA AACCATCTCC AAAGCCAAAG GGCAGCCCCG AGAGCCACAG GTGTACACCC TGCCCCCATC CCAGGAGGAG ATGACCAAGA ACCAGGTCAG CCTGAGCTGC GCTGTCAAAG GCTTCTACCC CAGCGACATC GCCGTGGAGT GGGAGAGCAA TGGGCAGCCG GAGAACAACT ACAAGACCAC GCCTCCCGTG CTGGACTCCG ACGGCTCCTT CTTCCTCGTT AGCAGGCTAA CCGTGGACAA GAGCAGGTGG CAGGAGGGGA ATGTCTTCTC ATGCTCCGTG ATGCATGAGG CTCTGCACAA CCACTACACA CAGAAGAGCC TCTCCCTGTC TCTGGGT |

(continued)

| SEQ ID NO: | Description | Sequence |
|---|---|---|
| 43 | Anti-IL17A and F cross reactive IgG4 T366S/ L368A/Y407V/ S228P heavy chain codon optimized coding sequence | GAAGTTCAGC TGGTTGAAAG CGGTGGTGGT CTGGTTCAGC CTGGTCGTAG CCTGCGTCTG AGCTGTGCAG CAAGCGGTTT TACCTTTGAT GATTATGCCA TGCATTGGGT TCGTCAGGCA CCGGGTAAAG GTCTGGAATG GGTTAGCGGT ATTAATTGGA GCAGCGGTGG TATTGGTTAT GCAGATAGCG TTAAAGGTCG TTTTACCATT AGCCGTGATA ATGCCAAAAA TAGCCTGTAC CTGCAGATGA ATAGTCTGCG TGCAGAAGAT ACCGCACTGT ATTATTGTGC ACGTGATATT GGTGGTTTTG GCGAATTCTA TTGGAATTTT GGTCTGTGGG GTCGTGGCAC CCTGGTTACC GTTAGCAGCG CCTCCACCAA GGGCCCATCG GTCTTCCCCC TGGCACCCTG CTCCCGCAGT ACTTCTGAGT CCACAGCGGC CCTGGGCTGC CTGGTCAAGG ACTACTTCCC CGAACCGGTG ACGGTGTCGT GGAACTCAGG CGCCCTGACC AGCGGCGTGC ACACCTTCCC GGCTGTCCTA CAGTCCTCAG GACTCTACTC CCTCAGCAGC GTGGTGACTG TGCCCTCTAG CAGCTTGGGC ACCAAGACCT ACACGTGCAA CGTGGATCAC AAGCCCAGCA ACACCAAGGT GGACAAACGC GTTGAGTCCA AATATGGTCC CCCATGCCCA CCATGCCCAG CACCTGAGTT CCTGGGGGGA CCATCAGTCT TCCTGTTCCC CCCAAAACCC AAGGACACTC TCATGATCTC CCGGACCCCT GAGGTCACGT GCGTGGTGGT GGACGTGAGC CAGGAAGACC CCGAGGTCCA GTTCAACTGG TACGTGGATG GCGTGGAGGT GCATAATGCC AAGACAAAGC CGCGGGAGGA GCAGTTCAAC AGCACGTACC GTGTGGTCAG CGTCCTCACC GTCCTGCACC AGGACTGGCT GAACGGCAAG GAGTACAAGT GCAAGGTCTC CAACAAAGGC CTCCCGTCCT CCATCGAGAA AACCATCTCC AAAGCCAAAG GGCAGCCCCG AGAGCCACAG GTGTACACCC TGCCCCCATC CCAGGAGGAG ATGACCAAGA ACCAGGTCAG CCTGAGCTGC GCTGTCAAAG GCTTCTACCC CAGCGACATC GCCGTGGAGT GGGAGAGCAA TGGGCAGCCG GAGAACAACT ACAAGACCAC GCCTCCCGTG CTGGACTCCG ACGGCTCCTT CTTCCTCGTT AGCAGGCTAA CCGTGGACAA GAGCAGGTGG CAGGAGGGGA ATGTCTTCTC ATGCTCCGTG ATGCATGAGG CTCTGCACAA CCACTACACA CAGAAGAGCC TCTCCCTGTC TCTGGGTAAA TAA |
| 44 | Anti-IL17A and F cross reactive light chain codon optimized coding sequence | GAAATTGTTC TGACCCAGAG TCCGGCAACC CTGAGCCTGA GTCCGGGTGA ACGTGCCACC CTGAGCTGTC GTGCAAGCCA GAGCGTTCGT AGCTATCTGG CATGGTATCA GCAGAAACCG GGTCAGGCAC CGCGTCTGCT GATTTATGAT GCAAGCAATC GTGCAACCGG TATTCCGGCA CGTTTTAGCG GTAGCGGTAG TGGCACCGAT TTTACCCTGA CCATTAGCAG CCTGGAACCG GAAGATTTTG CAGTGTATTA TTGTCAGCAG CGTAGCAATT GGCCACCGGC AACCTTTGGT GGTGGCACCA AAGTTGAAAT TAAACGAACT GTGGCTGCAC CATCTGTCTT CATCTTCCCG CCATCTGATG AGCAGTTGAA ATCTGGAACT GCTTCTGTTG TGTGCCTGCT GAATAACTTC TATCCCGTG AGGCCAAAGT ACAGTGGAAG GTGGATAACG CCCTCCAATC GGGTAACTCC CAGGAGAGTG TCACAGAGCA GGACAGCAAG GACAGCACCT ACAGCCTCAG CAGCACCCTG ACGCTGAGCA AAGCAGACTA CGAGAAACAC AAAGTCTACG CCTGCGAAGT CACCCATCAG GGCCTGAGCT CGCCCGTCAC AAAGAGCTTC AACAGGGGAG AGTGTTAA |

V. Vectors, Host Cells, and Recombinant Methods

For recombinant production of a heterologous polypeptide (*e.g.*, a multispecific antibody) using FkpA, the nucleic acid encoding the polypeptide (*e.g.*, multispecific antibody) is isolated and inserted into a replicable vector for further cloning (amplification of the DNA) or for expression. DNA encoding the polypeptide (*e.g.*, multispecific antibody) is readily isolated and sequenced using conventional procedures (*e.g.*, by using oligonucleotide probes that are capable of binding specifically to genes encoding the heavy and light chains of the antibody). Many vectors are available. The choice of vector depends in part on the host cell to be used. Generally, preferred host cells are of either prokaryotic origin. It will be appreciated that constant regions of any isotype can be used for this purpose, including IgG, IgM, IgA, IgD, and IgE constant regions, and that such constant regions can be obtained from any human or animal species.

A. Generating antibodies using prokaryotic host cells

i. Vector Construction

Polynucleotide sequences encoding polypeptide components of the multispecific antibody of the invention can be obtained using standard recombinant techniques. Desired polynucleotide sequences may be isolated and sequenced from antibody producing cells such as hybridoma cells. Alternatively, polynucleotides can be synthesized using nucleotide synthesizer or PCR techniques. Once obtained, sequences encoding the polypeptides are inserted into a recombinant vector capable of replicating and expressing heterologous polynucleotides in prokaryotic hosts. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication, a selection marker gene, a promoter, a ribosome binding site (RBS), a signal sequence, the heterologous nucleic acid insert and a transcription termination sequence.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, *E. coli* is typically transformed using pBR322, a plasmid derived from an *E. coli* species. pBR322 contains genes encoding ampicillin (Amp) and tetracycline (Tet) resistance and thus provides easy means for identifying transformed cells. pBR322, its derivatives, or other microbial plasmids or bacteriophage may also contain, or be modified to contain, promoters which can be used by the microbial organism for expression of endogenous proteins. Examples of pBR322 derivatives used for expression of particular antibodies are described in detail in Carter et al., U.S. Patent No. 5,648,237.

In addition, phage vectors containing replicon and control sequences that are compatible with the host microorganism can be used as transforming vectors in connection with these hosts. For example, bacteriophage such as GEM™-11 may be utilized in making a recombinant vector which can be used to transform susceptible host cells such as *E. coli* LE392.

The expression vector of the invention may comprise two or more promoter-cistron pairs, encoding each of the polypeptide components. A promoter is an untranslated regulatory sequence located upstream (5') to a cistron that modulates its expression. Prokaryotic promoters typically fall into two classes, inducible and constitutive. Inducible promoter is a promoter that initiates increased levels of transcription of the cistron under its control in response to changes in the culture condition, *e.g.,* the presence or absence of a nutrient or a change in temperature.

A large number of promoters recognized by a variety of potential host cells are well known. The selected promoter can be operably linked to cistron DNA encoding the light or heavy chain by removing the promoter from the source DNA via restriction enzyme digestion and inserting the isolated promoter sequence into the vector of the invention. Both the native promoter sequence and many heterologous promoters may be used to direct amplification and/or expression of the target genes. In some embodiments, heterologous promoters are utilized, as they generally permit greater transcription and higher yields of expressed target gene as compared to the native target polypeptide promoter.

Promoters suitable for use with prokaryotic hosts include the PhoA promoter, the -lactamase and lactose promoter systems, a tryptophan (trp) promoter system and hybrid promoters such as the tac or the trc promoter. However, other promoters that are functional in bacteria (such as other known bacterial or phage promoters) are suitable as well. Their nucleotide sequences have been published, thereby enabling a skilled worker operably to ligate them to cistrons encoding the target light and heavy chains (Siebenlist et al., (1980) Cell 20: 269) using linkers or adaptors to supply any required restriction sites.

The translational initiation region (TIR) is a major determinant of the overall translation level of a protein. The TIR includes the polynucleotide that encodes the signal sequence, and extends from immediately upstream of the Shine-Delgarno sequence to approximately twenty nucleotides downstream of the initiation codon. Generally, the vector will comprise a TIR, TIRs and variant TIRs are known in the art and methods for generating TIRs are known in in the art A series of nucleic acid sequence variants can be created with a range of translational strengths, thereby providing a convenient means by which to adjust this factor for the optimal secretion of many different polypeptides. The use of a reporter gene fused to these variants, such as PhoA, provides a method to quantitate the relative translational strengths of different translation initiation regions. The variant or mutant TIRs can be provided in the background of a plasmid vector thereby providing a set of plasmids into which a gene of interest may be inserted and its expression measured, so as to establish an optimum range of translational strengths for maximal expression of mature polypeptide. Variant TIRs are disclosed in USP 8,241,901.

In one aspect of the invention, each cistron within the recombinant vector comprises a secretion signal sequence component that directs translocation of the expressed polypeptides across a membrane. In general, the signal sequence may be a component of the vector, or it may be a part of the target polypeptide DNA that is inserted into the vector. The signal sequence selected for the purpose of this invention should be one that is recognized and processed (*i.e.*, cleaved by a signal peptidase) by the host cell. For prokaryotic host cells that do not recognize and process the signal sequences native to the heterologous polypeptides, the signal sequence is substituted by a prokaryotic signal sequence selected, for example, from the signal polypeptides of the present invention. In addition, the vector may comprise a signal sequence selected from the group consisting of alkaline phosphatase, penicillinase, Lpp, or heat-stable enterotoxin II (STII) leaders, LamB, PhoE, PelB, OmpA, and MBP.

In one aspect, one or more polynucleotides (*e.g.*, expression vectors) collectively encode an antibody. In one embodiment, a single polynucleotide encodes the light chain of the antibody and a separate polynucleotide encodes the heavy chain of the antibody. In one embodiment, a single polynucleotide encodes the light chain and heavy chain of the antibody. In some embodiments, one or more polynucleotides (*e.g.*, expression vectors) collectively encode a one-armed antibody. In one embodiment, a single polynucleotide encodes (a) the light and heavy chain of the one armed antibody, and (b) the Fc polypeptide. In one embodiment, a single polynucleotide encodes the light and heavy chain of the one armed antibody, and a separate polynucleotide encodes the Fc polypeptide. In one embodiment, separate polynucleotides encode the light chain component of the one-armed antibody, the heavy chain component of the one-armed antibody and the Fc polypeptide, respectively. Production of a one-armed antibody is described in, for example, in WO2005063816.

Prokaryotic host cells suitable for expressing antibodies of the invention include Archaebacteria and Eubacteria, such as Gram-negative or Gram-positive organisms. Examples of useful bacteria include Escherichia (*e.g.*, *E. coli*), Bacilli (*e.g., B. subtilis*), *Enterobacteria*, *Pseudomonas* species (*e.g.*, *P. aeruginosa*), *Salmonella typhimurium, Serratia marcescans,* Klebsiella, Proteus, Shigella, Rhizobia, Vitreoscilla, or Paracoccus. In one embodiment, gram-negative cells are used. In one embodiment, *E. coli* cells are used as hosts for the invention. Examples of *E. coli* strains include strain W3110 (Bachmann, Cellular and Molecular Biology, vol. 2 (Washington, D.C.: American Society for Microbiology, 1987), pp. 1190-1219; ATCC Deposit No. 27,325) and derivatives thereof, including strain 33D3 having genotype W3110 ΔfhuA (ΔtonA) ptr3 lac Iq lacL8 ΔompTΔ (nmpc-fepE) degP41 kanR (U.S. Pat. No. 5,639,635) and strains 63C1 and 64B4. In some embodiment, the *E. coli* strain is a W3110 derivative named 62A7 (ΔfhuA (ΔtonA) ptr3, lacIq, lacL8, ompTΔ(nmpc-fepE) ΔdegP ilvG repaired). Other strains and derivatives thereof, such as *E. coli* 294 (ATCC 31,446), *E. coli* B, *E. coli* λ 1776 (ATCC 31,537) and *E. coli* RV308(ATCC 31,608) are also suitable. These examples are illustrative rather than limiting. Methods for constructing derivatives of any of the above-mentioned bacteria having defined genotypes are known in the art and described in, for example, Bass et al., Proteins, 8:309-314 (1990). It is generally necessary to select the appropriate bacteria taking into consideration replicability of the replicon in the cells of a bacterium. For example, *E. coli,* Serratia, or Salmonella species can be suitably used as the host when well known plasmids such as pBR322, pBR325, pACYC177, or pKN410 are used to supply the replicon. Typically the host cell should secrete minimal amounts of proteolytic enzymes, and additional protease inhibitors may desirably be incorporated in the cell culture.

To improve the production yield and quality of the polypeptides in bacterial cultures, the bacterial cells can be modified. For example, to improve the proper assembly and folding of the secreted antibody polypeptides, the bacteria host cell may comprise additional vectors expressing chaperone proteins, such as FkpA and Dsb proteins (DsbB, DsbC, DsbD, and/or DsbG) can be used to co-transform the host prokaryotic cells. The chaperone proteins have been demonstrated to facilitate the proper folding and solubility of heterologous proteins produced in bacterial host cells.

ii. Antibody Production

Host cells are transformed with the above-described expression vectors and cultured in conventional nutrient media modified as appropriate for inducing promoters, selecting transformants, or amplifying the genes encoding the desired sequences.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cell-wall barriers. Another method for transformation employs polyethylene glycol/DMSO. Yet another technique used is electroporation.

Prokaryotic cells used to produce the polypeptides of the invention are grown in media known in the art and suitable for culture of the selected host cells. Examples of suitable media include Luria broth (LB) plus necessary nutrient supplements. In some embodiments, the media also contains a selection agent, chosen based on the construction of the expression vector, to selectively permit growth of prokaryotic cells containing the expression vector. For example, ampicillin is added to media for growth of cells expressing ampicillin resistant gene.

Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithioerythritol and dithiothreitol.

The prokaryotic host cells are cultured at suitable temperatures. For *E. coli* growth, for example, the preferred temperature ranges from about 20 °C to about 39 °C, more preferably from about 25 °C to about 37 °C, even more preferably at about 30 °C. The pH of the medium may be any pH ranging from about 5 to about 9, depending mainly on the host organism. For *E. coli,* the pH is preferably from about 6.8 to about 7.4, and more preferably about 7.0.

If an inducible promoter is used in the expression vector of the invention, protein expression is induced under conditions suitable for the activation of the promoter. In one aspect of the invention, PhoA promoters are used for controlling transcription of the polypeptides. Accordingly, the transformed host cells are cultured in a phosphate-limiting medium for induction. Preferably, the phosphate-limiting medium is the C.R.A.P medium (see, *e.g.*, Simmons et al., J. Immunol. Methods (2002), 263: 133-147) or media described in WO2002/061090. A variety of other inducers may be used, according to the vector construct employed, as is known in the art.

In one embodiment, the expressed polypeptides of the present invention are secreted into and recovered from the periplasm of the host cells. Protein recovery typically involves disrupting the microorganism, generally by such means as osmotic shock, sonication or lysis. Once cells are disrupted, cell debris or whole cells may be removed by centrifugation or filtration. The proteins may be further purified, for example, by affinity resin chromatography. Alternatively, proteins can be transported into the culture media and isolated therein. Cells may be removed from the culture and the culture supernatant being filtered and concentrated for further purification of the proteins produced. The expressed polypeptides can be further isolated and identified using commonly known methods such as polyacrylamide gel electrophoresis (PAGE) and Western blot assay.

In one aspect of the invention, antibody production is conducted in large quantity by a fermentation process. Various large-scale fed-batch fermentation procedures are available for production of recombinant polypeptides. Large-scale fermentations have at least 1000 liters of capacity, preferably about 1,000 to 100,000 liters of capacity. These fermentors use agitator impellers to distribute oxygen and nutrients, especially glucose (the preferred carbon/energy source). Small scale fermentation refers generally to fermentation in a fermenter that is no more than approximately 100 liters in volumetric capacity, and can range from about 1 liter to about 100 liters.

In a fermentation process, induction of protein expression is typically initiated after the cells have been grown under suitable conditions to a desired density, *e.g.,* an OD550 of about 180-220, at which stage the cells are in the early stationary phase. A variety of inducers may be used, according to the vector construct employed, as is known in the art and described above. Cells may be grown for shorter periods prior to induction. Cells are usually induced for about 12-50 hours, although longer or shorter induction time may be used.

To improve the production yield and quality of the polypeptides of the invention, various fermentation conditions can be modified. For example, to improve the proper assembly and folding of the secreted antibody polypeptides, additional vectors expressing chaperone proteins, such as FkpA, DsbA and/or DsbC can be used to co-transform the host prokaryotic cells. The chaperone proteins have been demonstrated to facilitate the proper folding and solubility of heterologous proteins produced in bacterial host cells. In some embodiments, FkpA, DsbA and/or DsbC are expressed in the bacterial host cell.

To minimize proteolysis of expressed heterologous proteins (especially those that are proteolytically sensitive), certain host strains deficient for proteolytic enzymes can be used for the present invention. For example, host cell strains may be modified to effect genetic mutation(s) in the genes encoding known bacterial proteases such as Protease III, OmpT, DegP, Tsp, Protease I, Protease Mi, Protease V, Protease VI, and combinations thereof. Some *E. coli* protease-deficient strains are available and described in, for example, Joly *et al.*, (1998), *supra;* Georgiou et al., U.S. Patent No. 5,264,365; Georgiou et al., U.S. Patent No. 5,508,192; Hara et al., Microbial Drug Resistance, 2:63-72 (1996).

In one embodiment, *E. coli* strains deficient for proteolytic enzymes and transformed with plasmids expressing one or more chaperone proteins are used as host cells in the expression system of the invention.

B. Antibody Purification

Standard protein purification methods known in the art can be employed. The following procedures are exemplary of suitable purification procedures: fractionation on immunoaffinity or ionexchange columns, ethanol precipitation, reverse phase HPLC, chromatography on silica or on a cation-exchange resin such as SP or an anion exchange such as DEAE, chromatofocusing, SDS-PAGE, ammonium sulfate precipitation, and gel filtration using, for example, Sephadex G-75.

In one aspect, Protein A immobilized on a solid phase is used for immunoaffinity purification of the antibody products of the invention. Protein A is a 41kD cell wall protein from Staphylococcus aureas which binds with a high affinity to the Fc region of antibodies. Lindmark et al., (1983) J. Immunol. Meth. 62:1-13. The solid phase to which Protein A is immobilized is preferably a column comprising a glass or silica surface, more preferably a controlled pore glass column or a silicic acid column. In some applications, the column has been coated with a reagent, such as glycerol, in an attempt to prevent nonspecific adherence of impurities.

As the first step of purification, the preparation derived from the cell culture as described above is applied onto the Protein A immobilized solid phase to allow specific binding of the antibody of interest to Protein A. The solid phase is then washed to remove impurities non-specifically bound to the solid phase. Finally the antibody of interest is recovered from the solid phase by elution.

In some embodiments of the invention, fractions derived from one or more of the purification steps are analyzed for the removal of FkpA. In some embodiments, the removal of FkpA is determined by immunoassay; for example, by the immunoassays described herein.

The invention also provides immunoconjugates (interchangeably termed "antibody-drug conjugates" or "ADC"), comprising any of the antibodies described herein conjugated to, *e.g.*, a cytotoxic agent such as a chemotherapeutic agent, a drug, a growth inhibitory agent, a toxin (*e.g.*, an enzymatically active toxin of bacterial, fungal, plant, or animal origin, or fragments thereof), or a radioactive isotope (*i.e.*, a radioconjugate).

Purification schemes for bispecific antibodies of the invention are described below.

VI. Compositions of FkpA

In some aspects, the invention provides compositions comprising ultrapure FkpA. In some aspects of the disclosure, the composition comprises FkpA wherein the FkpA is more than about any of 95.0%, 96.0%, 97.0%, 98.0%, 99.0%, or 99.5% FkpA. In some aspects of the disclosure, a composition comprising 95% FkpA is a composition where less than 5% of the material in the preparation are other substances that were present in the cells or cell lysates (*e.g.*, protein, nucleic acids, lipids, *etc.*) present during the production of the FkpA. In some aspects of the disclosure, the percentage of FkpA polypeptide is measured by size exclusion chromatography (*e.g.*, HPLC-SEC). In some aspects of the disclosure, the composition comprising ultrapure FkpA comprises less than about any of 5%, 4%, 3%, 2%, 1%, 0.5% or 0.1% low molecular weight species (*e.g.*, species with a molecular weight less than FkpA as measured by SEC or SDS-PAGE). In some aspects of the disclosure, the composition comprising ultrapure FkpA comprises less than about 2% low molecular weight species. In some aspects of the disclosure, the composition comprising ultrapure FkpA comprises less than about any of 5%, 4%, 3%, 2%, 1%, 0.5% or 0.1% high molecular weight species (*e.g.*, species with a molecular weight greater than FkpA as measured by SEC or SDS-PAGE). In some aspects of the disclosure, the composition comprising ultrapure FkpA comprises less than about 1% high molecular weight species.

In some aspects of the disclosure, the composition comprising highy purified FkpA is formulated for use. In some aspects of the disclosure, the composition comprising ultrapure FkpA is formulated for immunizing animals to generate antibodies to FkpA; for example, by adding adjuvants to facilitate the generation of an immune response. In other aspects of the disclosure, the composition comprising ultrapure FkpA is formulated for use in immunoassays; for example, as a positive control or as a standard curve.

VII. Generation of antibodies to FkpA

In certain aspects, the disclosure provides ultrapure FkpA to use as an immunogen to generate antibodies (*e.g.*, polyclonal antibodies and/or monoclonal antibodies) for use in immunoassays for analyzing recombinant polypeptide samples for the presence of FkpA. For example, the antibodies may be used in immunoassays to detect and/or quantitate FkpA in recombinant polypeptide samples where the recombinant polypeptide (*e.g.*, a multispecific antibody) was produced in bacterial cells that express exogenous FkpA. In some aspects of the disclosure, the disclosure provides polyclonal antibodies that specifically bind ultrapure FkpA. In some aspects, polyclonal antibodies specifically bind different epitopes of FkpA and therefore provide utility to detect FkpA fragments, variant, misfolded protein, *etc.* In order to minimize the generation of rabbit antibodies against host cell protein impurities, which might result in an over-quantification or over-estimation of the level of FkpA in samples being measured in the immunoassay, animals (*e.g.*, rabbits) are immunized with ultrapure FkpA.

Disclosed herein are antibodies that specifically bind FkpA. In some aspects of the disclosure, antibodies are polyclonal antibodies. Polyclonal antibodies are preferably raised in animals by multiple subcutaneous (sc) or intraperitoneal (ip) injections of the relevant antigen and an adjuvant. It may be useful to conjugate the relevant antigen to a polypeptide that is immunogenic in the species to be immunized, *e.g.*, keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl2, or R1N=C=NR, where R and R1 are different alkyl groups.

Animals are immunized against the antigen, immunogenic conjugates, or derivatives by combining, *e.g.*, 100 $\mu$g or 5 $\mu$g of the polypeptide or conjugate (for rabbits or mice, respectively) with 3 volumes of Freund's complete adjuvant and injecting the solution intradermally at multiple sites. One month later the animals are boosted with 115 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. In some aspects of the disclosure, the animal is boosted with the conjugate of the same antigen, but conjugated to a different polypeptide and/or through a different cross-linking reagent. Conjugates also can be made in recombinant cell culture as polypeptide fusions. Also, aggregating agents such as alum are suitably used to enhance the immune response.

In some aspects of the disclosure, the animal immunized with FkpA is a goat, a sheep, a rabbit, a mouse, a guinea pig, a hamster, a rat, a donkey or a chicken.

In some aspects of the disclosure, the antibodies that specifically bind FkpA are monoclonal antibodies. Monoclonal antibodies are obtained from a population of substantially homogeneous antibodies, i.e., the individual antibodies comprising the population are identical and/or bind the same epitope except for possible variants that arise during production of the monoclonal antibody, such variants generally being present in minor amounts. Thus, the modifier "monoclonal" indicates the character of the antibody as not being a mixture of discrete or polyclonal antibodies. As described above, the monoclonal antibodies may be made using the hybridoma method first described by Kohler et al., Nature 256:495 (1975), or may be made by recombinant DNA methods (U.S. Patent No. 4,816,567).

In some aspects, the invention provides methods for purifying antibodies that specifically bind FkpA, comprising contacting a composition comprising anti-FkpA antibodies to chromatography material comprising ultrapure FkpA attached to a support material, washing the chromatography material to remove unbound compounds, and eluting the anti-FkpA antibodies. In some aspects of the disclosure, the ultrapure FkpA that is attached to the support material comprises less than about any of 1%, 0.9%, 0.8%, 0.7%, 0.6%, 0.5%, 0.4%, 0.3%, 0.2%, 0.1%, 0.05%, 0.01% impurities. In some aspects of the disclosure, the ultrapure FkpA is prepared by the methods described herein. In some aspects of the disclosure, the invention provides methods to purify polyclonal antibodies that specifically bind FkpA. In some aspects of the disclosure, less than about any of 1%, 0.9%, 0.8%, 0.7%, 0.6%, 0.5%, 0.4%, 0.3%, 0.2%, 0.1%, 0.05%, 0.01% of the polyclonal antibodies specifically bind non-FkpA compounds.

In some aspects of the disclosure, anti-FkpA antibodies are purified by contacting the antibodies to ultrapure FkpA immobilized on a chromatography material (*e.g.*, ultrapure FkpA immobilized to activated glyceryl-controlled pore glass). In some aspects of the disclosure, the antibodies are concentrated, for example, by ammonium sulfate precipitation prior to contact with the immobilized FkpA. In some aspects of the disclosure, the antibodies are bound to the immobilized FkpA and then the immobilized FkpA-antibody complexes are washed to remove non-bound impurities. In further embodaspects of the disclosureiments, the antibodies are eluted from the immobilized FkpA by eluting with a buffer at a different pH as the load buffer; for example, the antibodies are bound to the immobilized FkpA at neutral pH (*e.g.,* pH 7.2) and eluted at acidic pH (*e.g.,* pH 2.0). In some further aspects of the disclosure, the anti-FkpA antibodies are subject to further chromatography; for example size exclusion chromatography. In some aspects of the disclosure, the purified anti-FkpA antibodies (*e.g.,* polyclonal antibodies) are assayed for their binding to ultrapure FkpA, respectively.

VIII. Immunoassays using antibodies to FkpA

In some aspects, the disclosure provides a method for analyzing a recombinant polypeptide (*e.g.*, a multispecific antibody) sample for the presence of and/or quantity of FkpA, comprising detecting FkpA in the sample using an immunoassay and comparing the amount of FkpA detected in the sample with the detection of one or more concentrations of an ultrapure FkpA reference standard. In some aspects of the disclosure, the preparation comprises less than about any of 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, or 0.01% of impurities. In some aspects of the disclosure, the ultrapure FkpA reference standard is prepared by the methods described herein. In some aspects of the disclosure, the immunoassay comprises antibodies that specifically bind ultrapure FkpA. In some aspects of the disclosure, the antibodies that specifically bind ultrapure FkpA bind less than about any of 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, or 0.01% of non-FkpA compounds. In some aspects of the disclosure, the antibodies that specifically bind ultrapure FkpA are polyclonal antibodies. In other aspects of the disclosure, the antibodies that specifically bind ultrapure FkpA are monoclonal antibodies. In some aspects of the disclosure, the antibodies that specifically bind ultrapure FkpA are used as capture antibodies in the immunoassay. In some aspects of the disclosure, the antibodies that specifically bind ultrapure FkpA are used as detection antibodies. In some aspects of the disclosure, the detection antibodies are conjugated to a detection agent (*e.g.*, a horseradish peroxidase). In some aspects of the disclosure, the FkpA is an *E. coli* FkpA. In some aspects of the disclosure, the recombinant polypeptide is prepared in a host cell (*e.g.,* an E. *coli* host cell). In some embodiments, the host cell overexpresses FkpA (*e.g.,* an *E. coli* host cell that overexpressed FkpA). In some aspects of the disclosure, the host cell expresses exogenous FkpA (*e.g.,* an *E. coli* host cell that expressed exogenous FkpA). In some aspects of the disclosure, the sample is cell lysate or is obtained from a recombinant polypeptide preparation and wherein the recombinant polypeptide preparation has been subjected to one or more chromatographic purification steps. In some aspects of the disclosure, the recombinant polypeptide preparation is a final purified product. In some aspects of the disclosure, the antibodies that specifically bind ultrapure FkpA are capable of detecting less than about and/or about any of 50 ng/mL, 25 ng/mL, 15 ng/mL, 10 ng/mL, 5 ng/mL, 2.5 ng/mL, and/or 1.5 ng/mL of FkpA in an immunoassay.

In some aspects, the disclosure provides immunoassay methods for detection and quantification of FkpA. Such methods may be used for the detection and quantification of FkpA in recombinant polypeptide preparations produced in host cells, for example *E. coli* where FkpA is expressed to facilitate polypeptide folding and assembly. In some aspects of the disclosure, the immunoassay methods use capture and detection anti-FkpA antibodies described herein. In some aspects of the disclosure, the antibodies are used in any immunoassay method known in the art, including but not limited to, sandwich assay, enzyme-linked immunosorbent assay (ELISA) assay, electrochemical assay (ECL) assay, magnetic immunoassay. In certain aspects of the disclosure, the method comprises contacting a sample of the recombinant polypeptide preparation with an anti-FkpA antibody as described herein under conditions permissive for binding of the anti-FkpA antibody to FkpA, and detecting whether a complex is formed between the anti-FkpA antibody and FkpA.

In certain aspects of the disclosure, labeled anti-FkpA antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, *e.g.*, through an enzymatic reaction or molecular interaction. Exemplary labels include, but are not limited to, the radioisotopes 32P, 14C, 125I, 3H, and 131I, fluorophores such as rare earth chelates or fluorescein and its derivatives, rhodamine and its derivatives, dansyl, umbelliferone, luceriferases, *e.g.*, firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, *e.g.*, glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

In certain aspects of the disclosure, a capture anti-FkpA antibody is immobilized on a solid phase. In some aspects of the disclosure, the solid phase used for immobilization is any inert support or carrier that is essentially water insoluble and useful in immunometric assays, including supports in the form of, *e.g.*, surfaces, particles, porous matrices, beads and the like. Examples of commonly used supports include small sheets, SEPHADEX®, gels, polyvinyl chloride, plastic beads, and assay plates or test tubes manufactured from polyethylene, polypropylene, polystyrene, and the like, including 96-well microtiter plates, as well as particulate materials such as filter paper, agarose, cross-linked dextran, and other polysaccharides. Alternatively, reactive water-insoluble matrices such as cyanogen-bromide-activated carbohydrates and the reactive substrates described in U.S. Pat. Nos. 3,969,287; 3,691,016; 4,195,128; 4,247,642; 4,229,537; and 4,330,440 are suitably employed for capture-reagent immobilization. In some aspects of the disclosure, the immobilized capture reagents are coated on a microtiter plate that can be used to analyze several samples at one time. Exemplary microtiter plates include, but are not limited to, MICROTEST®, MAXISORP®, NUNC MAXISORB®, and IMMULON®. The solid phase is coated with the capture reagents as defined above, which may be linked by a non-covalent or covalent interaction or physical linkage as desired. Techniques for attachment include those described in U.S. Pat. No. 4,376,110 and the references cited therein. If covalent, the plate or other solid phase is incubated with a cross-linking agent together with the capture reagent under conditions well known in the art such as for one hour at room temperature. In some aspects of the disclosure, the plates are stacked and coated long in advance of the assay itself, and then the assay is carried out simultaneously on several samples in a manual, semi-automatic, or automatic fashion, such as by using robotics.

In some aspects of the disclosure, the coated plates are treated with a blocking agent that binds non-specifically to and saturates the binding sites to prevent unwanted binding of the free ligand to the excess sites on the wells of the plate. Examples of appropriate blocking agents for this purpose include but are not limited to, *e.g.*, gelatin, bovine serum albumin, egg albumin, casein, and non-fat milk. The blocking treatment typically takes place under conditions of ambient temperatures for a period of time, typically about 1-4 hours.

In some aspects of the disclosure, after coating and blocking, the sample to be analyzed, appropriately diluted, is added to the immobilized phase. Exemplary buffers that may be used for dilution for this purpose include, but are not limited to, (a) phosphate-buffered saline (PBS) containing 0.5% BSA, 0.05% TWEEN 20® detergent (P20), 0.05% PROCLIN® 300 antibiotic, 5 mM EDTA, 0.25% 3-((3-cholamidopropyl)dimethylammonio)-1-propanesulphonate (CHAPS) surfactant, 0.2% beta-gamma globulin, and 0.35M NaCl; (b) PBS containing 0.5% bovine serum albumin (BSA), 0.05% P20, and 0.05% PROCLIN® 300, pH 7; (c) PBS containing 0.5% BSA, 0.05% P20, 0.05% PROCLIN® 300, 5 mM EDTA, and 0.35 M NaCl, pH 6.35; (d) PBS containing 0.5% BSA, 0.05% P20, 0.05% PROCLIN® 300, 5 mM EDTA, 0.2% beta-gamma globulin, and 0.35 M NaCl; and (e) PBS containing 0.5% BSA, 0.05% P20, 0.05% PROCLIN® 300, 5 mM EDTA, 0.25% CHAPS, and 0.35 M NaCl.

The conditions for incubation of sample and immobilized capture reagent are selected to maximize sensitivity of the assay and to minimize dissociation, and to ensure that any analyte of interest present in the sample (such as FkpA) binds to the immobilized capture reagent. Optionally, the sample is separated (for example, by washing) from the immobilized capture reagents to remove uncaptured material. The solution used for washing is generally a buffer (*e.g.*, "washing buffer"). A cross-linking agent or other suitable agent may also be added at this stage to allow the now-bound material of interest (*e.g.*, FkpA) to be covalently attached to the capture reagents if there is any concern that the captured material of interest may dissociate to some extent in the subsequent steps.

The immobilized capture reagents with any bound material of interest present are contacted with a detection anti-FkpA antibody. In some aspects of the disclosure, the detection antibody is biotinylated. In some aspects of the disclosure, the detection means for the biotinylated label is avidin or streptavidin-HRP. In some aspects of the disclosure, the readout of the detection means is fluorimetric or colorimetric.

The level of any free material of interest from the sample (*e.g.*, FkpA) that is now bound to the capture reagents is measured or quantified using a detection means for the detection antibody. In some aspects of the disclosure, the measuring or quantifying comprises comparing the reaction that occurs as a result of the above steps with a standard curve to determine the level of material of interest (*e.g.*, FkpA) compared to a known amount.

The antibody added to the immobilized capture reagents will be either directly labeled, or detected indirectly by addition, after washing off of excess first antibody, of a molar excess of a second, labeled antibody directed against IgG of the animal species of the first antibody. In the latter, indirect assay, labeled antisera against the first antibody are added to the sample so as to produce the labeled antibody in situ.

The label used for either the first or second antibody is any detectable functionality that does not interfere with the binding of free material of interest (*e.g.*, FkpA) to the first or second antibodies. Examples of suitable labels include those known for use in immunoassay, such as those enumerated above.

Conventional methods are available to bind these labels covalently to proteins or polypeptides. For instance, coupling agents such as dialdehydes, carbodiimides, dimaleimides, bis-imidates, bis-diazotized benzidine, and the like may be used to tag the antibodies with the above-described fluorescent, chemiluminescent, and enzyme labels. See, for example, U.S. Pat. No. 3,940,475 (fluorimetry) and U.S. Pat. No. 3,645,090 (enzymes); Hunter et al., Nature 144:945 (1962); David et al., Biochemistry, 13: 1014-1021 (1974); Pain et al., J. Immunol. Methods 40:219-230 (1981); and Nygren, J. Histochem. and Cytochem., 30:407-412 (1982).

IX. Obtaining Multispecific Antibodies for Use in the Formulations and Methods

The multispecific antibodies used in the methods of purification described herein may be obtained using methods well-known in the art, including the recombination methods. The following sections provide guidance regarding these methods.

(A) Polynucleotides

Polynucleotides encoding polypeptides may be obtained from any source including, but not limited to, a cDNA library prepared from tissue believed to possess the polypeptide mRNA and to express it at a detectable level. Accordingly, polynucleotides encoding polypeptide can be conveniently obtained from a cDNA library prepared from human tissue. The polypeptide-encoding gene may also be obtained from a genomic library or by known synthetic procedures (*e.g.*, automated nucleic acid synthesis).

For example, the polynucleotide may encode an entire immunoglobulin molecule chain, such as a light chain or a heavy chain. A complete heavy chain includes not only a heavy chain variable region ($V_H$) but also a heavy chain constant region ($C_H$), which typically will comprise three constant domains: $C_H1$, $C_H2$ and $C_H3$; and a "hinge" region. In some situations, the presence of a constant region is desirable.

Other polypeptides which may be encoded by the polynucleotide include antigen-binding antibody fragments such as single domain antibodies ("dAbs"), Fv, scFv, Fab' and $F(ab')_2$ and "minibodies." Minibodies are (typically) bivalent antibody fragments from which the $C_H1$ and $C_K$ or $C_L$ domain has been excised. As minibodies are smaller than conventional antibodies they should achieve better tissue penetration in clinical/diagnostic use, but being bivalent they should retain higher binding affinity than monovalent antibody fragments, such as dAbs. Accordingly, unless the context dictates otherwise, the term "antibody" as used herein encompasses not only whole antibody molecules but also antigen-binding antibody fragments of the type discussed above. Preferably each framework region present in the encoded polypeptide will comprise at least one amino acid substitution relative to the corresponding human acceptor framework. Thus, for example, the framework regions may comprise, in total, three, four, five, six, seven, eight, nine, ten, eleven, twelve, thirteen, fourteen, or fifteen amino acid substitutions relative to the acceptor framework regions.

Suitably, the polynucleotides described herein may be isolated and/or purified. In some embodiments, the polynucleotides are isolated polynucleotides.

The term "isolated polynucleotide" is intended to indicate that the molecule is removed or separated from its normal or natural environment or has been produced in such a way that it is not present in its normal or natural environment. In some embodiments, the polynucleotides are purified polynucleotides. The term purified is intended to indicate that at least some contaminating molecules or substances have been removed.

Suitably, the polynucleotides are substantially purified, such that the relevant polynucleotides constitutes the dominant (*i.e.*, most abundant) polynucleotides present in a composition.

(B) Expression of Polynucleotides

The description below relates primarily to production of polypeptides by culturing cells transformed or transfected with a vector containing polypeptide-encoding polynucleotides. It is, of course, contemplated that alternative methods, which are well known in the art, may be employed to prepare polypeptides. For instance, the appropriate amino acid sequence, or portions thereof, may be produced by direct peptide synthesis using solid-phase techniques (*see, e.g.,* Stewart et al., Solid-Phase Peptide Synthesis W.H. Freeman Co., San Francisco, Calif. (1969); Merrifield, J. Am. Chem. Soc. 85:2149-2154 (1963)). In vitro protein synthesis may be performed using manual techniques or by automation. Automated synthesis may be accomplished, for instance, using an Applied Biosystems Peptide Synthesizer (Foster City, Calif.) using manufacturer's instructions. Various portions of the polypeptide may be chemically synthesized separately and combined using chemical or enzymatic methods to produce the desired polypeptide.

Polynucleotides as described herein are inserted into an expression vector(s) for production of the polypeptides. The term "control sequences" refers to DNA sequences necessary for the expression of an operably linked coding sequence in a particular host organism. The control sequences include, but are not limited to, promoters (*e.g.,* naturally-associated or heterologous promoters), signal sequences, enhancer elements, and transcription termination sequences.

A polynucleotide is "operably linked" when it is placed into a functional relationship with another polynucleotide sequence. For example, nucleic acids for a presequence or secretory leader is operably linked to nucleic acids for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the nucleic acid sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

For antibodies, the light and heavy chains can be cloned in the same or different expression vectors. The nucleic acid segments encoding immunoglobulin chains are operably linked to control sequences in the expression vector(s) that ensure the expression of immunoglobulin polypeptides.

The vectors containing the polynucleotide sequences (*e.g.,* the variable heavy and/or variable light chain encoding sequences and optional expression control sequences) can be transferred into a host cell by well-known methods, which vary depending on the type of cellular host. For example, calcium chloride transfection is commonly utilized for prokaryotic cells, whereas calcium phosphate treatment, electroporation, lipofection, biolistics or viral-based transfection may be used for other cellular hosts. (*See generally* Sambrook et al., Molecular Cloning: A Laboratory Manual (Cold Spring Harbor Press, 2nd ed., 1989). Other methods used to transform mammalian cells include the use of polybrene, protoplast fusion, liposomes, electroporation, and microinjection. For production of transgenic animals, transgenes can be microinjected into fertilized oocytes, or can be incorporated into the genome of embryonic stem cells, and the nuclei of such cells transferred into enucleated oocytes.

Vectors

The term "vector" includes expression vectors and transformation vectors and shuttle vectors.

The term "expression vector" means a construct capable of *in vivo* or *in vitro* expression.

The term "transformation vector" means a construct capable of being transferred from one entity to another entity - which may be of the species or may be of a different species. If the construct is capable of being transferred from one species to another - such as from an *Escherichia coli* plasmid to a bacterium, such as of the genus *Bacillus,* then the transformation vector is sometimes called a "shuttle vector". It may even be a construct capable of being transferred from an *E. coli* plasmid to an *Agrobacterium* to a plant.

Vectors may be transformed into a suitable host cell as described below to provide for expression of a polypeptide. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Construction of suitable vectors containing one or more of these components employs standard ligation techniques which are known to the skilled artisan.

The vectors may be for example, plasmid, virus or phage vectors provided with an origin of replication, optionally a promoter for the expression of the said polynucleotide and optionally a regulator of the promoter. Vectors may contain one or more selectable marker genes which are well known in the art.

These expression vectors are typically replicable in the host organisms either as episomes or as an integral part of the host chromosomal DNA.

Host Cells

The host cell may be a bacterium, a yeast or other fungal cell, insect cell, a plant cell, or a mammalian cell, for example.

A transgenic multicellular host organism which has been genetically manipulated may be used to produce a polypeptide. The organism may be, for example, a transgenic mammalian organism (*e.g.,* a transgenic goat or mouse line).

Suitable prokaryotes include but are not limited to eubacteria, such as Gram-negative or Gram-positive organisms, for example, *Enterobacteriaceae* such as *E. coli.* Various E. *coli* strains are publicly available, such as E. *coli* K12 strain MM294 (ATCC 31,446); E. *coli* X1776 (ATCC 31,537); *E. coli* strain W3110 (ATCC 27,325) and K5 772 (ATCC 53,635). Other suitable prokaryotic host cells include *Enterobacteriaceae* such as *Escherichia, e.g., E. coli, Enterobacter, Erwinia, Klebsiella, Proteus, Salmonella, e.g., Salmonella typhimurium, Serratia, e.g., Serratia marcescans,* and *Shigella*, as well as *Bacilli* such as *B. subtilis and B. licheniformis* (*e.g., B. licheniformis* 41P), *Pseudomonas* such as *P. aeruginosa,* and *Streptomyces.* These examples are illustrative rather than limiting. Strain W3110 is one particularly preferred host or parent host because it is a common host strain for recombinant polynucleotide product fermentations. Preferably, the host cell secretes minimal amounts of proteolytic enzymes. For example, strain W3110 may be modified to effect a genetic mutation in the genes encoding polypeptides endogenous to the host, with examples of such hosts including *E. coli* W3110 strain 1A2, which has the complete genotype *tonA; E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompt kan'; E. coli* W3110 strain 37D6, which has the complete genotype *tonA ptr3 phoA E15 (argF-lac)169 degP ompT rbs7 ilvG kan ; E. coli* W3110 strain 40B4, which is strain 37D6 with a non-kanamycin resistant *degP* deletion mutation; and an E. *coli* strain having mutant periplasmic protease. Alternatively, in vitro methods of cloning, *e.g.,* PCR or other nucleic acid polymerase reactions, are suitable. In some embodiments, the prokaryotic host cell *(e.g.,* an E. *coli* host cell) expresses one or more chaperones to facilitate folding and assembly of the antibody. In some embodiments, the chaperone is one or more of FkpA, DsbA or DsbC. In some embodiments, the chaperone is expressed from an endogenous chaperone gene. In some embodiments, the chaperone is expressed from an exogenous chaperone gene. In some embodiments, the chaperone gene is an *E. coli* chaperone gene (*e.g.,* an *E. coli* FkpA gene, an *E. coli* DsbA gene and/or an *E. coli* DsbC gene).

In these prokaryotic hosts, one can make expression vectors, which will typically contain expression control sequences compatible with the host cell (*e.g.,* an origin of replication). In addition, any number of a variety of well-known promoters will be present, such as the lactose promoter system, a tryptophan (trp) promoter system, a beta-lactamase promoter system, or a promoter system from phage lambda. The promoters will typically control expression, optionally with an operator sequence, and have ribosome binding site sequences and the like, for initiating and completing transcription and translation.

Eukaryotic microbes may be used for expression. Eukaryotic microbes such as filamentous fungi or yeast are suitable cloning or expression hosts for polypeptide-encoding vectors. *Saccharomyces cerevisiae* is a commonly used lower eukaryotic host microorganism. Others include *Schizosaccharomyces pombe; Kluyveromyces hosts* such as, *e.g., K. lactis* (MW98-8C, CBS683, CBS4574), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans,* and *K. marxianus; yarrowia* (EP 402,226); *Pichia pastoris; Candida; Trichoderma reesia; Neurospora crassa; Schwanniomyces* such as *Schwanniomyces occidentalis;* and filamentous fungi such as, *e.g., Neurospora, Penicillium, Tolypocladium,* and Aspergillus hosts such as *A. nidulans,* and *A. niger.* Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula. Saccharomyces* is a preferred yeast host, with suitable vectors having expression control sequences (*e.g.*, promoters), an origin of replication, termination sequences and the like as desired. Typical promoters include 3-phosphoglycerate kinase and other glycolytic enzymes. Inducible yeast promoters include, among others, promoters from alcohol dehydrogenase, isocytochrome C, and enzymes responsible for maltose and galactose utilization.

In addition to microorganisms, mammalian tissue cell culture may also be used to express and produce the polypeptides as described herein and in some instances are preferred (*See* Winnacker, From Genes to Clones VCH Publishers, N.Y., N.Y. (1987). For some embodiments, eukaryotic cells may be preferred, because a number of suitable host cell lines capable of secreting heterologous polypeptides (*e.g.*, intact immunoglobulins) have been developed in the art, and include CHO cell lines, various Cos cell lines, HeLa cells, preferably, myeloma cell lines, or transformed B-cells or hybridomas. In some embodiments, the mammalian host cell is a CHO cell.

In some embodiments, the host cell is a vertebrate host cell. Examples of useful mammalian host cell lines are monkey kidney CV1 line transformed by SV40 (COS-7, ATCC CRL 1651); human embryonic kidney line (293 or 293 cells subcloned for growth in suspension culture); baby hamster kidney cells (BHK, ATCC CCL 10); Chinese hamster ovary cells/-DHFR(CHO or CHO-DP-12 line); mouse sertoli cells; monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells; MRC 5 cells; FS4 cells; and a human hepatoma line (Hep G2).

X. Production of multispecific antibodies-assembly of half-antibodies

In some aspects, the disclosure provides methods and reagents for producing multispecific antibodies. In some embodiments, multispecific antibodies are produced by separately producing half-antibodies, each half antibody comprising a VH/VL unit that binds a specific epitope. In some embodiments, each half-antibody is produced separately in a host cell. In some embodiments, each half-antibody is produced together in the same host cell. The half-antibodies are mixed and allowed to assemble into the multispecific antibody. In some embodiments, each half-antibody is produced together in the same host cell. In some embodiments the host cell expresses a chaperone, such as FkpA, DsbA and/or DsbC, to facilitate folding of the half-antibody. In some embodiments, the multispecific antibody is assembled from a half-antibody that binds IL13 and a half-antibody that binds IL17.

Each half-antibody can have either a knob (protuberance) or a hole (cavity) engineered into the heavy chain as described in U.S. Pat. No. 7,642,228. Briefly, a CH3 knob mutant can be generated first. A library of CH3 hole mutants can be then created by randomizing residues 366, 368 and 407 that are in proximity to the knob on the partner CH3 domain. In certain embodiments, the knob mutation comprises T366W, and the hole had mutations comprise T366S, L368A and Y407V in an IgG1or IgG4 backbone. Equivalent mutations in other immunoglobulin isotypes can be made by one skilled in the art. Further, the skilled artisan will readily appreciate that it is preferred that the two half-antibodies used for the bispecific be the same isotype.

In some embodiments, half-antibodies containing either the knob or hole mutations are generated in separate cultures by expressing the heavy and light chains constructs in a bacterial host cell, *e.g.*, *E. coli.* Each half-antibody can be purified separately by Protein A affinity chromatography. Clarified cell extracts from the knob and hole half-antibody can be purified by a HiTrap® MaBSelect™ SuRe™ column. Protein A purified half antibodies with different specificity can be assembled to form a bispecific antibody in a redox reaction *in vitro* in the presence of a reducing agent.

In some embodiments, half-antibodies containing either the knob or hole mutations are generated in the same culture by expressing the heavy and light chains constructs in the same bacterial host cell, *e.g., E. coli.* The half-antibodies can be purified by Protein A affinity chromatography. Clarified cell extracts from the knob and hole half-antibodies can be purified by a HiTrap® MaBSelect™ SuRe™ column. Protein A purified half antibodies with different specificity can be assembled to form a bispecific antibody in a redox reaction in vitro in the presence of a reducing agent.

Any suitable methods can be used to prepare a desired reducing condition. For example, a desired reducing condition can be prepared by adding a reductant/reducing agent to the reaction (such as an assembly mixture of the invention). Suitable reductants include without limitation dithiothreitol (DTT), tris(2-carboxyethyl)phosphine (TCEP), thioglycolic acid, ascorbic acid, thiol acetic acid, glutathione (GSH), Beta-MercaptoEthylAmine, cysteine/cystine, GSH/glutathione disulfide (GSSG), cysteamine/cystamine, glycylcysteine, and beta-mercaptoethanol, preferably GSH. In certain particular embodiments, the reductant is a weak reductant including without limitation GSH, Beta-MercaptoEthylAmine, cysteine/cystine, GSH/GSSG, cysteamine/cystamine, glycylcysteine, and beta-mercaptoethanol, preferably GSH. In certain preferred embodiments, the reductant is GSH. In certain embodiments, the reductant is not DTT. It is within the ability of one of ordinary skill in the art to select suitable reductants at suitable concentrations and under suitable experimental conditions to achieve in a reaction the desired reducing condition. For example, 10 mM L-reduced glutathione in a solution with a bispecific antibody protein concentration of 10g/L at 20 °C will result in a starting redox potential of about - 400mV. For example, glutathione added to an assembly mixture creates a weakly reducing condition that is advantageous for knob-into-hole bispecific assembly. Other reductants in a similar class such as BMEA (Beta-Mercapto-ToEthylAmine) may have a similar effect. See WO2013/055958. The reducing condition of the reaction can be estimated and measured using any suitable methods known in the art. For example, the reducing condition can be measured using a resazurin indicator (discolorization from blue to colorless in reducing conditions). For more precise measurement, a redox-potential meter (such as an ORP Electrode made by BROADLEY JAMES®) can be used.

In certain particular embodiments, the reducing condition is a weak reducing condition. The term "weak reductant" or "weak reducing condition" as used herein refers to a reducing agent or a reducing condition prepared by the reducing agent having a negative oxidation potential at 25°C. The oxidation potential of the reductant is preferably between -50 to -600 mV, -100 to -600 mV, -200 to -600 mV, - 100 to -500 mV, -150 to -300 mV, more preferably between about -300 to -500 mV, most preferably about -400mV, when the pH is between 7 and 9, and the temperature is between 15°C and 39°C. One skilled in the art will be able to select suitable reductants for preparing a desired reducing condition. The skilled researcher will recognize that a strong reductant, i.e., one that has a more negative oxidation potential than above mentioned reductants for the same concentration, pH and temperature, may be used at a lower concentration. In a preferred embodiment, the proteins will be able to form disulfide bonds in the presence of the reductant when incubated under the above-recited conditions. Examples of a weak reductant include without limitation glutathione, Beta-MercaptoEthylAmine, cystine/cysteine, GSH/GSSG, cysteamine/cystamine, glycylcysteine, and beta-mercaptoethanol. In certain embodiments, an oxidation potential similar to that of 200X molar ratio of GSH:Antibody can be used as a point of reference for a weakly reducing condition at which efficient assembly using other reductants can be expected.

Glutathione concentrations can be expressed in terms of molarity or in terms of molar ratio or molar excess with respect to the amount of the half-antibodies present in the assembly mixture. Using a target molar ratio of reductant controls for the protein concentration in the assembly mixture; this prevents over reducing or under reducing as a result of variable protein concentrations. In certain other embodiments, the reductant is added to the assembly mixture in 2-600X, 2-200X, 2-300X, 2-400X, 2-500X, 2-20X, 2-8X, 20-50X, 50-600X, 50-200X, or 100-300X molar excess, preferably 50-400X, more preferably 100-300X, and most preferably 200X, molar excess with respect to the total amount of the half antibodies. In certain embodiments, the assembly mixture has a pH of between 7 and 9, preferably pH 8.5.

XI. Purification of Polypeptides Including Multispecific Antibodies

Provided herein are methods for purifying a polypeptides (*e.g.*, multispecific antibodies) generated in *E. coli* using chaperones (*e.g.*, FkpA, Dsba and/or DsbC). In some aspects, the purification of the multispecific antibody comprises the sequential steps of affinity chromatography, mixed-mode chromatography and hydrophobic interaction chromatography. In some embodiments, the multispecific antibody is assembled before affinity chromatography. In other aspects, the multispecific antibody is assembled after affinity chromatography. In some embodiments, the multispecific antibody is a bispecific antibody wherein one arm of the antibody (*i.e.*, one VH/VL unit) binds IL13 and the other arm of the antibody binds IL17.

In some embodiments, the multispecific antibody is comprised of two or more antibody arms wherein different antibody arms bind different epitopes. In some embodiments, antibody arms comprise VH/VL units. In some embodiments, the antibody arms comprise hemimers, also known as half-antibodies. To facilitate assembly, in some embodiments the heavy chain of one antibody arm is modified to comprise a "knob" and the heavy chain of another antibody arm comprises a "hole" such that the knob of the first heavy chain fits into the hole of the second heavy chain.

In some embodiments, each arm of the multispecific antibody is produced in a separate cell culture. Following expression of the antibody arm in the host cell, whole cell broth is collected and homogenized and the antibody arm is extracted. In some embodiments, polyethyleneimine (PEI) is added to the cell lysate prior to chromatography. In some embodiments, the cell lysate is centrifuged prior to chromatography. Each arm of the multispecific antibody is then purified by affinity chromatography. In some embodiments, the affinity chromatography is protein A chromatography. At this point, purified antibody arms may be analyzed; for example, by SDS-PAGE SEC chromatography, mass spectrometry, *etc.* The purified arms of the multispecific antibody are then combined and allowed to assemble by the methods described herein.

In other embodiments, each arm of the multispecific antibody is produced in a separate cell culture. Following expression of the antibody arm in the host cell, whole cell broth is collected and homogenized. The cell homogenates from each culture are then mixed and the combined antibody arms are extracted. In some embodiments, polyethyleneimine (PEI) is added to the cell lysate prior to chromatography. In some embodiments, the cell lysate is centrifuged prior to chromatography. The combined arms of the multispecific antibody are then purified by affinity chromatography. In some embodiments, the affinity chromatography is protein A chromatography. At this point, purified antibody arms may be analyzed; for example, by SDS-PAGE SEC chromatography, mass spectrometry, *etc.* The purified arms of the multispecific antibody are then combined and allowed to assemble by the methods described herein.

In other embodiments, each arm of the multispecific antibody is produced in the same cell culture. Following expression of the antibody arm in the host cell, whole cell broth is collected and homogenized and the antibody arms are extracted. In some embodiments, polyethyleneimine (PEI) is added to the cell lysate prior to chromatography. In some embodiments, the cell lysate is centrifuged prior to chromatography. The arms of the multispecific antibody are then purified by affinity chromatography. In some embodiments, the affinity chromatography is protein A chromatography. At this point, purified antibody arms may be analyzed; for example, by SDS-PAGE SEC chromatography, mass spectrometry, *etc.* The purified arms of the multispecific antibody are then allowed to assemble by the methods described herein.

In some embodiments, the final concentration of PEI in the cell lysate is at least about any of 0.1%, 0.2%, 0.3%, 0.4%, 0.5%, 0.6%, 0.7%, 0.8%, 0.9%, 1.0%, 2.0%, 3.0%, 4.0%, or 5.0. In some embodiments, the final concentration of PEI in the cell lysate is between about any one of 0.1% and 5%, 0.1% and 1%, 0.1% and 0.5%, 0.5% and 5%, 0.5% and 1%, or 1% and 5%. In some embodiments, the cell lysate comprising PEI is held for more than about any of 1 hr, 2 hr, 3 hr, 4 hr, 5 hr, 6 hr, 7 hr, 8 hr, 9 hr, 10 hr, 12 hr, 14 hr, 16 hr, 18 hr, 20 hr, or 24 hr. In some embodiments, the cell lysate comprising PEI is held for between about any one of 1 hr and 24 hrs, 1 hr and 6 hrs, 6 hr and 12 hrs, 12 hrs and 18 hrs, 18 hrs and 24 hrs. In some embodiments, the cell lysate comprising PEI is held for between about any one of 10 hr and 14 hrs. In some embodiments, the cell lysate comprising PEI is held at between about 4 °C and 37 °C. In some embodiments, the cell lysate comprising PEI is held at about ambient temperature.

In some embodiments, the cell lysate is clarified by centrifugation prior to chromatography. In some embodiments, the cell lysate is filtered prior to chromatography. In some embodiments, the cell lysate is filtered through a 0.22 μm filter prior to chromatography.

Examples of affinity chromatography include, but are not limited to chromatography using derivatized with protein A or protein G. Examples of affinity chromatography material include, but are not limited to, ProSep®-vA, ProSep® Ultra Plus, Protein A Sepharose® Fast Flow, Toyopearl® AF-rProtein A, MabSelect™, MabSelect SuRe™ and MabSelect SuRe™ LX. In some embodiments of the above, the affinity chromatography material is in a column. In some embodiments of the above, the affinity chromatography material is a membrane. The affinity chromatography as defined in the claims is protein A chromatography. In some embodiments, the protein A chromatography is MabSelect SuRe™ chromatography.

In some embodiments, the eluate from the affinity chromatography step is subsequently applied to mixed-mode chromatography. In some embodiments, the mixed-mode material comprises functional groups capable of one of more of the following functionalities: anionic exchange, cation exchange, hydrogen bonding, and hydrophobic interactions. In some embodiments, the mixed-mode material comprises functional groups capable of anionic exchange and hydrophobic interactions. In some embodiments, the mixed-mode material comprises functional groups capable of cationic exchange and hydrophobic interactions. The mixed-mode material may contain N-benzyl-N-methyl ethanol amine, 4-mercapto-ethyl-pyridine, hexylamine, or phenylpropylamine as ligand or contain cross-linked polyallylamine. Examples of the mixed-mode materials include Capto™ adhere resin, Accell™ Plus Quaternary Methyl Amine (QMA) resin, Capto™ MMC resin, MEP HyperCel™ resin, HEA HyperCel™ resin, PPA HyperCel™ resin, or ChromaSorb™ membrane or Sartobind STIC®. In some embodiments, the mixed-mode material is Capto™ adhere resin. In some embodiments, the mixed-mode chromatography is performed in bind and elute mode where the multispecific antibody binds the mixed-mode chromatography material and then eluted from the mixed-mode chromatography material. In some embodiments of the above, the mixed-mode material is in a column. In some embodiments of the above, the mixed-mode material is in a membrane.

In some aspects of the disclosure, the eluate from the mixed-mode chromatography step is subsequently applied to hydrophobic interaction chromatography (HIC) chromatography. In some embodiments, the eluate from the mixed-mode chromatography step is applied to anion exchange chromatography prior to hydrophobic interaction chromatography. Hydrophobic interaction chromatography is a liquid chromatography technique that separates biomolecules according to hydrophobicity. Examples of HIC chromatography materials include, but are not limited to, Toyopearl® Hexyl-650, Toyopearl® Hexyl-650C, Toyopearl® Butyl-650, Toyopearl® Phenyl-650, Toyopearl® Ether-650, HiTrap® Sepharose, Octyl Sepharose®, Phenyl Sepharose® or Butyl Sepharose®. In some embodiments, the HIC chromatography material comprises Phenyl Sepharose®. In other embodiments, the HIC chromatography material comprises Butyl Sepharose®. In some embodiments, the HIC chromatography is performed in flow through mode where the multispecific antibody flows through the HIC chromatography. In some embodiments of the above, the HIC chromatography material is in a column. In some embodiments of the above, the HIC chromatography material is in a membrane.

In some embodiments of the invention, the mixed-mode chromatography eluate is applied to anion exchange chromatography prior to HIC chromatography. In some embodiments, the anion exchange chromatography material is a solid phase that is positively charged and has free anions for exchange with anions in an aqueous solution passed over or through the solid phase. In some embodiments of any of the methods described herein, the anion exchange material may be a membrane, a monolith, or resin. In an embodiment, the anion exchange material may be a resin. In some embodiments, the anion exchange material may comprise a primary amine, a secondary amine, a tertiary amine or a quarternary ammonium ion functional group, a polyamine functional group, or a diethylaminoaethyl functional group. Examples of anion exchange materials are known in the art and include, but are not limited to POROS® HQ 50, POROS® PI 50, POROS® D, Mustang® Q, Q Sepharose® Fast Flow (QSFF), and DEAE-Sepharose®. In some embodiments, the anion exchange chromatography material is QSFF chromatography material. In some embodiments, the anion exchange chromatography is performed in bind and elute mode. In some embodiments of the above, the anion exchange chromatography material is in a column. In some embodiments of the above, the anion exchange chromatography material in a membrane.

In some embodiments of the invention, the fraction comprising the multispecific antibody from the HIC chromatography is applied to cation exchange chromatography. In some embodiments, the cation exchange chromatography material is a solid phase that is positively charged and has free cations for exchange with cations in an aqueous solution passed over or through the solid phase. In some embodiments of any of the methods described herein, the cation exchange material may be a membrane, a monolith, or resin. In some embodiments, the cation exchange material may be a resin. The cation exchange material may comprise a carboxylic acid functional group or a sulfonic acid functional group such as, but not limited to, sulfonate, carboxylic, carboxymethyl sulfonic acid, sulfoisobutyl, sulfoethyl, carboxyl, sulphopropyl, sulphonyl, sulphoxyethyl, or orthophosphate. In some embodiments of the above, the cation exchange chromatography material is a cation exchange chromatography column. In some embodiments of the above, the cation exchange chromatography material is a cation exchange chromatography membrane. Examples of cation exchange materials are known in the art include, but are not limited to Mustang® S, Sartobind® S, SOs Monolith, CIM®, CIMmultus® and CIMac® SO3, S Ceramic HyperD®, POROS® XS, POROS® HS 50, POROS® HS 20, sulphopropyl-Sepharose® Fast Flow (SPSFF), SP-Sepharose® XL (SPXL), CM Sepharose® Fast Flow, Capto™ S, Fractogel® EMD Se Hicap, Fractogel® EMD SO$_3^-$, or Fractogel® EMD COO$^-$. In some embodiments of any of the methods described herein, the cation exchange material is POROS® HS 50. In some embodiments, the cation exchange chromatography is performed in bind and elute mode. In some embodiments of the above, the cation exchange chromatography material is in a column. In some embodiments of the above, the cation exchange chromatography material is in a membrane.

In some aspects of the disclosure, the fraction comprising the polypeptide (e.g., the multispecific antibody) from the anion exchange chromatography is applied to hydroxyapatite chromatography. In some aspects of the disclosure, the hydroxyapatite comprises (Ca$_5$(PO$_4$)$_3$OH)2. In some aspects of the disclosure, the hydroxyapatite chromatography material is a solid phase chromatography. In some aspects of the disclosure of any of the methods described herein, the hydroxyapatite material may be a membrane, a monolith, or resin. In some embodiments, the cation exchange material may be a resin. In some aspects of the disclosure, the resin is a ceramic. In some aspects of the disclosure, the hydroxyapatite chromatography is ceramic hydroxyapatite chromatography. In some aspects of the disclosure, the hydroxyapatite chromatography is CHT ceramic hydroxyapatite chromatography. Examples of hydroxyapatite materials are known in the art include, but are not limited to CHT ceramic hydroxyapatite, Type I and Type II. In some aspects of the disclosure, the hydroxyapatite chromatography is performed in bind and elute mode. In some aspects of the disclosure of the above, the hydroxyapatite chromatography material is in a column. In some aspects of the disclosure of the above, the hydroxyapatite chromatography material is in a membrane.

Loading of a solution comprising the multispecific antibody on any of the chromatography materials described herein may be optimized for separation of the multispecific antibody from impurities. In some embodiments, loading on the solution comprising the multispecific antibody onto the chromatography material is optimized for binding of the multispecific antibody to the chromatography material when used in bind and elute mode (*e.g.*, affinity chromatography, mixed-mode chromatography and ion exchange chromatography where designated herein). For example, the solution comprising the multispecific antibody may be loaded onto the chromatography material, *e.g.* a chromatography column, in a load buffer at a number of different pH while the conductivity of the load buffer is constant. Alternatively, the solution comprising the multispecific antibody may be loaded onto the chromatography material in a load buffer at a number of different conductivities while the pH of the load buffer is constant. Upon completion of loading the solution comprising the multispecific antibody on the chromatography material and elution of the multispecific antibody from the chromatography material into a pool fraction, the amount of contaminant in the pool fraction provides information regarding the separation of the product from the contaminants for a given pH or conductivity. Likewise, for chromatography where the multispecific antibody flows through the chromatography material (*e.g.*, HIC) the load buffer is optimized for pH and conductivity such that the multispecific antibody flows through the chromatography whereas impurities are retained by the chromatography material or flow through the chromatography material at a different rate than the multispecific antibodies.

In some embodiments, the loading density of the solution comprising the multispecific antibody or antibody arms is greater than about any of 10 g/L, 20 g/L, 30 g/L, 40 g/L, 50 g/L, 60 g/L, 70 g/L, 80 g/L, 90 g/L, 100 g/L, 110 g/L, 120 g/L, 130 g/L, 140 g/L, or 150 g/L of the affinity chromatography material (*e.g.*, protein A chromatography material). In some embodiments, the loading density of the solution comprising the multispecific antibody or antibody arms is between about any of 10 g/L and 20 g/L, 20 g/L and 30 g/L, 30 g/L and 40 g/L, 40 g/L and 50 g/L, 50 g/L and 60 g/L, 60 g/L and 70 g/L, 70 g/L and 80 g/L, 80 g/L and 90 g/L, 90 g/L and 100 g/L, of the affinity chromatography material (*e.g.*, protein A chromatography material).

In some embodiments of any of the methods described herein, the eluate of the affinity chromatography is loaded onto a mixed-mode chromatography material at a loading density of the polypeptide of greater than about any of 30 g/L, 40 g/L, 50 g/L, 60 g/L, 70 g/L, 80 g/L, 90 g/L, 100 g/L, 110 g/L, 120 g/L, 130 g/L, 140 g/L, or 150 g/L of the mixed-mode chromatography material (*e.g.*, Capto™ adhere chromatography material). In some embodiments, the loading density of the affinity chromatography eluate is between about any of 10 g/L and 20 g/L, 20 g/L and 30 g/L, 30 g/L and 40 g/L, 40 g/L and 50 g/L, 50 g/L and 60 g/L, 60 g/L and 70 g/L, 70 g/L and 80 g/L, 80 g/L and 90 g/L, 90 g/L and 100 g/L, of the mixed-mode chromatography material (*e.g.*, Capto™ adhere chromatography material).

In some embodiments of any of the methods described herein, the eluate of the mixed-mode chromatography or anion exchange chromatography is loaded onto a HIC chromatography material at a loading density of the polypeptide of greater than about any of 30 g/L, 40 g/L, 50 g/L, 60 g/L, 70 g/L, 80 g/L, 90 g/L, 100 g/L, 110 g/L, 120 g/L, 130 g/L, 140 g/L, or 150 g/L of the HIC chromatography material (*e.g.*, Phenyl Sepharose® or Butyl Sepharose® chromatography material). In some embodiments, the loading density of the eluate of the mixed-mode chromatography or anion exchange chromatography is between about any of 10 g/L and 20 g/L, 20 g/L and 30 g/L, 30 g/L and 40 g/L, 40 g/L and 50 g/L, 50 g/L and 60 g/L, 60 g/L and 70 g/L, 70 g/L and 80 g/L, 80 g/L and 90 g/L, 90 g/L and 100 g/L, of the mixed-mode chromatography material (*e.g.*, Phenyl Sepharose® or Butyl Sepharose® chromatography material).

In some embodiments of any of the methods described herein, the eluate of the mixed-mode chromatography is loaded onto an anion exchange chromatography material at a loading density of the polypeptide of greater than about any of 30 g/L, 40 g/L, 50 g/L, 60 g/L, 70 g/L, 80 g/L, 90 g/L, 100 g/L, 110 g/L, 120 g/L, 130 g/L, 140 g/L, or 150 g/L of the anion exchange chromatography material (e.g., QSFF chromatography material). In some embodiments, the loading density of the eluate of the mixed-mode chromatography is between about any of 10 g/L and 20 g/L, 20 g/L and 30 g/L, 30 g/L and 40 g/L, 40 g/L and 50 g/L, 50 g/L and 60 g/L, 60 g/L and 70 g/L, 70 g/L and 80 g/L, 80 g/L and 90 g/L, 90 g/L and 100 g/L, of the anion exchange chromatography material (*e.g.*, QSFF chromatography material).

In some embodiments of any of the methods described herein, the fraction comprising the multispecific antibody from the HIC chromatography is loaded onto a cation exchange chromatography material at a loading density of the polypeptide of greater than about any of 30 g/L, 40 g/L, 50 g/L, 60 g/L, 70 g/L, 80 g/L, 90 g/L, 100 g/L, 110 g/L, 120 g/L, 130 g/L, 140 g/L, or 150 g/L of the cation exchange chromatography material (*e.g.*, POROS® HS 50 chromatography material). In some embodiments, the loading density of the fraction comprising the multispecific antibody from the HIC chromatography is between about any of 10 g/L and 20 g/L, 20 g/L and 30 g/L, 30 g/L and 40 g/L, 40 g/L and 50 g/L, 50 g/L and 60 g/L, 60 g/L and 70 g/L, 70 g/L and 80 g/L, 80 g/L and 90 g/L, 90 g/L and 100 g/L, of the cation exchange chromatography material (*e.g.*, POROS® HS 50 chromatography material).

In some aspects of the disclosure of any of the methods described herein, the fraction comprising the multispecific antibody from the anion exchange chromatography is loaded onto a hydroxyapatite chromatography material at a loading density of the polypeptide of less than about any of 5 g/L, 10 g/L, 15 g/L, 20 g/L, 25 g/L, 30 g/L, 40 g/L, 50 g/L, 60 g/L, 70 g/L, 80 g/L, 90 g/L, 100 g/L, 110 g/L, 120 g/L, 130 g/L, 140 g/L, or 150 g/L of the hydroxyapatite chromatography material (*e.g.*, CHT Type I ceramic hydroxyapatite chromatography material). In some aspects of the disclosure, the loading density of the fraction comprising the multispecific antibody from the HIC chromatography is between about any of 5 g/L and 10 g/L, 10 g/L and 15 g/L, 15 g/L and 20 g/L, 20 g/L and 25 g/L, 25 g/L and 30 g/L, 30 g/L and 50 g/L, 50 g/L and 100 g/L, and 100 g/L and 150 g/L of the hydroxyapatite chromatography material (*e.g.*, CHT Type I ceramic hydroxyapatite chromatography material).

Elution of the multispecific antibody from the chromatography material may be optimized for yield of product with minimal contaminants and at minimal pool volume. For example, the solution containing the multispecific antibody or antibody arms may be loaded onto the chromatography material, *e.g.* a chromatography column, in a load buffer. Upon completion of load, the multispecific antibody or antibody arm is eluted with buffers at a number of different pH while the conductivity of the elution buffer is constant. Alternatively, the multispecific antibody or antibody arm may be eluted from the chromatography material in an elution buffer at a number of different conductivities while the pH of the elution buffer is constant. Upon completion of elution of the multispecific antibody or antibody arm from the chromatography material, the amount of impurities in the pool fraction provides information regarding the separation of the multispecific antibody or antibody arm from the impurities for a given pH or conductivity. Elution of the multispecific antibody or antibody arm in a high number of fractions (*e.g.* eight column volumes) indicates "tailing" of the elution profile. In some embodiments of the invention, tailing of the elution is minimized.

Various buffers which can be employed depending, for example, on the desired pH of the buffer, the desired conductivity of the buffer, the characteristics of the protein of interest, and the purification method. In some embodiments of any of the methods described herein, the methods comprise using a buffer. The buffer can be a loading buffer, an equilibration buffer, or a wash buffer. In some embodiments, one or more of the loading buffer, the equilibration buffer, and/or the wash buffer are the same. In some embodiments, the loading buffer, the equilibration buffer, and/or the wash buffer are different. In some embodiments of any of the methods described herein, the buffer comprises a salt. The loading buffer may comprise sodium chloride, sodium acetate, or a mixture thereof. In some embodiments, the loading buffer is a sodium chloride buffer. In some embodiments, the loading buffer is a sodium acetate buffer. In some embodiments, the buffer comprises Tris. In some embodiments, the buffer comprises arginine.

Load, as used herein, is the composition loaded onto a chromatography material. Loading buffer is the buffer used to load a solution comprising the multispecific antibody or antibody arm onto a chromatography material. The chromatography material may be equilibrated with an equilibration buffer prior to loading the solution which is to be purified. In some examples, the wash buffer is used after loading the solution onto a chromatography material and before elution of the multispecific antibody or antibody arm from the solid phase. However, some of the product of interest, e.g. a multispecific antibody, may be removed from the chromatography material by the wash buffer (e.g. flow-through mode in the case of HIC).

Elution, as used herein, is the removal of the product, e.g. multispecific antibody or antibody arm, from the chromatography material. Elution buffer is the buffer used to elute the multispecific antibody or other product of interest from a chromatography material. In many cases, an elution buffer has a different physical characteristic than the load buffer. For example, the elution buffer may have a different conductivity than load buffer or a different pH than the load buffer. In some embodiments, the elution buffer has a lower conductivity than the load buffer. In some embodiments, the elution buffer has a higher conductivity than the load buffer. In some embodiments, the elution buffer has a lower pH than the load buffer. In some embodiments, the elution buffer has a higher pH than the load buffer. In some embodiments the elution buffer has a different conductivity and a different pH than the load buffer. The elution buffer can have any combination of higher or lower conductivity and higher or lower pH.

Conductivity refers to the ability of an aqueous solution to conduct an electric current between two electrodes. In solution, the current flows by ion transport. Therefore, with an increasing amount of ions present in the aqueous solution, the solution will have a higher conductivity. The basic unit of measure for conductivity is the Siemen (or mho), mho (mS/cm), and can be measured using a conductivity meter, such as various models of Orion conductivity meters. Since electrolytic conductivity is the capacity of ions in a solution to carry electrical current, the conductivity of a solution may be altered by changing the concentration of ions therein. For example, the concentration of a buffering agent and/or the concentration of a salt (*e.g.* sodium chloride, sodium acetate, or potassium chloride) in the solution may be altered in order to achieve the desired conductivity. Preferably, the salt concentration of the various buffers is modified to achieve the desired conductivity.

In some embodiments of any of the methods described herein, the load buffer has a conductivity of greater than about any of 1.0 mS/cm, 1.5 mS/cm, 2.0 mS/cm, 2.5 mS/cm, 3.0 mS/cm, 3.5 mS/cm, 4.0 mS/cm, 4.5 mS/cm, 5.0 mS/cm, 5.5 mS/cm, 6.0 mS/cm, 6.5 mS/cm, 7.0 mS/cm, 7.5 mS/cm, 8.0 mS/cm, 8.5 mS/cm, 9.0 mS/cm, 9.5 mS/cm, 10 mS/cm or 20 mS/cm. The conductivity may be between about any of 1 mS/cm and 20 mS/cm, 4 mS/cm and 10 mS/cm, 4 mS/cm and 7 mS/cm, 5 mS/cm and 17 mS/cm, 5 mS/cm and 10 mS/cm, or 5 mS/cm and 7 mS/cm. In some embodiments, the conductivity is about any of 1.0 mS/cm, 1.5 mS/cm, 2.0 mS/cm, 2.5 mS/cm, 3.0 mS/cm, 3.5 mS/cm, 4 mS/cm, 4.5 mS/cm, 5.0 mS/cm, 5.5 mS/cm, 6.0 mS/cm, 6.5 mS/cm, 7.0 mS/cm, 7.5 mS/cm, 8.0 mS/cm, 8.5 mS/cm, 9.0 mS/cm, 9.5 mS/cm, 10 mS/cm or 20 mS/cm. In one aspect, the conductivity is the conductivity of the loading buffer, the equilibration buffer, and/or the wash buffer. In some embodiments, the conductivity of one or more of the loading buffer, the equilibration buffer, and the wash buffer are the same. In some embodiments, the conductivity of the loading buffer is different from the conductivity of the wash buffer and/or equilibration buffer.

In some embodiments, the elution buffer has a conductivity less than the conductivity of the load buffer. In some embodiments of any of the methods described herein, the elution buffer has a conductivity of less than about any of 0.0 mS/cm, 0.5 mS/cm, 1.0 mS/cm, 1.5 mS/cm, 2.0 mS/cm, 2.5 mS/cm, 3.0 mS/cm, 3.5 mS/cm, 4.0 mS/cm, 4.5 mS/cm, 5.0 mS/cm, 5.5 mS/cm, 6.0 mS/cm, 6.5 mS/cm, or 7.0 mS/cm. The conductivity may be between about any of 0 mS/cm and 7 mS/cm, 1 mS/cm and 7 mS/cm, 2 mS/cm and 7 mS/cm, 3 mS/cm and 7 mS/cm, or 4 mS/cm and 7 mS/cm, 0 mS/cm and 5.0 mS/cm, 1 mS/cm and 5 mS/cm, 2 mS/cm and 5 mS/cm, 3 mS/cm and 5 mS/cm, or 4 mS/cm and 5 mS/cm. In some embodiments, the conductivity of the elution buffer is about any of 0.0 mS/cm, 0.5 mS/cm, 1.0 mS/cm, 1.5 mS/cm, 2.0 mS/cm, 2.5 mS/cm, 3.0 mS/cm, 3.5 mS/cm, 4 mS/cm, 4.5 mS/cm, 5.0 mS/cm, 5.5 mS/cm, 6.0 mS/cm, 6.5 mS/cm, or 7.0 mS/cm.

In some embodiments, the elution buffer has a conductivity greater than the conductivity of the load buffer. In some embodiments of any of the methods described herein, the elution buffer has a conductivity of greater than about any of 5.5 mS/cm, 6.0 mS/cm, 6.5 mS/cm, 7.0 mS/cm, 7.5 mS/cm, 8.0 mS/cm, 8.5 mS/cm, 9.0 mS/cm, 9.5 mS/cm, 10 mS/cm, 11 mS/cm, 12 mS/cm, 13 mS/cm, 14 mS/cm, 15 mS/cm, 16 mS/cm, 17.0 mS/cm, 18.0 mS/cm, 19.0 mS/cm, 20.0 mS/cm, 21.0 mS/cm, 22.0 mS/cm, 23.0 mS/cm, 24.0 mS/cm, 25.0 mS/cm, 26.0 mS/cm, 27.0 mS/cm, 28.0 mS/cm, 29.0 mS/cm, or 30.0 mS/cm. The conductivity may be between about any of 5.5 mS/cm and 30 mS/cm, 6.0 mS/cm and 30 mS/cm, 7 mS/cm and 30 mS/cm, 8 mS/cm and 30 mS/cm, 9 mS/cm and 30 mS/cm, or 10 mS/cm and 30 mS/cm. In some embodiments, the conductivity of the elution buffer is about any of 5.5 mS/cm, 6.0 mS/cm, 6.5 mS/cm, 7.0 mS/cm, 7.5 mS/cm, 8.0 mS/cm, 8.5 mS/cm, 9.0 mS/cm, 9.5 mS/cm, 10 mS/cm, 11 mS/cm, 12 mS/cm, 13 mS/cm, 14 mS/cm, 15 mS/cm, 16 mS/cm, 17.0 mS/cm 18.0 mS/cm, 19.0 mS/cm, 20.0 mS/cm, 21.0 mS/cm, 22.0 mS/cm, 23.0 mS/cm, 24.0 mS/cm, 25.0 mS/cm, 26.0 mS/cm, 27.0 mS/cm, 28.0 mS/cm, 29.0 mS/cm, or 30.0 mS/cm. In some aspects of any of the above embodiments, the conductivity of the elution buffer changed from the load and/or wash buffer by step gradient or by linear gradient.

In some embodiments of the invention, the solution comprising the multispecific antibody is loaded onto a mixed-mode chromatography material in a buffer with a conductivity of about <6.5 mS/cm and the polypeptide is eluted from the chromatography material in an elution buffer with a conductivity of about 1.5 mS/cm. In some embodiments, the load buffer has a conductivity of about 6.5 mS/cm and the elution buffer has a conductivity of about 3 mS/cm. In some embodiments, the load buffer has a conductivity of about 5.5 mS/cm and the elution buffer has a conductivity of about 2 mS/cm. In some embodiments, the load buffer has a conductivity of about 5.5 mS/cm and the elution buffer has a conductivity of about 1 mS/cm. In further embodiments of the above embodiments, the chromatography material is a Capto™ adhere resin.

In some aspects of any of the above embodiments, the conductivity of the elution buffer changed from the load and/or wash buffer by step gradient or by linear gradient. In some embodiments, the composition comprising a multispecific antibody is loaded onto a mixed-mode chromatography (*e.g,* a Capto™ ahere chromatography) at <6.5 mS/cm and the multispecific antibody is eluted from the mixed-mode chromatography by a step conductivity gradient to about 1.5 mS/cm. In some embodiments, the composition comprising a multispecific antibody is loaded onto an anion exchange chromatography (*e.g.,* a QSFF chromatography) at <2.5 mS/cm and the multispecific antibody is eluted from the anion exchange chromatography by a step conductivity gradient to about 8.6 mS/cm. In some embodiments, the composition comprising a multispecific antibody is loaded onto a cation exchange chromatography (*e.g.,* a POROS® HS 50 chromatography) at about 5.0 mS/cm and the multispecific antibody is eluted from the cation exchange chromatography by a step conductivity gradient to about 27.5 mS/cm.

In some embodiments, the composition comprising a multispecific antibody is loaded onto a hydroxyapatite chromatography (*e.g,* a CHT Type I ceramic hydroxyapatite chromatography) and the multispecific antibody is eluted from the hydroxyapatite chromatography by a linear conductivity gradient. In some embodiments, the multispecific antibody is loaded onto a hydroxyapatite chromatography in a hydroxyapatite buffer A comprising about 10 mM sodium phosphate. In some embodiments, the linear gradient is formed by gradually adding hydroxyapatite buffer B comprising about 10 mM sodium phosphate, about 1 M sodium chloride. In some embodiments, the hydroxyapaptite buffer A and/or B is about any of pH 6.3, 6.4, 6.5, 6.6, 6.7, 6.8, 6.9, 7.0, 7.1, 7.2, 7.3. In some embodiments, the hydroxyapaptite buffer A and/or B is about pH 6.8. In some embodiments, the elution buffer increases in hydroxyapatite buffer B content over the gradient. In some emobiments, the linear gradient begins at about 0% hydroxyapatite buffer B and ends at about 100% hydroxyapatite buffer B. In some embodiments, the linear gradient begins at about 0% hydroxyapatite buffer B and ends at about 100% hydroxyapatite buffer B over any of about 10, 15, 20, 25 or 30 column volumes (CV). In some embodiments, the linear gradient begins at about 0% hydroxyapatite buffer B and ends at about 100% hydroxyapatite buffer B over about 20 column volumes (CV).

In some embodiments of any of the methods described herein, the load buffer has a pH of less than about any of 10, 9, 8, 7, 6, or 5. In some embodiments of any of the methods described herein, the load buffer has a pH of greater than about any of 4, 5, 6, 7, 8, or 9. The load buffer may have a pH of between about any of 4 and 9, 4 and 8, 4 and 7, 5 and 9, 5 and 8, 5 and 7, 5 and 6. In some embodiments, the pH of the load buffer is about any of 4, 4.5, 5, 5.5, 6, 6.5, 7, 7.5, or 8. The pH can be the pH of the loading buffer, the equilibration buffer, or the wash buffer. In some embodiments, the pH of one or more of the loading buffer, the equilibration buffer, and/or the wash buffer are the same. In some embodiments, the pH of the loading buffer is different from the pH of the equilibration buffer and/or the wash buffer.

In some embodiments, the elution buffer has a pH less than the pH of the load buffer. In some embodiments of any of the methods described herein, the elution buffer has a pH of less than about any of 8, 7, 6, 5, 4, 3 or 2. The pH of the elution buffer may be between about any of 4 and 9, 4 and 8, 4 and 7, 4 and 6, 4 and 5, 5 and 9, 5 and 8, 5 and 7, 5 and 6, 6 and 9, 6 and 8, 6 and 7. In some embodiments, the pH of the elution buffer is about any of 4.0, 4.5, 5.0. 5.5, 6.0, 6.5, 7.0, 7.5, 8.0, 8.5 or 9.0.

In some embodiments, the elution buffer has a pH greater than the pH of the load buffer. In some embodiments of any of the methods described herein, the elution buffer has a pH of greater than about any of 5, 6, 7, 8, or 9. The pH of the elution buffer may be between about any of 4 and 9, 5 and 9, 6 and 9, 7 and 9, 8 and 9, 4 and 8, 5 and 8, 6 and 8, 7 and 8, 4 and 7, 5 and 7, and 6 and 7. In some embodiments, the pH of the elution buffer is about any of 4.0, 4.5, 5.0. 5.5, 6.0, 6.5, 7/0, 7.5, 8.0, 8.5 or 9.0.

In some embodiments, the solution comprising a multispecific antibody or antibody arm is loaded onto an affinity chromatography (*e.g.,* a Protein A chromatography) at about pH 7.7 and the multispecific antibody or antibody arm is eluted from the affinity chromatography by a step gradient to pH of about 2.9.

In some aspects of any of the above embodiments, the pH of the elution buffer changed from the load and/or wash buffer by step gradient or by linear gradient.

In some embodiments of any of the methods described herein, the flow rate is less than about any of 50 CV/hr, 40 CV/hr, or 30 CV/hr. The flow rate may be between about any of 5 CV/hr and 50 CV/hr, 10 CV/hr and 40 CV/hr, or 18 CV/hr and 36 CV/hr. In some embodiments, the flow rate is about any of 9 CV/hr, 18 CV/hr, 25 CV/hr, 30 CV/hr, 36 CV/hr, or 40 CV/hr. In some embodiments of any of the methods described herein, the flow rate is less than about any of 100 cm/hr, 75 cm/hr, or 50 cm/hr. The flow rate may be between about any of 25 cm/hr and 150 cm/hr, 25 cm/hr and 100 cm/hr, 50 cm/hr and 100 cm/hr, or 65 cm/hr and 85 cm/hr.

Bed height is the height of chromatography material used. In some embodiments of any of the method described herein, the bed height is greater than about any of 5 cm, 10 cm, 15 cm, 20 cm, 25 cm, 30 cm, 35 cm, 40 cm, 45 cm, or 50 cm. In some embodiments, the bed heigt is between about 5 cm and 50 cm. In some embodiments, bed height is determined based on the amount of polypeptide or contaminants in the load.

In some embodiments, the chromatography is in a column of vessel with a volume of greater than about 1 mL, 2 mL, 3 mL, 4 mL, 5 mL, 6 mL, 7 mL, 8 mL, 9 mL, 10 mL, 15 mL, 20 mL, 25 mL, 30 mL, 40 mL, 50 mL, 75 mL, 100 mL, 200 mL, 300 mL, 400 mL, 500 mL, 600 mL, 700 mL, 800 mL, 900 mL, 1 L, 2 L, 3 L, 4 L, 5 L, 6 L, 7 L, 8 L, 9 L, 10 L, 25 L, 50 L, 100 L, 200 L, 300 L, 400 L, 500 L, 600 L, 700 L, 800 L, 900 L or 100 L.

In some embodiments of the invention, fractions are collected from the chromatography. In some embodiments, fractions collected are greater than about 0.01 CV, 0.02 CV, 0.03 CV, 0.04 CV, 0.05 CV, 0.06 CV, 0.07 CV, 0.08 CV, 0.09 CV, 0.1 CV, 0.2 CV, 0.3 CV, 0.4 CV, 0.5 CV, 0.6 CV, 0.7 CV, 0.8 CV, 0.9 CV, 1.0 CV, 2.0 CV, 3.0 CV, 4.0 CV, 5.0 CV, 6.0 CV, 7.0 CV, 8.0 CV, 9.0 CV, or 10.0 CV. In some embodiments, fractions containing the product, *e.g.* multispecific antibody or antibody arm, are pooled. The amount of polypeptide in a fraction can be determined by one skilled in the art; for example, the amount of polypeptide in a fraction can be determined by UV spectroscopy. In some embodiments, fractions are collected when the $OD_{280}$ is greater than about any of 0.5, 0.6, 0.7, 0.8, 0.9 and 1.0. In some embodiments, fractions are collected when the $OD_{280}$ is between about any of 0.5 and 1.0, 0.6 and 1.0, 0.7 and 1.0, 0.8 and 1.0, or 0.9 and 1.0. In some embodiments, fractions containing detectable multispecific antibodies or antibody arms are pooled.

In some embodiments of any of the methods described herein, the impurity is a product specific impurity. Examples of product specific impurities include but are not limited to unpaired half-antibodies, un-paired antibody chains, antibody fragments, homodimers, aggregates, high molecular weight species, very high molecular weight species, low molecular weight species, and charge variants such as acidic variants and basic variants of the antibody.

In some embodiments, the methods provide for the reduction or removal unpaired half antibodies and/or homodimers (*e.g.*, paired half dimers that comprise the same binding domains). Methods of measuring the presence of unpaired half-antibodies and homodimers are known in the art; for example, by hydrophobic interaction chromatography including hydrophobic interaction chromatography high performance liquid chromatography (HIC-HPLC). In some embodiments of any of the methods described herein, the amount of half-antibodies and/or homodimers is reduced by greater than about any of 5%, 10%, 20%, 30%, 40%, 50%, 60%, 70%, 80%, 90%, or 95%. The amount of half-antibodies and/or homodimers may be reduced by between about any of 10% and 99%, 30% and 95%, 30% and 99%, 50% and 95%, 50% and 99%, 75% and 99%, or 85% and 99%. In some embodiments, the reduction is determined by comparing the amount of half-antibodies and/or homodimers in the composition recovered from a purification step(s) to the amount of half-antibodies and/or homodimers in the composition before the purification step(s).

Aggregated polypeptide (*e.g.*, aggregated multispecic antibody or aggregated antibody arm) can be high molecular weight (HMW) protein. In some embodiments, the aggregated polypeptide is multimers of the polypeptide of interest. The HMW protein may be a dimer, up to 8x monomer, or larger of the polypeptide of interest. Methods of measuring aggregated protein (*e.g.*, HMW protein) are known in the art and described in the examples section. In some embodiments of any of the methods described herein, the amount of aggregated protein is reduced by greater than about any of 5%, 10%, 20%, 30%, 40%, 50%, 60%, 70%, 80%, 90%, or 95%. The amount of aggregated protein may be reduced by between about any of 10% and 99%, 30% and 95%, 30% and 99%, 50% and 95%, 50% and 99%, 75% and 99%, or 85% and 99%. The amount of aggregated protein may be reduced by about any of 5%, 10%, 20%, 30%, 40%, 50%, 60%, 70%, 80%, 90%, or 95%. In some embodiments, the reduction is determined by comparing the amount of aggregated protein (e.g., HMW protein) in the composition recovered from a purification step(s) to the amount of aggregated protein (e.g., HMW protein) in the composition before the purification step(s).

Fragment polypeptide can be low molecular weight (LMW) protein. In some embodiments, the fragmented polypeptide is a fragment of the multispecific antibody or antibody arm of interest. Examples of LMW protein include, but not limited to, a Fab (Fragment antigen binding), Fc (fragment, crystallizable) regions or combination of both or any random fragmented part of an antibody of interest. Methods of measuring fragmented protein (*e.g.*, LMW protein) are known in the art and described in the examples section. In some embodiments of any of the methods described herein, the amount of LMW protein is reduced by greater than about any of 5%, 10%, 20%, 30%, 40%,50%, 60%, 70%, 80%, 90%, or 95%. The amount of LMW protein may be reduced by between about any of 10% and 99%, 30% and 95%, 30% and 99%, 50% and 95%, 50% and 99%, 75% and 99%, or 85% and 99%. The amount of LMW protein may be reduced by about any of 5%, 10%, 20%, 30%, 40%, 50%, 60%, 70%, 80%, 90%, or 95%. In some embodiments, the reduction is determined by comparing the amount of fragmented protein (*e.g.*, LMW protein) in the composition recovered from a purification step(s) to the amount of fragmented protein (*e.g.*, LMW protein) in the composition before the purification step(s).

Acidic variants of an antibody are variants in which the pI of the antibody is less than the pI of the native intact antibody. Basic variants of an antibody are variants in which the pI of the antibody is greater that the pI of the native intact antibody. Charge variants (*e.g.*, acidic and basic variants) may be the result of natural processes such as oxidation, deamidation, C-terminal processing of lysine residues, N-terminal pyroglutamate formation, and glycation of the antibody.

Methods of measuring charge variants are known in the art; for example, imaging capillary IsoElectric Focusing (cIEF). In some embodiments of any of the methods described herein, the amount of charge variants (acidic and/or basic variants) is reduced by greater than about any of 5%, 10%, 20%, 30%, 40%, 50%, 60%, 70%, 80%, 90%, or 95%. The amount of charge variants (acidic and/or basic variants) may be reduced by between about any of 10% and 99%, 30% and 95%, 30% and 99%, 50% and 95%, 50% and 99%, 75% and 99%, or 85% and 99%. In some embodiments, the reduction is determined by comparing the amount of charge variants (acidic and/or basic variants) in the composition recovered from a purification step(s) to the amount of charge variants in the composition before the purification step(s).

In some embodiments of any of the methods described herein, the impurity is a process specific impurity. For example, the impurity may be any one or more of host cell materials; a chaperone such as FkpA, DsbA and DspC; leached Protein A; nucleic acid; another polypeptide; endotoxin; viral contaminant; cell culture media component, carboxypeptidase B, gentamicin, *etc.* In some examples, the contaminant may be a host cell protein (HCP) from, for example but not limited to, a bacterial cell such as an *E. coli* cell, an insect cell, a prokaryotic cell, a eukaryotic cell, a yeast cell, a mammalian cell, an avian cell, a fungal cell.

Host cell proteins (HCP) are proteins from the cells in which the polypeptide was produced. For example, ECP are proteins from host cells, *i.e.*, *E. coli* Proteins. The amount of CHOP may be measured by enzyme-linked immunosorbent assay ("ELISA"). In some embodiments of any of the methods described herein, the amount of HCP (e.g. ECP) is reduced by greater than about any of 10%, 20%, 30%, 40%, 50%, 60%, 70%, 80%, 90%, or 95%. The amount of HCP may be reduced by between about any of 10% and 99%, 30% and 95%, 30% and 99%, 50% and 95%, 50% and 99%, 75% and 99%, or 85% and 99%. In some embodiments, the amount of HCP is reduced by about any of 10%, 20%, 30%, 40%, 50%, 60%, 70%, 80%, 85%, 90%, 95%, or 98%. In some embodiments, the reduction is determined by comparing the amount of HCP in the composition recovered from a purification step(s) to the amount of HCP in the composition before the purification step(s).

Chaperone proteins, *e.g.*, FkpA, DsbA and/or DsbC, are often used in the production of antibodies in cells to facilitate folding and assembly of antibodies. Chaperones may be detected by ELISA using antibodies that specifically bind FkpA, DsbA or DsbC. For example, antibodies may be generated against ultrapure compositions of FkpA, DsbA or DsbC. In some embodiments, FkpA, DsbA and/or DsbC are determined by mass spectrometry.

Leached Protein A is Protein A detached or washed from a solid phase to which it is bound. For example, leached Protein A can be leached from Protein A chromatography column. The amount of Protein A may be measured, for example, by ELISA. In some embodiments of any of the methods described herein, the amount of leached Protein A is reduced by greater than about any of 10%, 20%, 30%, 40%, 50%, 60%, 70%, 80%, or 90%. The amount of leached Protein A may be reduced by between about any of 10% and 99%, 30% and 95%, 30% and 99%, 50% and 95%, 50% and 99%, 75% and 99%, or 85% and 99%. In some embodiments, the amount of leached Protein A is reduced by about any of 10%, 20%, 30%, 40%, 50%, 60%, 70%, 80%, 90%, or 95%. In some embodiments, the reduction is determined by comparing the amount of leached Protein A in the composition recovered from a purification step(s) to the amount of leached Protein A in the composition before the purification step(s).

Methods of measuring DNA such as host cell DNA are known in the art and described in the examples section. In some embodiments of any of the methods described herein, the amount of DNA is reduced by greater than about any of 10%, 20%, 30%, 40%, 50%, 60%, 70%, 80%, or 90%. The amount of DNA may be reduced by between about any of 10% and 99%, 30% and 95%, 30% and 99%, 50% and 95%, 50% and 99%, 75% and 99%, or 85% and 99%. The amount of DNA may be reduced by about any of 10%, 20%, 30%, 40%, 50%, 60%, 70%, 80%, 90%, 95%, or 99%. In some embodiments, the reduction is determined by comparing the amount of DNA in the composition recovered from a purification step(s) to the amount of DNA in the composition before the purification step(s).

Cell culture media component refers to a component present in a cell culture media. A cell culture media may be a cell culture media at the time of harvesting cells. In some embodiments, the cell culture media component is gentamicin. The amount of gentamicin may be measured by ELISA. In some embodiments of any of the methods described herein, the amount of cell culture media component is reduced by greater than about any of 10%, 20%, 30%, 40%, 50%, 60%, 70%, 80%, or 90%. The amount of cell culture media component may be reduced by between about any of 10% and 99%, 30% and 95%, 30% and 99%, 50% and 95%, 50% and 99%, 75% and 99%, or 85% and 99%. In some embodiments, the amount of cell culture media component is reduced by about any of 10%, 20%, 30%, 40%, 50%, 60%, 70%, 80%, 90%, 95%, or 98%. In some embodiments, the reduction is determined by comparing the amount of cell culture media component in the composition recovered from a purification step(s) to the amount of cell culture media component in the composition before the purification step(s).

In some embodiments, the multispecific antibodies are further purified by viral filtration. Viral filtration is the removal of viral contaminants in a polypeptide purification feedstream. Examples of viral filtration include ultrafiltration and microfiltration. In some embodiments the polypeptide is purified using a parvovirus filter.

In some embodiments, the multispecific antibody is concentrated after chromatography (*e.g.*, after HIC chromatography or after cation exchange chromatography). Examples of concentration methods are known in the art and include but are not limited to ultrafiltration and diafiltration. In some embodiments, the multispecific antibody is concentrated by a first ultrafiltraton, a diafiltration and a second ultrafiltration. In some embodiments, the ultrafiltration and/or diafiltration uses a filter with a cut off of less than about any of 5 kDal, 10 kDal, 15 kDal, 20 kDal, or 25 kDal. In some embodiments, the retentate of the first ultrafiltration is diafiltered into a pharmaceutical formulation.

In some embodiments of any of the methods described herein, the methods further comprise combining the purified polypeptide of the methods of purification with a pharmaceutically acceptable carrier. In some embodiments, the multispecific antibody is formulated into a pharmaceutical formulation by ultrafiltration/diafiltration.

In some embodiments, the concentration of the polypeptide (*e.g.*, multispecific antibody) is more than about any of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, 150 mg/mL, 160 mg/mL, 170 mg/mL, 180 mg/mL, 190 mg/mL, 200 mg/mL, or 300 mg/mL. In some embodiments, the concentration of multispecific antibody is between about any of 10 mg/mL and 20 mg/mL, 20 mg/mL and 30 mg/mL, 30 mg/mL and 40 mg/mL, 40 mg/mL and 50 mg/mL, 50 mg/mL and 60 mg/mL, 60 mg/mL and 70 mg/mL, 70 mg/mL and 80 mg/mL, 80 mg/mL and 90 mg/mL, 90 mg/mL and 100 mg/mL, 100 mg/mL and 110 mg/mL, 110 mg/mL and 120 mg/mL, 120 mg/mL and 130 mg/mL, 130 mg/mL and 140 mg/mL, 140 mg/mL and 150 mg/mL, 150 mg/mL and 160 mg/mL, 160 mg/mL and 170 mg/mL, 170 mg/mL and 180 mg/mL, 180 mg/mL and 190 mg/mL, 190 mg/mL and 200 mg/mL, 200 mg/mL and 300 mg/mL.

XII. Formulations and Methods of Making of the Formulations

Disclosed herein are also formulations and methods of making the formulation comprising the multispecific antibodies purified by the methods described herein. For example, the purified polypeptide may be combined with a pharmaceutically acceptable carrier.

The polypeptide formulations in some aspects of the disclosuremay be prepared for storage by mixing a polypeptide having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution.

Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.* Zn-protein complexes); and/or non-ionic surfactants such as TWEEN™, PLURONICS™ or polyethylene glycol (PEG).

In some aspects of the disclosure, the polypeptide in the polypeptide formulation maintains functional activity.

The formulations to be used for *in vivo* administration must be sterile. This is readily accomplished by filtration through sterile filtration membranes.

The formulations herein may also contain more than one active compound as necessary for the particular indication being treated, preferably those with complementary activities that do not adversely affect each other. For example, in addition to a polypeptide, it may be desirable to include in the one formulation, an additional polypeptide (*e.g.*, antibody). Alternatively, or additionally, the composition may further comprise a chemotherapeutic agent, cytotoxic agent, cytokine, growth inhibitory agent, anti-hormonal agent, and/or cardioprotectant. Such molecules are suitably present in combination in amounts that are effective for the purpose intended.

XIII. Compositions of Polypeptides Produced in Cells Expressing FkpA

In some aspects, the disclosed herein are compositions comprising a recombinant polypeptide produced in a host cell that expresses FkpA. In some aspects of the disclosure, the recombinant polypeptide in the composition has purified from host cell proteins including FkpA. In some aspects of the disclosure, the composition comprises a recombinant polypeptide produced in a host cell that expresses FkpA and a pharmaceutically acceptable excipient.

In some aspects of the disclosure, the composition comprising the recombinant polypeptide comprises less than about or no more than about any one of 50 ppm, 40 ppm, 30 ppm, 20 ppm, 10 ppm, 9 ppm, 8 ppm, 7 ppm, 6 ppm, 5 ppm, 4 ppm, 3 ppm, 2 ppm, 1 ppm, 0.9 ppm, 0.8 ppm, 0.7 ppm, 0.6 ppm, 0.5 ppm, 0.4 ppm, 0.3 ppm, 0.2 ppm, or 0.1 ppm FkpA. In some aspects of the disclosure, the composition comprising the recombinant polypeptide comprises about 0.2 ppm FkpA. In some aspects of the disclosure, the composition comprising the recombinant polypeptide comprises about 0.1 ppm FkpA. In some aspects of the disclosure, the composition comprises the recombinant polypeptide at a concentration of at least about any of 5 mg/ml, 10 mg/ml, 15 mg/ml, 20 mg/ml, 25 mg/ml, 50 mg/ml, 75 mg/ml, 100 mg/ml, 125 mg/ml, 150 mg/ml, 175 mg/ml, 200 mg/ml, 250 mg/ml, or 300 mg/ml. In some aspects of the disclosure, the composition comprises the recombinant polypeptide at a concentration of at least about any of 5 mg/ml, 10 mg/ml, 15 mg/ml, 20 mg/ml, 25 mg/ml, 50 mg/ml, 75 mg/ml, 100 mg/ml, 125 mg/ml, 150 mg/ml, 175 mg/ml, 200 mg/ml, 250 mg/ml, or 300 mg/ml; and less than about any one of 50 ppm, 40 ppm, 30 ppm, 20 ppm, 10 ppm, 9 ppm, 8 ppm, 7 ppm, 6 ppm, 5 ppm, 4 ppm, 3 ppm, 2 ppm, 1 ppm, 0.9 ppm, 0.8 ppm, 0.7 ppm, 0.6 ppm, 0.5 ppm, 0.4 ppm, 0.3 ppm, 0.2 ppm, or 0.1 ppm FkpA. In some aspects of the disclosure, the composition comprising the recombinant polypeptide comprises about 0.2 ppm FkpA. In some aspects of the disclosure, the composition comprising the recombinant polypeptide comprises about 0.1 ppm FkpA. In some aspects of the disclosure, the composition comprises the recombinant polypeptide at a concentration of between about 5 mg/ml and about 100 mg/ml, about 50 mg/ml and about 150 mg/ml, about 100 mg/ml and about 200 mg/ml, about 150 mg/ml and about 250 mg/ml, about 200 mg/ml and about 300 mg/ml; and less than about any one of 50 ppm, 40 ppm, 30 ppm, 20 ppm, 10 ppm, 9 ppm, 8 ppm, 7 ppm, 6 ppm, 5 ppm, 4 ppm, 3 ppm, 2 ppm, 1 ppm, 0.9 ppm, 0.8 ppm, 0.7 ppm, 0.6 ppm, 0.5 ppm, 0.4 ppm, 0.3 ppm, 0.2 ppm, or 0.1 ppm FkpA. In some aspects of the disclosure, the composition comprising the recombinant polypeptide comprises about 0.2 ppm FkpA. In some aspects of the disclosure, the composition comprising the recombinant polypeptide comprises about 0.1 ppm FkpA.

In some aspects of the disclosure, the composition comprising the recombinant polypeptide comprises less than about or no more than about any one of 50 ppm, 40 ppm, 30 ppm, 20 ppm, 10 ppm, 9 ppm, 8 ppm, 7 ppm, 6 ppm, 5 ppm, 4 ppm, 3 ppm, 2 ppm, 1 ppm, 0.9 ppm, 0.8 ppm, 0.7 ppm, 0.6 ppm, 0.5 ppm, 0.4 ppm, 0.3 ppm, 0.2 ppm, or 0.1 ppm DsbA and/or DsbC. In some embodiments, the composition comprises the recombinant polypeptide at a concentration of at least about any of 5 mg/ml, 10 mg/ml, 15 mg/ml, 20 mg/ml, 25 mg/ml, 50 mg/ml, 75 mg/ml, 100 mg/ml, 125 mg/ml, 150 mg/ml, 175 mg/ml, 200 mg/ml, 250 mg/ml, or 300 mg/ml. In some aspects of the disclosure, the composition comprises the recombinant polypeptide at a concentration of at least about any of 5 mg/ml, 10 mg/ml, 15 mg/ml, 20 mg/ml, 25 mg/ml, 50 mg/ml, 75 mg/ml, 100 mg/ml, 125 mg/ml, 150 mg/ml, 175 mg/ml, 200 mg/ml, 250 mg/ml, or 300 mg/ml; and less than about any one of 50 ppm, 40 ppm, 30 ppm, 20 ppm, 10 ppm, 9 ppm, 8 ppm, 7 ppm, 6 ppm, 5 ppm, 4 ppm, 3 ppm, 2 ppm, 1 ppm, 0.9 ppm, 0.8 ppm, 0.7 ppm, 0.6 ppm, 0.5 ppm, 0.4 ppm, 0.3 ppm, 0.2 ppm, or 0.1 ppm DsbA and/or DsbC. In some aspects of the disclosure, the composition comprises the recombinant polypeptide at a concentration of between about 5 mg/ml and about 100 mg/ml, about 50 mg/ml and about 150 mg/ml, about 100 mg/ml and about 200 mg/ml, about 150 mg/ml and about 250 mg/ml, about 200 mg/ml and about 300 mg/ml; and less than about any one of 50 ppm, 40 ppm, 30 ppm, 20 ppm, 10 ppm, 9 ppm, 8 ppm, 7 ppm, 6 ppm, 5 ppm, 4 ppm, 3 ppm, 2 ppm, 1 ppm, 0.9 ppm, 0.8 ppm, 0.7 ppm, 0.6 ppm, 0.5 ppm, 0.4 ppm, 0.3 ppm, 0.2 ppm, or 0.1 ppm DsbA and/or DsbC. Reagents and methods to detect DsbA and DsbC are provided by U.S. Provisional Application No. 62/129,701.

In some aspects of the disclosure, the recombinant polypeptide is an antibody. In some aspects of the disclosure, the recombinant polypeptide is a multispecific antibody. In some aspects of the disclosure, the recombinant polypeptide is a bispecific antibody. In some aspects of the disclosure, the composition comprises a multispecific antibody produced in a cell expressing FkpA; *e.g.*, expressing exogenous FkpA. In some embodaspects of the disclosureiments, the composition comprises a multispecific antibody produced in a cell expressing FkpA, wherein the composition is essentially free of FkpA. In some aspects of the disclosure, the composition comprises a multispecific antibody produced in a cell expressing FkpA, wherein the composition comprises less than about 50 ppm, 40 ppm, 30 ppm, 20 ppm, 10 ppm, 9 ppm, 8 ppm, 7 ppm, 6 ppm, 5 ppm, 4 ppm, 3 ppm, 2 ppm, 1 ppm, 0.9 ppm, 0.8 ppm, 0.7 ppm, 0.6 ppm, 0.5 ppm, 0.4 ppm, 0.3 ppm, 0.2 ppm, or 0.1 ppm FkpA. In some aspects of the disclosure, the composition comprising the recombinant polypeptide comprises about 0.2 ppm FkpA. In some aspects of the disclosure, the composition comprising the recombinant polypeptide comprises about 0.1 ppm FkpA. In some aspects of the disclosure, the composition comprises a multispecific antibody at a concentration of at least about any of 5 mg/ml, 10 mg/ml, 15 mg/ml, 20 mg/ml, 25 mg/ml, 50 mg/ml, 75 mg/ml, 100 mg/ml, 125 mg/ml, 150 mg/ml, 175 mg/ml, 200 mg/ml, 250 mg/ml, or 300 mg/ml. In some aspects of the disclosure, the composition comprises a multispecific antibody at a concentration of at least about any of 5 mg/ml, 10 mg/ml, 15 mg/ml, 20 mg/ml, 25 mg/ml, 50 mg/ml, 75 mg/ml, 100 mg/ml, 125 mg/ml, 150 mg/ml, 175 mg/ml, 200 mg/ml, 250 mg/ml, or 300 mg/ml; and less than about any one of 50 ppm, 40 ppm, 30 ppm, 20 ppm, 10 ppm, 9 ppm, 8 ppm, 7 ppm, 6 ppm, 5 ppm, 4 ppm, 3 ppm, 2 ppm, 1 ppm, 0.9 ppm, 0.8 ppm, 0.7 ppm, 0.6 ppm, 0.5 ppm, 0.4 ppm, 0.3 ppm, 0.2 ppm, or 0.1 ppm FkpA. In some aspects of the disclosure, the composition comprising the recombinant polypeptide comprises about 0.2 ppm FkpA. In some aspects of the disclosure, the composition comprising the recombinant polypeptide comprises about 0.1 ppm FkpA. In some aspects of the disclosure, the composition comprises a multispecific antibody at a concentration of between about 5 mg/ml and about 100 mg/ml, about 50 mg/ml and about 150 mg/ml, about 100 mg/ml and about 200 mg/ml, about 150 mg/ml and about 250 mg/ml, about 200 mg/ml and about 300 mg/ml; and less than about any one of 50 ppm, 40 ppm, 30 ppm, 20 ppm, 10 ppm, 9 ppm, 8 ppm, 7 ppm, 6 ppm, 5 ppm, 4 ppm, 3 ppm, 2 ppm, 1 ppm, 0.9 ppm, 0.8 ppm, 0.7 ppm, 0.6 ppm, 0.5 ppm, 0.4 ppm, 0.3 ppm, 0.2 ppm, or 0.1 ppm FkpA. In some aspects of the disclosure, the composition comprising the recombinant polypeptide comprises about 0.2 ppm FkpA. In some aspects of the disclosure, the composition comprising the recombinant polypeptide comprises about 0.1 ppm FkpA.

In some aspects of the disclosure, the composition comprises a multispecific antibody produced in a cell expressing FkpA; *e.g.*, expressing exogenous FkpA, DsbA and/or DsbC. In some aspects of the disclosure, the composition comprises a multispecific antibody produced in a cell expressing FkpA, wherein the composition is essentially free of FkpA. In some aspects of the disclosure, the composition comprises a multispecific antibody produced in a cell expressing FkpA, wherein the composition comprises less than about 50 ppm, 40 ppm, 30 ppm, 20 ppm, 10 ppm, 9 ppm, 8 ppm, 7 ppm, 6 ppm, 5 ppm, 4 ppm, 3 ppm, 2 ppm, 1 ppm, 0.9 ppm, 0.8 ppm, 0.7 ppm, 0.6 ppm, 0.5 ppm, 0.4 ppm, 0.3 ppm, 0.2 ppm, or 0.1 ppm DsbA and/or DsbC. In some aspects of the disclosure, the composition comprises a multispecific antibody at a concentration of at least about any of 5 mg/ml, 10 mg/ml, 15 mg/ml, 20 mg/ml, 25 mg/ml, 50 mg/ml, 75 mg/ml, 100 mg/ml, 125 mg/ml, 150 mg/ml, 175 mg/ml, 200 mg/ml, 250 mg/ml, or 300 mg/ml. In some aspects of the disclosure, the composition comprises a multispecific antibody at a concentration of at least about any of 5 mg/ml, 10 mg/ml, 15 mg/ml, 20 mg/ml, 25 mg/ml, 50 mg/ml, 75 mg/ml, 100 mg/ml, 125 mg/ml, 150 mg/ml, 175 mg/ml, 200 mg/ml, 250 mg/ml, or 300 mg/ml; and less than about any one of 50 ppm, 40 ppm, 30 ppm, 20 ppm, 10 ppm, 9 ppm, 8 ppm, 7 ppm, 6 ppm, 5 ppm, 4 ppm, 3 ppm, 2 ppm, 1 ppm, 0.9 ppm, 0.8 ppm, 0.7 ppm, 0.6 ppm, 0.5 ppm, 0.4 ppm, 0.3 ppm, 0.2 ppm, or 0.1 ppm DsbA and/or DsbC. In some aspects of the disclosure, the composition comprises a multispecific antibody at a concentration of between about 5 mg/ml and about 100 mg/ml, about 50 mg/ml and about 150 mg/ml, about 100 mg/ml and about 200 mg/ml, about 150 mg/ml and about 250 mg/ml, about 200 mg/ml and about 300 mg/ml; and less than about any one of 50 ppm, 40 ppm, 30 ppm, 20 ppm, 10 ppm, 9 ppm, 8 ppm, 7 ppm, 6 ppm, 5 ppm, 4 ppm, 3 ppm, 2 ppm, 1 ppm, 0.9 ppm, 0.8 ppm, 0.7 ppm, 0.6 ppm, 0.5 ppm, 0.4 ppm, 0.3 ppm, 0.2 ppm, or 0.1 ppm DsbA and/or DsbC.

In some aspects of the disclosure, the composition comprises a bispecific antibody produced in a cell expressing FkpA, wherein the composition is essentially free of FkpA. In some aspects of the disclosure the bispecific antibodies comprise a first half antibody and a second half antibody, wherein the first half-antibody comprises a first VH/VL unit that binds IL-17 and the second half antibody comprises a second VH/VL unit that binds IL-13. In some aspects of the disclosure, the first half antibody does not bind IL-13, and wherein the second half antibody does not bind IL-17. In certain particular aspects of the disclosure, the multispecific antibody provided herein binds to IL-17AA, IL-17AF and IL-17FF, inhibits IL-17AA-, IL-17AF, and IL-17FF - induced activity, and inhibits IL-13-induced activity. In certain such aspects of the disclosure, the anti-IL-13/IL-17AA, AF and FF bispecific antibody advantageously blocks activities induced by all of IL-17A and F cytokines as opposed to activities induced by IL-17A or IL-17F alone. In some aspects of the disclosure, a multispecific antibody provided herein binds to IL-17AA, IL-17AF, and IL-17FF. In some aspects of the disclosure, the IL-17AA-induced activity is IL-17AA-induced gene expression and/or proliferation of cells in vivo or in vitro. In some aspects of the disclosure, the IL-17AF-induced activity is IL-17AF-induced gene expression and/or proliferation of cells in vivo or in vitro. In some such aspects of the disclosure, the IL-17FF-induced activity is IL-17FF-induced gene expressing and/or proliferation of cells in vivo or in vitro. In some aspects of the disclosure, the IL-13-induced activity is IL-13-induced gene expression and/or proliferation of cells in vivo or in vitro. In some aspects of the disclosure, a multispecific antibody provided herein does not inhibit binding of IL-13 to IL-13R$\alpha$1.

In some aspects of the disclosure, the anti-IL13/IL17AA AF FF bispecific antibody in the composition comprises a first half antibody and a second half antibody, wherein the first half-antibody comprises a first VH/VL unit that binds IL-17AA, AF and FF and the second half antibody comprises a second VH/VL unit that binds IL-13, wherein the first VH/VL unit comprises HVR-H1 comprising the amino acid sequence of SEQ ID NO: 23, HVR-H2 comprising the amino acid sequence of SEQ ID NO: 24, HVR-H3 comprising the amino acid sequence of SEQ ID NO: 25, HVR-L1 comprising the amino acid sequence of SEQ ID NO: 26, HVR-L2 comprising the amino acid sequence of SEQ ID NO: 27, and HVR-L3 comprising the amino acid sequence of SEQ ID NO: 28, and wherein the second VH/VL unit comprises HVR-H1 comprising the amino acid sequence of SEQ ID NO: 12, HVR-H2 comprising the amino acid sequence of SEQ ID NO: 13, HVR-H3 comprising the amino acid sequence of SEQ ID NO: 14, HVR-L1 comprising the amino acid sequence of SEQ ID NO: 15, HVR-L2 comprising the amino acid sequence of SEQ ID NO: 16, and HVR-L3 comprising the amino acid sequence of SEQ ID NO: 17. In some aspects of the disclosure, the first half antibody does not bind IL-13, and the second half antibody does not bind IL-17.

In some aspects of the disclosure, the first VH/VL unit comprises a VH sequence having at least 95%, at least 96%, at least 97%, at least 98%, or at least 99% sequence identity to the amino acid sequence of SEQ ID NO:21 and a VL sequence having at least 95%, at least 96%, at least 97%, at least 98%, or at least 99% sequence identity to the amino acid sequence of SEQ ID NO: 22, and wherein the second VH/VL unit comprises a VH sequence having at least 95%, at least 96%, at least 97%, at least 98%, or at least 99% sequence identity to the amino acid sequence of SEQ ID NO:10 and a VL sequence having at least 95%, at least 96%, at least 97%, at least 98%, or at least 99% sequence identity to the amino acid sequence of SEQ ID NO: 11. In some aspects of the disclosure, a multispecific antibody is provided, wherein the first VH/VL unit comprises a VH sequence having the amino acid sequence of SEQ ID NO:21 and a VL sequence having the amino acid sequence of SEQ ID NO: 22, and wherein the second VH/VL unit comprises a VH sequence having the amino acid sequence of SEQ ID NO:10 and a VL sequence having the amino acid sequence of SEQ ID NO:11.

In certain aspects of the disclosure, the first half antibody binds IL17AA AF and FF comprising a heavy chain that comprises the amino acid sequence of SEQ ID NO:29 and a light chain that comprises the amino acid sequence of SEQ ID NO: 31, and the second half antibody binds IL13 comprising a heavy chain that comprises the amino acid sequence of SEQ ID NO:18 and a light chain that comprises the amino acid sequence of SEQ ID NO:20. In certain aspects of the disclosure, the first half antibody binds IL17AA AF and FF comprising a heavy chain that comprises the amino acid sequence of SEQ ID NO:30 and a light chain that comprises the amino acid sequence of SEQ ID NO:31, and the second half antibody binds IL13 comprising a heavy chain that comprises the amino acid sequence of SEQ ID NO: 19 and a light chain that comprises the amino acid sequence of SEQ ID NO: 20.

In some aspects of the disclosure, the multispecific antibody is prepared by assembling half-antibodies as described herein. The half antibodies are produced in a prokaryotic host cell by culturing the host cell to express the two chains of the half-antibody, whereby upon expression the two chains fold and assemble to form a biologically half antibody in the host cell. In some aspects of the disclosure, the host cell is an *E. coli* host cell. In some aspects of the disclosure, the host cell comprises one or more polynucleotides encoding a first translational unit encoding a first chain of the polypeptide, a second translational unit encoding a second chain of the polypeptide; and translational units encoding at least one chaperone protein selected from the group consisting of peptidyl-prolyl isomerases (*e.g.*, FkpA), protein disulfide oxidoreductases (*e.g*, DsbA and DsbC). The translational units may be on the same polypeptide molecule or different polypeptide molecules, or any combination. For example, the first and second translational units may be on first polypeptide molecule and the translation units encoding the one or more chaperone proteins may be on a second polypeptide molecule. The host cell is cultured in a culture medium under conditions comprising: a growth phase comprising a growth temperature and a growth agitation rate and a production phase comprising a production temperature and a production agitation rate. In some aspects of the disclosure, the growth temperature is from 2 to 10°C above the production temperature, and the growth agitation rate is from 50 to 250 rpm above the production agitation rate. The half antibodies are recovered from the cells and assembled as described above. In some aspects of the disclosure, the half-antibodies are purified by protein A chromatography prior to assembly. See US provisional application No. 62/075,792.

The assembled multispecific antibody is further purified to remove impurities including, for example, FkpA. In some aspects of the disclosure, the assembled multispecific antibodies are purified by mixed-mode chromatography (*e.g.*, anion exchange mixed-mode chromatography) followed by hydrophobic interaction chromatography. In some aspects of the disclosure, the assembled multispecific antibodies are purified by mixed-mode chromatography (*e.g.*, anion exchange mixed-mode chromatography) followed by anion exchange chromatography followed by hydrophobic interaction chromatography. In some aspects of the disclosure, the assembled multispecific antibodies are purified by mixed-mode chromatography (*e.g.*, anion exchange mixed-mode chromatography) followed by anion exchange chromatography followed by hydrophobic interaction chromatography followed by cation exchange chromatography. In some aspects of the disclosure, the purified multispecific antibodies are analyzed for removal of FkpA using the assays described herein.

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

Further details of the invention are illustrated by the following non-limiting Examples.

EXAMPLES

The examples below are intended to be purely exemplary of the invention and should therefore not be considered to limit the invention in any way. The following examples and detailed description are offered by way of illustration and not by way of limitation.

**Example 1. Assay for *E. coli* protein does not adequately measure FkpA.**

FkpA is a chaperone protein in *E. coli* that is typically not expressed at high levels, but *E. coli* overexpressing FkpA have been used to improve the proper assembly and folding of heteromultimeric eukaryotic proteins including antibodies (Zhang et al., 2003, BioTechniques, 35:1032-1042).

To determine if the assay for *E. coli* protein (ECP) can adequately detect and quantitate the presence of FkpA, samples of ultra purified FkpA (see Example 2) were added to assay diluent (0.15M NaCl/0.1 M NaPO4/0.1% fish gelatin/0.05% Polysorbate 20/0.05% Proclin 300) at different concentrations and tested in the ECP assay (Zhu-Shimoni, J. et al., 2014, Biotech and Bioeng. 111:2367-2379). Bovine serum albumin was used as a negative control as an assay for *E. coli* protein should not detect this mammalian protein. Results are shown in Table 4.

Table 4. ECP assay detection.

| FkpA | | | Bovine Serum Albumin | | |
|---|---|---|---|---|---|
| Theoretical ($\mu$g/mL) | Observed ($\mu$g/mL) | % Recovery | Theoretical ($\mu$g/mL) | Observed ($\mu$g/mL) | % Recovery |
| 1000.0 | 15.9 | 1.6 | 2000.0 | LTR | ND |
| 500.0 | 8.6 | 1.7 | 1000.0 | LTR | ND |
| 250.0 | 4.5 | 1.8 | 500.0 | LTR | ND |
| 125.0 | 2.0 | 1.6 | 250.0 | LTR | ND |
| 62.5 | 0.8 | 1.3 | 125.0 | LTR | ND |
| 31.3 | 0.1 | 0.3 | 62.5 | LTR | ND |
| 15.6 | LTR | ND | 31.3 | LTR | ND |
| 7.8 | LTR | ND | 15.6 | LTR | ND |
| LTR = less than range, ND = not detected | | | | | |

The results show that the ECP assay does not adequately detect and quantitate FkpA. Further, commercial assays to detect *E. coli* remnants (*e.g.*, *E. coli* proteins and nucleic acids) as part of release assays for therapeutic proteins prepared in *E. coli* do not specifically identify FkpA. As such, there is a need to generate polyclonal antibodies that are highly specific to FkpA with minimal reactivity to other *E. coli* proteins and/or nucleic acid that could interfere with FkpA detection assays. In addition, ultrapure preparations of FkpA are useful in the generation of FkpA standards for accurate detection of FkpA in a therapeutic polypeptide preparation and in the generation of FkpA positive controls to insure assay quality for research and commercial polypeptide production.

Example 2. Preparation of Ultrapure FkpA

Materials and Methods

Extraction Step

FkpA was expressed in *E. coli* (W3110 derivative named 66F8: ΔfhuA ΔphoA ilvG2096 (Valr) Δprc spr43H1 ΔdegP ΔmanA lacIQ ΔompT ΔmenE). To do so, compatible plasmids (pACYC, Novagen, Madison, WI) were constructed containing the ORF for FkpA, as described in EP1356 052 (see *e.g.*, Examples 9-10, in particular paragraph [0207]). 0.5 mL of frozen stock culture (containing 10-15% DMSO) was thawed and used to inoculate a 2L shake flask containing 500 mL of Soy LB medium supplemented with 2 mL of kanamycin solution (5 mg/mL) and 2.5 mL 1 M sodium phosphate solution. The culture was expanded to large scale culture. A 50 mL bolus addition of 200 mM IPTG was added to the fermentation at an approximate OD of 200. IPTG was used to induce the tacII promoter used to control the expression of the FkpA open reading frame (ORF). The fermentation run duration was 50 hours.

All cell lysis and centrifugation steps were carried out at 2-8°C, unless otherwise noted.

A 304 g aliquot of the cell paste recovered from the 10 L *E. coli* fermentation was thawed overnight. The cell paste was suspended in 10 volumes of 25 mM 2-(N-morpholino)ethanesulfonic acid (MES), pH 6.0 (lysis buffer) and mixed for 60 min to generate a uniform suspension. A Microfluidizer HC-8000 homogenizer (Microfluidics Corporation) equipped with a temperature control unit (initial set point of ≤ 5°C) was used for the cell lysis. The Microfluidizer was rinsed with 1 L of chilled lysis buffer prior to the introduction of the cell suspension. The cell suspension was transferred to the Microfluidizer, and a total of four passes under house pressure (6 - 8 K psi) were carried out. The residual suspension was chased from the Microfluidizer using chilled lysis buffer. A 200 mL aliquot of the lysed cell suspension was taken for centrifugation and subsequent purification. The remaining ~ 3.2 L of homogenate was stored at ≤ -60°C.

The 200 mL aliquot was divided into six equal volumes and transferred into 50 mL centrifuge tubes. The tubes were centrifuged using a RC6 Plus centrifuge equipped with an SS-34 rotor (Thermo Corporation) at a speed of 15,000 rpm for 30 min at a temperature of 20°C. The supernatant, containing FkpA, was stored at a temperature of 2-8°C and the pellets were discarded.

Analytical Evaluation of SP Sepharose® Flow cation-exchange chromatography

The calculated isoelectric point (pI) of FkpA (based on the amino acid sequence of the mature protein) is 7.01. Cation exchange chromatography, carried out at pH 6.0, should result in good binding of the target protein while the majority of the *E. coli* protein (ECP) impurities (with pI values of 3-6) should flow through the column.

An analytical evaluation of this potential first chromatography step was performed using a 1 mL gravity-flow column packed with the strong cation-exchange media SP Sepharose® Fast Flow (GE Healthcare).

The column was pre-equilibrated with 3 column volumes (CV) of 50 mM MES, 1.0 M NaCl, pH 6.0 (elution buffer) and then equilibrated with 3 CV of 50 mM MES, pH 6.0 (equilibration buffer). A 1.0 mL aliquot of the lysate supernatant was loaded to the column, followed by a 5 CV wash with equilibration buffer. The column was step eluted using increasing NaCl concentrations from 100 mM to 1000 mM (made by blending the equilibration and elution buffers).

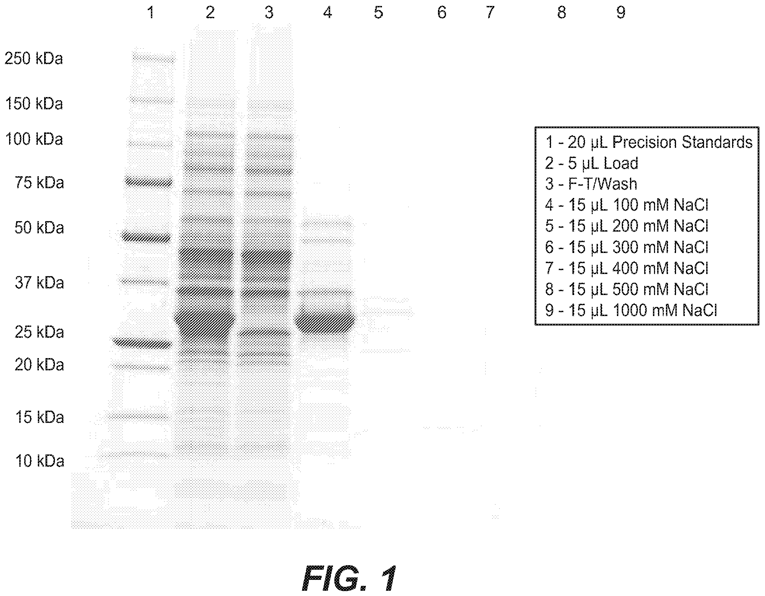

Sodium dodecyl sulfate polyacrylamide gel electrophoresis (SDS-PAGE) without disulfide bond reduction was used to determine the relative purity of FkpA in column fractions. Results show that the FkpA is completely bound to the cation exchange media under these conditions (as evidenced by the lack of FkpA in the flow-through and wash fraction) (FIG. 1, lanes 2 and 3). The FkpA was completely eluted from the column by the 100 mM NaCl step, indicating that the FkpA is weakly bound to the cation-exchange media, and confirming the hypothesis that the majority of the ECPs flow-through the column. The resulting FkpA-containing pool is very clean, with only small amounts of residual ECPs (FIG. 1, lane 4).

*Step Elution Evaluation of SP Sepharose® Flow cation-exchange chromatography*

The preparative evaluation of FkpA purification, using SP Sepharose® Fast Flow cation-exchange chromatography, was done on an AKTA Explorer 100 (GE Healthcare). A 2.6 cm (width) x 27.3 cm (length) column (1 CV = 145 mL) was used for this operation. All steps were carried out at a flow rate of 8.85 mL/min (100 cm/hr). The column was pre-equilibrated with 3 CV of elution buffer (25 mM MES, 1.0 M NaCl, pH 6.0) and then equilibrated with 3 CV of equilibration buffer (25 mM MES, pH 6.0). The lysate supernatant was loaded to the column and chased with 20 mL of equilibration buffer. The column was washed with 5 CV of equilibration buffer and then step eluted with 50 mM MES, 100 mM NaCl, pH 6.0. Following the step elution, the column was stripped with 3 CV of elution buffer.

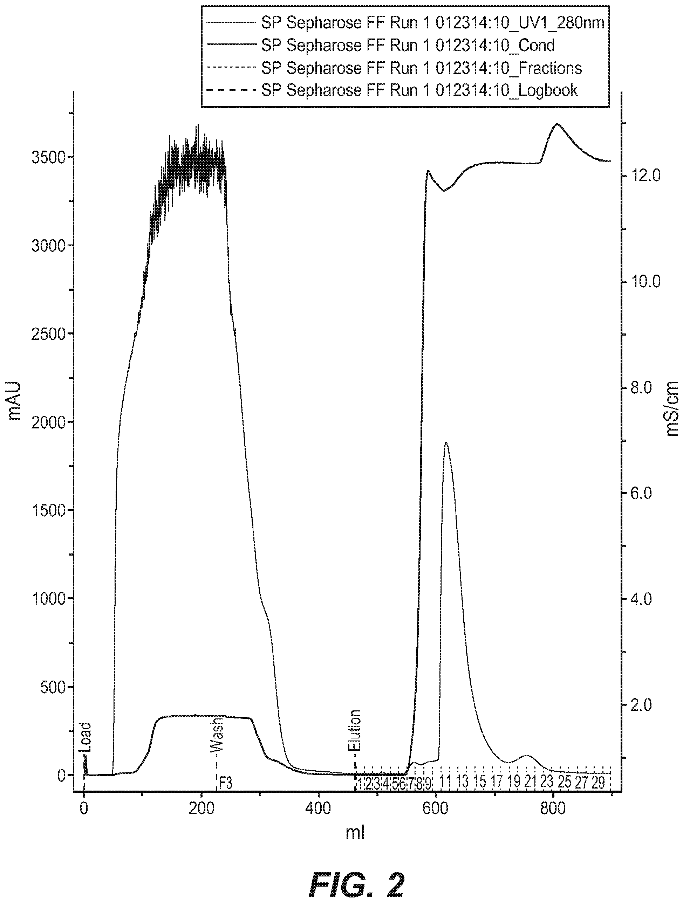

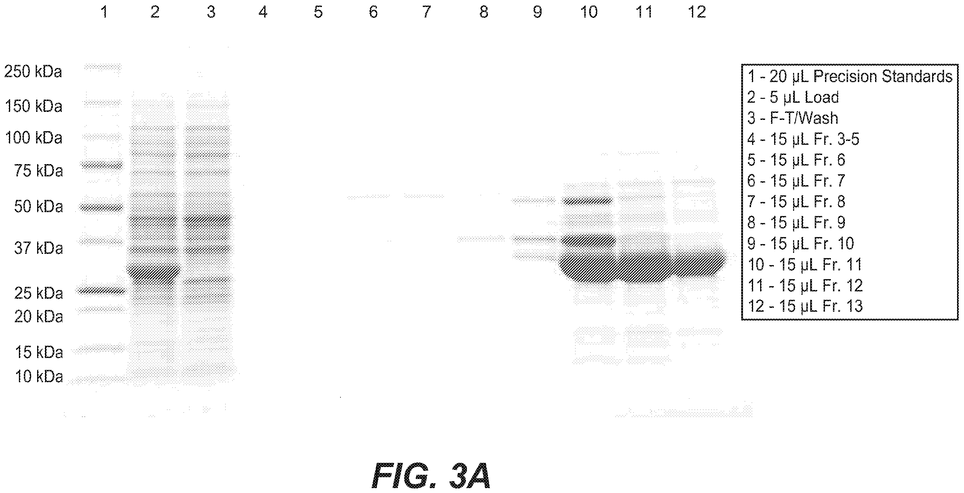

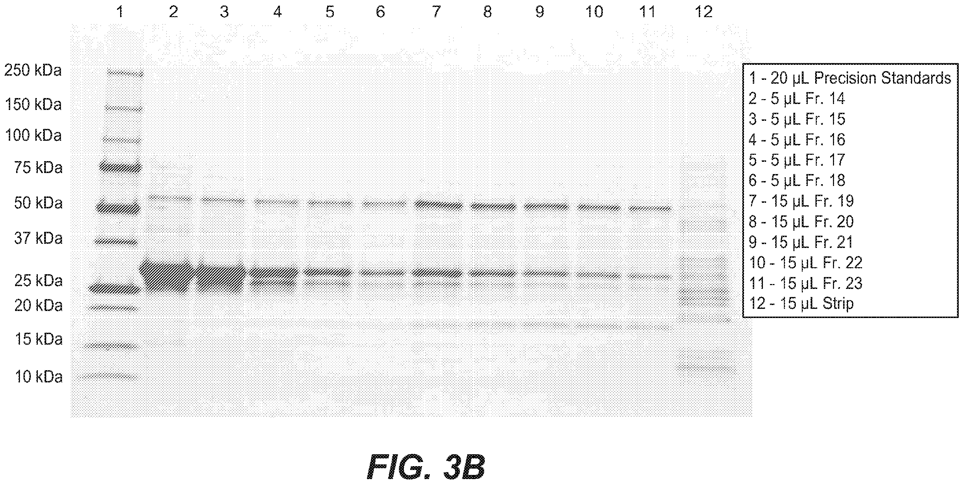

The chromatogram is shown in FIG. 2. SDS-PAGE analysis of the column fractions (FIGs. 3A and 3B) show that the FkpA is associated with the major elution peak, and is primarily found in fractions 11-17. There are also small amounts of FkpA in fractions 18-23.

Gradient Elution Evaluation of SP Sepharose® Flow cation-exchange chromatography

In order to further evaluate the potential of SP Sepharose® Fast Flow to remove residual ECPs, re-chromatography of fractions 11-18 from the first preparative evaluation was carried out.

Fractions 11-18 were pooled and buffer-exchanged back into SP Sepharose® Fast Flow equilibration buffer using Sephadex® G-25.

The buffer-exchanged fractions were re-chromatographed on the same SP Sepharose® Fast Flow column used in the earlier preparative run, following the same load and wash parameters. The elution was carried out using a 0 - 30% gradient (0 - 300 mM NaCl) over 9 CV, which takes into account the relative salt amount required for the original step elution of FkpA.

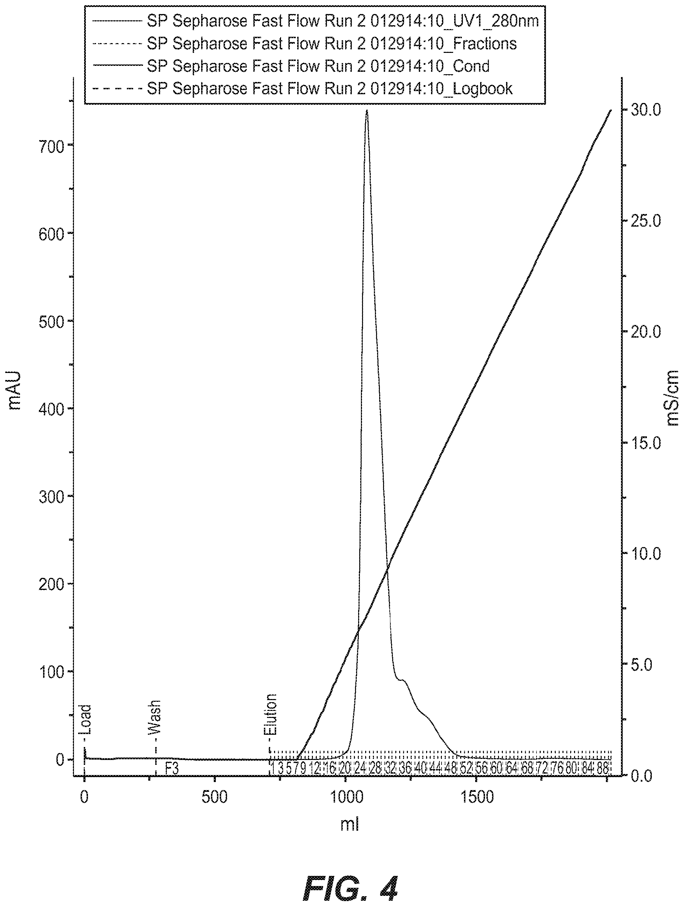

The chromatogram (FIG. 4) shows a large major peak eluting under relatively low (5-11 mS/cm) ionic strength conditions. The gradient profile shows strong similarities to the step elution profile (FIG. 2), with the absence of the smaller pre-peak (those fractions were not included in those chosen for re-chromatography).

SDS-PAGE analysis of the elution profile. Examination of the ECP bands relative to the FkpA shows that only partial resolution of some of these impurities are achieved by using a linear gradient (data not shown). This likely indicates that these ECP may have comparable pI values to FkpA, or that some other mode(s) of interaction may be responsible for their co-purification.

Analytical Evaluation of Potential Second Purification Step: Comparision of Q Sepharose® Fast Flow and Phenyl Sepharose®

*Q Sepharose® Fast Flow*

An analytical evaluation of this potential second chromatography step was performed using a 1 mL gravity-flow column packed with the strong anion-exchange media Q Sepharose® Fast Flow (QSFF) (GE Healthcare).

The column was pre-equilibrated with 5 mL of 50 mM Tris, 1.0 M NaCl, pH 8.0 (QSFF elution buffer) and then equilibrated with 5 mL of 50 mM Tris, pH 8.0 (QSFF equilibration buffer). A 2.5 mL aliquot of a mini-pool of fractions 22-50 from the gradient evaluation of SP Sepharose® described above was buffer-exchanged into 3.5 mL of QSFF equilibration buffer using a PD-10 column (GE Healthcare) and was loaded to the column. The column was washed with 3.5 mL QSFF equilibration buffer, and was subsequently eluted with 3.0 mL of QSFF elution buffer.

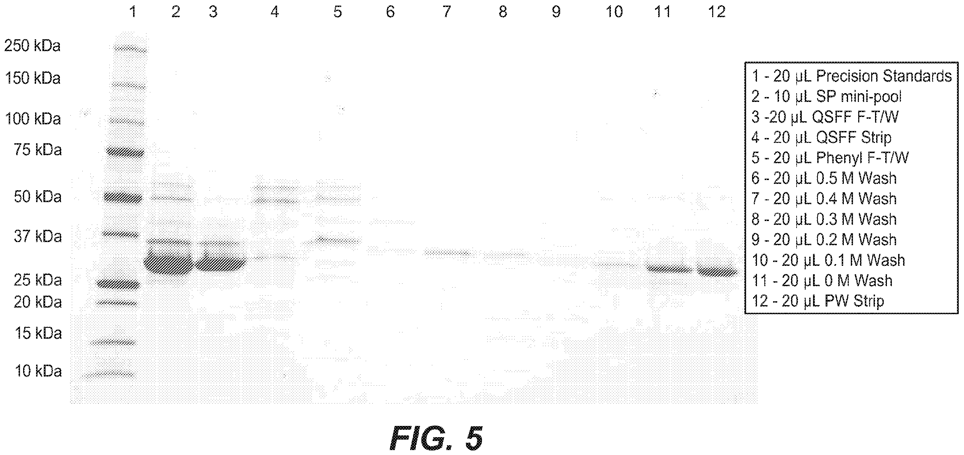

Column fractions from the evaluation of QSFF and Phenyl Sepharose® (described below) were analyzed by SDS-PAGE (FIG. 5). The Q Sepharose® column does not bind FkpA, but was able to remove three of the higher molecular weight (MW) species that were bound to the column. However, bands at ~ 35 kDa, ~ 40 kDa, and ~ 26 kDa can still be detected in the column flow through along with FkpA. The recovery data is summarized in Table 5 and 6. The Q Sepharose® Fast Flow gel gave good overall recovery (95%).

Table 5. Spectrophotometric Data from QSFF and Phenyl Sepharose® Column Fractions

| Sample | Dilution Factor | FkpA (mg/mL) | Abs <280 nm> | Abs <320 nm> |
|---|---|---|---|---|
| PW Blank | 1.0 | 1.1656E-3 | -8.3447E-5 | -7.1287E-4 |
| SP Pool | 1.0 | 1.51360 | 0.82628 | 8.9335E-3 |
| Q F-T/Wash | 1.0 | 0.46137 | 0.24948 | 3.4523E-4 |
| Q Strip | 1.0 | 0.12278 | 6.5184E-2 | -1.1196E-3 |
| Ph F-T/W | 1.0 | 6.8952E-2 | 3.2589E-2 | -4.6453E-3 |
| Ph 0.5 MW | 1.0 | 1.8189E-2 | 7.5068E-3 | -2.3150E-3 |
| Ph 0.4 MW | 1.0 | 2.3830E-2 | 1.1497E-2 | -1.3714E-3 |
| Ph 0.3 MW | 1.0 | 2.0948E-2 | 8.4639E-3 | -2.8481E-3 |
| Ph 0.2 MW | 1.0 | 1.8924E-2 | 9.6922E-3 | -5.2691E-4 |
| Ph 0.1 MW | 1.0 | 3.4419E-2 | 2.0305E-2 | 1.7190E-3 |
| Ph 0 MW | 1.0 | 0.14326 | 8.2129E-2 | 4.7674E-3 |
| Ph PW Strip | 1.0 | 0.20207 | 0.11301 | 3.8896E-3 |
| FkpA (mg/mL)=(Abs(280 nm) - Abs (320 nm))/0.54 | | | | |

Table 6. Q Sepharose® Fast Flow recovery data

| Fraction | Vol. (mL) | Conc. (mg/mL) | mg | % Recovery |
|---|---|---|---|---|
| SP mini-pool fr. 22-50 | 2.5 | 1.51 | 3.8 | 100% |
| F-T/Wash | 7.0 | 0.46 | 3.2 | 85% |
| 1 M NaCl strip | 3.0 | 0.12 | 0.4 | 10% |
| **Mass Balance** | | | **3.6** | **95%** |

*Phenyl Sepharose®*

An analytical evaluation of this potential second chromatography step was performed using a 1 mL gravity-flow column packed with the hydrophobic interation media Phenyl Sepharose® Fast Flow (low substitution) (GE Healthcare).
The column was pre-equilibrated with 5 mL of 50 mM phosphate, pH 7.0 (Phenyl elution buffer) and then equilibrated with 5 mL of 0.6 M sodium sulfate, 50 mM phosphate, pH 7.0 (Phenyl equilibration buffer). 2.5 mL of 1.2 M sodium sulfate was added to a 2.5 mL aliquot of a mini-pool of fractions 22-50 from the gradient evaluation of SP Sepharose® described above. The mini-pool was gently mixed and allowed to stand for 30 min at room temperature and then was loaded to the column. The column was washed with 3.0 mL Phenyl equilibration buffer, and was subsequently step eluted with 3.0 mL volumes of decreasing sodium sulfate (0.5 M, 0.4 M, 0.3 M, 0.2 M, 0.1 M and 0 M) in Phenyl elution buffer. Lastly, the column was stripped using 3.0 mL of purified water (PW).
The Phenyl Sepharose® (low substitution) gel showed the opposite behavior to Q Sepharose® Fast Flow: the impurities that were bound to QSFF are able to flow through the Phenyl Sepharose® (low substitution) column. The ~ 35 kDa band that co-purified with FkpA on QSFF flows through the Phenyl Sepharose® column as well. The FkpA is eluted very late in the decreasing salt profile, at 0 M sodium sulfate, as well as in the PW strip, indicating that it is very hydrophobic. The FkpA-containing fractions appear to be devoid of the ECP impurities that were present in the SP Sepharose® Fast Flow pool. The recovery data is summarized in Table 5 and 6. The Phenyl Sepharose® Fast Flow (low substitution) gel was only able to recover 51%. The remaining protein is likely bound to the column, indicating that the Phenyl Sepharose® (low substitution) gel binds the FkpA too tightly. The Phenyl Sepharose® Fast Flow (low substitution) was able to successfully remove residual ECP, but at a cost of lower recovery.

Table 6. Pheny Sepharose® (low sub) recovery data

| Fraction | Vol. (mL) | Conc. (mg/mL) | mg | % Recovery |
|---|---|---|---|---|
| SP mini-pool fr. 22-50 | 2.5 | 1.51 | 3.8 | 100% |
| F-T/Wash | 8.0 | 0.07 | 0.6 | 15% |
| 0.5 M Wash | 3.0 | 0.02 | 0.1 | 1% |
| 0.4 M Wash | 3.0 | 0.02 | 0.1 | 2% |
| 0.3 M Wash | 3.0 | 0.02 | 0.1 | 2% |
| 0.2 M Wash | 3.0 | 0.02 | 0.1 | 2% |
| 0.1 MWash | 3.0 | 0.03 | 0.1 | 3% |
| 0 M Wash | 3.0 | 0.14 | 0.4 | 11% |
| PW strip | 3.0 | 0.20 | 0.6 | 16% |
| **Mass Balance** | | | **1.9** | **51%** |

*Analytical Evaluation of Potential Second Purification Step: Comparision of Phenyl Sepharose®, Butyl Sepharose®, and Octyl Sepharose®*

Having demonstrated the potential of hydrophobic interaction chromatography (HIC) for the second step of purification; a second evaluation was performed comparing three HIC media with different hydrophobicities: Phenyl Sepharos®e (low sub), Octyl Sepharose® Fast Flow, and Butyl Sepharose® 4 Fast Flow (GE Healthcare).

Chromatography was performed for each column as described above for Phenyl Sepharose® with an additional step wherein following the stripping of the column using 3.0 mL of purified water (PW), each column was regenerated using 3 mL of 2% sodium hydroxide.

The column regeneration fraction from each column was compared (Table 7 and 8). The different columns are listed in Tables 7 and 8 in order of increasing hydrophobicity with respect to FkpA. The Butyl Sepharose® 4 Fast Flow gel has the lowest regeneration value of the three (15%), whereas the Octyl Sepharose® Fast Flow and Phenyl Sepharose® Fast Flow (low substitution) show 45 and 56% recoveries, respectively. Therefore, the Octyl Sepharose® Fast Flow and Phenyl Sepharose® Fast Flow (low substitution) gels are too hydrophobic for use as a second chromatography step.

Table 7. Spectrophotometric Data from Column Regeneration Fractions

| Sample | Dilution Factor | FkpA (mg/mL) | Abs <280 nm> | Abs <320 nm> |
|---|---|---|---|---|
| PW Blank | 1.0 | -5.6867E-4 | 6.1989E-5 | 3.6907E-4 |
| Butyl regen | 1.0 | 0.18870 | 0.10798 | 6.0782E-3 |
| Octyl regen | 1.0 | 0.56642 | 0.31557 | 9.7098E-3 |
| Phenyl regen | 1.0 | 0.70106 | 0.38781 | 9.2392E-3 |
| FkpA (mg/mL)=(Abs(280 nm) - Abs (320 nm))/0.54 | | | | |

Table 8. HIC Gel Regeneration

| Fraction | Vol. (mL) | Conc. (mg/mL) | mg | % Recovery |
|---|---|---|---|---|
| SP mini 22-50 | 2.5 | 1.51 | 3.8 | 100% |

(continued)

| Fraction | Vol. (mL) | Conc. (mg/mL) | mg | % Recovery |
|---|---|---|---|---|
| Butyl regeneration | 3.0 | 0.19 | 0.6 | 15% |
| Octyl regeneration | 3.0 | 0.57 | 1.7 | 45% |
| Phenyl regeneration | 3.0 | 0.70 | 2.1 | 56% |
| *Analytical Evaluation of Potential Second Purification Step: Butyl-S Sepharose® 6 Fast Flow* | | | | |

Following the comparision of three HIC media with varying hydrophobicities; an additional HIC gel was evaluated: Butyl-S Sepharose® 6 Fast Flow (GE Healthcare). This gel has the weakest HIC ligand in the chromatography media of the HIC gels evaluated.

A 1 mL gravity-flow column was packed with Butyl-S Sepharose® 6 Fast Flow (GE Healthcare). The column was pre-equilibrated with 5 mL of 50 mM phosphate, pH 7.0 (Butyl-S elution buffer) and then equilibrated with 5 mL of 0.6 M sodium sulfate, 50 mM phosphate, pH 7.0 (Butyl-S equilibration buffer). 2.5 mL of 1.2 M sodium sulfate was added to a 2.5 mL aliquot of a mini-pool of fractions 22-50 from the gradient evaluation of SP Sepharose® described above. The mini-pool was gently mixed and allowed to stand for 30 min at room temperature and then was loaded to the column. The column was washed with 3.0 mL Butyl-S equilibration buffer, and was subsequently step eluted with 3.0 mL volumes of decreasing sodium sulfate (0.5 M, 0.4 M, 0.3 M, 0.2 M, 0.1 M and 0 M) in Butyl-S elution buffer. The column was stripped twice using 3.0 mL of purified water (PW) and then was regenerated using 3 mL of 2% sodium hydroxide.

Column fractions from the evaluation of Butyl-S were analyzed by SDS-PAGE (FIG. 6). ECPs from the SP Sepharose® Fast Flow mini-pool are not captured by the column under equilibration conditions (0.6 M sodium sulfate). In addition, there appears to be very little FkpA in the NaOH regeneration fraction, indicating that this chromatography media may be able to remove residual ECPs from the SP Sepharose® Fast Flow pool while not sustaining large recovery losses.

Analytical Evaluation of Potential Second Purification Step: Comparison of Butyl Sepharose® 4 Fast Flow and Butyl-S Sepharose® 6 Fast Flow

1 mL gravity flow columns containing either Butyl-S Sepharose® 6 Fast Flow or Butyl Sepharose® 4 Fast Flow were directly compared. The procedure was as described above for Butyl-S Sepharose® 6 Fast Flow except that the columns were stripped once using 6.0 mL of PW rather than 2 x 3 mL PW.

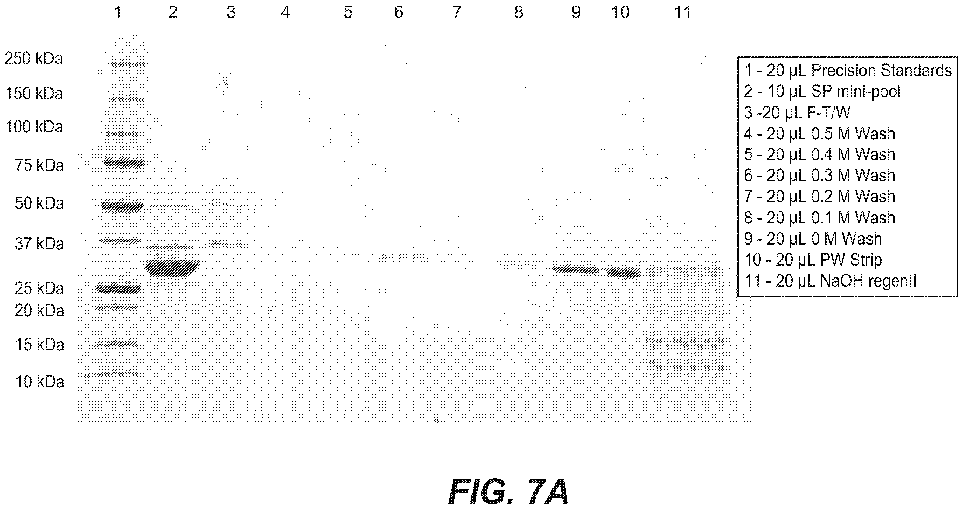

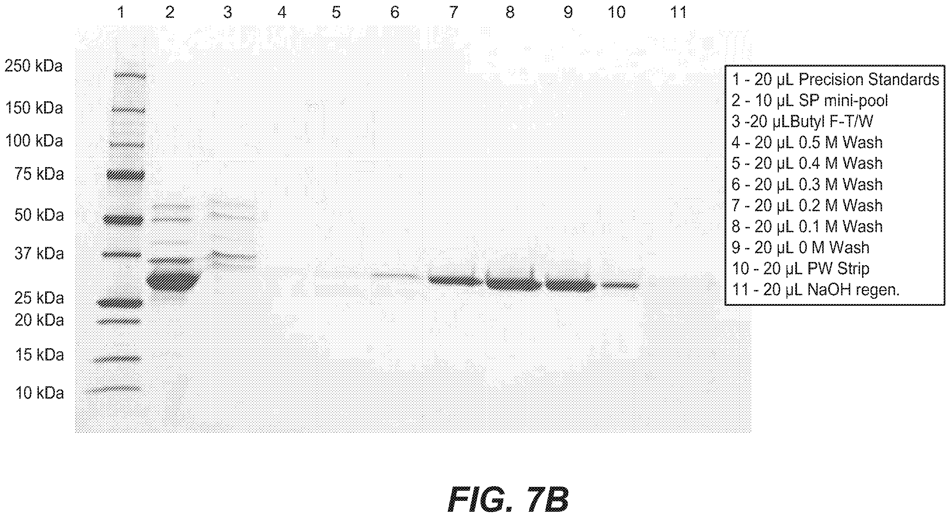

SDS-PAGE analyses of the column fractions from Butyl Sepharose® 4 Fast Flow and Butyl-S Sepharose® 6 Fast Flow are shown in FIGs. 7A and 7B, respectively. For the Butyl Sepharose® 4 Fast Flow samples, FkpA elutes very late (50 mM phosphate, followed by PW). There is a noticeable amount of FkpA and lower MW impurities in the regeneration fraction. For the Butyl-S Sepharose® 6 Fast Flow samples, the FkpA elutes over the course of several wash concentrations, with most eluting prior to the PW strip step. The regeneration fraction still contains some lower MW impurities and a small amount of FkpA. The recovery data for the Butyl-S Sepharose® 6 Fast Flow arm of the experiment is shown in Tables 9 and 10. Mass balance is quantitative, and only 6% of the total protein is found in the regeneration fraction. The Butyl-S Sepharose® 6 Fast Flow gel provides excellent clearance of residual ECPs from the SP Sepharose® Fast Flow pool, as well as very high recoveries.

Table 9. Spectrophotometric date: Butyl-S Sepharose® 6 Fast Flow

| Sample | Dilution Factor | FkpA (mg/mL) | Abs <280 nm> | Abs <320 nm> |
|---|---|---|---|---|
| PW Blank | 1.0 | -1.0596E-4 | -1.0586E-4 | -4.8637E-5 |
| F-T/Wash | 1.0 | 9.4522E-2 | 4.9919E-2 | -1.1234E-3 |
| 0.5 M Wash | 1.0 | 2.1272E-2 | 7.1135E-3 | -4.3736E-3 |
| 0.4 M Wash | 1.0 | 1.9341E-2 | 9.2669E-3 | -1.1773E-3 |
| 0.3 M Wash | 1.0 | 4.0865E-2 | 2.0347E-2 | -1.7200E-3 |
| 0.2 M Wash | 1.0 | 0.17278 | 9.4492E-2 | 1.1907E-3 |
| 0.1 M Wash | 1.0 | 0.31258 | 0.17033 | 1.5364E-3 |
| 0 M Wash | 1.0 | 0.27013 | 0.14822 | 2.3418E-3 |

(continued)

| Sample | Dilution Factor | FkpA (mg/mL) | Abs <280 nm> | Abs <320 nm> |
|---|---|---|---|---|
| PW elute | 1.0 | 6.1637E-2 | 3.2298E-2 | -9.8085E-4 |
| 2% NaOH regen | 1.0 | 7.7314E-2 | 4.6334E-2 | 4.5843E-3 |
| FkpA (mg/mL)=(Abs(280 nm) - Abs (320 nm))/0.54 | | | | |

Table 10. Butyl-S Sepharose® Recovery Data

| Fraction | Vol. (mL) | Conc. (mg/mL) | mg | % Recovery |
|---|---|---|---|---|
| SP mini 22-50 | 2.5 | 1.51 | 3.8 | 100% |
| Butyl-S F-T/Wash | 8.0 | 0.09 | 0.8 | 20% |
| 0.5 MWash | 3.0 | 0.02 | 0.1 | 2% |
| 0.4 M Wash | 3.0 | 0.02 | 0.1 | 2% |
| 0.3 M Wash | 3.0 | 0.04 | 0.1 | 3% |
| 0.2 M Wash | 3.0 | 0.17 | 0.5 | 14% |
| 0.1 MWash | 3.0 | 0.31 | 0.9 | 25% |
| 0 M Wash | 3.0 | 0.27 | 0.8 | 21% |
| PW strip | 6.0 | 0.06 | 0.4 | 10% |
| 2% NaOH regen. | 3.0 | 0.08 | 0.2 | 6% |
| **Mass Balance** | | | **3.9** | **102%** |

*Butyl-S Sepharose® 6 Fast Flow Optimization*

The purpose of this experiment was to evaluate whether an intermediate 0.3 M sodium sulfate wash of the column would provide a comparable purification (recovery and purity) to a potential decreasing sodium sulfate gradient.

A 1 mL gravity-flow column was packed with Butyl-S Sepharose® 6 Fast Flow (GE Healthcare). The column was pre-equilibrated with 5 mL of 50 mM phosphate, pH 7.0 (Butyl-S elution buffer) and then equilibrated with 5 mL of 0.6 M sodium sulfate, 50 mM phosphate, pH 7.0 (Butyl-S equilibration buffer). 2.5 mL of 0.6 M sodium sulfate was added to a 2.5 mL aliquot of a mini-pool of fractions 22-50 from the gradient evaluation of SP Sepharose® described above. The mini-pool was gently mixed and allowed to stand for 30 min at room temperature and then was loaded to the column. The column was washed with 5.0 mL of 0.3 M sodium sulfate, 50 nM phosphate, pH 7.0, and was subsequently eluted with 5.0 mL purified water (PW), and then was regenerated using 3 mL of 2% sodium hydroxide.

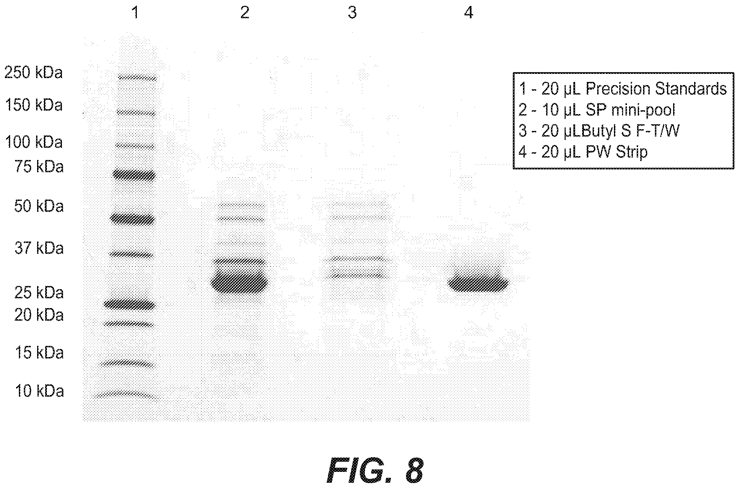

Analysis of the column fractions by SDS-PAGE (FIG. 8) shows that the higher MW impurities in the SP Sepharose® Fast Flow pool flowed through the Butyl-S Sepharose® column under the reduced sodium sulfate conditions, leaving a very clean elution pool. The few lower MW bands in the load lane bound to the column and were removed via regeneration (as seen in the earlier gels; FIG. 3).

The recovery data is shown in Tables 11 and 12. The recovery in the F-T/Wash pool is about 5% higher than the recovery values from the previous experiment. The PW elution recovery is a little lower. Additional PW elutions may have increased the yield. The regeneration step is also a little higher than the step elution consistent with a possible need for a longer elution step prior to regeneration.

Table 11. Butyl-S Sepharose® Fast Flow Recovery Data

| Sample | Dilution Factor | FKpA (mg/mL) | Abs <280 nm> | Abs <320 nm> |
|---|---|---|---|---|
| PW Blank | 1.0 | -1.8632E-4 | -2.6512E-4 | -1.6451E-4 |
| F-T/Wash | 1.0 | 0.11878 | 6.3139E-2 | -1.0047E-3 |
| PW elute | 1.0 | 0.45328 | 0.24602 | 1.2512E-3 |

(continued)

| Sample | Dilution Factor | FKpA (mg/mL) | Abs <280 nm> | Abs <320 nm> |
|---|---|---|---|---|
| 2% NaOH regen | 1.0 | 0.12895 | 7.2400E-2 | 2.7652E-3 |
| FkpA (mg/mL)=(Abs(280 nm) - Abs (320 nm))/0.54 | | | | |

Table 12. Butyl-S Sepharose® Fast Flow Recovery Data

| Fraction | Vol. (mL) | Conc. (mg/mL) | mg | % Recovery |
|---|---|---|---|---|
| SP mini 22-50 | 2.5 | 1.51 | 3.8 | 100% |
| Butyl-S F-T/Wash | 10.0 | 0.12 | 1.2 | 31% |
| PW elute | 5.0 | 0.45 | 2.3 | 60% |
| 2% NaOH regen. | 3.0 | 0.13 | 0.4 | 10% |
| **Mass Balance** | | | **3.8** | **102%** |

The Butyl-S Sepharose® HIC media shows excellent separation of the ECPs from the SP Sepharose® Fast Flow pool, with very good recovery of FkpA. Based on the data from these analytical evaluations, it will be scaled-up and used as the second recovery step in the FkpA purification process.

Scale up of Butyl-S Sepharose® Fast Flow Step

The Butyl-S Sepharose® chromatography step was scaled up to compare recoveries and purities to the analytical Butyl-S Sepharose® chromatography step. A series of developmental runs were carried out to optimize the step. The analytical HIC runs showed that the FkpA bound relatively tightly to the Butyl-S Sepharose® gel. An extended elution step was evaluated to see if an increase in FkpA recovery can be obtained.

Buffers and solutions

Conditioning buffer: 0.6 M sodium sulfate, 50 mM phosphate, pH 7.0
Equilibration buffer: 0.3 M sodium sulfate, 50 mM phosphate, pH 7.0 (A11)
Elution solution: purified water (PW, A12)
Regeneration solution: 2% NaOH

Column preparation

A 2.6 cm x 9.4 cm (50 mL) column was prepared for this evaluation. The column was equilibrated with 3 CV of equilibration buffer.

Load conditioning

140 mL of conditioning buffer was gradually added to 140 mL of SP Sepharose® FF pool, with gentle mixing throughout the addition. The mixing was continued at room temperature for 30 minutes after all of the conditioning buffer was added.

Chromatography

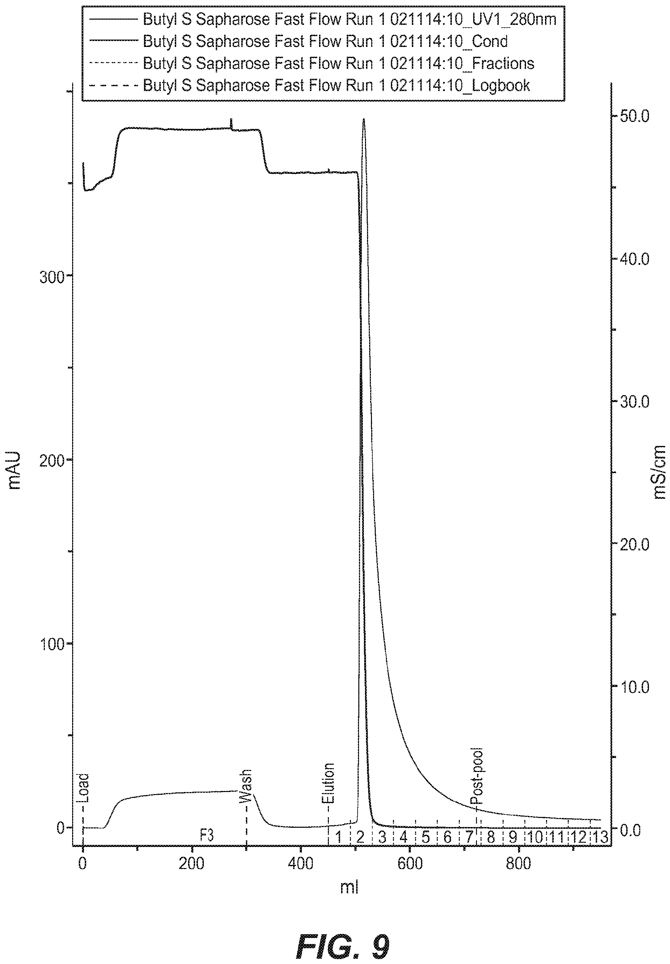

The chromatogram is shown in FIG. 9. A small amount of breakthrough OD was observed, which was expected based on the analytical evaluations. The elution peak has a pronounced tailing, and reaches 10 mAUFS after about 6 CV of elution. The post-pool was collected as a separate fraction. Column regeneration was carried out using 2% (0.5 M) sodium hydroxide as the regeneration solution. UV-Vis spectrophotometric analysis column fractions are shown in Table 13. The mass balance was higher than for analytical runs, but the data is similar to that generated from the previous analytical runs. The scale-up run shows similar column performance with respect to purity and recovery of FkpA. A second column run showed a comparable column profile.

Table 13. Butyl-S Sepharose® recovery

| Fraction | Vol. (mL) | Conc. (mg/mL) | mg | % Recovery |
|---|---|---|---|---|
| SP FF fr. 22-50 | 140 | 1.48 | 207 | 100% |
| F-T/Wash | 450 | 0.12 | 53 | 26% |
| Pre-pool | 54 | 0.03 | 1 | 1% |
| Pool | 232 | 0.60 | 140 | 67% |
| Post-pool | 232 | 0.03 | 8 | 4% |
| 2% NaOH regen. | 150 | 0.16 | 23 | 11% |
| **Mass Balance** | | | **225** | **109%** |

SDS-PAGE

Analysis of the column fractions by SDS-PAGE (FIG. 10A) shows that the purification step is comparable to the analytical evaluation, demonstrating that the scale-up of this HIC step was able to replicate the removal of host cell protein impurities present in the SP Sepharose® Fast Flow pool. The higher MW impurities flowed through the column and the lower MW impurities were tightly bound to the column (as evidenced by the earlier gels). The elution pool was very clean, with a very small amount of FkpA being observed in the post pool. The same gel was imaged using SYPRO Ruby reagent (Bio-Rad Laboratories) after overnight incubation in the staining solution. SYPRO Ruby imaging is a more sensitive detection method for analysis of protein-containing samples. The SYPRO Ruby imaging (FIG. 10B) shows that the fractions of purified FkpA still have a number of lower MW bands that may be host cell protein impurities and/or clipped forms of FkpA.

Conclusions

Scale-up of the Butyl-S Sepharose® chromatography step was linear from the parameters chosen from the analytical evaluation of different HIC media. The Butyl-S Sepharose® offers the best recovery of FkpA due to it having the weakest ligand of the HIC media that were evaluated for this second purification step. Molecule purity was greatly enhanced by this unit operation, with the major impurity bands either present in the flow-through or being more highly retained, based on SDS-PAGE analysis.

HPLC-SEC Analytical Evaluation

Based on its primary sequence, FkpA has a molar mass of ~26 kDa. Previously published information on FkpA suggests that the molecule runs at a higher MW through non-covalent association. An analytical evaluation was carried out using protein references alongside the Butyl-S Sepharose® pool containing FkpA in order to determine where FkpA migrates on a size-exclusion chromatography column.

Materials and methods

A TSKgel G3000SWxl HPLC-SEC column (7.8 mm x 30 cm; Tosoh Bioscience) was used for the evaluation. The mobile phase was 0.25 M KCl, 0.2 M potassium phosphate, pH 6.2. The column flow rate was 0.5 mL/min. FkpA was compared to the protein standards: an antibody (-150 kDa), bovine serum albumin (~66.5 kDa) and Nutropin® (~22 kDa).

Chromatography

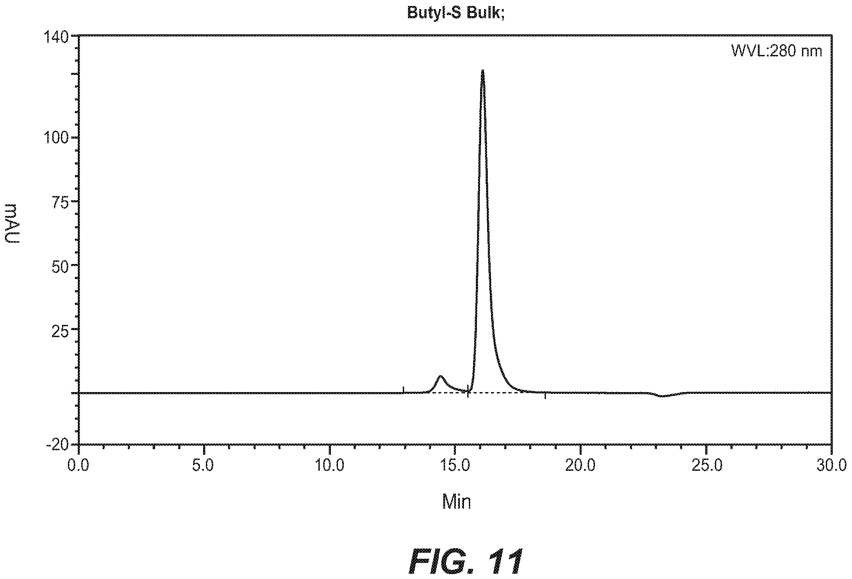

HPLC-SEC results are shown in FIG. 11. FkpA has a retention time between antibody and BSA, confirming the earlier literature references that FkpA has a much larger solution molar mass than would be predicted from its primary sequence (Table 14).

Table 14. HPLC-SEC of FkpA and Standards

|  | Predicted MW | Ret. time (min) |
|---|---|---|
| antibody | ~150 kDa | 13.420 |
|  |  | 14.733 |
|  |  | 15.577 |
| BSA | ~66.5 kDa | 17.787 |
| Nutropin | ~22 kDa | 20.730 |
| FkpA | ~26 kDa | 14.430 |
|  |  | 16.100 |

Superdex 200 size-exclusion chromatography

Superdex 200 size-exclusion chromatography (SEC) was chosen to separate the residual higher MW species and lower MW species from FkpA, as well as to formulate the protein.

Run 1: pilot method using 1.6 cm x 60 cm Superdex 200 column

The initial Superdex 200 run was performed on a 120 mL column. Column fractions were characterized for purity prior to scaling the Superdex 200 SEC step up for generation of the final formulated bulk.

Materials and methods

A 1.6 cm x 60 cm (1 CV = 120 mL) Superdex 200 column (GE Healthcare) was used for this pilot run. The column was cleaned with 1 CV of 0.1 M NaOH and then equilibrated with 3 CV of phosphate-buffered saline (PBS), pH 7.5 (equilibration buffer).

Sample preparation

30 mL of Butyl-S Sepharose® pool was concentrated using a pair of Amicon Ultra 10 kDa units (Millipore Corporation). The Amicon units were centrifuged in an Eppendorf 5810 R centrifuge at 3000 rpm using 20 min cycles until the retentate volume was ≤ 6 mL (≤ 5% of 1 CV).

Chromatography

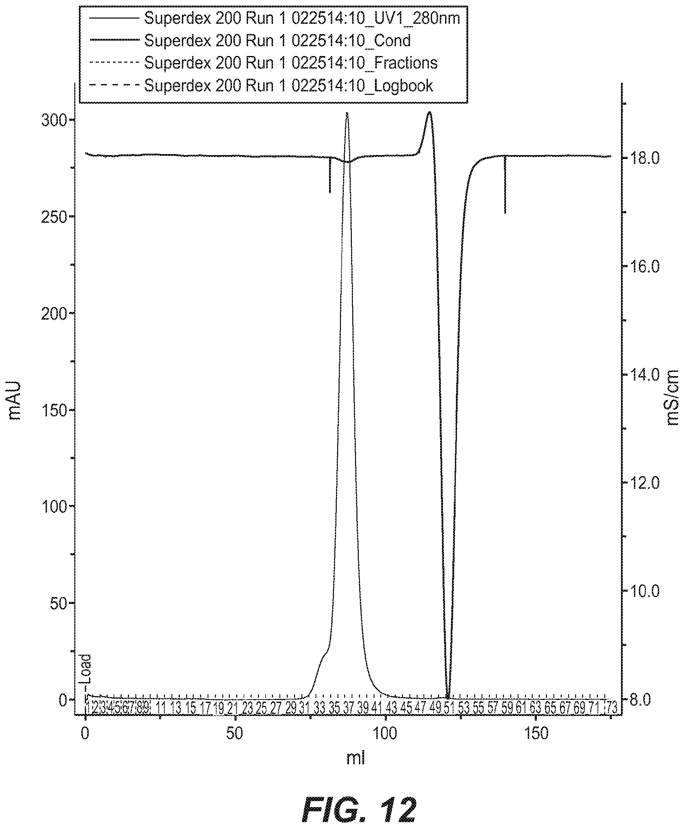

The chromatogram for the Superdex 200 chromatography step is shown in FIG. 12. The profile does not appear substantially different than the HPLC-SEC profile using the TSKgel G3000SWxl HPLC-SEC column, except that the TSKgel G3000SWxl HPLC-SEC column yielded better resolution of the higher MW species.

SDS-PAGE

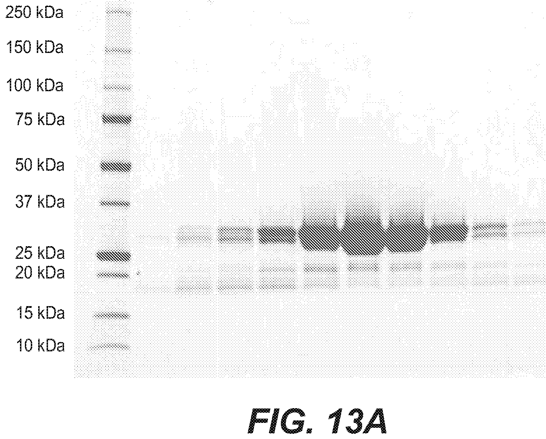

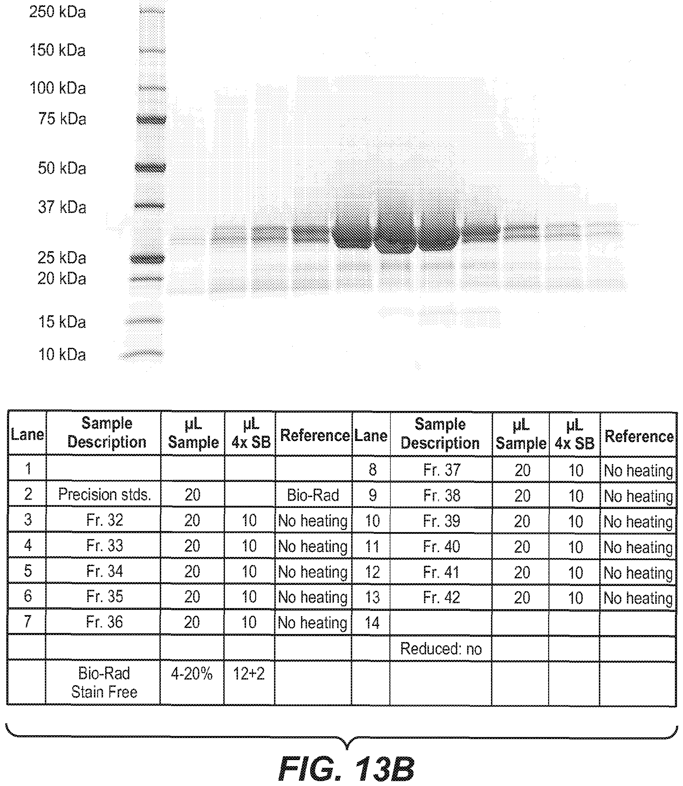

Column fractions 32-42 were analyzed by SDS-PAGE. The samples were prepared for electrophoresis by incubation with SDS sample buffer for 1 hr at room temperature (no heating). This modification to the usual sample preparation was done to see if heating the samples was responsible for the presence of the lower MW species.
The stain-free image (FIG. 13A) and SYPRO Ruby imaging of the same gel (FIG. 13B) show the presence of lower MW bands co-migrating with the intact protein. It may be concluded that the conventional sample preparation of heating for 5 min at 85°C is not responsible for the presence of the lower MW bands since they are present in the unheated samples.

Western Blot analysis

Western blot analysis was carried out to determine whether the material was sufficiently pure to be used to immunize rabbits or whether additional purification work needed to be done. For this analysis, Fraction 37 from the Superdex 200 column (the peak fraction; FIG. 12) was used.

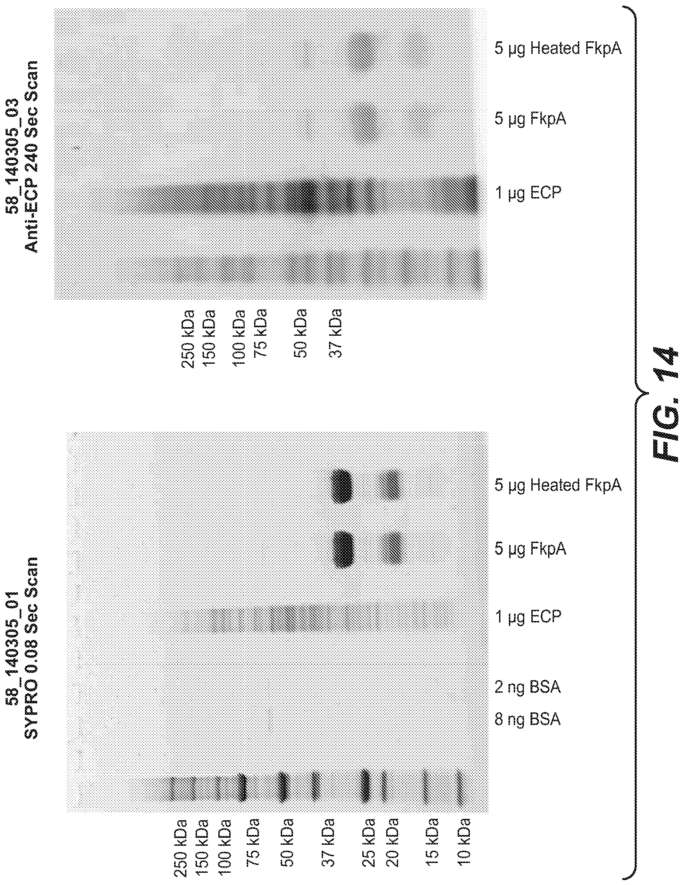

The gel and the western blot results are shown in FIG. 14. The antibody for the western blot was anti-ECP. Proteins are transferred onto PVDF sheets and each PVDF sheet is incubated with goat anti-ECP. A second incubation follows with a rabbit anti-goat antibody conjugated to horseradish peroxidase. The PVDF sheet is then incubated with an enhanced chemiluminescent substrate that luminesces by reaction with horseradish peroxidase. The molecular weight markers also luminesce with a StrepTactin and horseradish peroxidase conjugate system. The immunoblot incubated with anti-ECP reveals ECP in the sample. The gel results confirm the SYPRO Ruby imaging results previously generated (FIG. 13B). The western blot shows a faint band between 37 kDa and 50 kDa that is not seen with Sypro Ruby imaging.

Mass spectrometric analysis

The protein was digested with trypsin and analyzed by UPLC-MS/MS to determine 1) the FkpA peptide sequences present in the material, and 2) that no other protein impurities are present in the sample. The analysis consisted of a 45 minute UPLC gradient from 0-40% acetonitrile which was sprayed directly into a high resolution quadrupole-Time of Flight mass spectrometer which collected a MS survey scan followed by MS/MS of the top 20 most-abundant precursor ions from each successive MS scan during the chromatographic separation. The LC-MS/MS raw data file was searched against the Uniprot E coli canonical and isoform database, identifying 356FkpA peptides (many being multiple instances of the same peptide due to the high MS/MS sampling rate). Also found were 2 β-galactosidase peptides, which originate from the internal standard used for calibration of the mass spectrometer, and thus not contained in the FkpA sampleMass spectrometry was used to further characterize the FkpA and beta-galactosidase was used as an internal control.

Results are shown in Table 15. Mass spectrometry analysis showed -91% coverage of the FkpA sequence, with the signal sequence representing the 9% absent in FkpA peptide coverage, as expected. No other peptides from other proteins were observed, leading to the conclusion that the lower MW bands observed by SDS-PAGE and western blot analysis are FkpA-related. If there are other impurities present, their level is below that of the detection limit of the mass spectrometer and/or their composition is not present in the database that the results were compared against.

Table 15. Results of Mass Spectrometric Analysis

| % Coverage | Accession # | Name | Species | Peptides (95%) |
|---|---|---|---|---|
| 90.7 | tr\|E2QFK2\|E2QFK2_ECOLX | Peptidyl-prolyl cis-trans isomerase OS=Escherichia coli GN=fkpA PE=4 SV=1 | ECOLX | 356 |
| 4.1 | tr\|E2QGA4\|E2QGA4_ECOLX | Beta-galactosidase OS=Escherichia coli GN=lacZ PE=3 SV=1 | ECOLX | 2 |

Superdex 200 Runs 2-4

Following the initial pilot run, subsequent runs 2-4 were performed on a 320 mL column for generation of the final formulated bulk.

Column preparation

The 2.6 cm x 60 cm column (320 mL bed volume) was cleaned with 1 CV of 0.1 M NaOH and was then equilibrated with 3 CV of PBS, pH 7.5 (equilibration buffer).

Sample preparation

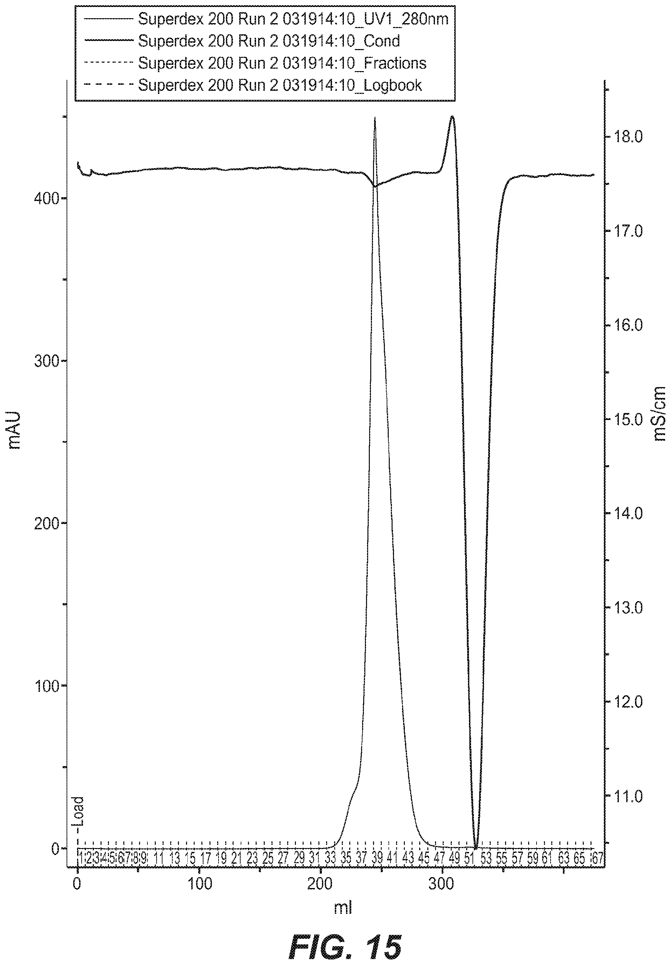

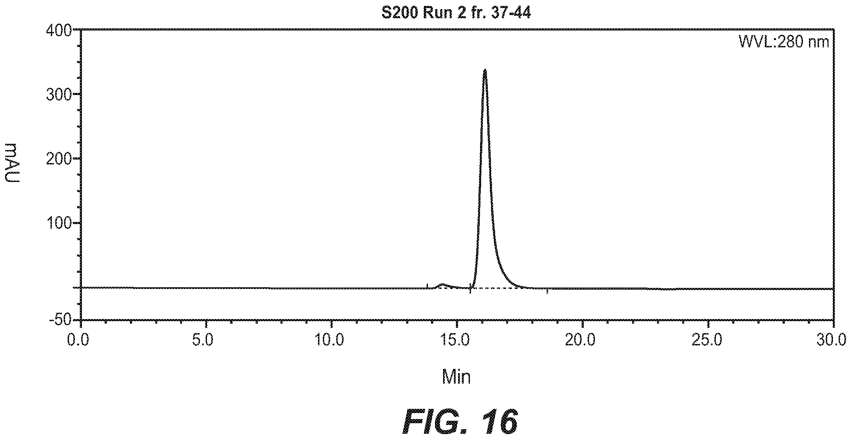

Aliquots of 60 mg - 95 mg were used for runs 2-4. The same procedure used for Run 1 was used to prepare the aliquots for SEC. The targeted final volume was ≤ 16.0 mL (≤ 5% of the bed volume). FIG. 15 shows the chromatogram obtained during Run 2. Fractions 37-44 were pooled and reanalysed using a TSKgel G3000SWxl HPLC-SEC column. FIG. 16 shows the TSKgel G3000SWxl HPLC-SEC chromatogram of Run 2 fractions 37-44. About 2% aggregate can be seen. This amount of aggregation is desirable, so as to have antibodies generated against the aggregates. Results were similar for Runs 3 and 4.

SDS-PAGE

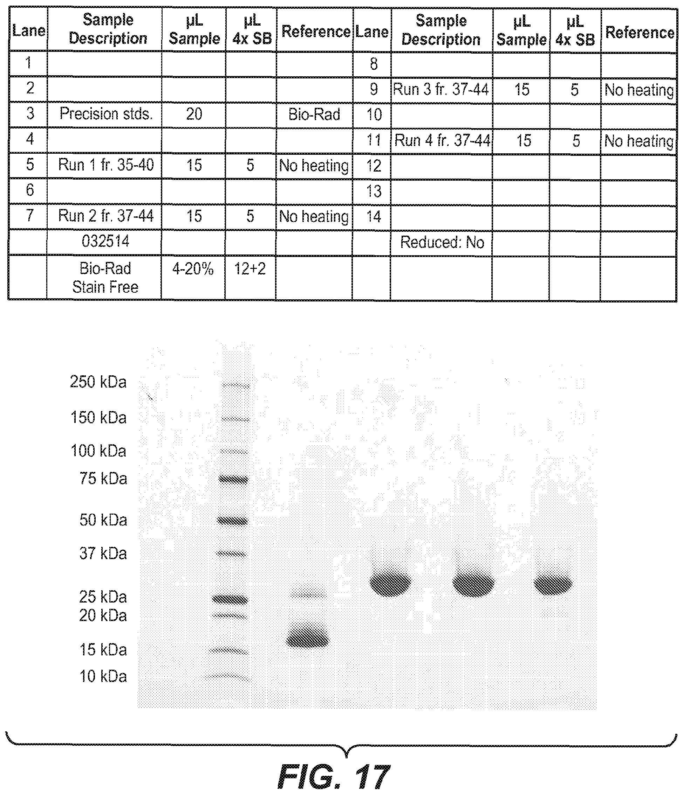

Column fractions 35-40 from Run 1 and 37-44 from Runs 2-4 were analyzed by SDS-PAGE. See FIG. 17 (lanes 2-5, respectively). Material from Run 1 appears to have degraded, and was not used for subsequent experiments.

Ultrafiltration and Filtration

The Superdex 200 pool was concentrated to a target protein concentration of 2.0 mg/mL using Amicon Ultra 10 kDa units. Only material from Runs 2-4 was included for the final bulk, due to the unforeseen and unknown cause of degradation of the Run 1 material.

Two Amicon Ultra 10 kDa units were used. 13 mL of the combined fractions from Runs 2-4 (fr. 37-44 from each run) were transferred into each unit. The samples were centrifuged as previously described. The filtrates were poured off and the UF retentates were mixed with additional material from the combined fractions (in order to minimize higher protein concentrations in the units). The process was repeated until the protein concentration was slightly above the target of 2.0 mg/mL. The concentrated sample was filtered through a 0.2 $\mu$ filter and chased with 5 mL of UF filtrate. Recovery of FkpA at the target protein concentration was virtually quantitative as shown in Table 16.

Table 16. Recovery from Ultrafiltration and Filtration Steps

| Fraction | Vol. (mL) | Conc. (mg/mL) | mg | % Recovery |
|---|---|---|---|---|
| Superdex pools | 154 | 1.49 | 228 | 100% |
| UF filtrate | 44 | 0.00 | 0 | 0% |
| Form. Pool | 110 | 2.02 | 223 | 98% |

Freeze-Thaw Stability of Ultrapure FkpA

The purpose behind freeze-thaw stability testing is to ensure that the reagent is stable for up to three freeze-thaws without loss of activity or change in electrophoretic or SEC profiles.

Materials and methods

75 $\mu$L aliquots of Ultrapure FkpA were used for the study. One aliquot was kept in the coldroom and the remaining three were placed in the $\leq$ -80°C freezer. After one hour, the three frozen aliquots were thawed and gently mixed. One aliquot ("one freeze-thaw") was placed in the coldroom and the other two were put back into the freezer. The process was repeated for the remaining two samples, with the second freeze-thaw sample transferred to the coldroom and the third freeze-thaw sample being kept overnight in the $\leq$ -80°C freezer. The third sample was thawed on the following day.

SDS-PAGE

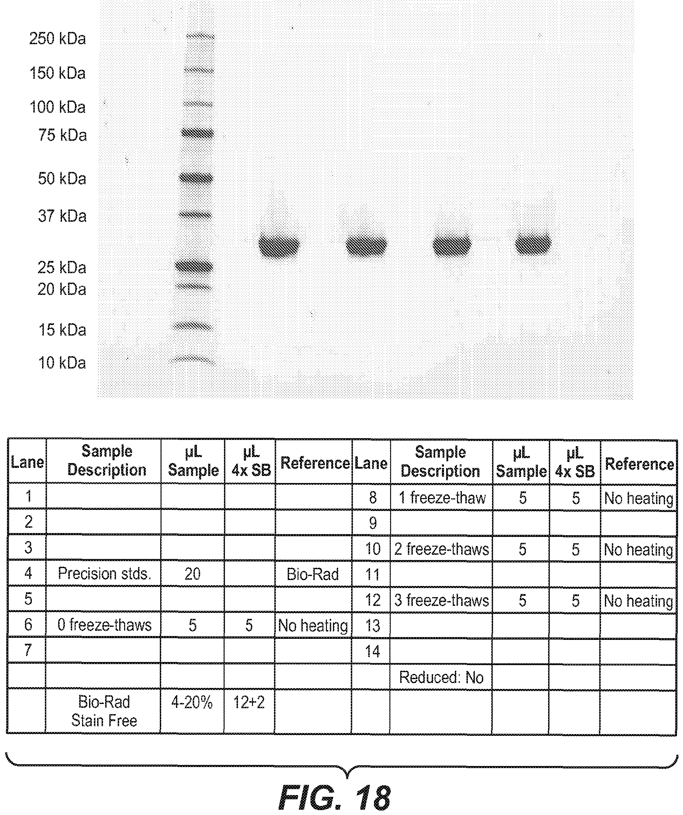

10 $\mu$g aliquots of each of the freeze-thaw samples were analyzed by SDS-PAGE. The gel results (FIG. 18) show comparability between each of the four samples.

HPLC-SEC

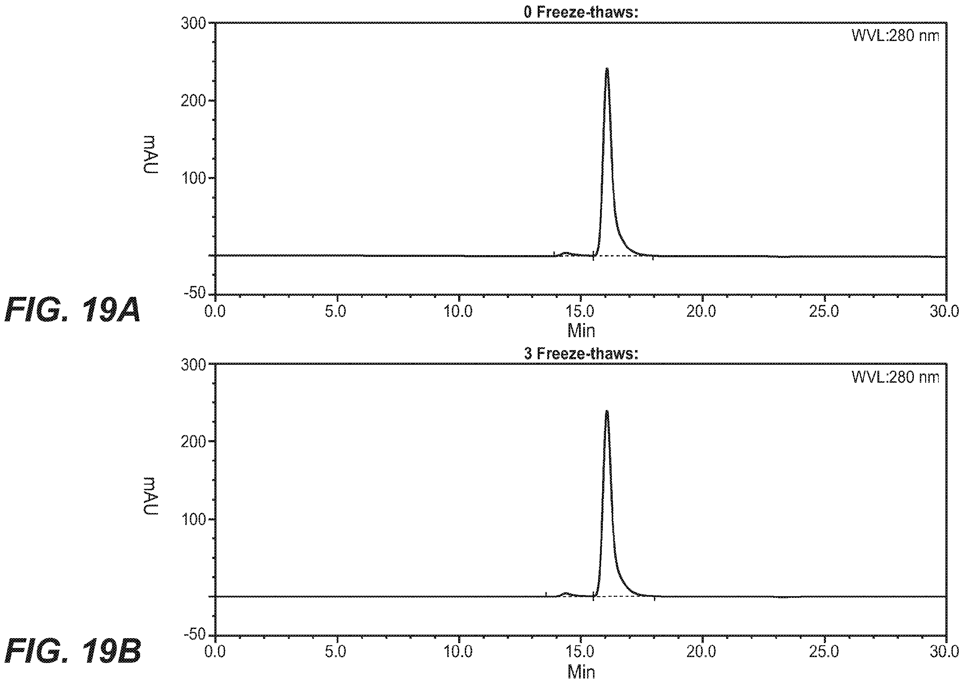

100 $\mu$g aliquots of each of the freeze-thaw samples were analyzed by HPLC-SEC. The sizing results (FIG. 19A and 19B and Table 17) show comparability between the four samples. The very small differences in higher MW species and monomer are probably due to manual integration of the peaks. The ultrapure FkpA bulk from Superdex 200 Runs 2-4 is stable to three freeze-thaws with no obvious changes in the protein. The reagent was deemed suitable to be used as the immunogen to raise antibodies in rabbits, as well as to serve as the reference for the ELISA assay and as the ligand to construct an affinity column to purify anti-FkpA from rabbit antisera.

Table 17. HPLC-SEC Analysis of Freeze-Thaw Assay of FkpA

| Freeze/Thaws | Peak ID | min | mAU*min | mAU | % |
|---|---|---|---|---|---|
| 0 | 1 | 14.377 | 2.3194 | 3.972 | 2.06 |

(continued)

| Freeze/Thaws | Peak ID | min | mAU*min | mAU | % |
|---|---|---|---|---|---|
| | 2 | 16.070 | 110.5102 | 241.565 | 97.94 |
| | Total: | | 112.8296 | 245.537 | 100.00 |
| 1 | 1 | 14.367 | 2.4097 | 4.303 | 2.13 |
| | 2 | 16.053 | 110.5952 | 239.389 | 97.87 |
| | Total: | | 113.0049 | 243.691 | 100.00 |
| 2 | 1 | 14.380 | 2.3730 | 4.255 | 2.10 |
| | 2 | 16.067 | 110.5798 | 241.087 | 97.90 |
| | Total: | | 112.9529 | 245.342 | 100.00 |
| 3 | 1 | 14.373 | 2.4747 | 4.286 | 2.20 |
| | 2 | 16.067 | 110.2633 | 238.686 | 97.80 |
| | Total: | | 112.738 | 242.972 | 100.00 |

The final product was characterized by SDS-PAGE (FIG. 14), HPLC-SEC (Table 17), N-terminal sequence analysis, and peptide mass fingerprinting (Table 15). The concentration was 2.02 mg/mL as determined spectrophotometrically using the extinction coefficient for FkpA and was diluted to 100 μg/mL. The diluted solution was designated Standard Stock I and stored at a temperature of -70 °C or less.

## Example 3. Generation and purification of anti-FkpA antibodies

Polyclonal antibodies were generated against ultrapure FkpA for use in assays to measure the removal of FkpA in the preparation of therapeutic polypeptides. The following example describes the purification methods used to generate the polyclonal FkpA antibodies. These reagents, along with the FkpA immunogens, were required for the development of the specific FkpA ELISA.

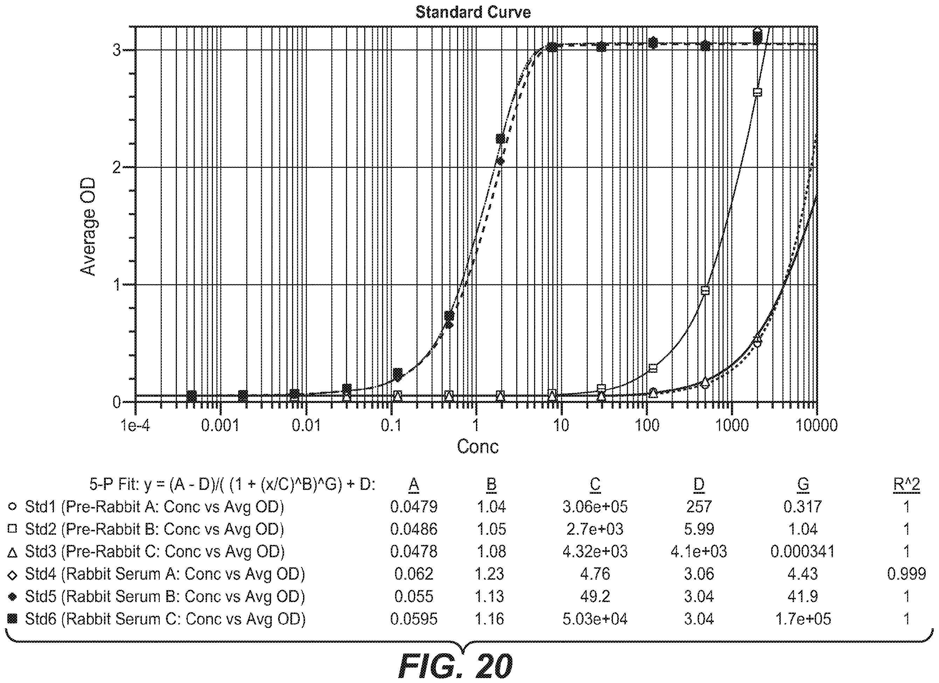

Three rabbits were immunized with the ultrapure FkpA. At day 42, blood was drawn from individual rabbits and the FkpA antisera were used for the purification of anti-FkpA antibodies. Antisera and pre-immunization sera from rabbits A, B, and C were assayed by direct binding ELISA. Ultrapure FkpA was diluted to 4 μg/mL in carbonate buffer, pH 9.6 and coated directly on a Costar 96-well plate (catalog #9018) and incubated at 2-8°C for 12 to 72 hours. Each well was washed and aspirated with approximately 400 μL of Wash Buffer (phosphate buffered saline (PBS) and 0.05% polysorbate 20) 3 times using platewasher and blotted thoroughly. Solution was not removed from wells by inverting plate over waste reservoir. Approximately 200 μL of Assay Diluent was added to each well and incubated for 1 to 2 hours at ambient temperature with agitation. The wash step was repeated. Samples were diluted 1:500 in Assay Diluent (0.15 M NaCl, 0.1M NaPO4 pH 7.2, 0.05% polysorbate 20, 0.1% fish gelatin; 0.05% proclin 300) and then serially diluted four fold up to 12 times and added to the corresponding well. The plate was incubated at room temperature for two hours with agitation. Plates were washed with ELISA wash buffer and goat anti-rabbit-HRP diluted in assay diluent was added to each well and incubated at room temperature for two hours with agitation. Plates were washed with ELISA wash buffer and the SureBlue Reserve™ TMB Microwell Peroxidase Substrate (Kirkegaard & Perry Labs [KPL], catalog # 53-00-00) (Substrate Solution) was added to the plate and color was allowed to develop for approximately 20 minutes at room temperature. The reaction was stopped with 0.6N Sulfuric acid. The OD's were read at a wavelength of 450 nm and data was analyzed using Softmax Pro data reduction software. The immunoreactivity of antisera from rabbits A, B, and C against FkpA was comparable (FIG. 20), therefore the samples were pooled.

*Purification of FkpA antibodies*

A 60% ammonium sulfate precipitation (ASP) step was performed on the pooled antisera. 52.7 mL ammonium sulfate solution (3.8 M ammonium sulfate, 50 mM phosphate, pH 7.0) was slowly added to 73.0 mL anti-FkpA rabbit antisera with gentle stirring throughout the addition. Once the addition was complete, the solution was transferred to the coldroom and gently mixed overnight.

Sodium dodecyl sulfate polyacrylamide gel electrophoresis (SDS-PAGE) was then used to determine the relative purity of the ASP anti-FkpA antibodies as well as to confirm the molar mass of the proteins. 2 x 10 μL aliquots of the

AS suspension were transferred into separate Eppendorf tubes. 90 $\mu$L of PBS, pH 7.2 was added to each sample and gently mixed to dissolve the suspensions. The 100 $\mu$L samples were transferred to an Eppendorf tube. The sample was microfuged at top speed for 5 min. The supernatant was withdrawn and transferred to a separate Eppendorf tube. The remaining pellet was reconstituted in 100 $\mu$L of PBS, pH 7.2. 2 x 10 $\mu$L aliquots of the supernatant were transferred into separate Eppendorf tubes. 90 $\mu$L of PBS, pH 7.2 was added to each supernatant sample and gently mixed.

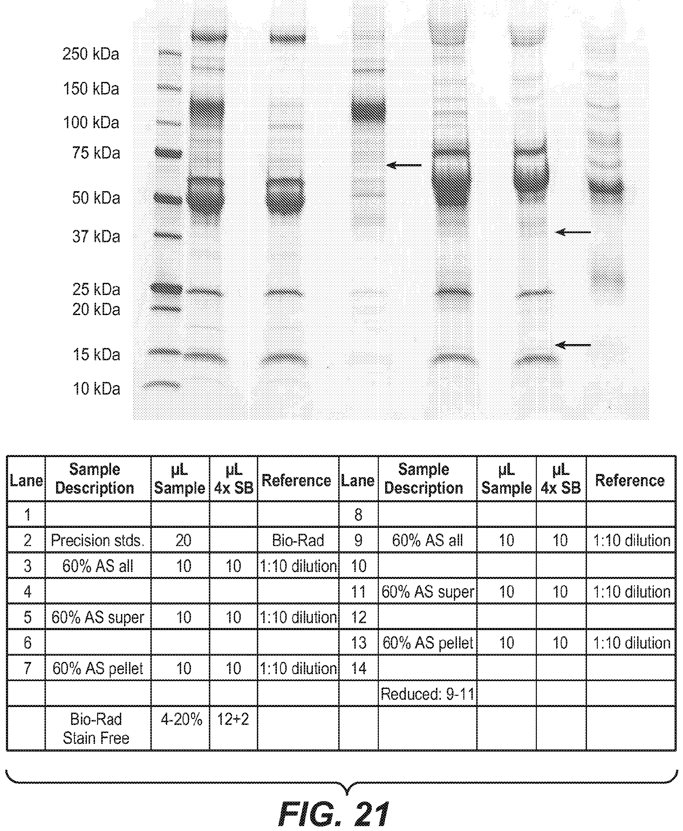

10 $\mu$L of samples from each condition were analyzed by SDS-PAGE. Electrophoresis was performed with and without disulfide bond reduction to assess for covalent aggregation of the antibodies. SDS-PAGE was performed using Bio-Rad Criterion™ 4-20% TGX (Tris-Glycine eXtended) Stain-Free™ gels and imaged with a Bio-Rad Criterion Stain Free™ Imager (Bio-Rad Laboratories). Gel results are shown in FIG. 21. The 60% fractionation step successfully depleted the antisera of all of the rabbit antibodies, as evidenced by the absence of antibodies in the supernatants as marked by arrows.

Following analysis by SDS-PAGE, the remaining ammonium sulfate suspension was centrifuged at 11,000 rpm, at a temperature of 2-8°C for 30 minutes in a Sorvall RC 6 Plus centrifuge. The supernatant was removed and the pellet was stored at -80°C.

The pellets were then dissolved in phosphate buffer. The concentration of the ASP anti-FkpA antibodies was determined by UV spectrophotometry to be 6.69 mg/mL.

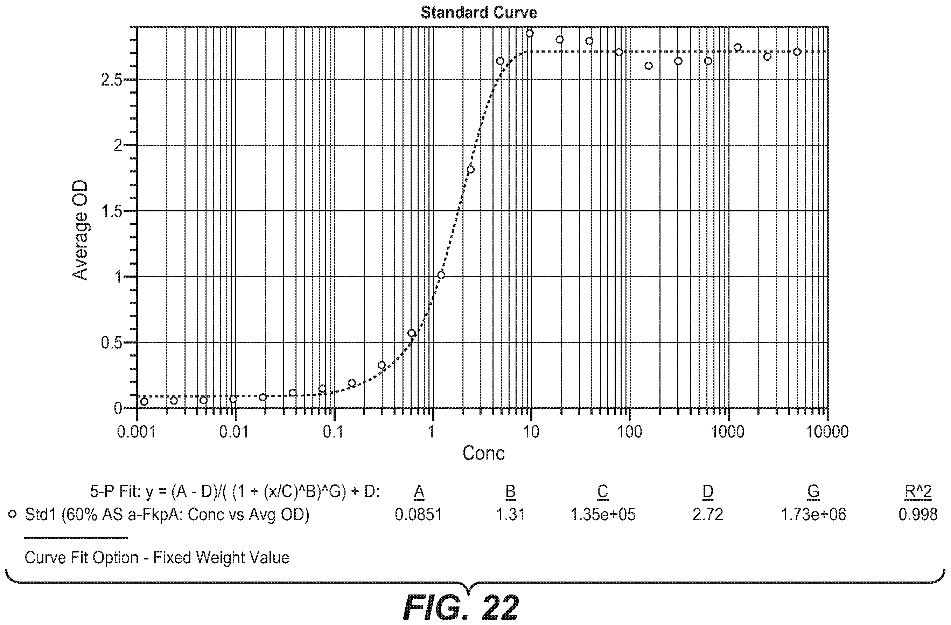

ASP anti-FkpA antibodies were assayed by direct binding ELISA as described above. Ultrapure FkpA was diluted to 4 $\mu$g/mL in carbonate buffer, pH 9.6 and coated directly on a Costar 96-well plate (catalog #9018). Samples were diluted 1:500 in assay diluent and then serially diluted two fold up to 12 times. Goat anti-rabbit-HRP was used to detect binding. The ASP anti-FkpA antibodies show a linear dose response to ultrapure FkpA starting at a dilution of 1:256,000 (FIG. 22). These results prompted the pursuit of an ELISA sandwich assay.

*Development of ELISA Sandwich assay with ASP anti-FkpA antibodies*

1 mg of ASP anti-FkpA antibodies were conjugated to HRP using a Pierce Kit EZ-Link Peroxidase (cat#31489) per manufacturer's instructions. HRP conjugated ASP anti-FkpA antibodies were further diluted 1/20 in assay diluent and was designated ASP HRP Conjugate Stock I. ASP anti-FkpA antibodies were aliquoted and were designated as ASP Coat Source. ASP Coat Source was then diluted in PBS to 1 mg/mL and was designated ASP Coat Stock I.

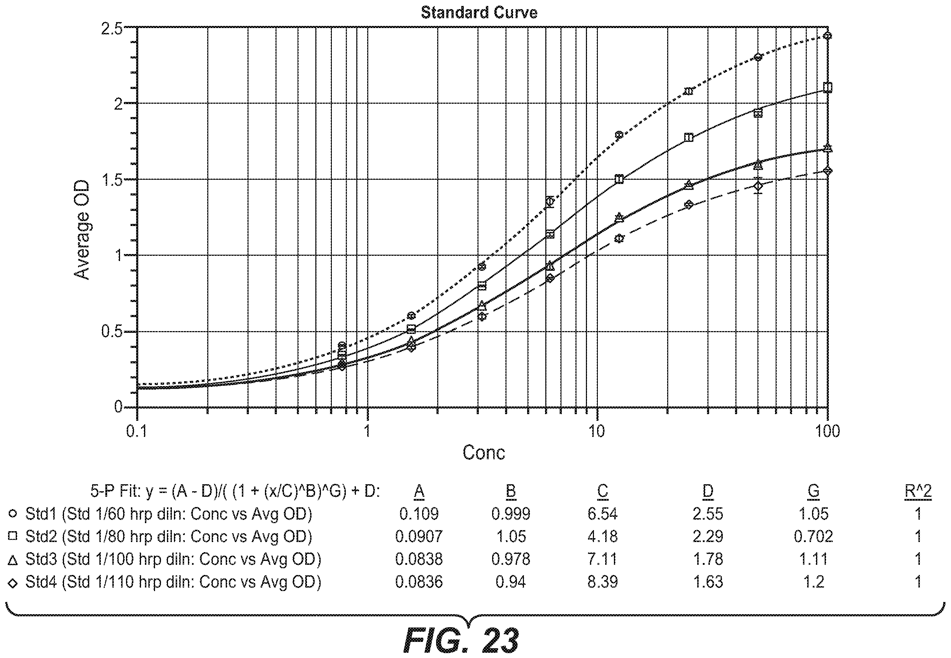

ASP Coat Stock I was diluted to 4 $\mu$g/mL in carbonate buffer, pH 9.6 and coated directly on a Costar 96-well plate (catalog #9018). FkpA was diluted to various concentrations ranging from 0.78-100 ng/mL and was added to each corresponding well. ASP HRP Conjugate Stock I was diluted to 1:60, 1:80, 1:100, and 1:110 and was added to each corresponding well. 100 $\mu$L of diluted ASP Coat Stock I was pipetted into each well of microtiter plate and incubated at 2-8°C for 12 to 72 hours. Each well was washed and aspirated with approximately 400 $\mu$L of Wash Buffer 3 times using platewasher and blotted thoroughly. Solution was not removed from wells by inverting plate over waste reservoir. Approximately 200 $\mu$L of Assay Diluent was added to each well and incubated for 1 to 2 hours at ambient temperature with agitation. The wash step was repeated. 100 $\mu$L per well of diluted standards (in duplicate), controls (in duplicate), and samples was pipetted into appropriate wells. The plate was incubated at ambient temperature for 2 hours $\pm$ 10 minutes with agitation. Wells were washed as above, the plate was rotated, and the wash step was repeated. The ASP HRP-Conjugate Stock I was diluted with Assay Diluent to yield a significant OD range between the highest and lowest standards, targeting a maximum OD value of 1.5 - 2.0 OD. 100 $\mu$L of diluted HRP-Conjugate Stock I was pipetted to each well and incubated at ambient temperature for 2 hours $\pm$ 10 minutes with agitation. Wells were washed as above, the plate was rotated, and the wash step was repeated. 100 $\mu$L per well of Substrate Solution was pipetted to the wells and incubated for a sufficient time in the dark at ambient temperature to allow optimal standard curve color development. 100 $\mu$L of 0.6 N Sulfuric Acid was pipetted into each well. ODs were read using a platereader using two filters, 450 nm for detection absorbance and 620-630 nm for reference absorbance (reference wavelength was optional). Sample concentrations were determined by using data-processing software with a minimum 4-parameter logistic curve-fitting program. Results are shown in FIG. 23. The standard curves appear to plateau at ~25 ng/mL. The working range was determined to be -0.156-20 ng/mL and the optimal ASP HRP Conjugate Stock I dilution was 1:90 as determined by the expected maximum OD of ~1.7.

*Use of ELISA Sandwich assay with ASP anti-FkpA antibodies*

An ELISA sandwich assay was performed to assay the amount of FkpA in an anti-IL13/IL17 bispecific antibody sample generated as described in Example 6. ASP HRP Conjugate Stock I was diluted 1:90. The results show non-linearity (Table 18); therefore suggesting that additional antibody purification may be preferred.

Table 18. ASP ELISA Sandwich Assay

| Concentration of αIL 13 and/or αIL17 at Each Step (mg/mL) | | | | | |
|---|---|---|---|---|---|
| αIL13 | αIL17 | Assembly | Capto™ | QSFF | PHS F/T |
| 10.26 | 9.01 | 8.26 | 4.8 | 5.31 | 4.37 |

| Concentration of FkpA at Each Step (ng/mL) | | | | | | |
|---|---|---|---|---|---|---|
| Dilution | αIL13 | αIL17 | Assembly | Capto™ | QSFF | PHS F/T |
| 10 | GTR | GTR | GTR | GTR | 28.505* | 1.437 |
| 40 | GTR | GTR | GTR | GTR | 11.554 | LTR |
| 160 | GTR | GTR | GTR | GTR | LTR | LTR |
| 640 | GTR | 30.962 | 30.725 | 82.828* | LTR | LTR |
| 2560 | 20.783 | 8.694 | 7.616 | 15.317 | LTR | LTR |
| 10240 | 4.555 | 2.301 | 1.743 | 3.700 | LTR | LTR |
| 40960 | 1.163 | LTR | LTR | 0.857 | LTR | LTR |
| 163840 | LTR | LTR | LTR | LTR | LTR | LTR |

| Concentration of FkpA/Concentration of αIL13 and/or αIL17 (ng/mg) | | | | | | |
|---|---|---|---|---|---|---|
| Dilution | αIL13 | αIL17 | Assembly | Capto™ | QSFF | PHS F/T |
| 10 | | | | | 53.7* | 3.3 |
| 40 | | | | | 87.0 | |
| 160 | | | | | 109.7 | |
| 640 | | 2199.3 | 2380.6 | 11043.7* | 126.1 | |
| 2560 | 5185.6 | 2470.2 | 2360.4 | 8167.1 | | |
| 10240 | 4546.1 | 2615.1 | 2160.8 | 7893.3 | | |
| 40960 | 4642.9 | | | 7313.1 | | |
| 163840 | | | | | | |
| Mean | 4791.6 | 2428.2 | 2300.6 | 7791.8 | 107.6 | 3.3 |
| SD | 344.7 | 211.1 | 121.5 | 436.9 | 19.6 | N/A |
| CV | 7 | 9 | 5 | 6 | 18 | N/A |
| *=data not included in calculation, N/A=not applicable, GTR=greater than range, LTR=less than range | | | | | | |

*Affinity purification of FkpA antibodies*

To improve the linearity of the ELISA sandwich assay, an aliquot of ASP anti-FkpA antibodies were further purified by affinity (AF) purification and size-exclusion chromatography (SEC). An affinity column was constructed using ultrapure FkpA covalently coupled to activated Glyceryl CPG in order to separate the target anti-FkpA antibodies from non-FkpA antibodies.

Preparation of Ultrapure FkpA for Coupling to Glyceryl Controlled Pore Glass

20 x 1 mL vials of 2.02 mg/mL Ultrapure FkpA were thawed overnight at 2-8°C and the contents were pooled. A 125 mL Sephadex G-25 column (GE Healthcare) was used to buffer exchange the FkpA into coupling buffer (0.1 M sodium bicarbonate, 0.5 M sodium chloride, pH 8.3). The column was cleaned with 1 CV of 0.1 M NaOH and then equilibrated with 3 CV of coupling buffer.

Coupling procedure

Glyceryl-CPG (Millipore Corporation) was chosen as the support for construction of an affinity column due to its high flow rate characteristics and its ability to withstand low pH conditions during the elution phase. The Glyceryl moiety of the support has alcohol functional groups that are oxidized to aldehydes prior to amino chemistry coupling. All coupling steps were done at room temperature.

Dry Glyceryl-CPG was added to an appropriate amount of purified water in the fume hood (1 gram of Glyceryl-CPG will produce about 3 mL of hydrated gel). The Glyceryl-CPG was gently mixed with the water to fully hydrate the gel. The hydrated gel was allowed to settle for 15-30 minutes. A volume of 10 - 12 mL of settled volume was targeted for the hydrated gel.

The liquid layer (containing fines) was removed and purified water was added to the settled gel. The gel was gently mixed and then settled for 15-30 minutes. The liquid layer was removed and the step was repeated, as needed, until no fines were observed in the liquid layer.

Sodium meta-periodate (NaIO4) was chosen to oxidize the hydroxyl groups on the Glyceryl-CPG gel to aldehydes. An equal volume of freshly prepared 1% (m/v) sodium meta-periodate was added to the hydrated Glyceryl-CPG, and then transferred to a suitably sized container. The container was secured to an orbital rotator and gently mixed for 30 minutes. At the end of the oxidation step, the container was removed from the rotator and the gel was allowed to settle.

The liquid was removed, and 3-4 volumes of phosphate buffered saline (PBS), pH 7.2, was added to the gel. The gel was gently suspended and then allowed to settle for 15 minutes. The liquid was removed and PBS was was repeated 3 additional times in order to remove residual sodium metaperiodate prior to the addition of the protein to be coupled. Finally, 3-4 volumes of 0.1 M sodium bicarbonate, 0.5 M NaCl, pH 8.3 (coupling buffer) were added to the settled gel, mixed gently, and allowed to settle for 15 minutes. The liquid was removed prior to the coupling step.

The buffer-exchanged FkpA (from the Sephadex G-25 step) was added to the oxidized Glyceryl-CPG. Sodium cyanoborohydride (NaBH3CN) was used to reduce the intermediate Schiff base and form a stable covalent bond between FkpA and the support. NaBH3CN was added at ~ 1 mg of sodium cyanoborohydride per mL of settled Glyceryl-CPG to the reaction mixture. The reaction mixture was transferred into a sealed container, secured to the orbital rotator, and gently mixed overnight at room temperature.

The coupled gel was removed from the rotator and allowed to settle for 15-30 minutes. After settling, a 2 mL aliquot of the supernatant was removed and transferred to a pair of 1 mL Eppendorf tubes. The tubes were microfuged at top speed for 2 minutes in order to remove any residual solids. The supernatant was measured for residual protein to determine coupling efficiency. Binding of the FkpA to the oxidized Glyceryl-CPG should be quantitative, depleting the supernatant of protein. The protein concentration in the supernatant showed no signs of residual FkpA, indicating that the coupling efficiency was 100% (data not shown).

The FkpA-CPG gel was washed to remove residual NaBH3CN as described above for removing NaIO4.

An equal volume of 1.0 M Tris, pH 8.0, was added to the FkpA-CPG gel. Coupling of FkpA was used to introduce additional amine groups to block any remaining reactive sites prior to the use of the gel as an affinity support.

The FkpA-CGP was again washed as described above.

Affinity column preparation

The affinity gel was packed in a 1.6 cm XK column (GE Healthcare). The final dimensions were 1.6 cm x 6.3 cm (CV = 12.6 mL). The column was washed with 5 CV of PBS, 0.02% sodium azide, pH 7.2 and then stored in the coldroom until used.

*FkpA-CPG affinity chromatography*

Run 1: initial evaluation of FkpA-CPG affinity gel

An initial evaluation of affinity column performance was carried out using 25% of the 60% ammonium sulfate pellet. The pellet was thawed from $\leq$ -60°C storage and warmed to room temperature. 120 mL of 3.8 M ammonium sulfate, 50 mM sodium phosphate, pH 7.0 was added to the pellet and the pellet was gently mixed to suspend it. The suspension was divided between four SS-34 centrifuge tubes and centrifuged for 30 min at 15 K rpm in the Sorvall RC 6 Plus centrifuge. The supernatants were discarded and three of the four centrifuge tubes were transferred back into the $\leq$ - 60°C freezer for purification at a later date.

30 mL of 50 mM sodium phosphate, pH 7.0 was added to the remaining centrifuge tube. The tube was secured to the laboratory rotator and gently mixed for 1 hr at room temperature to completely solubilize the proteins in the pellet.

The sample was centrifuged at 15 K rpm for 15 min and then filtered through a 0.2 μ filter. The filtered sample was chased with 30 mL of PBS, 0.02% sodium azide, pH 7.5 (the equilibration buffer for the affinity column) to generate the feedstock to be loaded to the affinity column.

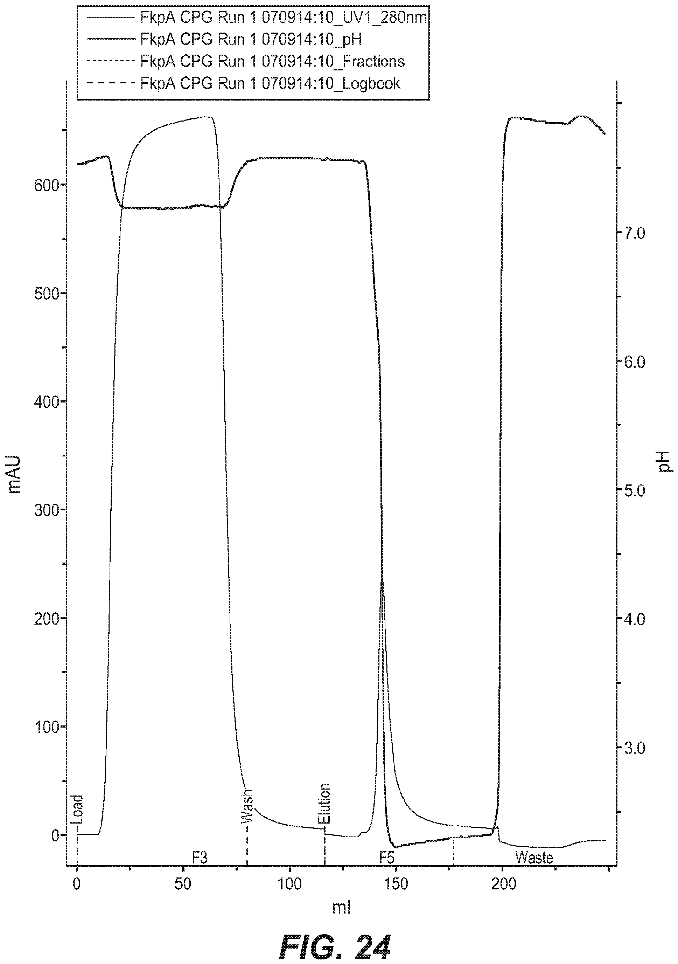

The sample was loaded to the column and chased with 20 mL of equilibration buffer. After washing, the elution pool was collected into a beaker that contained 17 mL of 1.0 M Tris, pH 8.0 with gentle mixing throughout collection of the pool. The chromatogram is shown in FIG. 24.

The pH-adjusted pool was filtered using a 0.2 μ filter and chased with 5.0 mL of equilibration buffer prior to measuring the protein concentration and recovery in the pool. The concentration was determined by UV-VIS spectrophotometry to be 0.058 mg/mL. The pH-adjusted pool was stored at 2-8°C.

Superdex 200 chromatography

SEC was performed using a Superdex 200 column (GE) to remove aggregates and to formulate the pH-adjusted affinity pools.

A 1.6 cm x 60 cm (1 CV = 120 mL) Superdex 200 column (GE Healthcare) was cleaned with 1 CV of 0.1 M NaOH and then equilibrated with 3 CV of PBS, pH 7.5 (equilibration buffer).

Run 1: chromatography of FkpA-CPG Run 1

The 83 mL pH-adjusted pool from FkpA-CPG Run 1 was concentrated to a volume of ≤ 6.0 mL (≤ 5% of 1 CV) using two Amicon Ultra 10 kDa units. 13 mL of the pH-adjusted pool was transferred into each unit. The concentrators were spun at 3 K rpm for 30 min in the Eppendorf centrifuge. The filtrates were removed and replenished with pH-adjusted pool. The process was repeated until the target volume of UF retentate was achieved (UF retentate volume = 3.8 mL).

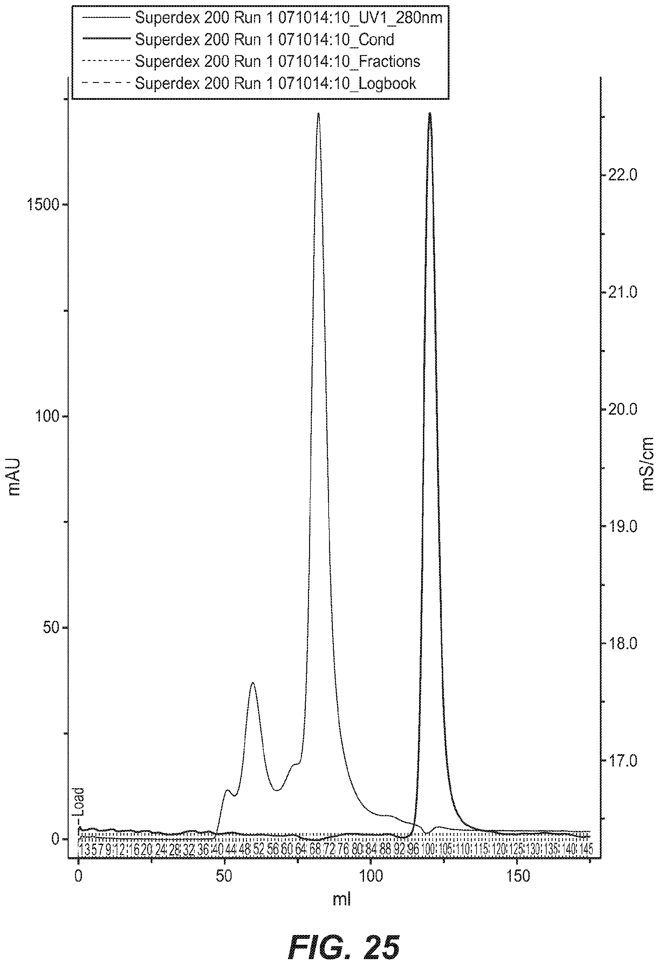

The UF retentate was loaded to the column and chased with PBS, pH 7.5. The chromatogram (FIG. 25) shows a number of higher MW species that are separated from the target monomeric species.

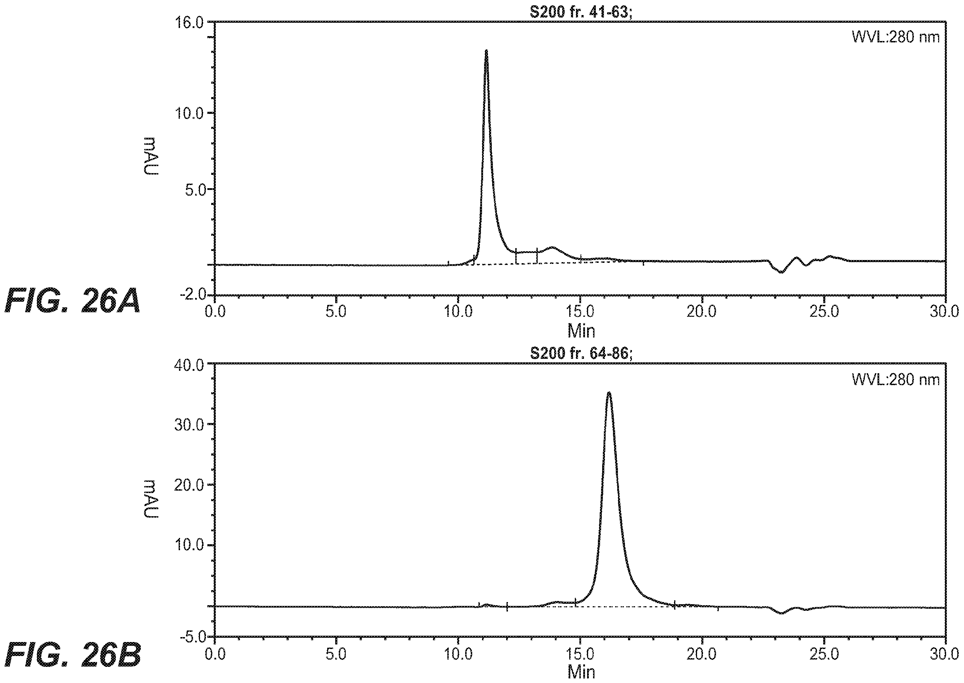

Fractions containing higher MW species (fr. 41-63) and those comprising the main peak (fr. 64-86) were analyzed by HPLC-SEC. The assay results (FIGs. 26A and 26B) show that the main peak contains ~ 96% monomeric species, greatly enhanced by the use of the size-exclusion chromatography step.

The Superdex 200 fractions were pooled and concentrated using Amicon Ultra concentration units (Millipore). The concentration was determined by UV spectrophotometry to be 3.1 mg/mL

1 mg of affinity purified anti-FkpA antibodies were conjugated to HRP using a Pierce Kit EZ-Link Peroxidase (cat#31489) per manufacturer's instructions. HRP conjugated affinity purified anti-FkpA antibodies were further diluted 1/20 in assay diluent and was designated AF HRP Conjugate Stock I. Affinity purified anti-FkpA antibodies were aliquoted and designated AF Coat Source. AF Coat Source was diluted in PBS to 1 mg/mL and was designated AF Coat Stock I.

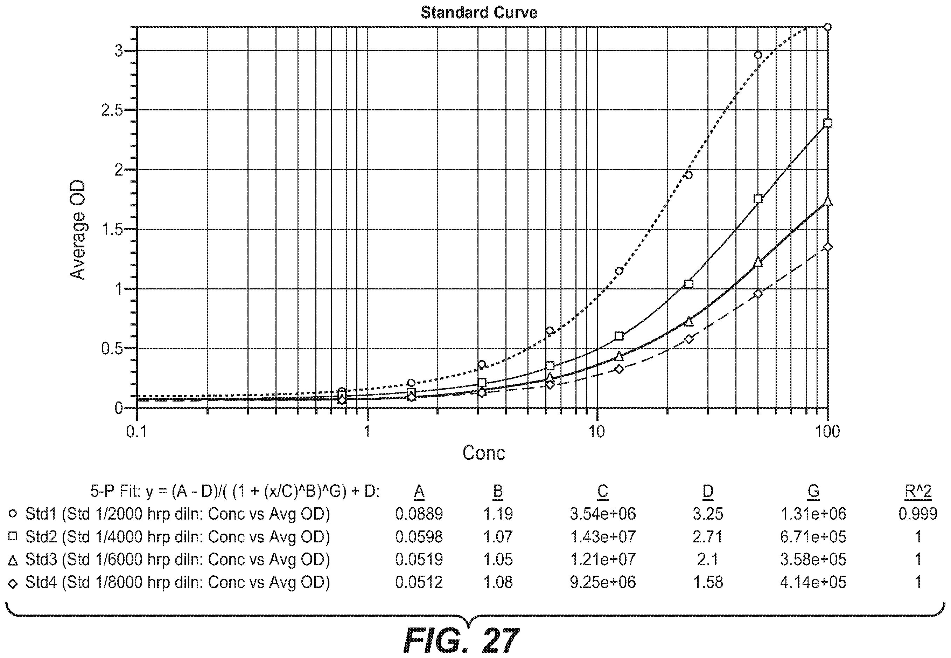

AF Coat Stock I was diluted to 4 μg/mL in carbonate buffer, pH 9.6 and coated directly on a Costar 96-well plate (catalog #9018). FkpA was diluted to various concentrations ranging from 0.78-100 ng/mL and was added to each corresponding well. AF HRP Conjugate Stock I was diluted to 1:2000, 1:4000, 1:6000, and 1:8000 and was added to each corresponding well. The assay was performed as described above. Results are shown in FIG. 27. The working range was determined to be 0.78-100 ng/mL and the optimal AF HRP Conjugate Stock I dilution was 1:6000 as the maximum OD was ~1.7.

*Comparison of ASP and Affinity Purified anti-FkpA Antibodies*

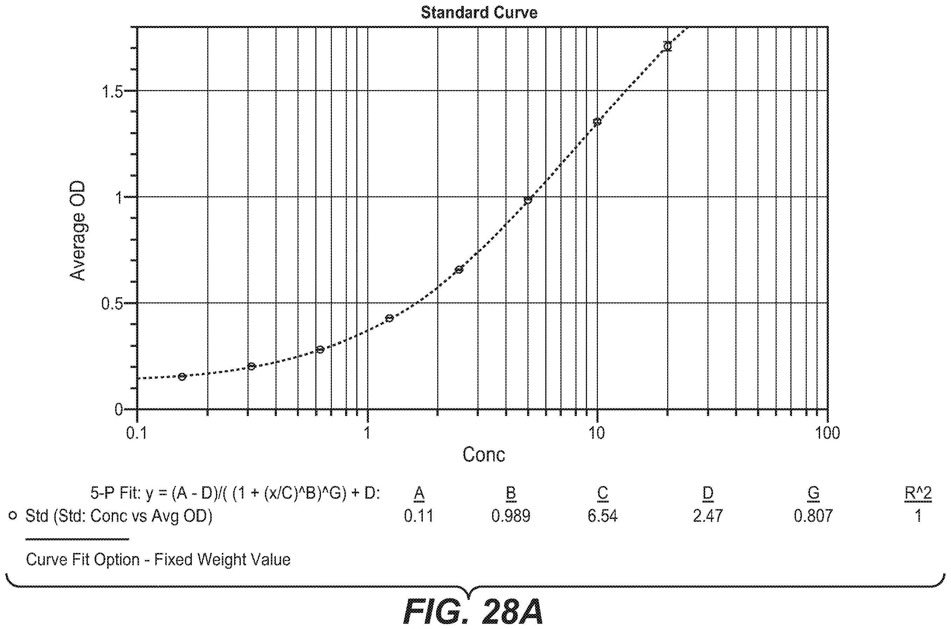

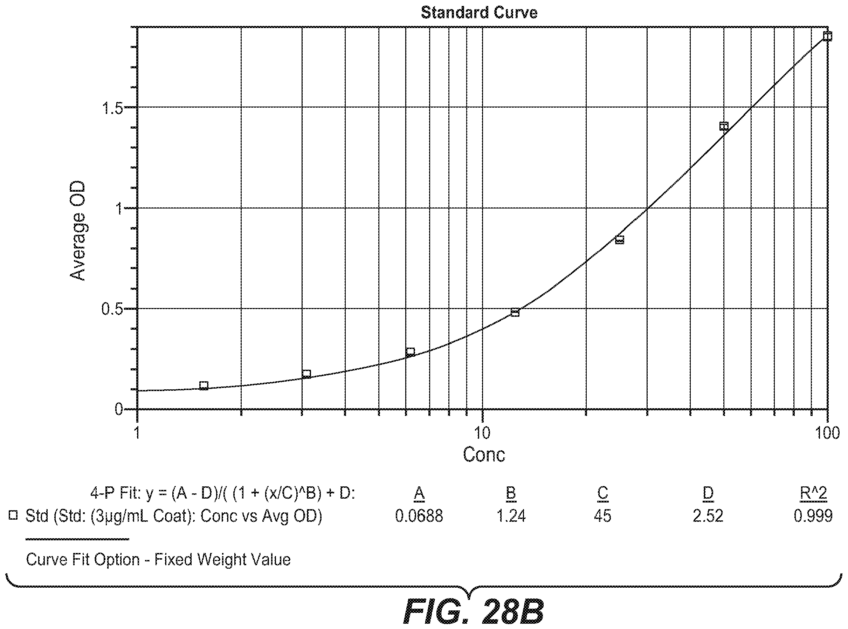

An ELISA sandwich assay was performed to compare the ability of ASP and affinity purified anti-FkpA antibodies to assay the amount of FkpA in an anti-IL13/IL17 bispecific antibody sample prepared as described in Example 6 containing 5.31 mg/mL anti-IL13/IL17. Assay parameters are shown in Table 19. The resulting standard curves for ASP and AF anti-FkpA antibodies are shown in FIGs. 28A and 28B. As shown in FIGs. 28A, 28B, and Tables 19 and 20, affinity purified antibodies had a dynamic range of 0.78ng/mL-100ng/mL, had improved linearity, and required less HRP detection antibody.

Table 19. Parameters used in comparison of ASP and AF anti-FkpA antibodies

| Parameters | ASP-FkpA | AF-FkpA |
|---|---|---|
| Coat | 4 ug/mL | 4 ug/mL |
| Conjugate Dilution | 1/90 | 1/6000 |

(continued)

| Parameters | ASP-FkpA | AF-FkpA |
|---|---|---|
| Blank OD | 0.092 | 0.054 |
| OD at 0.156 or 0.78 ng/mL | 0.137 | 0.087 |
| Standard Curve Range | 0.156-20 ng/mL | 0.78-100ng/mL |
| Max OD | 1.537 | 1.753 |

Table 20. ASP and AF anti-FkpA antibodies results

| | Raw Results (ng/mL) | | Dilution Corrected Results (ng/mL) | |
|---|---|---|---|---|
| Dilution | AF-FkpA | AS-FkpA | AF-FkpA | AS-FkpA |
| 1/20 | 48.014 | 20.016 | 960 | 400 |
| 1/40 | 22.798 | 11.344 | 912 | 454 |
| 1/80 | 11.180 | 6.357 | 894 | 509 |
| 1/160 | 5.444 | 3.528 | 871 | 564 |
| 1/320 | 2.562 | 1.864 | 820 | 596 |
| 1/640 | 1.234 | 0.937 | 790 | 600 |
| Mean | | | 875 | 521 |
| SD | | | 62 | 81 |
| CV | | | 7 | 16 |
| FkpA/aIL13/aIL17 (ng/mg) | | | 165 | 98 |

*Affinity Purification of remaining ASP antibodies*

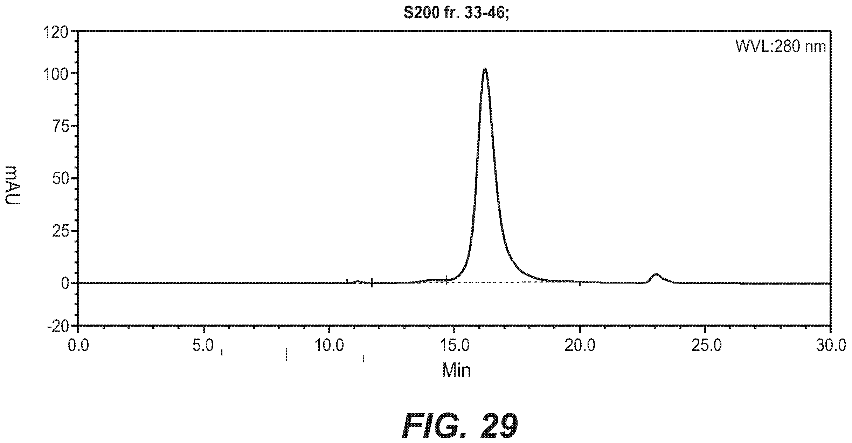

Based on the side-by-side comparison described above, the remaining ASP antibodies were purified using affinity chromatography, followed by size-exclusion chromatography. Fractions 33-46 contain -98% monomer (FIG. 29). The concentration was determined by UV spectrophotometry to be 0.386 mg/mL. Superdex 200 Run 2 fr. 33-46 were combined and concentrated using a single Amicon Ultra 10 kDa unit, as previously described. The concentration was determined by UV spectrophotometry to be 1.2 mg/mL and the sample was designated AF Coat Stock II.

*Freeze thaw stability of AF antibodies*

The purpose behind freeze-thaw stability testing is to ensure that the reagent is stable for up to three freeze-thaws without loss of activity or change in electrophoretic or SEC profiles. 100 μL aliquots of AF Coat Stock II were used for this experiment. One aliquot was kept in the coldroom and the remaining three were placed in the ≤ -60°C freezer. After one hour, the three frozen aliquots were thawed and gently mixed. One aliquot ("one freeze-thaw") was placed in the coldroom and the other two were put back into the freezer. The process was repeated for the remaining two samples, with the second freeze-thaw sample transferred to the coldroom and the third freeze-thaw sample being kept overnight in the ≤ -60°C freezer. The third sample was thawed on the following day.

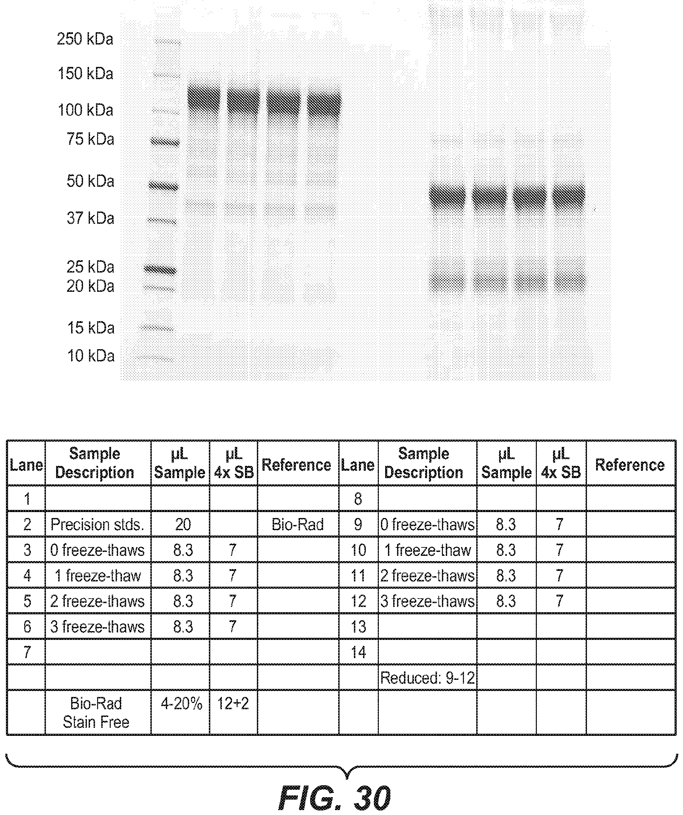

10 μg aliquots of each of the freeze-thaw samples were analyzed by SDS-PAGE. The gel results are shown in FIG. 30. The freeze-thaw samples appear comparable between freeze-thaws on both sides of the gel. The reduced side shows some very high MW bands in each of the samples. These bands may be gel artifacts associated with the reduction step.

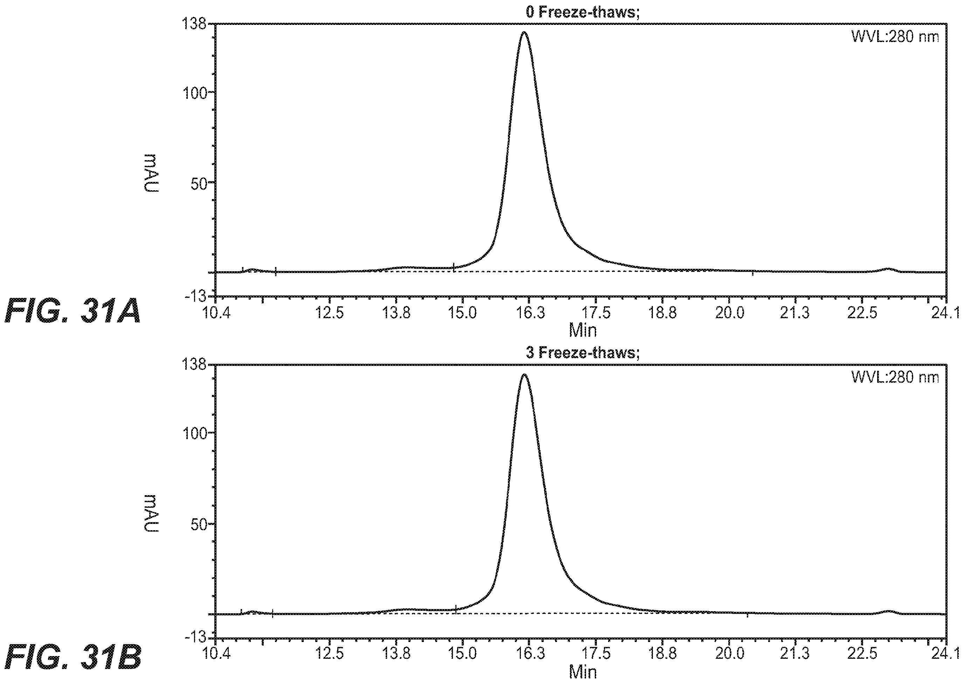

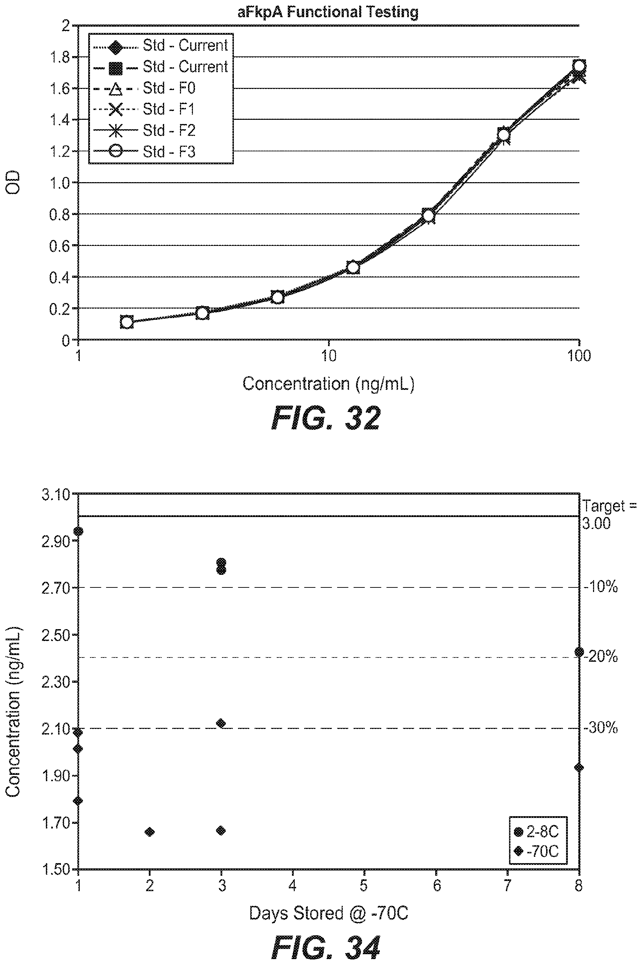

The four freeze-thaw samples were analyzed by HPLC-SEC. 50 μg aliquots were used for each of the samples. Assay results are in FIG. 31A and 31B and Table 21. The profiles are fairly similar, but show a slight trend towards increased higher MW species with increasing freeze-thaws. Functional testing of these samples revealed that the increase in higher MW species had little impact on the use of the reagent (FIG. 32). Based on SDS-PAGE and HPLC-SEC analysis, there are no significant differences in protein purity and/or composition for up to 3 freeze-thaws.

Table 21. HPLC-SEC Analysis of Freeze-Thaw Assay of AF Purified Antibodies

| # of Freeze - Thaws | Peak # | min | mAU*min | mAU | % |
|---|---|---|---|---|---|
| 0 | 1 | 11.050 | 0.3134 | 1.199 | 0.26 |
| | 2 | 13.963 | 2.6242 | 2.278 | 2.18 |
| | 3 | 16.160 | 117.2764 | 132.181 | 97.56 |
| | Total: | | 120.2140 | 135.658 | 100.00 |
| 1 | 1 | 11.050 | 0.3102 | 1.178 | 0.26 |
| | 2 | 13.973 | 2.6683 | 2.295 | 2.22 |
| | 3 | 16.163 | 117.1783 | 131.670 | 97.52 |
| | Total: | | 120.1567 | 135.143 | 100.00 |
| 2 | 1 | 11.047 | 0.3140 | 1.208 | 0.26 |
| | 2 | 13.960 | 2.7041 | 2.287 | 2.26 |
| | 3 | 16.163 | 116.6066 | 131.513 | 97.48 |
| | Total: | | 119.6248 | 135.008 | 100.00 |
| 3 | 1 | 11.050 | 0.3583 | 1.243 | 0.30 |
| | 2 | 13.967 | 3.0928 | 2.338 | 2.59 |
| | 3 | 16.163 | 116.0707 | 131.455 | 97.11 |
| | Total: | | 119.5217 | 135.036 | 100.00 |

AF Coat Stock II was aliquoted and the stability of AF anti-FkpA antibodies was assessed over multiple freeze/thaw events.

*Sandwich ELISA Comparison of AF Coat Stock I and II*

AF Coat Stock I and II were diluted to 3 μg/mL in carbonate buffer, pH 9.6 and coated directly on a Costar 96-well plate (catalog #9018) and incubated overnight at 2-8 °C. Plates were washed with ELISA wash buffer (Phosphate Buffered Saline (PBS)/0.05% Polysorbate 20), blocked with assay diluent (0.15M Sodium chloride [NaCl]/0.1M Sodium phosphate [NaPO4]/0.1% fish gelatin/0.05% Polysorbate 20/0.05% Proclin 300) for 1-2 hours at room temperature with agitation and then washed again with ELISA wash buffer. Standard curves (100-1.56 ng/mL0 and blanks were added to the plates and incubated at room temperature for two hours with agitation. Plates were washed with ELISA wash buffer and AF HRP Conjugate Stock I was added to the plates at a 1:6000 dilution with assay diluent and incubated at room temperature for two hours with agitation. Plates were washed with ELISA wash buffer and the SureBlue Reserve™ TMB Microwell Peroxidase Substrate (Kirkegaard & Perry Labs [KPL], catalog # 53-00-00) was added to the plate and color was allowed to develop for approximately 20 minutes at room temperature. The reaction was stopped with 0.6N sulfuric acid. The OD's were read at a wavelength of 450 nm and data was analyzed using Softmax Pro data reduction software. Standard curves generated from AF Coat Stock I and II were nearly superimposable. Coat concentration, detection antibody dilution, and incubation times were screened and optimized. Coat concentration of 3 ug/mL and dilution of AF HRP Conjugate at 1/6000 were determined to be accurate and robust. Coat incubation times were robust up to 3 days.

*Purification and Analysis of Antibodies from Subsequent Bleeds*

Subsequent bleeds of rabbits A, B, and C were pooled on the basis of whether hemolysis of red blood cells had occurred, with lysed and non-lysed materials being purified separately. Both pools were purified by 60% ASP, followed by affinity chromatography and SEC as previously described. Based on SEC-HPLC data, there are no significant differences between the lysed pool (designated Lot B) and non-lysed pool (designated Lot C). Fractions 35-38 most closely resemble AF Coat Stock II (designated Lot A) material so Lot B fractions 35-38 were pooled together (Table 22).

Table 22. HPLC-SEC comparison of Lots A and B

| Fractions | Peak # | min | mAU*min | mAU | % |
|---|---|---|---|---|---|
| Lot B Fr. 34-38 | 1 | 11.080 | 1.7297 | 2.458 | 0.69 |
| | 2 | 14.767 | 7.0641 | 4.538 | 2.82 |
| | 3 | 16.277 | 242.0951 | 246.183 | 96.49 |
| | Total: | | 250.8889 | 253.178 | 100.00 |

| Fractions | Peak # | min | mAU*min | mAU | % |
|---|---|---|---|---|---|
| Lot B Fr. 35-38 | 1 | 11.080 | 1.3904 | 1.896 | 0.50 |
| | 2 | 14.867 | 5.2068 | 3.500 | 1.89 |
| | 3 | 16.280 | 269.3863 | 275.811 | 97.61 |
| | Total: | | 275.9836 | 281.206 | 100.00 |

| Fractions | Peak # | min | mAU*min | mAU | % |
|---|---|---|---|---|---|
| Lot A | 1 | 11.050 | 0.3134 | 1.199 | 0.26 |
| | 2 | 13.963 | 2.6242 | 2.278 | 2.18 |
| | 3 | 16.160 | 117.2764 | 134.181 | 97.56 |
| | Total: | | 120.2140 | 135.658 | 100.00 |

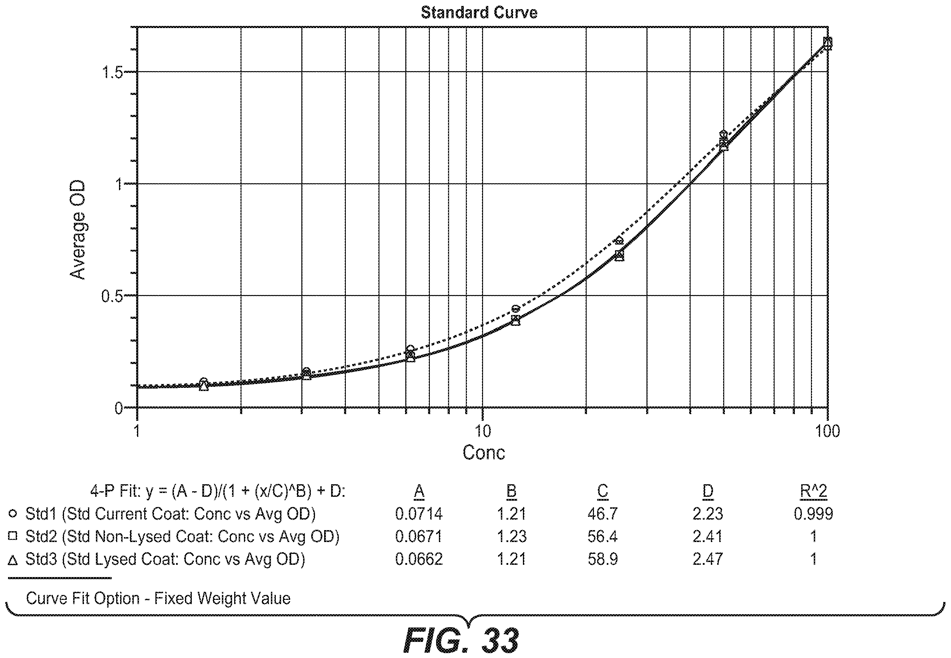

Lots A, B, and C were compared as the Coat antibody in a sandwich ELISA. Coat antibodies were diluted to 3 µg/mL. AF HRP Conjugate Stock I was used as the detection antibody at a dilution of 1:6000. The results of the three antibodies were comparable (FIG. 33). The concentration of Lot B was determined by UV spectrophotometry to be 2.13 mg/mL.

1 mg of Lot B antibodies were conjugated to HRP using a Pierce Kit EZ-Link Peroxidase (cat#31489) per manufacturer's instructions. Lot B HRP conjugated antibodies were further diluted 1/10 and was designated Lot B HRP Conjugate Stock I. Lot B was subaliquoted and was designated Lot B Coat Stock I.

*Assay Robustness*

A design of experiments was performed to screen assay conditions and demonstrate robustness. Assay parameters, such as coat concentration, conjugated dilutions, and TMB substrate incubation times, were varied. Minor variations in these parameters did not affect assay outcome. Optimal conditions are shown in Table 23.

Table 23.

| Y154 Parameters | |
|---|---|
| Standard Curve range | 1.56-100 ng/mL |
| AF Coat Stock II | 3ug/mL |
| Conjugate Stock I | 1/20,000 |
| Substrate Incubation Time | 20 min |

**Example 4. Stability of Ultrapure FkpA**

*Stability of ultrapure FkpA stored at -70 °C*

To test the stability of ultrapure FkpA as assay controls, the Standard Stock I, described in Example 2, was diluted in assay diluent to 3 (low), 15 (mid), 75 (high) ng/mL, aliquoted and stored at -70 °C. The concentration of one low, mid, and high aliquot was measured by ELISA using a standard curve prior to freezing. The stability of low, mid, and high ultrapure FkpA controls was monitored for a total of 18 days by thawing an aliquot and determining the concentration of FkpA. Low controls were unstable when stored at -70 °C. Mid controls showed similar instability, however, high controls appeared to be stable when stored at -70 °C.

*Stability of ultrapure FkpA stored at 2-8 °C compared to -70 °C*

A second test was performed on a new set of low and mid controls with half stored at -70 °C and the other half at 2-8 °C for a total of 8 days. Concentrations were not measured prior to freezing. As in previous experiment, the concentrations of frozen controls started dropping 30% from the calculated target concentration at Day 1. Concentrations of controls stored at 2-8 °C dropped at a slower rate than controls stored at -70 °C, but the drop was still considered to be outside of the acceptable range (FIG. 34).

*Stability of ultrapure FkpA prepared in varying amounts of polysorbate*

Standard Stock I was diluted to 3 (low), 15 (mid), or 75 (high) ng/mL in varying amounts of polysorbate in assay diluent for use in assay control. Assay diluent contains 0.05% polysorbate, so polysorbate concentrations of 0.1, 0.25, 0.5, and 1% were tested to see if it improved stability of controls frozen at -70 °C. The various solutions of FkpA were tested prior to freezing (T=0) and monitored for a total of 15 days as described above. For all samples diluted to 3 ng/mL, only samples containing 0.5% polysorbate appear to have a less than 20% drop in concentration when compared to T=0 for all 15 days. Greater variability was observed for the other 3 ng/mL conditions (Table 24). For 15 ng/mL solution, no conditions had concentrations that were greater than 30% below target concentration. The concentration of containing 0.5% or 0.25% polysorbate stayed within 20% of target for all 15 days (Table 24). 0.5% polysorbate yielded the best recovery of all controls tested. The percent difference (%Diff) from both target concentration and from Day 0 is between 1-13% and %CV is 1-10%. Controls diluted in 0.10% polysorbate did have a %Diff less than 20%, but there was more variability for low controls with %CV of 20%. Overall, increased amount of polysorbate was not deemed adequate to prevent ultrapure FkpA instability.

Table 24. Comparison of different concentration of Polysorbate

|  | 0.05% Polysorbate | | | 0.10% Polysorbate | | | 0.25% Polysorbate | | |
|---|---|---|---|---|---|---|---|---|---|
|  | Low | Mid | High | Low | Mid | High | Low | Mid | High |
| Mean | 2.334 | 12.482 | 69.327 | 2.625 | 12.499 | 70.272 | 2.403 | 13.256 | 70.126 |
| Std | 0.200 | 0.725 | 4.418 | 0.518 | 0.542 | 6.009 | 0.443 | 0.650 | 4.723 |
| %CV | 9% | 6% | 6% | 20% | 4% | 9% | 18% | 5% | 7% |
| %Diff From T=0 (Fresh) | -7% | 1% | -1% | -8% | -4% | -10% | -15% | 2% | -8% |
| %Diff from Target | -22% | -17% | -8% | -12% | -17% | -6% | -20% | -12% | -6% |
|  | 0.5% Polysorbate | | | 1.0% Polysorbate | | |  |  |  |
|  | Low | Mid | High | Low | Mid | High |  |  |  |
| Mean | 2.709 | 12.991 | 70.301 | 2.394 | 12.518 | 67.780 |  |  |  |
| Std | 0.082 | 0.730 | 5.665 | 0.343 | 0.399 | 8.412 |  |  |  |
| %CV | 3% | 6% | 8% | 14% | 3% | 12% |  |  |  |
| %Diff From T=0 (Fresh) | -2% | -1% | -10% | -11% | -1% | -9% |  |  |  |
| %Diff from Target | -10% | -13% | -6% | -20% | -17% | -10% |  |  |  |

*Stability of ultrapure FkpA prepared in fish gelatin, ApoMab, and buffer C*

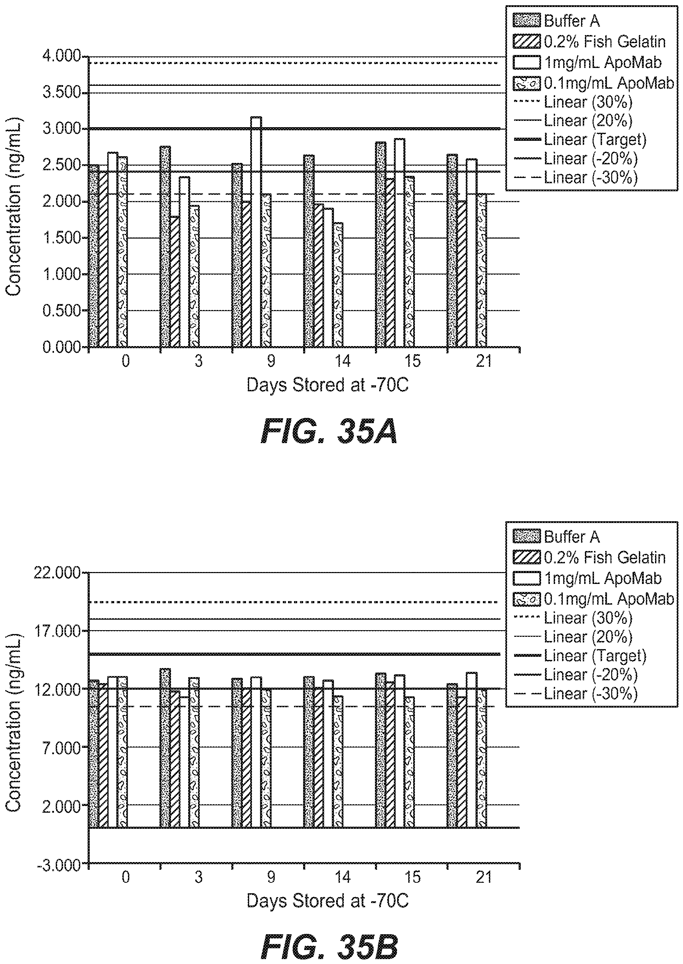

The stability of ultrapure FkpA at -70 °C when diluted to 3 (low), 15 (mid), or 75 (high) ng/mL in 0.2% fish gelatin, 1 mg/mL ApoMab (Ashkenazi, A, 2008, Nature Reviews Drug Discovery, 7: 1001-1012), or buffer C (20 mM histidine acetate, 240 mM sucrose, 0.02 % polysorbate 20, pH 5.5) was determined. (FIGs. 35A and 35B). The results show the stability of FkpA in several different compositions and that the controls remain stable at -70 °C when stored in buffer C in all cases. As an example, the robustness of control recovery in buffer C was demonstrated by repeating this assay 16 times (Table 25). Variability was within acceptable ranges.

Table 25. ELISA for detection of FkpA

|  | Low Control Recovery (ng/mL) | Mid Control Recovery (ng/mL) | High Control Recovery (ng/mL) |
|---|---|---|---|
| Mean | 2.897 | 13.584 | 74.535 |
| STDEV | 0.224 | 0.441 | 3.131 |
| CV | 8% | 3% | 4% |
| N | 16 | 16 | 16 |

**Example 5. Comparison of Ultrapure FkpA to commercial FkpA reagents**

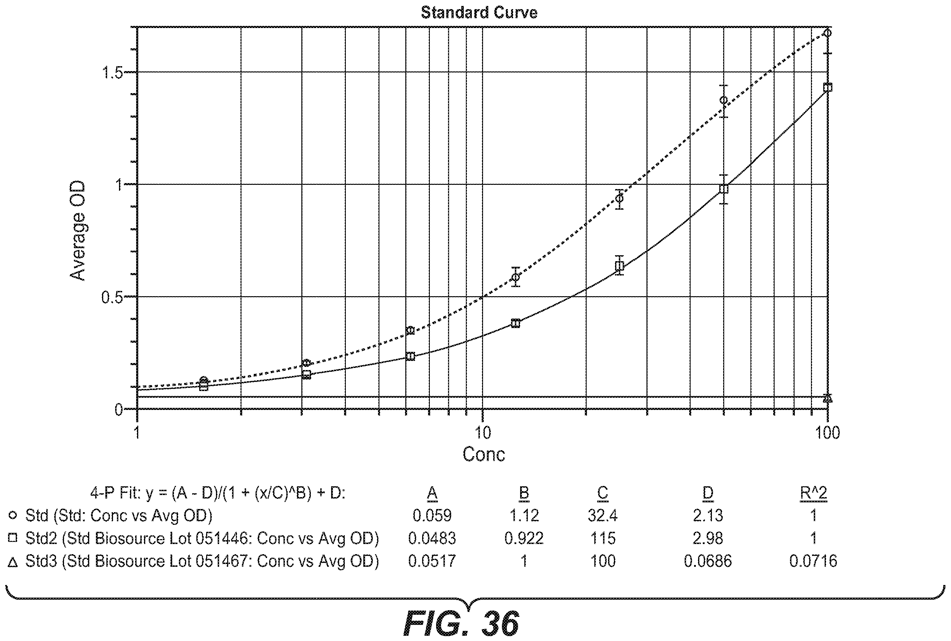

The ability of the sandwich ELISA assay described in Example 3 to detect different FkpA standards was determined. The various standards included Standard Stock I (described in Example 2), BioSource FkpA standard catalog number MBS 1037402, and BioSource FkpA standard catalog number MBS1182120. The standards used were 1.56 to 100 ng/mL. The BioSource FkpA standard catalog number MBS1037402 and MBS1182120 produced a standard curve with a lower signal than Standard Stock I. (FIG. 36).

Example 6. Generation of anti-IL13/anti-IL17 IgG4 Bispecific Antibody

Technology to generate human IgG1 bispecific antibodies with two different light chains in *E. coli* (Yu et al., 2011, Sci Transl Med 3, 84ra44). The method utilizes knobs-into-holes technology (Ridgway et al., 1996, Protein Eng. 9, 617-621; Atwell et al., 1997, J Mol Biol 270, 26-35) to promote hetero-dimerization of immunoglobulin heavy chains. To enable the use of two different light chains without light chain mispairing, each arm was cultured as a hemimer or half antibody in separate *E. coli* cells. This approach was used to generate the anti-IL13/anti-IL17 bispecific antibody by subcloning the anti-IL17 and anti-IL13 parental antibodies into vectors allowing the expression of the anti-IL17 arm as a human IgG4 hole and of the anti-IL13 arm as a human IgG4 knob. The sequence of the IgG4 knob heavy chain constant region is shown in SEQ ID NO:15 or 16 and the sequence of the IgG4 hole heavy chain constant region is shown in SEQ ID NO:26 or 27.

For initial antibody expression, *E. coli* strain 64B4 was used, that expressed only endogenous FkpA. During the initial assessment, we found that the anti-IL-17 half-antibody formed precipitates at pH higher than 7. The half-antibody was captured and then eluted from a protein A column at low pH and the eluate was adjusted to pH 8.5 to carry out the assembly of bispecific antibody in a redox reaction. About 20% of the anti-IL-17 half antibody eluate was lost to precipitation after pH adjustment. The decrease in available anti-IL-17 half antibody to pair with anti-IL-13 half antibody led to an imbalance between the ratio of the two half antibodies and reduced the yield of anti-IL-13/IL-17 bispecific. Subsequently, the half antibodies were produced in a different *E. coli* strain that overexpress exogenous FkpA, DsbA and DsbC. E. *coli* strain 67A6 (genotype: W3110 *ΔfhuA ΔphoA ilvG2096 (IlvG+; Valr) Δprc spr43H1 ΔmanA lacIQ ΔompT ΔmenE742 degPS210A)* was transformed with TIR (Translation initiation region) 2,2 single plasmids that encode the LC and HC of the anti-IL13 or anti-IL17 half Ab and the chaperones FkpA, DsbA and DsbC. On both plasmids, the expression of FkpA was under the control of a phoA promoter and the expression of DsbA and DsbC were under the control of a tacII promoter in a polycistronic fashion. Both LC and HC were under the control of independent phoA promoters. The plasmids were used to transform 67A6. The FkpA expressed contains its native signal peptide.

Half-antibodies anti-IL13.knob and anti-IL17.hole where each grown in cultures of 67A6 *E. coli* cells descried above. Large scale cultures for anti-IL13.knob were maintained at pH 6.7 ± 0.2 and large scale cultures of anti-IL17.hole were maintained at pH 7.0 ± 0.2. Cultures were grown at 34 °C at 220 rpm until the OD of the culture reached 150. Cultures were then shifted to 25 °C 160 rpm. The total fermentation was 72 hours.

Whole broths for each half antibody were separately homogenized via microfluidics. Each homogenate pool was then diluted with water and conditioned with PEI and incubated at room temperature. This conditioned homogenate pool was subsequently centrifuged to separate the solids followed by filtration of the half-antibody containing centrate through a series of filters.

The titer of each half antibody was about 10 to 20 fold higher than the titer produced by *E. coli* strain 64B4 without exogenous expression of FkpA, DsbA and DsbC (data not shown). Thus, exogenous expression of FkpA, DsbA and DsbC was adopted for preparing the anti-IL13/IL17 antibody.

Each half antibody was then separately captured on an affinity chromatography column using MabSelect SuRe™

resin (GE Healthcare Life Sciences). After column equilibration, the centrate was loaded onto the column and washed with equilibration buffer and a second wash buffer. The half antibodies were eluted from the column under acidic conditions (pH 2.8).

Assembly of anti-IL13/anti-IL17 antibody

The affinity-purified anti-IL13 and anti-IL17 half antibodies were combined in a 1:1 mass ratio followed by the titration to pH 8.5 . 100x excess molar ratio of L-glutathione, reduced (GSH) with respect to the theoretical maximum amount of bispecific antibody expected from the assembly process was added to the mixture and the temperature was adjusted from room temperature to 35°C. The two half antibodies assembled *in vitro* in the redox reaction to form the anti-IL13/anti-IL17 bispecific antibody. The redox reaction continued for 12 - 24 hrs at 35°C. The assembly reaction was quenched by adjusting the pH to 6.5 and the pool was diluted with water before the being subjected to the purification process described below.

Following the initial MabSelect SuRe™ purification and assembly, several sequential steps of purification of the bispecific antibody were performed. The assembled antibody was first purified on a mixed-mode anion exchange chromatography step operated in a bind-and-elute mode using CAPTO™ ADHERE resin (GE Healthcare Life Sciences). After column equilibration (50 mM acetate, pH 6.5), the adjusted assembly pool was loaded onto the column, washed with Equilibration buffer, and then washed with second wash buffer (200 mM Acetate, pH 6.5). The bispecific antibody was eluted by dropping the pH of the buffer (25 mM Acetate, pH 5.2).

Following CAPTO™ ADHEREchromatography the concentration of bispecific antibody product increased from about 80-85% to ≥ 92% and the high molecular weight species concentration decreased from about 12-15% to about ≤4% as measured by SEC-HPLC. In addition ECP in the preparation was reduced by about 2 logs, DsbA was decreased by about 1 log and FkpA decreased by 0.75 logs.

The pool was further purified over an anion exchange chromatography step operated in the bind-and-elute mode using Q SEPHAROSE® Fast Flow resin (GE Healthcare Life Sciences). Capto™ adhere eluate pool adjusted to pH 8.5 was loaded onto the equilibrated Q Sepharose® Fast Flow column and washed with Equilibration buffer (50 mM Tris, pH 8.5). The bispecific antibody was eluted from the column by increasing the conductivity of the buffer (50 mM Tris, 100 mM Sodium Acetate, pH 8.5).

Following QSFF chromatography the concentration of bispecific antibody product increased from about ≥ 92% to ≥ 94% and the high molecular weight species concentration decreased from ≤4% to about ≤2.5%, and the amount of very high molecular weight species could not be detected as measured by SEC-HPLC. In addition DsbC in the preparation was decreased by about 4 fold and FkpA decreased by 4-5 fold.

The QSFF eluate pool was adjusted to pH 5.5 and further purified on a hydrophobic interaction chromatography column using PHENYL SEPHAROSE® 6 Fast Flow (high sub) resin (GE Healthcare Life Sciences) and operated in the flow-through mode. The column was equilibrated (170 mM acetate, pH 5.5) and the adjusted QSFF pool was loaded onto the column. The anti-IL13/anti-IL17 bispecific antibody flowed through and the column was subsequently washed with Equilibration buffer. Product pool collection was initiated and terminated based on $OD_{280}$ with pooling occurring between 0.5 to 1 OD or a maximum of 10 CV of column wash volume. The pool was then concentrated and buffer exchanged using ultrafiltration/diafiltration (UF/DF).

A second run was performed with similar results.

Example 7. Detection of impurities in preparation of anti-IL13/anti-IL17 antibodies

Two lots of anti-IL13/anti-IL17 bispecific antibody were analyzed for the presence of impurities including non-paired half antibodies, homodimers, low molecular weight species (LMWS), high molecular weight species (HMWS), very high molecular weight species (vHMWS), acidic variants, basic variants, FkpA, DsbA, DsbC, ECP, leached protein A, *E. coli* DNA, and endotoxin. Product quality is presented in Table 26.

Table 26. Product quality

| Product Quality Attribute | Assay | Product Quality Targets | Run 1 | Run 2 |
|---|---|---|---|---|
| Bispecific (%) | HIC-HPLC | ≥90% | 97.2 | 97.3 |
| aIL17 species (%) | | ≤5% | 1.3 | 1.0 |
| aIL13 ½ mAb (%) | | ≤5% | 1.1 | 1.2 |
| aIL13 homodimer (%) | | ≤5% | 0.5 | 0.5 |

(continued)

| Product Quality Attribute | Assay | Product Quality Targets | Run 1 | Run 2 |
|---|---|---|---|---|
| Main (%) | NR CE-SDS | ≥85% | 94.0 | 95.5 |
| LMW (%) | | | 3.8 | 3.7 |
| LMW (%) | SEC-HPLC | | <LOQ | <LOQ |
| Main (%) | | ≥95% | 98.5 | 98.8 |
| HMWS (%) | | ≤5% | 1.5 | 1.2 |
| vHMWS (%) | | <LOQ | <LOQ | <LOQ |
| Acidic (%) | icIEF | | 21.0 | 21.0 |
| Main (%) | | | 75.8 | 76.4 |
| Basic (%) | | | 3.2 | 2.6 |
| ECP (ppm) | ELISA | <100 ppm | 19 | 20 |
| *E. coli* chaperones (ppm) | ELISA or LC-M/MS | ≤ 10 ppm (each) | DsbA=0.5 DsbC=0.7 FkpA=11 (LC-MS/MS) | DsbA=0.4 DsbC=0.2 FkpA=10 (LC-MS/MS) |
| Leached protein A (ppm) | ELISA | ≤50 | <2 ppm | <2 ppm |
| *E. coli* DNA (ppb) | qPCR | ≤8.3 | <0.1 ppb | <0.1 ppb |
| Endotoxin (EU/mL) | LAL | ≤4 | 0.3 | 0.2 |

Detection of FkpA in Purified anti-IL13/anti-IL17 bispecific antibodies

The concentration of FkpA was determined by ELISA using the antibodies against ultrapure FkpA as well as ultrapure FkpA for a standard and control. Samples were also analyzed for the presence of FkpA by LC-MS/MS (liquid chromatography-tandem mass spectrometry) after each production stage for the anti-IL13/anti-IL17 bispecific antibody.
Detection FkpA by ELISA and LC-MS/MS is described below. Results are shown in Table 27.

Table 27 FkpA Concentration in anti-IL13/anti-IL17 Samples

| Process Step | Run 1 | | | Run 2 | | |
|---|---|---|---|---|---|---|
| | LC-MS/MS (ppm) | ELISA (ppm) | Protein Conc. (mg/mL) | LC-MS/MS (ppm) | ELISA (ppm) | Protein Conc. (mg/mL) |
| αIL13 Centrate | not tested | 1449419 | 1.71 | not tested | 1202344 | 1.90 |
| αIL17 Centrate | not tested | 2588278 | 0.96 | not tested | 2483876 | 0.97 |
| αIL13 MabSelect SuRe™ | 3380 | 2859 | 16.7 | 3840 | 2677 | 14.9 |
| αIL17 MabSelect SuRe™ | 2330 | 1990 | 19.6 | 2510 | 1870 | 19.3 |
| Assembly | 3070 | 2447 | 13.6 | 3260 | 2294 | 13.0 |
| Capto™ adhere | 820 | 447 | 5.1 | 744 | 593 | 7.3 |
| QSFF | 243 | 116 | 9.9 | 160 | 117 | 9.6 |
| Phenyl HS (high sub) | 13 | 6 | 6.2 | 10 | 7 | 6.0 |
| UF/DF | 11 | 6 | 191.7 | 11 | 6 | 178.7 |

Detection of FkpA by ELISA

Polyclonal sera directed against ultrapure FkpA was diluted to 3 μg/mL in carbonate buffer, pH 9.6 and coated directly on a Costar 96-well plate (catalog #9018) and incubated overnight at 2-8 °C. Plates were washed with ELISA wash buffer (Phosphate Buffered Saline (PBS)/0.05% Polysorbate 20), blocked with assay diluent (0.15M Sodium chloride/0.1M Sodium phosphate/0.1% fish gelatin/0.05% Polysorbate 20/0.05% Proclin 300) for 1-2 hours at room temperature with agitation and then washed again with ELISA wash buffer. Standard curves of ultrapure FkpA (100-1.56 ng/mL) and blanks were added to the plates and incubated at room temperature for two hours with agitation. Plates were washed with ELISA wash buffer and affinity purified anti-FkpA HRP Conjugate Stock was added to the plates at a 1:6000 dilution with assay diluent and incubated at room temperature for two hours with agitation. Plates were washed with ELISA wash buffer and the SureBlue Reserve™ TMB Microwell Peroxidase Substrate (Kirkegaard & Perry Labs, catalog # 53-00-00) was added to the plate and color was allowed to develop for approximately 20 minutes at room temperature. The reaction was stopped with 0.6N Sulfuric acid. The OD's were read at a wavelength of 450 nm and data was analyzed using Softmax Pro data reduction software.

Detection of FkpA by LC-MS/MS

FkpA standards of 1, 5, 10, 25, and 50 ppm FkpA in 0.5 mg Apomab. 0.5 mg of each antibody sample and the standards were diluted with purified water to a final concentration of 5 mg/mL. 400 μL Denaturation Buffer (7.2 M guanidine hydrochloride, 0.3 M sodium acetate, pH 5.0) and 10 μL 0.5 M Bond-Breaker Tris(2-carboxyethyl)phosphine (TCEP), neutral pH (Pierce) were added to 100 μL of diluted sample, and samples were incubated for 15 minutes are 37 °C.

A NAP-5 desalting column was equilibrated with ~10 mL of desalting buffer (1 mM sodium acetate, 0.5 mM TCEP, pH 5.0). The samples were loaded and eluted with 800 μL of desalting buffer. 100 μL of 0.5 M MOPS (3-(N-Morpholino)propanesulfonic acid, 4-Morpholinepropane-sulfonic acid) was added to each sample.

20 μL of 0.5 mg/mL Trypsin was added to each sample and samples were incubated for 60 min at 37 °C. The trypsin was inactivated by adding 30 μL of 10% triflouroacetic acid (TFA).

100 μL of each digested sample was injected for LC-MS/MS analysis by Multiple Reaction Monitoring (MRM). The LC was performed on a Waters Acquity H-Class Bio UPLC with a 2.1 mm x 150 mm, 1.7 μm Waters CSH C18 UPLC Column. The column temperature was 60 °C, flow rate 0.3 mL/min, and with buffer A (water with 0.1% Formic Acid) and buffer B (acetonitrile with 0.1% Formic Acid) used as a gradient of 0-40% B for 45 min.

The MS/MS was performed with a Sciex 6500Qtrap Mass Spectrometer. MRM monitors transition of +2 precursor ion of the FkpA peptide SAYALGASLGR (SEQ ID NO: 45) peptide to y6 fragment ion. The collision energy was 28.1 V, declustering potential=70 V, and dwell time was 100 ms. Q1 Mass=533.3 for precursor, Q3 Mass=560.3 for y6 fragment ion. The FkpA standard curve was used to determine the FkpA concentration in the anti-IL13/anti-IL17 samples.

Example 8. 5-Column Purification of anti-IL13/anti-IL17 Bispecific Antibodies

In order to further reduce the concentration of FkpA in anti-IL13/anti-IL17 antibody, an additional step of purification was added to the 4-column procedure described above. A POROS® 50HS cation exchange chromatography was evaluated. A sample of the PHENYL SEPHAROSE® 6 Fast Flow (high sub) eluate pool adjusted to pH 5.0 was applied to a POROS® HS 50 (Applied Biosystems™) cation exchange column and purified in the bind-and-elute mode. The bispecific antibody was eluted using a linear salt gradient (0 to 500 mM acetate, pH 5.8 over 22 CV). Samples were then assayed for the removal of impurities. Results are presented in Table 28.

Table 28. Results of preliminary POROS® HS 50 fifth chromatography step

| Load Material | Pool | Load density (g/L) | Recovery (%) | ELISA | | | | LC-MS/MS | |
|---|---|---|---|---|---|---|---|---|---|
| | | | | FkpA (ppm) | ECP (ppm) | DsbA (ppm) | DsbC (ppm) | FkpA (ppm) | |
| Run 1 | Load | 20 | 75 | 7 | 24 | <LOQ | <LOQ | 13 | |
| | POROS® HS 50 | | | 1 | 9 | n.t. | n.t. | 1 | |
| Run 2 | Load | 20 | 79 | 6 | 22 | <LOQ | <LOQ | 10 | |
| | POROS® HS 50 | | | | 2 | 15 | n.t. | n.t. | 3 |

| Load Material | Pool | Pool vol. (CV) | SEC | | RPLC | HIC-HPLC | NR CE-SDS | icIEF | | |
|---|---|---|---|---|---|---|---|---|---|---|
| | | | HMWS (%) | Main Peak (%) | BsAb (%) | BsAb (%) | Purit y (%) | Acidic (%) | Main (%) | Basic (%) |
| Run 1 | Load | 2.5 | 1.6 | 96.6 | 95.8 | 97.0 | 95.0 | 2 1.4 | 74.9 | 3.8 |
| | POROS® HS 50 | | 0.4 | 98.6 | 98.0 | 98.4 | 97.6 | 2 0.5 | 77.1 | 2.4 |
| Run 2 | Load | 2.5 | 0.8 | 98.2 | 96.7 | 97.6 | 95.5 | 2 1.3 | 76.0 | 2.7 |
| | POROS® HS 50 | | 0.7 | 98.1 | 97.1 | n.t. | n.t. | n. t. | n.t. | n.t. |
| n.t.: not tested BsAb = bispecific antibody | | | | | | | | | | |

EP 3 337 819 B1

Based on these results, the POROS® HS 50 column was incorporated into the purification scheme for anti-IL13/anti-IL17. The half-antibodies were purified by protein A affinity chromatography on a MabSelect SuRe™ column and assembled as described in Example 6. The assembled anti-IL13/anti-IL17 antibody was further purified by CAPTO™ ADHERE, Q SEPHAROSE® Fast Flow and PHENYL SEPHAROSE® 6 Fast Flow (high sub) chromatography steps as described in Example 6.

Prior to the UF/DF step, the anti-IL13/anti-IL17 bispecific antibody pool from the PHENYL SEPHAROSE® 6 Fast Flow column was purified on a POROS® HS 50 column operated in the bind-and-elute mode. The column was equilibrated (50 mM acetate, pH 5.5) and the diluted PHENYL SEPHAROSE® 6 Fast Flow (high sub) flow through pool, was loaded onto the column. The column was washed with Equilibration buffer followed by a second wash buffer (50 mM acetate, pH 5.8). The bispecific antibody was eluted from the column using a linear salt gradient from 10-100%B over 22 CV (A: water, B: 500 mM acetate, pH 5.8). Eluate collection was initiated and terminated based on $OD_{280}$ with pooling occurring between 0.5 to 1.

Subsequently, concentration and buffer exchange of the POROS® HS 50 pool was performed using ultrafiltration/diafiltration (UF/DF).

The process was repeated with four different fermentations of $\alpha$IL13 and $\alpha$IL17.

Samples of the final product as well as samples after different steps of the purification process were analyzed for product specific impurities. Results of analysis of high molecular weight species (HMWS) in the samples is presented in Table 29. Briefly, the assembled bispecific antibody starting material contained about 91-94% product and about 6.0-7.5% HMWS. Following Capto™ adhere mixed-mode chromatography, the sample contained about 95-98% product and about 2.2% HMWS showing reduction in the amount of HMWS impurities in the samples. Following anion exchange, HIC and cation exchange the sample contained 98-99.6% product and 0.2-1.5% HMWS.

Table 29. Analytical SEC-HPLC results

| Process Step | Run 1 | | Run 2 | | Run 3 | | Run 4 | |
|---|---|---|---|---|---|---|---|---|
| | Main peak (%) | HMWS (%) | Main peak (%) | HMWS (%) | Main peak (%) | HMWS (%) | Main peak (%) | HMWS (%) |
| Assembly | 91.8 | 7.5 | 93.2 | 6.1 | 93.6 | 5.8 | 93.3 | 3.4 |
| Capto™ adhere | 97.6 | 2.2 | 96.1 | 2.2 | 95.4 | 2.1 | 95.2 | 2.3 |
| QSFF | 98.7 | 1.1 | 97.2 | 1.4 | 96.6 | 1.5 | 96.4 | 1.9 |
| Phenyl HS | 99.3 | 0.5 | 99.2 | 0.7 | 97.7 | 0.8 | 97.3 | 1.7 |
| POROS® HS 50 | 99.6 | 0.2 | 99.6 | 0.3 | 98.4 | 0.3 | 98.0 | 1.5 |

Samples of the 5-column process pools were analyzed for the presence of FkpA by using the ELISA presented in Example 4 using the antibodies against ultrapure FkpA. As shown in Tables 30 and 31, the 5-column process was effective in removing FkpA from the bispecific antibody formulation.

Thus, the purification processes described herein substantially removed or reduced FkpA levels to no more than 6 ppm as analyzed by the ELISA assay in a sample of an exemplary bispecific antibody, i.e., anti-IL13/anti-IL17 bispecific antibody, which was assembled from half antibodies produced by *E. coli* cells over-expressing exogenous FkpA. As a comparison, the level of FkpA remains 7 ppm as determined by the same ELISA assay in a sample of anti-IL13/anti-IL17 bispecific antibodies produced by *E. coli* cells that express only endogenous FkpA (such as strain 64B4 without harboring plasmids expressing exogenous FkpA or other chaperone proteins) and purified by the ProPac™ HIC-10 column (data not shown).

Table 30. FkpA Concentrations in anti-IL13/anti-IL17 samples determined by ELISA

| Process Step | Run 1 | Run 2 | Run 3 | Run 4 |
|---|---|---|---|---|
| $\alpha$IL13 Centrate | 1248255 | 532393 | 1782250 | 1621115 |
| $\alpha$IL17 Centrate | 1923183 | 786910 | 2511409 | 2381039 |
| $\alpha$IL13 MabSelect SuRe™ | 2138 | 2064 | 2411 | 2214 |
| $\alpha$IL17 MabSelect SuRe™ | 1425 | 1608 | 2003 | 1780 |

(continued)

| Process Step | Run 1 | Run 2 | Run 3 | Run 4 |
|---|---|---|---|---|
| Assembly | 2173 | 2073 | 2376 | 2131 |
| Capto™ Adhere | 277 | 283 | 404 | 354 |
| QSFF | 109 | 100 | 166 | 109 |
| Phenyl HS | 2 | 3 | 6 | 4 |
| POROS® 50HS | 0.2 | 0.4 | 0.7 | 0.5 |
| UF/DF | <0.3 | 0.6 | 0.9 | 0.5 |

Table 31 Protein Concentrations (mg/mL) in anti-IL13/anti-IL17 samples

| Process Step | Run 1 | Run 2 | Run 3 | Run 4 |
|---|---|---|---|---|
| αIL13 Centrate | 1.2692 | 1.2200 | 1.2124 | 1.3013 |
| αIL17 Centrate | 0.8394 | 0.8485 | 0.8143 | 0.8099 |
| αIL13 MabSelect SuRe™ | 13.83 | 13.78 | 14.04 | 13.49 |
| αIL17 MabSelect SuRe™ | 18.33 | 16.88 | 17.13 | 16.63 |
| Assembly | 12.30 | 10.52 | 10.89 | 11.78 |
| Capto™ adhere | 5.77 | 8.44 | 6.27 | 6.29 |
| QSFF | 5.86 | 11.25 | 11.64 | 11.97 |
| Phenyl HS | 2.78 | 5.64 | 5.86 | 6.01 |
| POROS® HS 50 | 4.36 | 4.64 | 5.64 | 5.68 |
| UF/DF | 159.05 | 176.38 | 174.73 | 179.70 |

Example 9. Evaluation of mixed-mode anion exchange chromatography for purifying polypeptides produced in *E. coli* expressing FkpA exogenously

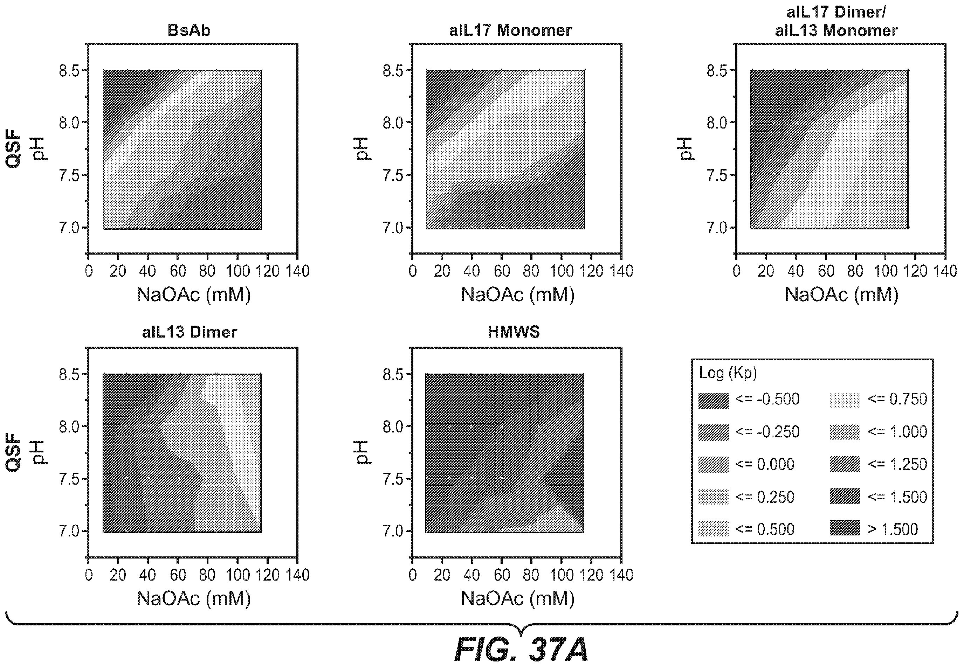

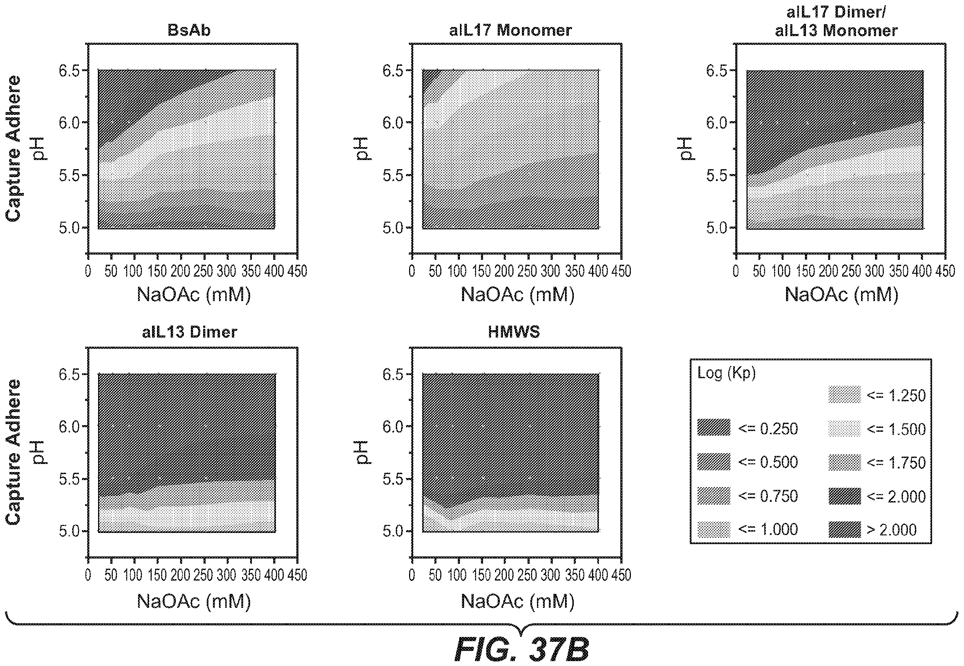

High throughput screening indicated enhanced resolution of product-related impurities on mixed-mode anion-exchange chromatography compared to traditional anion exchange chromatography (e.g., QSFF chromatography) (FIG.37). Little or no anti-IL17 monomer was separated by QSFF chromatography but improved separation of anti-IL13 monomer and anti-IL17 dimer species was observed with mixed-mode chromatography.

The use of mixed-mode chromatography in the purification of a bispecific antibody was evaluated. In one arm of the evaluation, half-antibodies that bound IL13 or IL17 were prepared separately in bacterial cultures. The half-antibodies that bound IL 13 or IL17 were then subject to affinity chromatography using protein A chromatography (Mab-Select SuRe™ chromatography). Partially purified antibodies were then assembled to form bispecific antibodies that bound IL13 and IL17 as described above. The assembled bispecific antibodies were subject to QSFF anion exchange chromatography and then Capto™ Adhere mixed-mode anion exchange chromatography (Train 1) or were subject Capto™ Adhere mixed-mode anion exchange chromatography and then QSFF anion exchange chromatography (Train 2). The results are presented in Table 32.

Table 32. Product Quality

| Step | A280 Yield (%) | | BsAb Yield (%) | | SEC-Total HMWS (%) | | RPLC-BsAb Purity (%) | |
|---|---|---|---|---|---|---|---|---|
| | Train 1 | Train 2 | Train 1 | Train 2 | Train 1 | Train 2 | Train 1 | Train 2 |
| αIL13 MSS | 90 | | 3.6 | | -- | | -- | |
| αIL17 MSS | 80 | | 9.1 | | | | -- | |
| Assembly | 100 | | 86 | | 7.8 | | 85.6 | |

(continued)

| Step | A280 Yield (%) | | BsAb Yield (%) | | SEC-Total HMWS (%) | | RPLC-BsAb Purity (%) | |
|---|---|---|---|---|---|---|---|---|
| | Train 1 | Train 2 | Train 1 | Train 2 | Train 1 | Train 2 | Train 1 | Train 2 |
| Column 2 | 71 | 72 | 76 | 80 | 1.4 | 1.6 | 92.8 | 94.6 |
| Column 3 | 77 | 97 | 80 | 98 | 0.6 | 0.7 | 96.7 | 96.2 |
| Overall | 39 | 50 | 53 | 67 | | | | |
| Step | CE-SDS BsAb Purity (%) | | iCIEF-Acidics (%) | | iCIEF-Main (%) | | iCIEF-Basics (%) | |
| | Train 1 | Train 2 | Train 1 | Train 2 | Train 1 | Train 2 | Train 1 | Train 2 |
| αIL13 MSS | -- | | -- | | -- | | -- | |
| αIL17 MSS | -- | | -- | | -- | | -- | |
| Assembly | 84.2 | | 36.6 | | 60.5 | | 2.9 | |
| Column 2 | 92.0 | 94.2 | 38.1 | 28.9 | 58.3 | 67.3 | 3.6 | 3.8 |
| Column 3 | 96.3 | 95.9 | 28.5 | 31.3 | 68.5 | 65.2 | 3.1 | 3.5 |

Subjecting the assembled bispecific antibodies to mixed-mode anion chromatography before anion exchange chromatography generally resulted in inproved product variant removal.

Clearance of process-related impurities is presented in Table 33.

Table 33. Process-related impurities

| Step | ECP (%) | | DsbA (ppm) | | DsbC (ppm) | |
|---|---|---|---|---|---|---|
| | Train 1 | Train 2 | Train 1 | Train 2 | Train 1 | Train 2 |
| αIL13 MSS | 1451 | | 39 | | 144 | |
| αIL17 MSS | 3060 | | 64 | | 350 | |
| Assembly | 972 | | 38 | | 156 | |
| Column 2 | 239 | 56 | 49 | 3 | 149 | 39 |
| Column 3 | 87 | 8 | 2 | 2 | 37 | 26 |

Improved impurity clearance was observed when mixed-mode preceded anion exchange chromatography, as shown by the lower amount of impurities in material purified using Train 2.

The bind and elute QSFF anion exchange conditions were as follows: The column was equilibrated with 50 mM Tris, pH 8.5. Assembly pool (Train 1) or Capto™ Adhere elution pool (Train 2) adjusted to pH 8.5 and ≤ 1.8 mS/cm was loaded onto the column to a load density of ≤ 20 g of protein per L of packed resin, washed with Equilibration buffer, and then washed with second wash buffer (50 mM Tris with 5 mM sodium acetate at pH 8.5). The bispecific antibody was eluted by increasing the conductivity of the buffer (50 mM Tris with 139 mM sodium acetate at pH 8.5). Pooling was initiated and terminated based on absorbance at 280 nm.

The bind and elute Capto™ Adhere mixed-mode anion exchange conditions were as follows: The column was equilibrated with 50 mM acetate, pH 6.5. QSFF elution pool (Train 1) or Assembly pool (Train 2) adjusted to pH 6.5 and ≤ 7.0 mS/cm was loaded onto the column to a load density of ≤ 24 g of protein per L of packed resin, washed with Equilibration buffer, and then washed with second wash buffer (200 mM Acetate, pH 6.5). The bispecific antibody was eluted by dropping the pH of the buffer (25 mM Acetate, pH 5.2). Pooling was initiated and terminated based on absorbance at 280 nm.

Example 10. Screening HIC resins for FkpA reduction

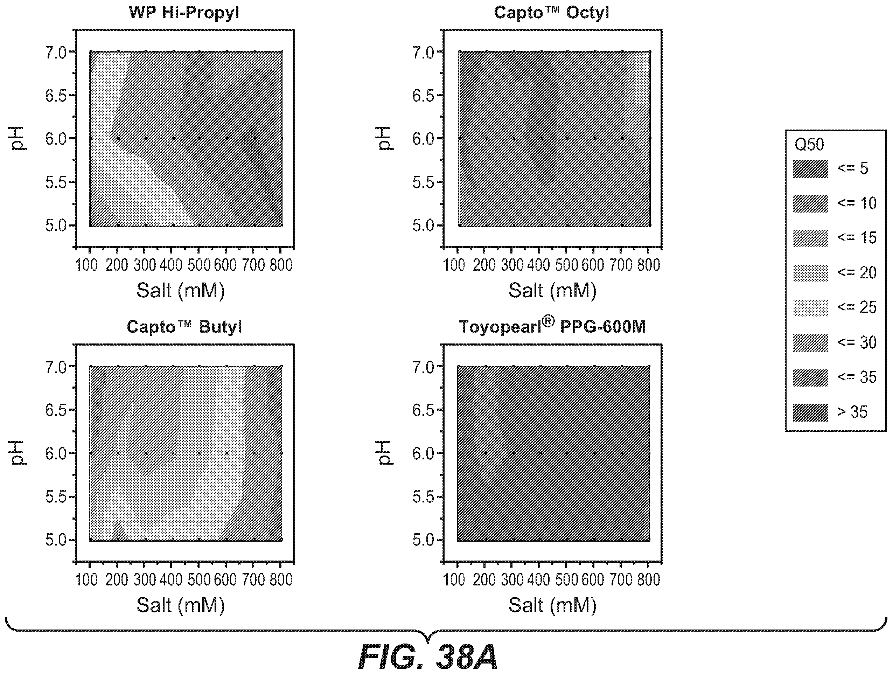

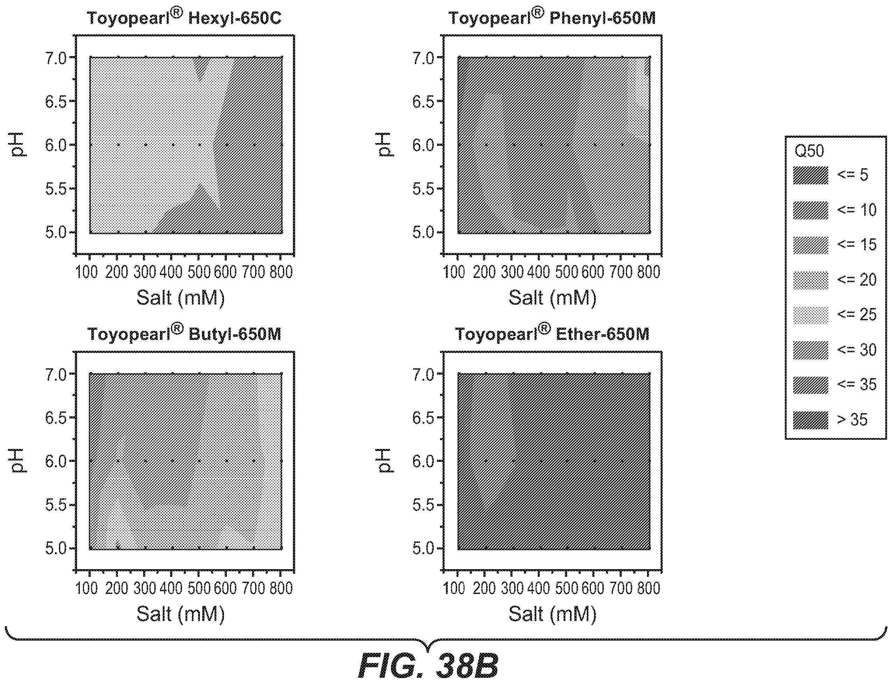

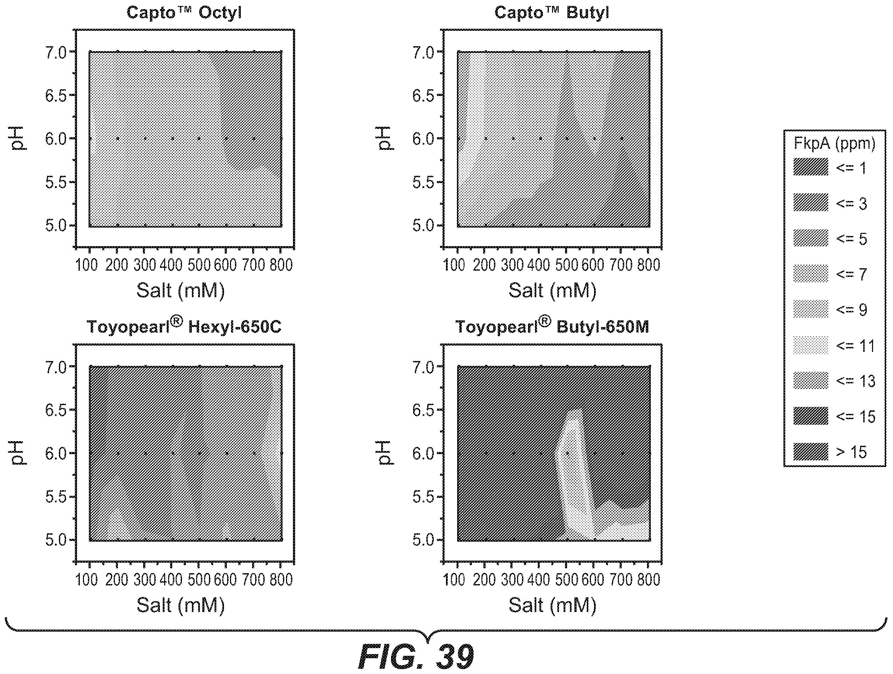

A number of HIC resins were screened for reduction of FkpA in polypeptide products such as bispecific antibody product. Resins included Bakerbond™ WP Hi-Propyl, Capto™ Octyl, Capto™ Butyl, Toyopearl® Hexyl-650C, Toyopearl® PPG-600M, Toyopearl® Phenyl-650M, Toyopearl® Butyl-650M, and Toyopearl® Ether-650M. Hexyl 650C produced the lowest FkpA levels in flowthrough mode, followed by Capto® Butyl (FIGs. 38 and 39). Robotic high throughput screening was performed to evaluate the batch binding of product and FkpA to these resins under varying salt type and concentration and pH conditions. Three different kosmotropic salts were examined to determine the effect of salt type on selectivity. The salts and concentration ranges tested were as follows: 100 - 800 mM sodium acetate, 100 - 400 mM sodium sulfate, 500 - 2000 mM sodium chloride. In addition, a pH range of 5.0 - 7.0 was evaluated. 96-well membrane-bottom plates were packed with approximately 50 $\mu$L of resin in each well and equilibrated at the appropriate binding condition (salt type, salt concentration, pH) for each experiment. Partially purified product pool containing FkpA (i.e., starting load material/feedstock) adjusted to the corresponding binding condition was applied to each well to target a load density of 50 $\mu$g of product per $\mu$L of resin. Following mixing and incubation to achieve equilibrium, the supernatant was collected and analyzed for relative product and FkpA content in order to assess the enrichment of product obtained relative to the starting load material/feedstock. Hexyl 650C produced the lowest FkpA levels in flowthrough mode, followed by Capto™Butyl.

Example 11. Process versions

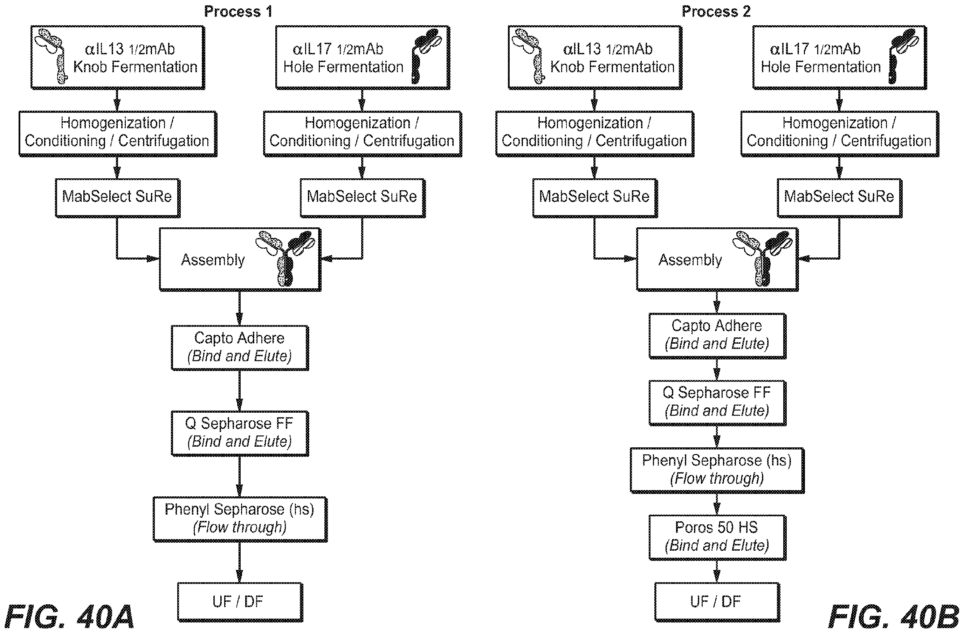

A number of process versions were evaluated. In a first example of a process, half antibodies were produced in *E. coli*. See FIG. 40A. *E. coli* whole cell broths for each half antibody were homogenized via microfluidics. Each homogenate pool was diluted with water and conditioned with PEI and incubated at room temperature. Conditioned homogenate pool was subsequently centrifuged to separate the solids followed by filtration of the half-antibody containing centrate through a series of filters. The half antibodies where then subject to affinity purification using a protein A chromatography column (MabSelect SuRe™). The bispecific antibodies where then assembled by combining the affinity purified half antibodies as described above. For this first process (Process 1), the assembled bispecific antibodies were subject to mixed-mode anion exchange chromatography (Capto™ Adhere) in bind and elute mode, then subject to anion exchange chromatography (Q Sepharose® Fast Flow) in bind and elute mode. The recovered fractions were then subjected to HIC (Phenyl Sepharose® 6 Fast Flow, high sub) in flow-through mode. Finally, the bispecific antibody was subject to ultrafiltration and diafiltration to formulate the bispecific antibody. In another example of a process, Process 2 (FIG. 40B), the assembled bispecific antibodies were prepared as described above, were subject to mixed-mode anion exchange chromatography (Capto™ Adhere), then subject to anion exchange chromatography (Q Sepharose® Fast Flow) in bind and elute mode. The recovered fractions were then subjected to HIC (Phenyl Sepharose® 6 Fast Flow, high sub) in flow-through mode and cation exchange chromatography (POROS® 50 HS) in bind and elute mode. Finally, the bispecific antibody was subject to ultrafiltration and diafiltration to formulate the bispecific antibody.

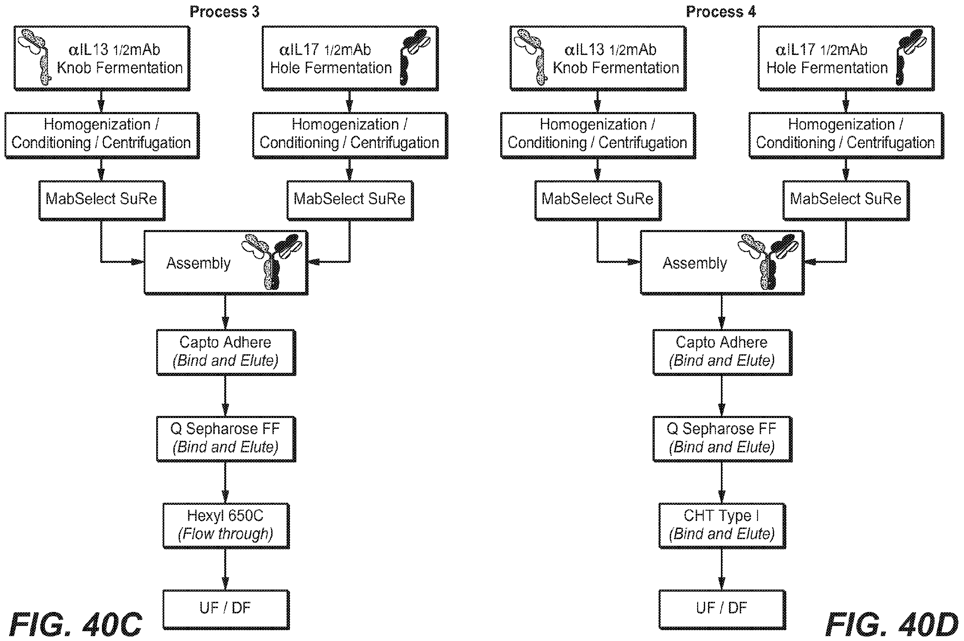

In a third process (Process 3, FIG. 40C), the bispecific antibodies were assembled as described above and were then subject to mixed-mode anion exchange chromatography (Capto™ Adhere), then subject to anion exchange chromatography (Q Sepharose® Fast Flow) in bind and elute mode, and HIC (Hexyl 650C) in flow-through mode . Finally, the bispecific antibody was subject to ultrafiltration and diafiltration to formulate the bispecific antibody.

In a fourth process (Process 4, FIG. 40D), the bispecific antibodies were assembled as described above and were then subject to mixed-mode anion exchange chromatography (Capto™ Adhere) in bind and elute mode, then subject to anion exchange chromatography (Q Sepharose® Fast Flow) in bind and elute mode, and Ceramic Hydroxyapatite Chromatography (CHT Type I, 80$\mu$m) in bind and elute mode.

Finally, for processes 1-3 the bispecific antibody was subject to ultrafiltration and diafiltration to formulate the bispecific antibody.

Each half antibody was then separately captured on an affinity chromatography column using MabSelect SuRe™ resin (GE Healthcare Life Sciences). After column equilibration, the centrate was loaded onto the column and washed with equilibration buffer and a second wash buffer. The half antibodies were eluted from the column under acidic conditions (pH 2.8).

Capto™ Adhere Process:

The bind and elute Capto™ Adhere mixed-mode anion exchange conditions were as follows: The column was equilibrated with 50 mM acetate, pH 6.5. Assembly pool adjusted to pH 6.5 and 6.5 mS/cm was loaded onto the column to a load density of $\leq$ 20 g of protein per L of packed resin, washed with Equilibration buffer, and then washed with second wash buffer (200 mM Acetate, pH 6.5). The bispecific antibody was eluted by dropping the pH of the buffer (25 mM Acetate, pH 5.2). Pooling was initiated and terminated based on absorbance at 280 nm.

Q Sepharose® FF Process:

The bind and elute QSFF anion exchange conditions were as follows: The column was equilibrated with 50 mM Tris, pH 8.5. Capto™ Adhere elution pool adjusted to pH 8.5 was loaded onto the column to a load density of $\leq$ 50

g of protein per L of packed resin and washed with Equilibration buffer. The bispecific antibody was eluted by increasing the conductivity of the buffer (50 mM Tris with 100 mM sodium acetate at pH 8.5). Pooling was initiated and terminated based on absorbance at 280 nm.

Phenyl Sepharose® Process:

The flow-through Phenyl Sepharose® 6 Fast Flow (high sub) HIC conditions were as follows: The column was equilibrated with 170 mM acetate, pH 5.5. Adjusted QSFF elution pool at pH 5.5 was loaded onto the column. The anti-IL13/anti-IL17 bispecific antibody flowed through and the column was subsequently washed with Equilibration buffer. Product pool collection was initiated and terminated based on absorbance at 280 nm.

POROS® HS 50 Process:

The bind and elute POROS® HS 50 cation exchange conditions were as follows: The column was equilibrated with 50 mM acetate, pH 5.5. Phenyl Sepharose® flow-through pool adjusted to 5.0 mS/cm was loaded onto the column to a load density of ≤ 20 g of protein per L of packed resin, washed with Equilibration buffer, and then washed with second wash buffer (50 mM acetate, pH 5.8). The bispecific antibody was eluted by increasing the conductivity in a linear gradient from 10-100%B in 22 column volumes (CV) (A: WFI, B: 500 mM acetate, pH 5.8). Pooling was initiated and terminated based on absorbance at 280 nm.

Hexyl-650C Process:

The flow-through Hexyl-650C HIC conditions were as follows: The column was equilibrated with 50 mM MOPS with 125 mM sodium acetate at pH 7.0. Adjusted QSFF elution pool at pH 7.0 was loaded onto the column. The anti-IL13/anti-IL17 bispecific antibody flowed through and the column was subsequently washed with Equilibration buffer. Product pool collection was initiated and terminated based on absorbance at 280 nm.

CHT Type I Process:

The bind and elute CHT Type I Ceramic Hydroxyapatite chromatography conditions were as follows: The column was equilibrated with 10 mM sodium phosphate, pH 6.8. QSFF elution pool conditioned to contain 10 mM sodium phosphate at pH 6.8 was loaded onto the column to a load density of ≤ 25 g of protein per L of packed resin and washed with Equilibration buffer. The bispecific antibody was eluted by increasing the conductivity in a linear gradient from 0-100%B in 20 column volumes (CV) (A: Equilibration buffer, B: 10 mM sodium phosphate containing 1 M sodium chloride at pH 6.8). Pooling was initiated and terminated based on absorbance at 280 nm.
Overall yield and product quality data in the final column pools for each of these exemplary processes (Process 1-4) are shown in the Table 34 below:

Table 34. Product Quality

| Attribute | Assay | Process 1 (N = 2) | Process 2 (N = 4) | Process 3 (N = 2) | Process 4 (N = 2) |
|---|---|---|---|---|---|
| Overall Yield | -- | 44 - 45 | 31 - 34 | 44 – 46 | 44 - 48 |
| HMWS (%) | SEC | 0.6 – 0.8 | 0.2 - 0.4 | 0.6, 0.6 | 0.4 - 0.5 |
| Main (%) | | 99.0 – 99.3 | 99.5 - 99.6 | 99.4 – 99.5 | 99.0 - 99.2 |
| Acidics (%) | iCIEF | 21.3 – 21.4 | 17.0 - 23.0 | 24.2* | n.a. |
| Main (%) | | 74.9 – 76.0 | 74.1 - 80.5 | 72.1* | n.a. |
| Basics (%) | | 2.7 – 3.8 | 2.5 - 2.9 | 3.7* | n.a. |
| Purity – 150 kDa species (%) | CE-SDS (NR) | 95.0 – 95.5 | 97.2 - 97.6 | 95.2 – 95.3 | n.a. |
| Purity - BsAb (%) | RPLC | 95.3 – 95.6 | 96.7 - 97.8 | 96.5 – 96.9 | 95.7 - 96.1 |
| Purity - BsAb (%) | HIC-HPLC | 97.0 – 97.6 | 97.3 - 97.9 | 97.5* | n.a. |
| ECP (ppm) | ELISAs | 20 - 21 | 7 - 17 | 18 - 20 | 6, 6 |
| FkpA (ppm) | | 6 - 8 | 0.2 - 0.7 | 1, 1 | 1, 1 |
| DsbA (ppm) | | 0.4 | < 0.6 | 0.4 – 0.5 | < 0.7 |
| DsbC (ppm) | | < 0.4 - 0.4 | < 0.6 | 0.4 – 0.7 | < 1.0 - 1.4 |

*: N=1 run analyzed

n.a.: Data not available

Example 12. Purification of αA/αB

A bispecific antibody that binds two antigens, A and B, was assembled and purified. Anti-A and anti-B half antibodies were produced in *E. coli* overexpressing FkpA, DsbA and DsbC as described above for the anti-IL13/anti-IL17 bispecific antibody (see Example 6). The anti-A half antibody included the "knob" amino acid substitutions in the Fc region and the anti-B half antibody included the "hole" amino acid substitutions.

Whole broths for each half antibody were separately homogenized via microfluidics. Each homogenate pool was then diluted with water and conditioned with PEI and incubated at room temperature. This conditioned homogenate pool was subsequently centrifuged to separate the solids followed by filtration of the half-antibody containing centrate through a series of filters.

Each half antibody was then separately captured on an affinity chromatography column using MabSelect SuRe™ resin (GE Healthcare Life Sciences). After column equilibration, the centrate was loaded onto the column and washed with equilibration buffer and a second wash buffer. The half antibodies were eluted from the column under acidic conditions (pH 2.8<3).

The bispecific antibody was assembled by combining equal masses of each half antibody. The sample was titrated up to pH 8.4. 100x excess molar ratio of L-glutathione, reduced (GSH) with respect to the theoretical maximum amount of bispecific antibody expected from the assembly process was added to the mixture and the temperature was adjusted from room temperature to 35°C. The two half antibodies assembled *in vitro* in the redox reaction to form the bispecific antibody. The redox reaction continued for 20 hrs at 35 °C . The assembly reaction was quenched by adjusting the pH to 6.5 and the pool was diluted with water before the being subjected to the purification process described below.

The assembled bispecific antibody was then subjected to Capto™ Adhere mixed-mode anion exchange chromatography. The bind and elute Capto™ Adhere mixed-mode anion exchange conditions were as follows: The column was equilibrated with 2 mM MES containing 50 mM sodium acetate, pH 6.5. Assembly pool adjusted to pH 6.5 and 6.5 mS/cm was loaded onto the column to a load density of ≤ 20 g of protein per L of packed resin, washed with Equilibration buffer, and then washed with second wash buffer (6 mM MES containing 150 mM Acetate, pH 6.5). The bispecific antibody was eluted by dropping the pH of the buffer (25 mM Acetate, pH 5.0). Pooling was initiated and terminated based on absorbance at 280 nm.

The pooled Capto™ Adhere eluate was adjusted to pH 8.5 and purified over the QSFF anion exchange chromatography column in bind and elute mode. The bind and elute QSFF anion exchange conditions were as follows: The column was equilibrated with 50 mM Tris, pH 8.5. Adjusted Capto™ Adhere elution pool was loaded onto the column to a load density of 41 g of protein per L of packed resin and washed with Equilibration buffer. The bispecific antibody was eluted by increasing the conductivity of the buffer (50 mM Tris with 100 mM sodium acetate at pH 8.5). Pooling was initiated and terminated based on absorbance at 280 nm.

The QSFF eluate adjusted to pH 5.5 was then subject to phenyl Sepharose® HS chromatography in the flow-through mode. The flow-through Phenyl Sepharose® 6 Fast Flow (high sub) HIC conditions were as follows: The column was equilibrated with 170 mM acetate, pH 5.5. Adjusted QSFF elution pool was loaded onto the column. The bispecific antibody flowed through and the column was subsequently washed with Equilibration buffer. Product pool collection was initiated and terminated based on absorbance at 280 nm. The Phenyl Sepharose® pool was then concentrated and buffer exchanged using ultrafiltration/diafiltration (UF/DF). The anti-A/anti-B bispecific antibody was analyzed for the presence of impurities including non-paired half antibodies, homodimers, low molecular weight species (LMWS), high molecular weight species (HMWS), very high molecular weight species (vHMWS), acidic variants, basic variants, FkpA, DsbA, DsbC, ECP, leached protein A, *E. coli* DNA, and endotoxin. Product quality and process performance (as shown by % yield) are detailed in Table 35.

Table 35. Anti-A/anti-B Product Quality and Process Performance

| Step | αA MSS | αB MSS | Assembly | Capto™ Adhere | QSFF | Phenyl Sepharose® | UFDF |
|---|---|---|---|---|---|---|---|
| Yield (%) | 77 | 75 | 85 | 77 | 85 | 84 | 80 |
| ECP (ppm) | 11817 | 10996 | 3447 | 94 | 44 | 26 | 16 |
| FkpA (ppm) | 3331 | 2751 | 1841 | 1265 | 216 | 8 | 8 |
| DsbA (ppm) | 59 | 29 | 28 | 5 | 1 | 1 | 0.3 |
| DsbC (ppm) | 94 | 144 | 122 | 4 | 9 | 2 | 0.1 |
| SEC-total HMWS (%) | 4.2 | 12.0 | 14.4 | 7.4 | 1.4 | 0.2 | 0.7 |
| SEC-main (%) | 18.1 | 29.4 | 85.1 | 92.6 | 98.5 | 99.8 | 99.3 |
| SEC-LMWS (%) | 77.3 | 58.5 | 0.5 | 0 | 0.09 | 0 | 0 |
| Purity, NR-CE-SDS (%) | 83.5 | 71.1 | 86.9 | 92.6 | 96.4 | 97 | 97 |

SEQUENCES

Unless otherwise designated, amino acid sequences are presented N-terminus to C-terminus and nucleic acid sequences are presented 5' to 3'.

*E. coli* FkpA

Amino Acid Sequence - without leader sequence

AFADKSKLSDQEIEQTLQAFEARVKSSAQAKMEKDAADNEAKGKEYREKFAKEKGVKTSSTGLVYQVVEAGKGEAPK
DSDTVVVNYKGTLIDGKEFDNSYTRGEPLSFRLDGVIPGWTEGLKNIKKGGKIKLVIPPELAYGKAGVPGIPPNSTL
VFDVELLDVKPAPKADAKPEADAKAADSAKK

(SEQ ID NO:1)


Amino Acid Sequence - full sequence


MKSLFKVTLLATTMAVALHAPITFAAEAAKPATTADSKAAFKNDDQKSAYALGASLGRYMENSLKEQEKLGIKLDKD
QLIAGVQDAFADKSKLSDQEIEQTLQAFEARVKSSAQAKMEKDAADNEAKGKEYREKFAKEKGVKTSSTGLVYQVVE
AGKGEAPKDSDTVVVNYKGTLIDGKEFDNSYTRGEPLSFRLDGVIPGWTEGLKNIKKGGKIKLVIPPELAYGKAGVP
GIPPNSTXVFDVELLDVKPAPKADAKPEADAKAADSAKK

(SEQ ID NO:2)


Nucleic acid sequence


atgaaatcactgtttaaagtaacgctgctggcgaccacaatggccgttgccctgcatgcaccaatcactttgctgc
tgaagctgcaaaacctgctacagctgctgacagcaaagcagcgttcaaaaatgacgatcagaaatcagcttatgcac
tgggtgcctcgctgggtcgttacatggaaaactctctaaaagaacaagaaaaactgggcatcaaactggataaagat
cagctgatcgctggtgttcaggatgcatttgctgataagagcaaactctccgaccaagagatcgaacagactctaca
agcattcgaagctcgcgtgaagtcttctgctcaggcgaagatggaaaaagacgcggctgataacgaagcaaaaggta
aagagtaccgcgagaaatttgccaaagagaaaggtgtgaaaacctcttcaactggtctggtttatcaggtagtagaa
gccggtaaaggcgaagcaccgaaagacagcgatactgttgtagtgaactacaaaggtacgctgatcgacggtaaaga
gttcgacaactcttacacccgtggtgaaccgctttctttccgtctggacggtgttatcccgggttggacagaaggtc
tgaagaacatcaagaaaggcggtaagatcaaactggttattccaccagaactggcttacggcaaagcgggtgttccg
gggatcccaccgaattctaccctggtgtttgacgtagagctgctggatgtgaaaccagcgccgaaggctgatgcaaa
gccggaagctgatgcgaaagccgcagattctgctaaaaataa

(SEQ ID NO:3)


**Claims**

1. A method for purifying a polypeptide from a composition comprising the polypeptide and one or more impurities, wherein the polypeptide is produced in a prokaryotic cell, and wherein the cell is engineered to express one or more chaperones, wherein the chaperone is one or more of FkpA, DsbA or DsbC and wherein the polypeptide is a multispecific antibody, wherein the multispecific antibody comprises

    i) multiple arms, each arm comprising a VH/VL unit, the method comprising the sequential steps of

        a) subjecting the composition to protein A chromatography to produce a protein A eluate,
        b) subjecting the protein A eluate to mixed-mode chromatography to generate a mixed-mode eluate, and
        c) subjecting the mixed-mode eluate to anion exchange chromatography to generate an anion exchange eluate,
        d) subjecting the anion exchange eluate to hydrophobic interaction chromatography and collecting a fraction comprising the multispecific antibody,
        e) subjecting the hydrophobic interaction eluate to cation exchange chromatography and collecting a fraction comprising the polypeptide,

    wherein the method reduces the amount of FkpA, DsbA and/or DsbC from the composition;
    or
    ii) multiple arms, each arm comprising a VH/VL unit, wherein each arm of the multispecific antibody is produced separately, the method comprising the sequential steps of

        a) subjecting each arm of the multispecific antibody to protein A chromatography to produce protein A eluates for each arm of the multispecific antibody,
        b) forming a mixture comprising protein A eluates of each arm of the multispecific antibody under conditions sufficient to produce a composition comprising the multispecific antibody

c) subjecting the composition comprising the multispecific antibody to mixed-mode chromatography to generate a mixed-mode eluate, and

d) subjecting the mixed-mode eluate to anion exchange chromatography to generate an anion exchange eluate,

e) subjecting the anion exchange eluate to hydrophobic interaction chromatography and collecting a fraction comprising the multispecific antibody,

f) subjecting the hydrophobic interaction eluate to cation exchange chromatography and collecting a fraction comprising the polypeptide,

wherein the method reduces the amount of FkpA, DsbA and/or DsbC from the composition.

2. The method of claim 1, wherein the mixed-mode chromatography is

a) a mixed-mode anion exchange chromatography; and/or
b) is carried out in bind and elute mode.

3. The method of claim 1, wherein the protein A chromatography is carried out in bind and elute mode.

4. The method of any one of claims 1 to 3 further comprising the step of subjecting

a) the hydrophobic interaction chromatography fraction comprising the multispecific antibody to ultrafiltration; or
b) the cation exchange fraction comprising the multispecific antibody to ultrafiltration; or
c) the hydrophobic interaction chromatography fraction comprising the multispecific antibody to ultrafiltration, wherein the ultrafiltration comprises sequentially a first ultrafiltration, a diafiltration and a second ultrafiltration; or
d) the cation exchange fraction comprising the multispecific antibody to ultrafiltration, wherein the ultrafiltration comprises sequentially a first ultrafiltration, a diafiltration and a second ultrafiltration.

5. The method of any one of claims 1 to 3, wherein the hydrophobic interaction chromatography (HIC) comprises a hydrophobic moiety.

6. The method of claim 5, wherein the hydrophobic interaction chromatography comprises

a) a phenyl moiety or a butyl moiety linked to crosslinked agarose; or
b) a phenyl moiety or a butyl moiety linked to crosslinked agarose, wherein the hydrophobic interaction chromatography is phenyl Sepharose® chromatography or butyl Sepharose® chromatography.

7. The method of any one of claims 5 to 6, wherein the hydrophobic interaction chromatography comprises a) a hexyl moiety; or b) a hexyl moiety wherein the hydrophobic interaction chromatography comprises a hydrophobic moiety linked to hydroxylated methacrylic polymer.

8. The method of claim 1 to 7 , where cation exchange chromatography comprises

a) a carboxylic acid moiety or a sulfonic acid moiety; or
b) a carboxylic acid moiety or a sulfonic acid moiety, wherein the cation exchange loading density is less than about 20 g/L volume of the chromatography material.

9. The method of any one of claims 1 to 8, wherein the cell is an E. coli cell.

10. The method of any one of claim 1 to 9, wherein the chaperone is an *E. coli* chaperone.

11. The method of any one of claims 1 to 10 wherein the multispecific antibody is a bispecific antibody.

**Patentansprüche**

1. Verfahren zum Reinigen eines Polypeptids aus einer Zusammensetzung, die das Polypeptid und eine oder mehrere Verunreinigung(en) umfasst, wobei das Polypeptid in einer prokaryotischen Zelle produziert wird und wobei die Zelle genetisch so modifiziert ist, dass sie ein oder mehrere Chaperon(e) exprimiert, wobei das Chaperon eines oder mehrere von FkpA, DsbA oder DsbC ist und wobei das Polypeptid ein multispezifischer Antikörper ist, wobei der multispezifische Antikörper Folgendes umfasst

i) mehrere Arme, wobei jeder Arm eine VH/VL-Einheit umfasst, wobei das Verfahren die folgenden aufeinanderfolgenden Schritte umfasst

a) Unterziehen der Zusammensetzung einer Protein-A-Chromatographie zum Produzieren eines Protein-A-Eluats,
b) Unterziehen des Protein-A-Eluats einer Mixed-Mode-Chromatographie zum Erzeugen eines Mixed-Mode-Eluats und
c) Unterziehen des Mixed-Mode-Eluats einer Anionenaustauschchromatographie zum Erzeugen eines Anionenaustauscheluats,
d) Unterziehen des Anionenaustauscheluats einer hydrophoben Wechselwirkungschromatographie und Sammeln einer Fraktion, die den multispezifischen Antikörper umfasst,
e) Unterziehen des hydrophoben Wechselwirkungseluats einer Kationenaustauschchromatographie und Sammeln einer Fraktion, die das Polypeptid umfasst,

wobei das Verfahren die Menge an FkpA, DsbA und/oder DsbC aus der Zusammensetzung reduziert; oder
ii) mehrere Arme, wobei jeder Arm eine VH/VL-Einheit umfasst, wobei jeder Arm des multispezifischen Antikörpers separat produziert wird, wobei das Verfahren die folgenden aufeinanderfolgenden Schritte umfasst

a) Unterziehen jedes Arms des multispezifischen Antikörpers einer Protein-A-Chromatographie zum Produzieren von Protein-A-Eluaten für jeden Arm des multispezifischen Antikörpers,
b) Bilden eines Gemisches, das Protein-A-Eluate von jedem Arm des multispezifischen Antikörpers umfasst, unter Bedingungen, die zum Produzieren einer Zusammensetzung ausreichen, die den multispezifischen Antikörper umfasst,
c) Unterziehen der Zusammensetzung, die den multispezifischen Antikörper umfasst, einer Mixed-Mode-Chromatographie zum Erzeugen eines Mixed-Mode-Eluats und
d) Unterziehen des Mixed-Mode-Eluats einer Anionenaustauschchromatographie zum Erzeugen eines Anionenaustauscheluats,
e) Unterziehen des Anionenaustauscheluats einer hydrophoben Wechselwirkungschromatographie und Sammeln einer Fraktion, die den multispezifischen Antikörper umfasst,
f) Unterziehen des hydrophoben Wechselwirkungseluats einer Kationenaustauschchromatographie und Sammeln einer Fraktion, die das Polypeptid umfasst,
wobei das Verfahren die Menge an FkpA, DsbA und/oder DsbC aus der Zusammensetzung reduziert.

2. Verfahren nach Anspruch 1, wobei die Mixed-Mode-Chromatographie

a) eine Mixed-Mode-Anionenaustauschchromatographie ist; und/oder
b) im Bindungs- und Elutionsmodus durchgeführt wird.

3. Verfahren nach Anspruch 1, wobei die Protein-A-Chromatographie im Bindungs- und Elutionsmodus durchgeführt wird.

4. Verfahren nach einem der Ansprüche 1 bis 3, ferner umfassend den Schritt des Unterziehens

a) der Fraktion aus der hydrophoben Wechselwirkungschromatographie, die den multispezifischen Antikörper umfasst, einer Ultrafiltration; oder
b) der Kationenaustauschfraktion, die den multispezifischen Antikörper umfasst, einer Ultrafiltration; oder
c) der Fraktion aus der hydrophoben Wechselwirkungschromatographie, die den multispezifischen Antikörper umfasst, einer Ultrafiltration, wobei die Ultrafiltration der Reihe nach eine erste Ultrafiltration, eine Diafiltration und eine zweite Ultrafiltration umfasst; oder
d) der Kationenaustauschfraktion, die den multispezifischen Antikörper umfasst, einer Ultrafiltration, wobei die Ultrafiltration der Reihe nach eine erste Ultrafiltration, eine Diafiltration und eine zweite Ultrafiltration umfasst.

5. Verfahren nach einem der Ansprüche 1 bis 3, wobei die hydrophobe Wechselwirkungschromatographie (HIC) eine hydrophobe Einheit umfasst.

6. Verfahren nach Anspruch 5, wobei die hydrophobe Wechselwirkungschromatographie Folgendes umfasst

   a) eine Phenyleinheit oder eine Butyleinheit, die mit vernetzter Agarose verknüpft ist; oder
   b) eine Phenyleinheit oder eine Butyleinheit, die mit vernetzter Agarose verknüpft ist, wobei die hydrophobe Wechselwirkungschromatographie Phenyl-Sepharose®-Chromatographie oder Butyl-Sepharose®-Chromatographie ist.

7. Verfahren nach einem der Ansprüche 5 bis 6, wobei die hydrophobe Wechselwirkungschromatographie a) eine Hexyleinheit; oder b) eine Hexyleinheit umfasst, wobei die hydrophobe Wechselwirkungschromatographie eine hydrophobe Einheit umfasst, die mit einem hydroxylierten Methacrylpolymer verknüpft ist.

8. Verfahren nach Anspruch 1 bis 7, wobei die Kationenaustauschchromatographie Folgendes umfasst

   a) eine Carbonsäureeinheit oder eine Sulfonsäureeinheit; oder
   b) eine Carbonsäureeinheit oder eine Sulfonsäureeinheit, wobei die Kationenaustauschladungsdichte weniger als etwa 20 g/l bezogen auf das Volumen des Chromatographiematerials beträgt.

9. Verfahren nach einem der Ansprüche 1 bis 8, wobei die Zelle eine E. coli-Zelle ist.

10. Verfahren nach einem der Ansprüche 1 bis 9, wobei das Chaperon ein E. coli-Chaperon ist.

11. Verfahren nach einem der Ansprüche 1 bis 10, wobei der multispezifische Antikörper ein bispezifischer Antikörper ist.

## Revendications

1. Méthode de purification d'un polypeptide provenant d'une composition comprenant le polypeptide et une ou plusieurs impuretés, dans laquelle le polypeptide est produit dans une cellule procaryote, et dans laquelle la cellule est modifiée pour exprimer un ou plusieurs chaperons, dans laquelle le chaperon est un ou plusieurs parmi FkpA, DsbA ou DsbC et dans laquelle le polypeptide est un anticorps multispécifique, dans laquelle l'anticorps multispécifique comprend

   i) de multiples bras, chaque bras comprenant une unité VH/VL, la méthode comprenant les étapes séquentielles de

      a) soumission de la composition à une chromatographie de protéine A pour produire un éluat de protéine A,
      b) soumission de l'éluat de protéine A à une chromatographie en mode mixte pour générer un éluat de mode mixte, et
      c) soumission de l'éluat de mode mixte à une chromatographie d'échange d'anions pour générer un éluat d'échange d'anions,
      d) soumission de l'éluat d'échange d'anions à une chromatographie d'interaction hydrophobe et collecte d'une fraction comprenant l'anticorps multispécifique,
      e) soumission de l'éluat d'interaction hydrophobe à une chromatographie d'échange de cations et collecte d'une fraction comprenant le polypeptide,

   dans laquelle la méthode réduit la quantité de FkpA, DsbA et/ou DsbC provenant de la composition ;
   ou
   ii) de multiples bras, chaque bras comprenant une unité VH/VL, dans laquelle chaque bras de l'anticorps multispécifique est produit séparément, la méthode comprenant les étapes séquentielles de

      a) soumission de chaque bras de l'anticorps multispécifique à une chromatographie de protéine A pour produire des éluats de protéine A pour chaque bras de l'anticorps multispécifique,
      b) formation d'un mélange comprenant des éluats de protéine A de chaque bras de l'anticorps multispécifique dans des conditions suffisantes pour produire une composition comprenant l'anticorps multispécifique
      c) soumission de la composition comprenant l'anticorps multispécifique à une chromatographie en mode mixte pour générer un éluat de mode mixte, et
      d) soumission de l'éluat de mode mixte à une chromatographie d'échange d'anions pour générer un éluat d'échange d'anions,

e) soumission de l'éluat d'échange d'anions à une chromatographie d'interaction hydrophobe et collecte d'une fraction comprenant l'anticorps multispécifique,

f) soumission de l'éluat d'interaction hydrophobe à une chromatographie d'échange de cations et collecte d'une fraction comprenant le polypeptide,

dans laquelle la méthode réduit la quantité de FkpA, DsbA et/ou DsbC provenant de la composition.

2. Méthode selon la revendication 1, dans laquelle la chromatographie en mode mixte est

a) une chromatographie d'échange d'anions en mode mixte ; et/ou
b) est effectuée en mode liaison et élution.

3. Méthode selon la revendication 1, dans laquelle la chromatographie de protéine A est effectuée en mode liaison et élution.

4. Méthode selon l'une quelconque des revendications 1 à 3 comprenant en outre l'étape de soumission

a) de la fraction de chromatographie d'interaction hydrophobe comprenant l'anticorps multispécifique à une ultrafiltration ; ou
b) de la fraction d'échange d'anions comprenant l'anticorps multispécifique à une ultrafiltration ; ou
c) de la fraction de chromatographie d'interaction hydrophobe comprenant l'anticorps multispécifique à une ultrafiltration, dans laquelle l'ultrafiltration comprend séquentiellement une première ultrafiltration, une diafiltration et une seconde ultrafiltration ; ou
d) de la fraction d'échange de cations comprenant l'anticorps multispécifique à une ultrafiltration, dans laquelle l'ultrafiltration comprend séquentiellement une première ultrafiltration, une diafiltration et une seconde ultrafiltration.

5. Méthode selon l'une quelconque des revendications 1 à 3, dans laquelle la chromatographie d'interaction hydrophobe (HIC) comprend un fragment hydrophobe.

6. Méthode selon la revendication 5, dans laquelle la chromatographie d'interaction hydrophobe comprend

a) un fragment phényle ou un fragment butyle lié à de l'agarose réticulée ; ou
b) un fragment phényle ou un fragment butyle lié à de l'agarose réticulée, dans laquelle la chromatographie d'interaction hydrophobe est une chromatographie phenyl Sepharose® ou une chromatographie butyl Sepharose®.

7. Méthode selon l'une quelconque des revendications 5 à 6, dans laquelle la chromatographie d'interaction hydrophobe comprend a) un fragment hexyle ; ou b) un fragment hexyle dans lequel la chromatographie d'interaction hydrophobe comprend un fragment hydrophobe lié à un polymère méthacrylique hydoxylé.

8. Méthode selon les revendications 1 à 7, où la chromatographie d'échange de cations comprend

a) un fragment acide carboxylique ou un fragment acide sulfonique ; ou
b) un fragment acide carboxylique ou un fragment acide sulfonique, dans lequel la densité de chargement d'échange de cations est inférieure à environ 20 g/L de volume de matériau chromatographique.

9. Méthode selon l'une quelconque des revendications 1 à 8, dans laquelle la cellule est une cellule E. coli.

10. Méthode selon l'une quelconque des revendications 1 à 9, dans laquelle le chaperon est un chaperon d'*E. coli.*

11. Méthode selon l'une quelconque des revendications 1 à 10 dans laquelle l'anticorps multispécifique est un anticorps bispécifique.

FIG. 1

**FIG. 2**

FIG. 3A

*FIG. 3B*

FIG. 4

FIG. 5

**FIG. 6**

FIG. 7A

FIG. 7B

FIG. 8

1 - 20 µL Precision Standards
2 - 10 µL SP mini-pool
3 - 20 µLButyl S F-T/W
4 - 20 µL PW Strip

*FIG. 9*

**FIG. 10A**

**FIG. 10B**

EP 3 337 819 B1

**Butyl-S Bulk;**

**FIG. 11**

**FIG. 12**

## FIG. 13A

| 250 kDa |
|---|
| 150 kDa |
| 100 kDa |
| 75 kDa |
| 50 kDa |
| 37 kDa |
| 25 kDa |
| 20 kDa |
| 15 kDa |
| 10 kDa |

| Lane | Sample Description | µL Sample | µL 4x SB | Reference | Lane | Sample Description | µL Sample | µL 4x SB | Reference |
|---|---|---|---|---|---|---|---|---|---|
| 1 | | | | | 8 | Fr. 37 | 20 | 10 | No heating |
| 2 | Precision stds. | 20 | | Bio-Rad | 9 | Fr. 38 | 20 | 10 | No heating |
| 3 | Fr. 32 | 20 | 10 | No heating | 10 | Fr. 39 | 20 | 10 | No heating |
| 4 | Fr. 33 | 20 | 10 | No heating | 11 | Fr. 40 | 20 | 10 | No heating |
| 5 | Fr. 34 | 20 | 10 | No heating | 12 | Fr. 41 | 20 | 10 | No heating |
| 6 | Fr. 35 | 20 | 10 | No heating | 13 | Fr. 42 | 20 | 10 | No heating |
| 7 | Fr. 36 | 20 | 10 | No heating | 14 | | | | |
| | | | | | | Reduced: no | | | |
| | Bio-Rad Stain Free | 4-20% | 12+2 | | | | | | |

## FIG. 13B

FIG. 14

*FIG. 15*

**FIG. 16**

EP 3 337 819 B1

| Lane | Sample Description | µL Sample | µL 4x SB | Reference | Lane | Sample Description | µL Sample | µL 4x SB | Reference |
|---|---|---|---|---|---|---|---|---|---|
| 1 | | | | | 8 | | | | |
| 2 | | | | | 9 | Run 3 fr. 37-44 | 15 | 5 | No heating |
| 3 | Precision stds. | 20 | | Bio-Rad | 10 | | | | |
| 4 | | | | | 11 | Run 4 fr. 37-44 | 15 | 5 | No heating |
| 5 | Run 1 fr. 35-40 | 15 | 5 | No heating | 12 | | | | |
| 6 | | | | | 13 | | | | |
| 7 | Run 2 fr. 37-44 | 15 | 5 | No heating | 14 | | | | |
| | 032514 | | | | | Reduced: No | | | |
| | Bio-Rad Stain Free | 4-20% | 12+2 | | | | | | |

**FIG. 17**

| Lane | Sample Description | µL Sample | µL 4x SB | Reference | Lane | Sample Description | µL Sample | µL 4x SB | Reference |
|------|-------------------|-----------|----------|-----------|------|-------------------|-----------|----------|-----------|
| 1 | | | | | 8 | 1 freeze-thaw | 5 | 5 | No heating |
| 2 | | | | | 9 | | | | |
| 3 | | | | | 10 | 2 freeze-thaws | 5 | 5 | No heating |
| 4 | Precision stds. | 20 | | Bio-Rad | 11 | | | | |
| 5 | | | | | 12 | 3 freeze-thaws | 5 | 5 | No heating |
| 6 | 0 freeze-thaws | 5 | 5 | No heating | 13 | | | | |
| 7 | | | | | 14 | | | | |
| | | | | | | Reduced: No | | | |
| | Bio-Rad Stain Free | 4-20% | 12+2 | | | | | | |

## FIG. 18

EP 3 337 819 B1

FIG. 19A

FIG. 19B

**Standard Curve**

5-P Fit: $y = (A - D)/((1 + (x/C)^B)^G) + D$:

| | A | B | C | D | G | R^2 |
|---|---|---|---|---|---|---|
| Std1 (Pre-Rabbit A: Conc vs Avg OD) | 0.0479 | 1.04 | 3.06e+05 | 257 | 0.317 | 1 |
| Std2 (Pre-Rabbit B: Conc vs Avg OD) | 0.0486 | 1.05 | 2.7e+03 | 5.99 | 1.04 | 1 |
| Std3 (Pre-Rabbit C: Conc vs Avg OD) | 0.0478 | 1.08 | 4.32e+03 | 4.1e+03 | 0.000341 | 1 |
| Std4 (Rabbit Serum A: Conc vs Avg OD) | 0.062 | 1.23 | 4.76 | 3.06 | 4.43 | 0.999 |
| Std5 (Rabbit Serum B: Conc vs Avg OD) | 0.055 | 1.13 | 49.2 | 3.04 | 41.9 | 1 |
| Std6 (Rabbit Serum C: Conc vs Avg OD) | 0.0595 | 1.16 | 5.03e+04 | 3.04 | 1.7e+05 | 1 |

**FIG. 20**

| Lane | Sample Description | µL Sample | µL 4x SB | Reference | Lane | Sample Description | µL Sample | µL 4x SB | Reference |
|------|------|------|------|------|------|------|------|------|------|
| 1 | | | | | 8 | | | | |
| 2 | Precision stds. | 20 | | Bio-Rad | 9 | 60% AS all | 10 | 10 | 1:10 dilution |
| 3 | 60% AS all | 10 | 10 | 1:10 dilution | 10 | | | | |
| 4 | | | | | 11 | 60% AS super | 10 | 10 | 1:10 dilution |
| 5 | 60% AS super | 10 | 10 | 1:10 dilution | 12 | | | | |
| 6 | | | | | 13 | 60% AS pellet | 10 | 10 | 1:10 dilution |
| 7 | 60% AS pellet | 10 | 10 | 1:10 dilution | 14 | | | | |
| | | | | | | Reduced: 9-11 | | | |
| | Bio-Rad Stain Free | 4-20% | 12+2 | | | | | | |

# FIG. 21

FIG. 22

**Standard Curve**

| 5-P Fit: y = (A - D)/( (1 + (x/C)^B)^G) + D: | $\underline{A}$ | $\underline{B}$ | $\underline{C}$ | $\underline{D}$ | $\underline{G}$ | $\underline{R^2}$ |
|---|---|---|---|---|---|---|
| ○ Std1 (Std 1/60 hrp diln: Conc vs Avg OD) | 0.109 | 0.999 | 6.54 | 2.55 | 1.05 | 1 |
| □ Std2 (Std 1/80 hrp diln: Conc vs Avg OD) | 0.0907 | 1.05 | 4.18 | 2.29 | 0.702 | 1 |
| △ Std3 (Std 1/100 hrp diln: Conc vs Avg OD) | 0.0838 | 0.978 | 7.11 | 1.78 | 1.11 | 1 |
| ◇ Std4 (Std 1/110 hrp diln: Conc vs Avg OD) | 0.0836 | 0.94 | 8.39 | 1.63 | 1.2 | 1 |

*FIG. 23*

*FIG. 24*

*FIG. 25*

FIG. 26A

FIG. 26B

Standard Curve

| 5-P Fit: y = (A - D)/( (1 + (x/C)^B)^G) + D: | A | B | C | D | G | R^2 |
|---|---|---|---|---|---|---|
| ○ Std1 (Std 1/2000 hrp diln: Conc vs Avg OD) | 0.0889 | 1.19 | 3.54e+06 | 3.25 | 1.31e+06 | 0.999 |
| □ Std2 (Std 1/4000 hrp diln: Conc vs Avg OD) | 0.0598 | 1.07 | 1.43e+07 | 2.71 | 6.71e+05 | 1 |
| △ Std3 (Std 1/6000 hrp diln: Conc vs Avg OD) | 0.0519 | 1.05 | 1.21e+07 | 2.1 | 3.58e+05 | 1 |
| ◇ Std4 (Std 1/8000 hrp diln: Conc vs Avg OD) | 0.0512 | 1.08 | 9.25e+06 | 1.58 | 4.14e+05 | 1 |

*FIG. 27*

**Standard Curve**

| 5-P Fit: y = (A - D)/( (1 + (x/C)^B)^G) + D: | A | B | C | D | G | R^2 |
|---|---|---|---|---|---|---|
| ○ Std (Std: Conc vs Avg OD) | 0.11 | 0.989 | 6.54 | 2.47 | 0.807 | 1 |

Curve Fit Option - Fixed Weight Value

*FIG. 28A*

FIG. 28B

*FIG. 29*

| Lane | Sample Description | μL Sample | μL 4x SB | Reference | Lane | Sample Description | μL Sample | μL 4x SB | Reference |
|------|--------------------|-----------|----------|-----------|------|--------------------|-----------|----------|-----------|
| 1 | | | | | 8 | | | | |
| 2 | Precision stds. | 20 | | Bio-Rad | 9 | 0 freeze-thaws | 8.3 | 7 | |
| 3 | 0 freeze-thaws | 8.3 | 7 | | 10 | 1 freeze-thaw | 8.3 | 7 | |
| 4 | 1 freeze-thaw | 8.3 | 7 | | 11 | 2 freeze-thaws | 8.3 | 7 | |
| 5 | 2 freeze-thaws | 8.3 | 7 | | 12 | 3 freeze-thaws | 8.3 | 7 | |
| 6 | 3 freeze-thaws | 8.3 | 7 | | 13 | | | | |
| 7 | | | | | 14 | | | | |
| | | | | | | Reduced: 9-12 | | | |
| | Bio-Rad Stain Free | 4-20% | 12+2 | | | | | | |

## FIG. 30

**FIG. 31A**

**FIG. 31B**

aFkpA Functional Testing

FIG. 32

FIG. 34

**Standard Curve**

| 4-P Fit: y = (A - D)/(1 + (x/C)^B) + D: | A | B | C | D | R^2 |
|---|---|---|---|---|---|
| o Std1 (Std Current Coat: Conc vs Avg OD) | 0.0714 | 1.21 | 46.7 | 2.23 | 0.999 |
| □ Std2 (Std Non-Lysed Coat: Conc vs Avg OD) | 0.0671 | 1.23 | 56.4 | 2.41 | 1 |
| △ Std3 (Std Lysed Coat: Conc vs Avg OD) | 0.0662 | 1.21 | 58.9 | 2.47 | 1 |

Curve Fit Option - Fixed Weight Value

*FIG. 33*

FIG. 35A

FIG. 35B

**Standard Curve**

| 4-P Fit: y = (A - D)/(1 + (x/C)^B) + D: | A | B | C | D | R^2 |
|---|---|---|---|---|---|
| ○ Std (Std: Conc vs Avg OD) | 0.059 | 1.12 | 32.4 | 2.13 | 1 |
| □ Std2 (Std Biosource Lot 051446: Conc vs Avg OD) | 0.0483 | 0.922 | 115 | 2.98 | 1 |
| △ Std3 (Std Biosource Lot 051467: Conc vs Avg OD) | 0.0517 | 1 | 100 | 0.0686 | 0.0716 |

*FIG. 36*

EP 3 337 819 B1

*FIG. 37A*

FIG. 37B

FIG. 38A

FIG. 38B

*FIG. 39*

*FIG. 40A*

*FIG. 40B*

FIG. 40C

FIG. 40D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015038888 A
- US 5641870 A
- EP 404097 A
- WO 9311161 A
- US 20110287009 A
- US 20070178552 A
- WO 96027011 A
- WO 98050431 A
- US 5731168 A
- US 5807706 A
- US 5821333 A
- US 7695936 B
- US 8216805 B
- WO 2011133886 A
- WO 2013055958 A
- WO 2005035572 A
- WO 03035694 A
- WO 0029004 A
- WO 02051870 A
- US 4816567 A
- US 5693780 A
- US 5500362 A
- US 5821337 A
- US 7521541 B
- WO 9316185 A
- US 5571894 A
- US 5587458 A
- WO 9308829 A
- WO 2009080251 A
- WO 2009080252 A
- WO 2009080253 A
- WO 2009080254 A
- WO 2009089004 A1
- US 4676980 A
- US 20060025576 A1
- US 20080069820 A
- WO 9404690 A
- WO 2009089004 A
- US 20090182127 A
- WO 2005063816 A
- WO 2005010044 A
- US 201501716 W
- WO 2015127405 A
- US 5648237 A, Carter
- US 8241901 B
- US 5639635 A
- WO 2002061090 A
- US 5264365 A, Georgiou
- US 5508192 A, Georgiou
- US 4737456 A
- US 3969287 A
- US 3691016 A
- US 4195128 A
- US 4247642 A
- US 4229537 A
- US 4330440 A
- US 4376110 A
- US 3940475 A
- US 3645090 A
- EP 402226 A
- US 7642228 B
- US 62129701
- US 62075792
- EP 1356052 A

### Non-patent literature cited in the description

- **MISSIAKAS, D. et al.** *Molecular Microbiology,* 1996, vol. 21 (4), 871-884
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **BURTON.** *Molec. Immunol.,* 1985, vol. 22, 161-206
- **ZAPATA et al.** *Protein Eng.,* 1995, vol. 8 (10), 1057-1062
- **PLÜCKTHUN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **ZHU et al.** *Protein Science,* 1997, vol. 6, 781-788
- *Nature,* 1989, vol. 341, 544-546
- *Dev Comp Immunol,* 2006, vol. 30, 43-56
- *Trend Biochem Sci,* 2001, vol. 26, 230-235
- *Trends Biotechnol,* 2003, vol. 21, 484-490
- *FEBS Lett,* 1994, vol. 339, 285-290

- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **MORRISON et al.** *Proc. Natl. Acad. Sci. USA,* 1984, vol. 81, 6851-6855
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **GAZZANO-SANTORO et al.** *J. Immunol. Methods,* 1996, vol. 202, 163
- **RAVETCH ; KINET.** *Annu. Rev. Immunol,* 1991, vol. 9, 457-92
- **CLYNES et al.** *Proc. Natl. Acad. Sci. (USA),* 1998, vol. 95, 652-656
- **DAËRON.** *Annu. Rev. Immunol.,* 1997, vol. 15, 203-234
- **CAPEL et al.** *Immunomethods,* 1994, vol. 4, 25-34
- **HAAS et al.** *J. Lab. Clin. Med.,* 1995, vol. 126, 330-41
- **GUYER et al.** *J. Immunol.,* 1976, vol. 117, 587
- **KIM et al.** *J. Immunol.,* 1994, vol. 24, 249
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **CARTER et al.** *Bio/Technology,* 1992, vol. 10, 163-167
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MUNSON et al.** *Anal. Biochem.,* 1980, vol. 107, 220
- **SKERRA et al.** *Curr. Opinion in Immunol.,* 1993, vol. 5, 256-262
- **PLÜCKTHUN.** *Immunol. Revs.,* 1992, vol. 130, 151-188
- **MCCAFFERTY et al.** *Nature,* 1990, vol. 348, 552-554
- **MARKS et al.** *Bio/Technology,* 1992, vol. 10, 779-783
- **WATERHOUSE et al.** *Nuc. Acids. Res.,* 1993, vol. 21, 2265-2266
- **MORRISON et al.** *Proc. Natl Acad. Sci. USA,* 1984, vol. 81, 6851
- **MORIMOTO et al.** *Journal of Biochemical and Biophysical Methods,* 1992, vol. 24, 107-117
- **BRENNAN et al.** *Science,* 1985, vol. 229, 81
- **CUNNINGHAM ; WELLS.** *Science,* 1989, vol. 244, 1081-1085
- **A. L. LEHNINGER.** Biochemistry. Worth Publishers, 1975, 73-75
- **MILSTEIN ; CUELLO.** *Nature,* 1983, vol. 305, 537
- **TRAUNECKER et al.** *EMBO J.,* 1991, vol. 10, 3655
- **KOSTELNY et al.** *J. Immunol.,* 1992, vol. 148 (5), 1547-1553
- **GRUBER et al.** *J. Immunol.,* 1994, vol. 152, 5368
- **TUTT et al.** *J. Immunol.,* 1991, vol. 147, 60
- **TRAUNECKER et al.** *EMBO J,* 1991, vol. 10, 3655
- **SURESH et al.** *Methods in Enzymology,* 1986, vol. 121, 210
- **MARVIN ; ZHU.** *Acta Pharmacologica Sincia,* 2005, vol. 26 (6), 649-658
- **KONTERMANN.** *Acta Pharacol. Sin.,* 2005, vol. 26, 1-9
- Molecular weight amino acid minus that of water. Handbook of Chemistry and Physics. Chemical Rubber Publishing Co, 1961
- **A.A. ZAMYATNIN.** *Prog. Biophys. Mol. Biol.,* 1972, vol. 24, 107-123
- **C. CHOTHIA.** *J. Mol. Biol.,* 1975, vol. 105, 1-14
- **ELLMAN et al.** *Meth. Enzym.,* 1991, vol. 202, 301-336
- **NOREN et al.** *Science,* 1989, vol. 244, 182
- **MUTAGENESIS.** Practical Approach, M.J. IRL Press, 1991
- **PONDERS ; RICHARDS.** *J. Mol. Biol.,* 1987, vol. 193, 775-791
- **KABAT et al.** Sequences of proteins of immunological interest. NIH, 1991, vol. 1, 688-696
- **GAFFEN, S. L.** *Nature Review,* 2009, vol. 9, 556-567
- **HYMOWITZ et al.** *EMBO J.,* 2001, vol. 20, 5332-41
- **SIEBENLIST et al.** *Cell,* 1980, vol. 20, 269
- **BACHMANN.** Cellular and Molecular Biology. American Society for Microbiology, 1987, vol. 2, 1190-1219
- **BASS et al.** *Proteins,* 1990, vol. 8, 309-314
- **SIMMONS et al.** *J. Immunol. Methods,* 2002, vol. 263, 133-147
- **HARA et al.** *Microbial Drug Resistance,* 1996, vol. 2, 63-72
- **LINDMARK et al.** *J. Immunol. Meth.,* 1983, vol. 62, 1-13
- **HUNTER et al.** *Nature,* 1962, vol. 144, 945
- **DAVID et al.** *Biochemistry,* 1974, vol. 13, 1014-1021

- **PAIN et al.** *J. Immunol. Methods,* 1981, vol. 40, 219-230
- **NYGREN.** *J. Histochem. and Cytochem.,* 1982, vol. 30, 407-412
- **STEWART et al.** Solid-Phase Peptide Synthesis. W.H. Freeman Co, 1969
- **MERRIFIELD.** *J. Am. Chem. Soc.,* 1963, vol. 85, 2149-2154
- **SAMBROOK et al.** Molecular Cloning: A Laboratory Manual. Cold Spring Harbor Press, 1989
- **WINNACKER.** From Genes to Clones. VCH Publishers, 1987

- Remington's Pharmaceutical Sciences. 1980
- **ZHANG et al.** *BioTechniques,* 2003, vol. 35, 1032-1042
- **ZHU-SHIMONI, J. et al.** *Biotech and Bioeng.,* 2014, vol. 111, 2367-2379
- **SHKENAZI, A.** *Nature Reviews Drug Discovery,* 2008, vol. 7, 1001-1012
- **YU et al.** *Sci Transl Med,* 2011, vol. 3, 82-44
- **RIDGWAY et al.** *Protein Eng.,* 1996, vol. 9, 617-621
- **ATWELL et al.** *J Mol Biol,* 1997, vol. 270, 26-35